# EUROPEAN PATENT SPECIFICATION

(11) **EP 1 954 697 B1**
(45) Date of publication and mention of the grant of the patent: **24.02.2010**
(21) Application number: 06849235.4
(22) Date of filing: 17.10.2006
(51) Int. Cl.: C07D 471/06, C07D 519/00, A61K 31/437, A61K 31/495, A61K 31/436, A61P 31/00

(54) **PERI CONDENSED TRICYCLIC COMPOUNDS USEFUL AS ANTIBACTERIAL AGENTS**
PERI-KONDENSIERTE TRICYCLISCHE VERBINDUNGEN VERWENDBAR ALS ANTIBAKTERIELLE MITTEL
COMPOSÉS TRICYCLIQUES PERI CONDENSÉS UTILES COMME AGENTS ANTIBACTÉRIENS

(30) Priority: 21.10.2005 US 728975 P; 22.09.2006 US 826590 P
(43) Date of publication of application: 13.08.2008
(73) Proprietor: GLAXO GROUP LIMITED, Greenford, Middlesex UB6 ONN (GB)
(72) Inventor: CAILLEAU, Nathalie, Harlow, Essex CM19 5AW (GB); DAVIES, David, Thomas, Stevenage, Hertfordshire SG1 2NY (GB); ESKEN, Joel, Michael, NC 27709 (US); HENNESSY, Alan, Joseph, Harlow, Essex CM19 5AW (GB); KUSALAKUMARI SUKUMAR, Senthil, Kumar, NC 27709 (US); MARKWELL, Roger, Edward, Harlow, Essex CM19 5AW (GB); MILES, Timothy, James, Harlow, Essex CM19 5AW (GB); PEARSON, Neil, David, Harlow, Essex CM19 5AW (GB)
(74) Representative: Valentine, Jill Barbara
(86) International application number: PCT/US2006/060023
(87) International publication number: WO 2007/081597

(56) References cited:
- WO-A-03/064431
- WO-A1-2007/071936

## Description

This invention relates to novel compounds, compositions containing them and their use as antibacterials.

WO02/08224, WO02/50061, WO02/56882, WO02/96907, WO2003087098, WO2003010138, W02003064421, W02003064431, WO2004002992, WO2004002490, WO2004014361, WO2004041210, WO2004096982, WO2002050036, WO2004058144, WO2004087145, WO2006002047, WO2006014580, WO2006010040, WO2006017326, WO2006012396, WO2006017468, WO2006020561, WO2006081179, WO2006081264, W02006081289, WO2006081178, W02006081182, WO01/25227, WO02/40474, WO02/07572, WO2004024712, WO2004024713, WO2004035569, WO2004087647, WO2004089947, WO2005016916, WO2005097781, W02006010831, WO2006021448, WO2006032466, W02006038172 and WO2006046552 disclose quinoline, naphthyridine, morpholine, cyclohexane, piperidine and piperazine derivatives having antibacterial activity. WO2004104000 discloses tricyclic condensed ring compounds capable of selectively acting on cannabinoid receptors.

This invention provides a compound of formula (I) or a pharmaceutically acceptable salt and/or solvate thereof: 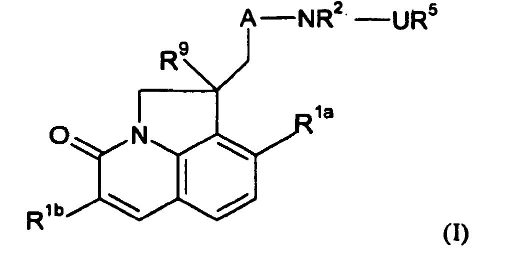 wherein:
R^{1a} and R^{1b} are independently selected from hydrogen; halogen; cyano; (C₁₋₆)alkyl; (C₁₋₆)alkylthio; trifluoromethyl; trifluoromethoxy; carboxy ; hydroxy optionally substituted with (C₁₋₆)alkyl or (C₁₋₆)alkoxy-substituted (C₁₋₆)alkyl; (C₁₋₆)alkoxy-substituted (C₁₋₆)alkyl; hydroxy (C₁₋₆)alkyl; an amino group optionally N-substituted by one or two (C₁₋₆)alkyl, formyl, (C₁₋₆)alkylcarbonyl or (C₁₋₆)alkylsulphonyl groups; or aminocarbonyl wherein the amino group is optionally substituted by (C₁₋₄)alkyl;
R² is hydrogen, or (C₁₋₄)alkyl, or together with R⁶ forms Y as defined below; A is a group (ia) or (ib): 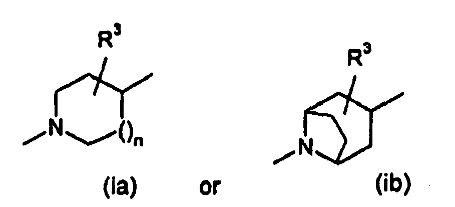
in which: R³ is as defined for R^{1a} or R^{1b} or is oxo and n is 1 or 2;
or A is a group (ii) 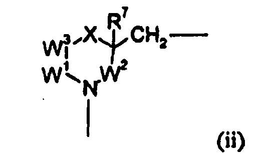 wherein :
W¹, W² and W3 are CR⁴R⁸;
or W² and W³ are CR⁴R⁸ and W¹ represents a bond between W³ and N;
X is O, CR⁴R⁸, or NR⁶;
one R⁴ is as defined for R^{1a} and R^{1b} and the remainder and R⁸ are hydrogen or one R⁴ and R⁸ are together oxo and the remainder are hydrogen;
R⁶ is hydrogen or (C₁₋₆)alkyl or together with R² forms Y;
R⁷ is hydrogen; halogen; hydroxy optionally substituted with (C ₁₋₆)alkyl; or (C₁₋₆)alkyl;
Y is CR⁴R⁸CH₂; CH₂CR⁴R⁸; (C-O); CR⁴R⁸; CR⁴R⁸(C=O); or (C=O)CR⁴R⁸;
or when X is CR⁴R⁸, R⁸ and R⁷ together represent a bond;
U is selected from CO and CH₂;
R⁵ is an optionally substituted bicyclic carbocyclic or heterocyclic ring system (B): 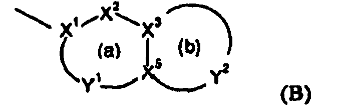 containing up to four heteroatoms in each ring in which
at least one of rings (a) and (b) is aromatic;
X 1 is C or N when part of an aromatic ring, or CR¹⁴ when part of a non-aromatic ring;
X² is N, NR¹³, O, S(O)ₓ, CO or CR¹⁴ when part of an aromatic or non-aromatic ring or may in addition be CR¹⁴R¹⁵ when part of a non-aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non-aromatic ring;
Y² is a 2 to 6 atom linker group, each atom of Y² being independently selected from N, NR¹³, O, S(O)ₓ, CO, CR¹⁴ when part of an aromatic or non-aromatic ring or may additionally be CR¹⁴R¹⁵ when part of a non-aromatic ring;
each of R¹⁴ and R¹⁵ is independently selected from: H; (C₁₋₄)alkylthio; halo; carboxy(C₁₋₄)alkyl; (C₁₋₄)alkyl; (C₁₋₄)alkoxycarbonyl; (C ₁₋₄)alkylcarbonyl; (C₁, ₄)alkoxy (C₁₋₄)alkyl; hydroxy; hydroxy(C₁₋₄)alkyl; (C₁₋₄)alkoxy; nitro; cyano; carboxy; amino or aminocarbonyl optionally mono- or di-substituted by (C₁₋₄)alkyl or
R¹⁴ and R¹⁵ may together represent oxo;
each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; (C₁₋₄)alkoxycarbonyl; (C₁₋₄)alkylcarbonyl; (C₁₋₆)alkylsulphonyl; or aminocarbonyl wherein the amino group is optionally mono or disubstituted by (C₁₋₄)alkyl;
each x is independently 0, 1 or 2; and
R⁹ is hydrogen or hydroxy.

This invention also provides a method of treatment of bacterial infections in mammals, particularly in man, which method comprises the administration to a mammal in need of such treatment an effective amount of a compound of formula (I), or a pharmaceutically acceptable salt and/or solvate thereof.

The invention also provides a compound of formula (I), or a pharmaceutically acceptable salt and/or solvate thereof, for use in the treatment of bacterial infections in mammals.

The invention also provides a pharmaceutical composition comprising a compound of formula (I), or a pharmaceutically acceptable salt and/or solvate thereof, and a pharmaceutically acceptable carrier.

In a particular aspect each R^{1a} and R^{1b} is independently hydrogen, (C₁₋₄)alkoxy, (C₁₋₄)alkylthio, (C₁₋₄)alkyl, cyano, carboxy, hydroxymethyl or halogen; more particularly hydrogen, methoxy, methyl, cyano, or halogen.

In certain embodiments each R^{1a} and R^{1b} is hydrogen, methoxy, methyl, or halogen, such as chloro or fluoro. In some embodiments only one group R^{1a} or R^{1b} is other than hydrogen. In particular embodiments R^{1a} is methoxy, fluoro or cyano and R^{1b} is hydrogen, more particularly R^{1a} is fluoro and R^{1b} is hydrogen.

In a particular aspect R² is hydrogen.

In a particular aspect R⁹ is hydrogen.

Particular examples of R³ include hydrogen; optionally substituted hydroxy; optionally substituted amino; halogen; (C₁₋₄) alkyl; 1-hydroxy-(C₁₋₄) alkyl; optionally substituted aminocarbonyl. More particular R³ groups are hydrogen; CONH₂; 1-hydroxyalkyl e.g. CH₂OH; optionally substituted hydroxy e.g. methoxy; optionally substituted amino; and halogen, in particular fluoro. Most particularly R³ is hydrogen, hydroxy or fluoro.

In a particular aspect, when A is (ia), n is 1. In a further aspect R³ is in the 3- or 4-position. In a more particular aspect, A is (ia), n is 1 and R³ is in the 3-position, and more particularly is *cis* to the NR² group.

In particular embodiments, A is a group (ia) in which n is 1 and R³ is hydrogen or hydroxy.

In a particular aspect, when A is (ii), X is CR⁴R⁸ and R⁸ is H or OH and more particularly OH is *trans* to R⁷. In a further aspect W¹ is a bond. In another aspect R⁷ is H. In particular embodiments W¹ is a bond, X, W² and W³ are each CH₂ and R⁷ is H.

In certain embodiments U is CH₂.

In certain embodiments R⁵ is an aromatic heterocyclic ring (B) having 8-11 ring atoms including 2-4 heteroatoms of which at least one is N or NR¹³ in which, in particular embodiments, Y² contains 2-3 heteroatoms, one of which is S and 1-2 are N, with one N bonded to X³.

In alternative embodiments the heterocyclic ring (B) has ring (a) aromatic selected from optionally substituted benzo, pyrido and pyridazino and ring (b) non-aromatic and Y² has 3-5 atoms, more particularly 4 atoms, including at least one heteroatom, with O, S, CH₂ or NR¹³ bonded to X⁵ where R¹³ is other than hydrogen, and either NHCO bonded via N to X³, or O, S, CH₂ or NH bonded to X³. In a particular aspect the ring (a) contains aromatic nitrogen, and more particularly ring (a) is pyridine or pyrazine. Examples of rings (B) include optionally substituted:

### (a) and (b) aromatic

1H-pyrrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl,benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, indan-2-yl, naphthalen-2-yl, 1,3-dioxo-isoindol-2-yl, benzimidazol-2-yl, benzothiophen-2-yl, 1H-benzotriazol-5-yl, 1H-indol-5-yl, 3H-benzooxazol-2-one-6-yl, 3H-benzooxazol-2-thione-6-yl, 3H-benzothiazol-2-one-5-yl, 3H-quinazolin-4-one-2-yl, 3H-quinazolin-4-one-6-yl, 4-oxo-4H-pyrido[1,2-a]pyrimidin-3-yl, benzo[1,2,3]thiadiazol-6-yl, benzo[1,2,5]thiadiazot-5-yl,benzo[1,4]oxazin-2-one-3-yl, benzothiazol-5-yl, benzothiazol-6-yl, cinnolin-3-yl, imidazo[1,2-a]pyridazin-2-yl, imidazo[1,2-b]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin-3-yl, pyrido[1,2-a]pyrimdin-4-one-2-yl, pyrido[1,2-a]pyrimidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, thiazolo[5,4-b]pyridin-6-yl, 4-oxo-4H-pyrido[1,2-a]pyrimidin-2-yl, 1-oxo-1,7-dihydro-isoquinolin-3-yl, thiazolo[4,5-b]pyridin-5-yl, [1,2,3]thiadiazolo[5,4-b]pyridin-6-yl, 2H-isoquinolin-1-one-3-yl

### (a) is non-aromatic

(2S)-2,3-dihydro-1H-indol-2-yl, (2S)-2,3-dihydro-benzo[1,4]dioxine-2-yl, 3-(R,S)-3,4-dihydro-2H-benzo[1,4]thiazin-3-yl, 3-(R)-2,3-dihydro-[1,4]dioxino[2,3-b]pyridin-3-yl, 3-(S)-2,3-dihydro-[1,4]dioxino[2,3-b]pyridin-3-yl, 2,3-dihydro-benzo[1,4]dioxan-2-yl, 3-substituted-3H-quinazolin-4-one-2-yl, (7S)-6,7-dihydro[1,4]dioxino[2,3*-c*]pyridazin-7-yl

### (b) is non-aromatic

1,1,3-trioxo-1,2,3,4-tetrahydrol 6-benzo[1,4] thiazin-6-yl, benzo[1,3]dioxol-5-yl, 2,3-dihydro-benzo[1,4]dioxin-6-yl, 2-oxo-2,3-dihydro-benzooxazol-6-yl, 3-substituted-3H-benzooxazol-2-one-6-yl, 3-substituted-3H-benzooxazole-2-thione-6-yl, 3-substituted-3H-benzothiazol-2-one-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-benzo[1,4]thiazin-6-yl), 4H-benzo[1,4]oxazin-3-one-7-yl, 4-oxo-2,3,4,5-tetrahydro-benzo[b][1,4]thiazepine-7-yl, 5-oxo-2,3-dihydro-5H-thiazolo[3,2-a]pyrimidin-6-yl, 1H-pyrido[2,3-b][1,4]thiazin-2-one-7-yl (2-oxo-2,3-dihydro-1H-pyrido[2,3-b]thiazin-7-yl), 2,3-dihydro-1H-pyrido[2,3-b][1,4]thiazin-7-yl, 2-oxo-2,3-dihydro-1H-pyrido[3,4-b]thiazin-7-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyndin-7-yl, 3,4-dihydro-2H-benzo[1,4]oxazin-6-yl, 3,4-dihydro-2H-benzo[1,4]thiazin-6-yl, 3-oxo-3,4-dihydro-2H-pyrido[3,2-b][1,4]oxazin-6-yl, 3,4-dihydro-2H-pyrido[3,2-b][1,4]thiazin-6-yl, 3-oxo-3,4-dihydro-2H-pyrido[3,2-b][1,4]thiazin-6-yl, 3,4-dihydro-1H-quinolin-2-one-7-yl, 3,4-dihydro-1H-quinoxalin-2-one-7-yl, 6,7-dihydro-4H-pyrazolo[1,5-a]pyrimidin-5-one-2-yl, 5,6,7,8-tetrahydro-[1,8]naphthyridin-2-yl, 2-oxo-3,4-dihydro-1*H*-[1,8]naphthyridin-6-yl, 6-oxo-6,7-dihydro-5H-8-thia-1,2,5-triaza-naphthalen-3-yl, 2-oxo-2,3-dihydro-1H-pyrido[3,4-b][1,4]oxazin-7-yl, 2-oxo-2,3-dihydro-1H-pyrido[2,3-b][1,4]oxazin-7-yl, 6,7-dihydro-[1,4]dioxino[2,3-d]pyrimidin-2,yl, [1,3]oxathiolo[5,4*-c*]pyridin-6-yl, 3,4-dihydro-2*H*-pyrano[2,3*-c*]pyridine-6-yl, 2,3-dihydro-1,4-benzodioxin-7-yl, 2,3-dihydro[1,4]oxathiino[2,3*-c*]pyridine-7-yl, 2,3-dihydrofuro[2,3*-c*]pyridin-5-yl, 2,3-dihydro-1-benzofuran-5-yl, 2,3-dihydro[1,4]oxathiino[2,3-*b*]pyridin-7-yl, 6,7-dihydro[1,4]oxathiino[3,2*-c*]pyridazin-3-yl, 6,7-dihydro[1,4]oxathiino[2,3*-c*]pyridazin-3-yl, 6,7-dihydro[1,4]dioxino[2,3*-c*]pyridazin-3-yl, 2,3-dihydro-1*H*-pyrido[3,4-b][1,4]oxazin-7-yl, 5-oxo-1,2,3,5-tetrahydroindolizin-7-yl, 6,7-dihydro-5H-pyrano[2,3-c]pyridazin-3-yl, 6-oxo-6,7-dihydro-5*H*-pyridazino[3,4-b][1,4]thiazin-3-yl, benzo[1,2,3]thiadiazol-5-yl, benzo[1,2,5]thiadiazol-5-yl, benzothiazol-5-yl, thiazolo-[5,4-b]pyridin-6-yl, 2-oxo-2,3-dihydro-1H-pyrido[2,3-b][1,4]thiazin-7-yl, 3-methyl-2-oxo-2,3-dihydro-benzooxazol-6-yl, 4-oxo-4H-pyrido[1,2-a]pyrimidin-2-yl, 7-oxo-1,5,6,7-tetrahydro-1,8-naphthyridin-2-yl.

In some embodiments R¹³ is H if in ring (a) or in addition (C₁₋₄)alkyl such as methyl or isopropyl when in ring (b). More particularly, in ring (b) R¹³ is H when NR¹³ is bonded to X³ and (C₁₋₄)alkyl when NR¹³ is bonded to X⁵.

In futher embodiments R¹⁴ and R ¹⁵ are independently selected from hydrogen, halo, hydroxy, (C₁₋₄) alkyl, (C₁₋₄)alkoxy, nitro and cyano. More particularly R¹⁵ is hydrogen.

More particularly each R¹⁴ is selected from hydrogen, chloro, fluoro, hydroxy, methyl, methoxy, nitro and cyano. Still more particularly R ¹⁴ is selected from hydrogen, fluorine or nitro,

Most particularly R¹⁴ and R¹⁵ are each H.

Particular groups R⁵ include:
[1,2,3]thiadiazolo[5,4-b]pyridin-6-yl
1H-pyrrolo[2,3-b]pyridin-2-yl
2,3-dihydro-[1,4]dioxino[2,3-b]pyridin-6-yl
2,3-dihydro-[1,4]dioxino[2,3-b]pyridin-7-yl
2,3-dihydro-[1,4]dioxino[2,3-c]pyridin-7-yl
2,3-dihydro-benzo[1,4]dioxin-6-yl
2-oxo-2,3-dihydro-1H-pyrido[2,3-b][1,4]oxazin-7-yl
2-oxo-2,3-dihydro-1H-pyrido[2,3-b][1,4]thiazin-7-yl
3,4-dihydro-2H-benzo[1,4]oxazin-6-yl
3-methyl-2-oxo-2,3-dihydro-benzooxazol-6-yl
3-oxo-3,4-dihydro-2H-benzo[1,4]oxazin-6-yl
3-oxo-3,4-dihydro-2H-pyrido[3,2-b][1,4]oxazin-6-yl
3-oxo-3,4-dihydro-2H-benzo[1,4]thiazin-6-yl (4H-benzo[1,4] thiazin-3-one-6-yl)
4-oxo-4H-pyrido[1,2-a]pyrimidin-2-yl
6-nitro-benzo[1,3]dioxol-5-yl
7-fluoro-3-oxo-3,4-dihydro-2H-benzo[1,4] oxazin-6-yl
8-hydroxy-1-oxo-1,2-dihydro-isoquinolin-3-yl
8-hydroxyquinolin-2-yl
benzo[1,2,3]thiadiazol-5-yl
benzo[1,2,5]thiadiazol-5-yl
benzothiazol-5-yl
thiazolo-[5,4-b]pyridin-6-yl
3-oxo-3,4-dihydro-2*H*-pyrido[3,2*-b*][1,4]thiazin-6-yl
7-chloro-3-oxo-3,4-dihydro-2*H*-pyrido[3,2*-b*][1,4]thiazin-6-yl
7-fluoro-3-oxo-3,4-dihydro-2*H*-pyrido[3,2*-b*][1,4]thiazin-6-yl
2-oxo-2,3-dihydro-1*H*-pyrido[3,4*-b*][1,4]thiazin-7-yl
6-oxo-6,7-dihydro-5H-pyridazino[3,4-b][1,4]thiazin-3-yl
7-oxo-1,5,6,7-tetrahydro-1,8-naphthyridin-2-yl
7-chloro-3-oxo-3,4-dihydro-2H-pyrido[3,2-b][1,4]oxazin-6-yl
(3S)-2,3-dihydro[1,4]dioxino[2,3-b]pyridin-3-yl
[1,3]oxathiolo[5,4*-c*]pyridin-6-yl
3,4-dihydro-2*H*-pyrano[2,3*-c*]pyridin-6-yl
5-carbonitro-2,3-dihydro-1,4-benzodioxin-7-yl
2,3-dihydro[1,4]oxathiino[2,3*-c*]pyridin-7-yl
5-fluoro-2,3-dihydro-1,4-benzodioxino-7-yl
2,3-dihydro-1-benzofuran-5-yl
2,3-dihydro[1,4]oxathiino[2,3*-b*]pyridin-7-yl
6,7-dihydro[1,4]oxathiino[3,2*-c*]pyridazin-3-yl
6,7-dihydro[1,4]oxathiino[2,3*-c*]pyridazin-3-yl
6,7-dihydro[1,4]dioxino[2,3*-c*]pyridazin-3-yl
2,3-dihydrofuro[2,3*-c*]pyridin-5-yl
2,3-dihydro-1*H*-pyrido[3,4*-b*][1,4]oxazin-7-yl
5-oxo- ,2,3,5-tetrahydroindolizin-7-yl
6,7-dihydro-5H-pyrano[2,3-c]pyridazin-3-yl
7-hydroxymethyl-6,7-dihydro[1,4]dioxino[2,3-c]pyridazin-3-yl
5,6-dihydrofuro[2,3-c]pyridazin-3-yl
   especially
3-oxo-3,4-dihydro-2H-pyrido[3,2-b][1,4]oxazin-6-yl
3-oxo-3,4-dihydro-2*H*-pyrido[3,2*-b*][1,4]thiazin-6-yl
6,7-Dihydro[1,4]dioxino[2,3*-c*]pyridazin-3-yl
6,7-dihydro[1,4]oxathiino[2,3*-c*]pyridazin-3-yl
6,7-dihydro[1,4]oxathiino[3,2*-c*]pyridazin-3-yl
2,3-dihydro-[1,4]dioxino[2,3-c]pyridin-7-yl
[1,3]oxathiolo[5,4*-c*]pyridin-6-yl.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Compounds within the invention contain a heterocyclyl group and may occur in two or more tautomeric forms depending on the nature of the heterocyclyl group; all such tautomeric forms are included within the scope of the invention.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Furthermore, it will be understood that phrases such as "a compound of formula (I) or a pharmaceutically acceptable salt and/or solvate thereof" are intended to encompass the compound of formula (I), a pharmaceutically acceptable salt of the compound of formula (I), a solvate of formula (I), or any pharmaceutically acceptable ' combination of these. Thus by way of non-limiting example used here for illustrative purpose, "a compound of formula (I) or a pharmaceutically acceptable salt or solvate thereof" may include a pharmaceutically acceptable salt of a compound of formula (I) that is further present as a solvate.

Since the compounds of formula (I) are intended for use in pharmaceutical compositions it will readily be understood that in particular embodiments they are provided in substantially pure form, for example at least 60% pure, more suitably at least 75% pure and particularly at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and more particularly from 10 to 59% of a compound of the formula (I) or pharmaceutically acceptable salt and/or solvate thereof.

Particular compounds according to the invention include those mentioned in the examples and their pharmaceutically acceptable salts and solvates.

Pharmaceutically acceptable salts of the above-mentioned compounds of formula (I) include the acid addition or quaternary ammonium salts, for example their salts-with mineral acids e.g. hydrochloric, hydrobromic, sulphuric nitric or phosphoric acids, or organic acids, e.g. acetic, fumaric ((2E)-2-butenedioic), succinic, maleic, citric, benzoic, p-toluenesulphonic (4-methylbenzene sulphonic), methanesulphonic, naphthalenesulphonic acid or tartaric acids. The invention extends to all such derivatives.

Certain of the compounds of formula (I) may exist in the form of optical isomers, e.g. diastereoisomers and mixtures of isomers in all ratios, e.g. racemic mixtures. The invention includes all such forms, in particular the pure isomeric forms. For example the invention includes enantiomers and diastereoisomers at the attachment points of NR², R³ and/or R⁹. The different isomeric forms may be separated or resolved one from the other by conventional methods, or any given isomer may be obtained by conventional synthetic methods or by stereospecific or asymmetric syntheses.

In a further aspect of the invention there is provided a process for preparing compounds of formula (I) in which R⁹ is H, and pharmaceutically acceptable salts and/or solvates thereof, which process comprises cyclising a compound of formula (11A): 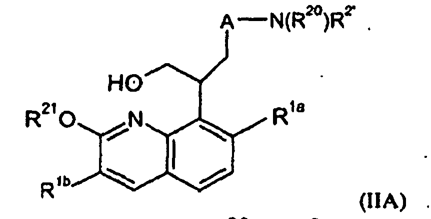 in which R²¹ is (C₁₋₆)alkyl such as methyl, R²⁰ is UR⁵ or a group convertible thereto and R²' is R² or a group convertible thereto, wherein A, R^{1a}, R^{1b}, R², U and R⁵ are as defined in formula (I),
to give a compound of formula (IIB): 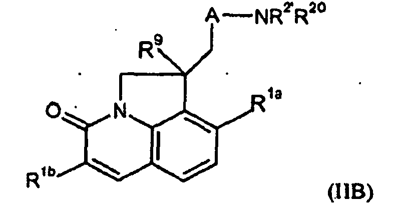 in which R⁹ is H, and thereafter optionally or as necessary converting R²⁰ and R^{2'} to UR⁵ and R², interconverting any variable groups, and/or forming a pharmaceutically acceptable salt and/or solvate thereof.

The cyclisation reaction is effected by treatment of the compound of formula (IIA) with an activating agent such as methanesulphonyl chloride, p-toluenesulphonyl chloride, methanesulfonic anhydride or p-toluene sulfonic anhydride and an organic base such as triethylamine or diisopropylethylamine. Mesylate or tosylate preparation takes place under standard conditions and the compound of formula (IIB) forms *in situ.*

In a further aspect of the invention there is provided a process for preparing compounds of formula (I) in which R⁹ is OH, and pharmaceutically acceptable salts and/or solvates thereof, which process comprises cyclising a compound of formula (IIC): 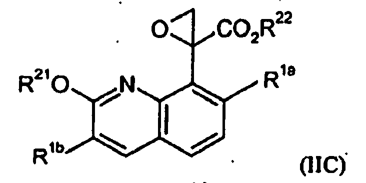 in which R²¹ is (C ₁₋₆)alkyl such as methyl R²² is H or (C ₁₋₆)alkyl such as methyl and R^{1a}, R^{1b} are as defined in formula (I),
to give a compound of formula (IID): 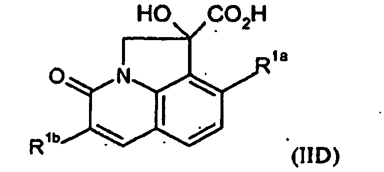 and thereafter converting -CO₂H to -CH₂-A-NR²-UR⁵, interconverting any variable groups, and/or forming a pharmaceutically acceptable salt and/or solvate thereof.

The cyclisation reaction may be effected by treatment of the compound of formula (IIC) with lithium perchlorate in acetonitrile or lithium hydroxide in water to give the tricyclic hydroxy-carboxylic acid (IID). Conversion of -CO₂H to --CH₂-A-NR²-UR⁵ may be effected by methylation using methanol in sulphuric acid, followed by reduction to the diol with sodium borohydride in methanol, and conversion to the tosyl derivative with tosyl chloride/dibutyltin oxide. Reaction with amine HN-A-NR²⁰R^{2'} R²⁰ where R²⁰ is UR⁵ or a group convertible thereto and R^{2'} is R² or a group convertible thereto, gives a compound of formula (IIB) in which R⁹ is OH.

Conveniently one of R²⁰ and R^{2'} is an N-protecting group, such as t-butoxycarbonyl, benzyloxycarbonyl or 9-fluorenylmethyloxycarbonyl. This may be removed by several methods well known to those skilled in the art (for examples see *"*Protective Groups in Organic Synthesis, T.W. Greene and P.G.M. Wuts, Wiley-Interscience. 1999), for example conventional acid hydrolysis with, for example trifluoroacetic acid or hydrochloric acid. The invention further provides compounds of formula (IIB) in which R²⁰ is hydrogen.

The free amine of formula (IIB) in which R²⁰ is hydrogen may be converted to NR²UR⁵ by conventional means such as amide or sulphonamide formation with an acyl derivative R⁵COW, for compounds where U is CO or, where U is CH₂, by alkylation with an alkyl halide R⁵CH₂-halide in the presence of base, acylation/reduction with an acyl derivative R⁵COW or reductive alkylation with an aldehyde R⁵CHO under conventional conditions (see for examples Smith, M.B.; March, J.M. Advanced Organic Chemistry, Wiley-lnterscience). The appropriate reagents containing the required R⁵ group are known compounds or may be prepared analogously to known compounds, see for example WO02/08224, WO02/50061, WO02/56882, WO02/96907, WO2003087098, WO2003010138, WO2003064421, W02003064431, WO2004002992, WO2004002490, W02004014361, WO2004041210, WO2004096982, WO2002050036, WO2004058144, W02004087145, WO06002047, WO06014580, WO06010040, WO06017326, WO06012396, WO06017468, WO06020561 and EP0559285.

Where R⁵ contains an NH group, this may be protected with a suitable N-protesting group such as t-butoxycarbonyt, benzyloxycarbonyl or 9-fluorenylmethyloxycarbonyl during the coupling of the R⁵ derivative with the free amine of formula (IIB). The protecting group may be removed by conventional methods, such as by treatment with trifluoroacetic acid.

Conveniently the resolution of enantiomers at the attachment position of R⁹ is carried out on the compound of formula (IIB), by any conventional method such as preparative high performance liquid chromatography.

The compound of formula (IIA) may be prepared by the following Scheme 1:

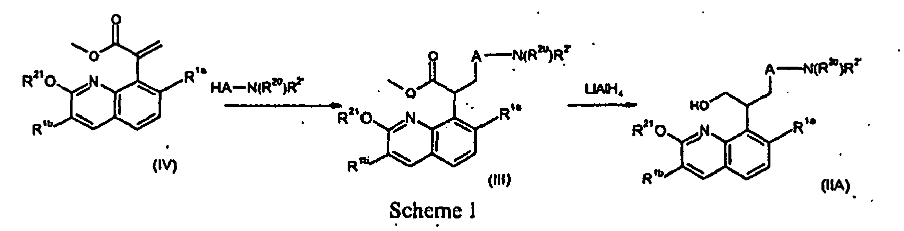

Compounds of general structure (III) may be prepared by reaction of acrylate ester (IV) with a compound HA-N(R²⁰)R²', such as a Boc protected amino-piperidine, under conventional conditions for Michael additions (see for examples Smith, M.B.; March, J.M. Advanced Organic Chemistry, Wiley-Interscience). Reduction of (III) to (IIA) occurs upon treatment with lithium aluminium hydride under conventional conditions (see for examples Smith, M.B.; March, J.M. Advanced Organic Chemistry, Wiley-Interscience).

The compound of formula (IIC) may be prepared by conventional epoxidation of the vinyl ester (IV) e.g. by oxidation with m-chloroperbenzoic acid or t-butyl hydrogen peroxide.

A route to intermediate (IV) is shown in Scheme 2:

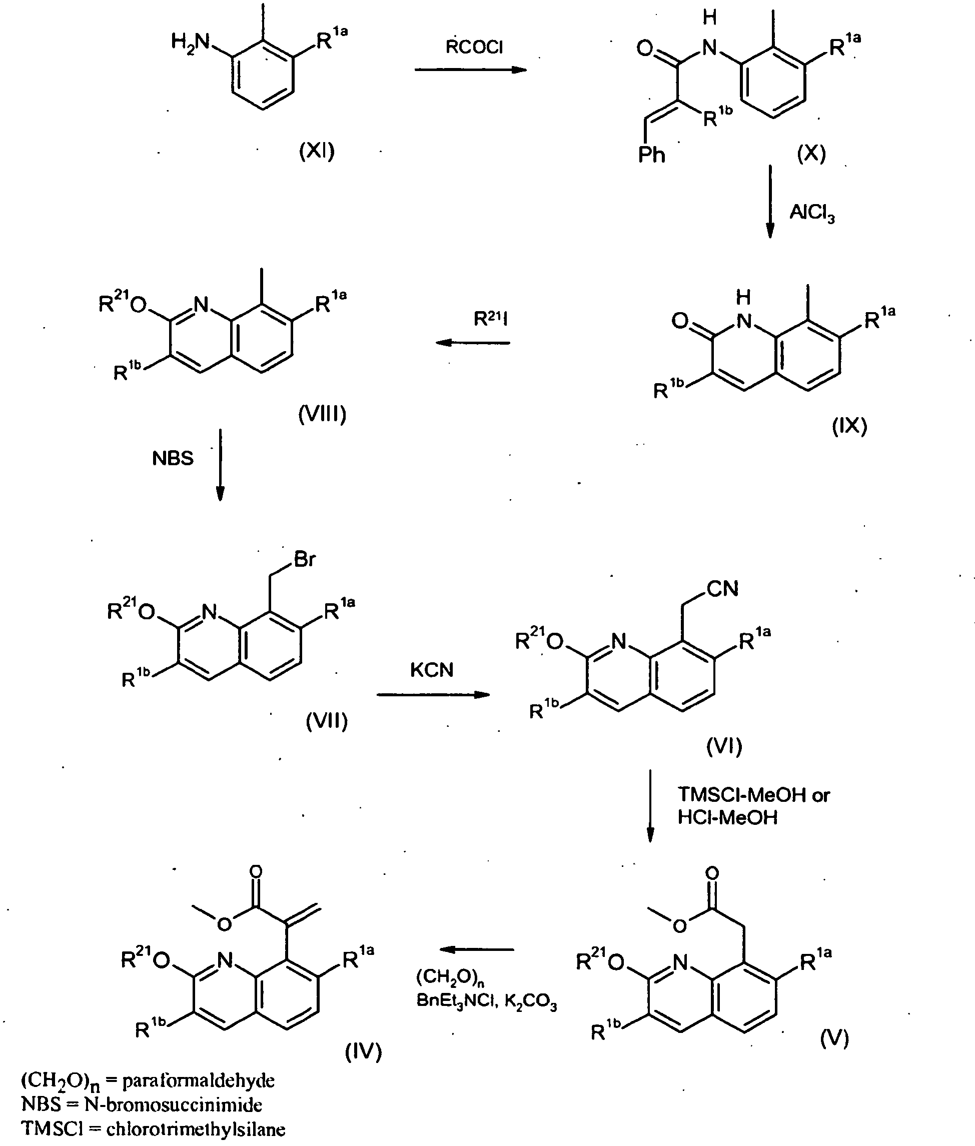

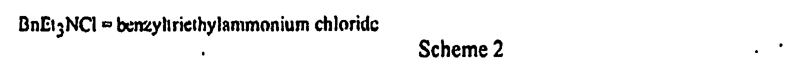

The aniline (XJ) is converted to the cinnamide (X), which is cyclised with aluminium chloride (with loss of the phenyl moiety - See M.C. Elliot et al. S.R. Inglis et al. J. Med. Chem. 47 (22) ,5405-5417 (2004)*,* Synlett, 5, 898-900 (2004)) to give (IX). This is selectively O-alkylated with e.g. methyl iodide or dimethylsulphate to give (VIII) and the methyl group functionalised with N-bromosuccinimide to give the bromomethyl analogue (VII). This is converted to the nitrile (VI) by treatment with KCN, or with NaCN and tetrabutylammonium bromide, which undergoes acid-catalysed methanolysis (TMS-chloride or HCl in methanol) to the methyl ester (V), and then vinylation with paraformaldehyde. Some demethylated material is formed with (V), but this can be re-methylated with TMS-diazomethane. This route is particularly suitable for R^{1a}=F.

An alternative route to intermediate (IV) is shown in Scheme 3:

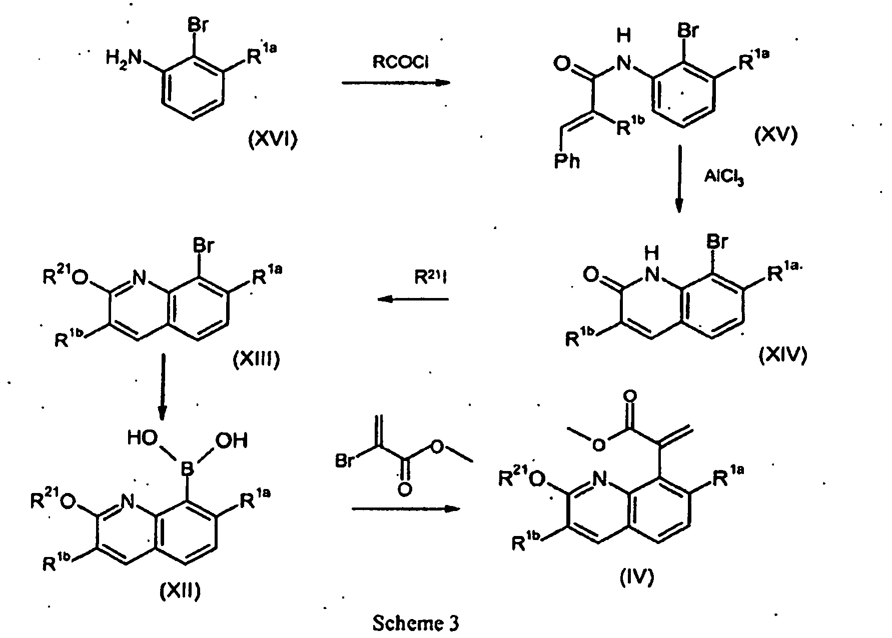

Quinolinone (XIV) may be prepared by reaction of commercially available aniline (XVI) with cinnamoyl chloride to give (XV) and its subsequent cyclisation (for an example of this procedure see Cottet, F.; Marull, M.; Lefebvre, O.; Schlosser, M European Journal of Organic Chemistry (2003), 8, 1559). (XIV) can be converted into the bromo-quinoline (XIII) under standard conditions (see for examples Smith, M.B.; March, J.M. Advanced Organic Chemistry, Wiley-Interscience). The boronic acid (XII) can be synthesised from (XIII) under standard conditions (for an example see Li, W.; Nelson, D.; Jensen, M.; Hoermer, R.; Cai, D.; Larsen, R.; Reider P. J Org Chem. (2002), 67(15), 5394). The coupling of (XII) with the known bromo-acrylate, (for synthesis see Rachon, J.; Goedken, V.; Walborsky, H. J. Org. Chem. (1989), 54(5), 1006) to give (IV) may be accomplished using a Suzuki coupling reaction (for conditions see Littke, A.; Dai, C.; Fu, G. J. Am. Chem. Soc. (2000), 122(17), 4020). This route is particularly suitable for R^{1a}-H.

In schemes 2 and 3, the RCOCI reagent in the first stage, cinnamoyl chloride, may be replaced by (2E)-3-ethyloxy-2-propenoyl chloride and the subsequent cyclisation effected with trifluoroacetic acid or sulfuric acid instead of aluminium trichloride (E. Baston et al, European J. Med. Chem., 2000 35(10), 931).

An alternative route to compounds of formula (I) in which A is (ia), n is I and R³ is H and U is CH₂, comprises reaction of a compound of formula (IIE): 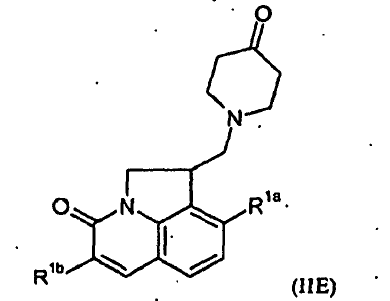 where R^{1a} and R^{1b} are as described in formula (I), with a compound R⁵CH₂NH₂, by reductive alkylation.

The compound of formula (IIE) may be prepared by the following Scheme 4:

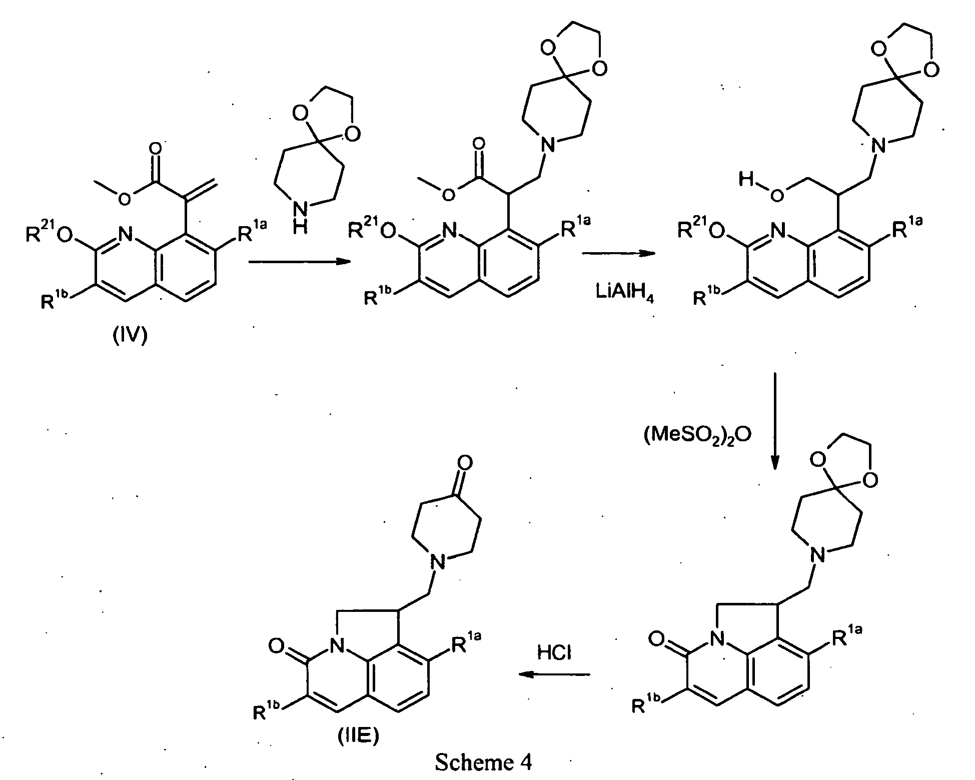

Reaction of (IV) with a suitable protected ketopiperidine such as 1,4-dioxa-8-azaspiro[4.5]decane followed by reduction of the ester and cyclisation with methane sulphonic anhydride gives the tricylic intermediate. Deprotection of the acetal with hydrochloric acid liberates the ketone.

Another alternative route to compounds of formula (IIB) in which, R^{1a} is F, R^{1b} is H, R⁹ is H, R²⁰ is H, R^{2'} is Boc, A is (ia), n is 1 and R³ is H (compound 5), comprises Scheme 5A:

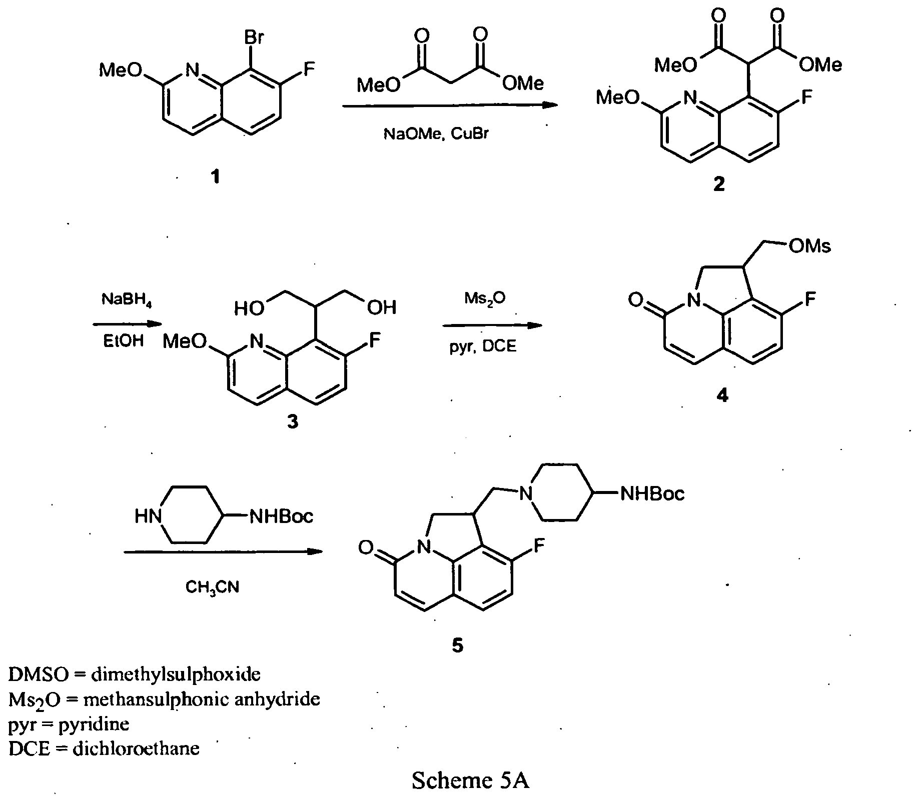

The diol 3 may be subjected to an enzymatic desymmetrization reaction to generate the desired E1 enantiomer of compound 4, by treatment with lipase TL and a vinyl ester (such as vinyl acetate or vinyl pivalate), followed by cyclisation with methanesulphonic anhydride, ester hydrolysis with sodium methoxide in methanol and activation of the resultant alcohol to mesylate 4 by conventional methods.

A variant of this process is shown in Scheme 5B:

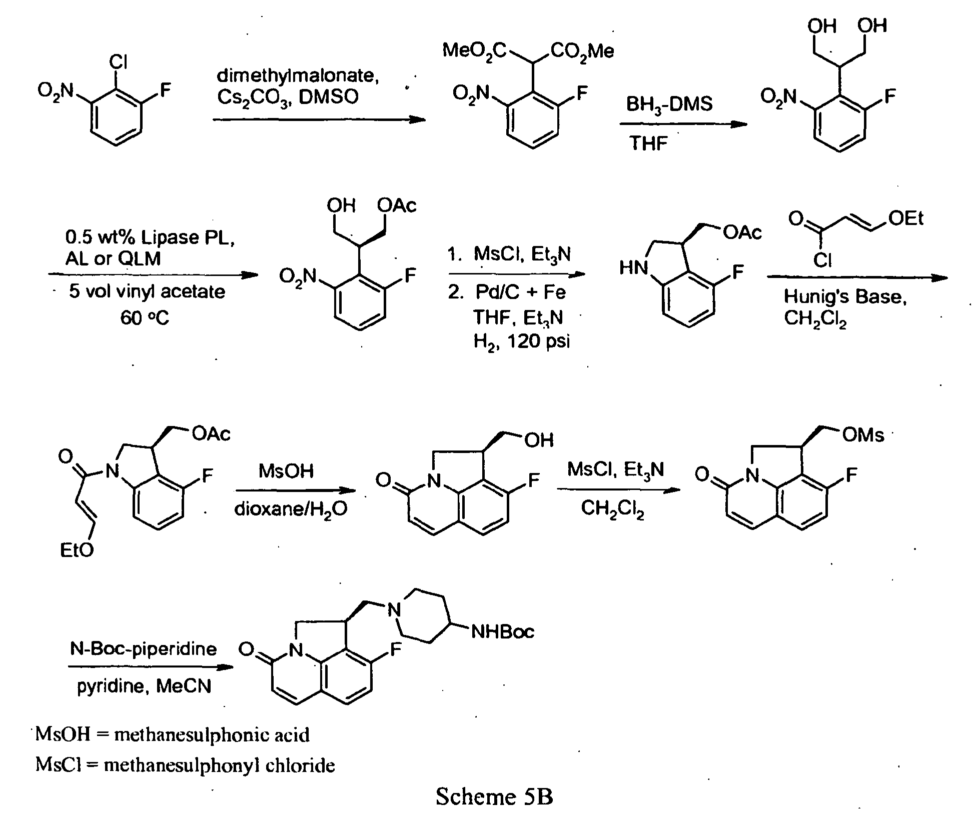

Interconversions of R^{1a}, R^{1b}, R², A and R⁵ are conventional. In compounds which contain an optionally protected hydroxy group, suitable conventional hydroxy protecting groups which may be removed without disrupting the remainder of the molecule include acyl and alkylsilyl groups. N-protecting groups are removed by conventional methods.

Interconversion of R^{1a} and R^{1b} groups may be carried out conventionally, on compounds of formula (I) or (IIB). For example R^{1a} or R^{1b} methoxy is convertible to R^{1a} or R^{1b} hydroxy by treatment with lithium and diphenylphosphine (general method described in Ireland et al, J. Amer. Chem: Soc., 1973, 7829) or HBr. Alkylation of the hydroxy group with a suitable alkyl derivative bearing a leaving group such as halide, yields R^{1a} or R^{1b} substituted alkoxy. R^{1a} halogen is convertible to other R^{1a} by conventional means, for example to hydroxy, alkylthiol (via thiol) and amino using metal catalysed coupling reactions, for example using copper as reviewed in Synlett (2003), 15, 2428-2439 and Angewandte Chemie, International Edition, 2003, 42(44), 5400-5449. R^{1b} halo such as bromo may be introduced by the method of M. A. Alonso et al, Tetrahedron 2003, 59(16), 2821. R^{1a} or R^{1b} halo such as bromo may be converted to cyano by treatment with copper (I) cyanide in N,N-dimethylformamide. R^{1a} or R^{1b} carboxy may be obtained by conventional hydrolysis of R^{1a} or R^{1b} cyano, and the carboxy converted to hydroxymethyl by conventional reduction.

Compounds of formula HA-N(R²⁰)R^{2'} and (V) are known compounds or may be prepared analogously to known compounds, see for example W02004/035569, WO2004/089947, WO02/08224, WO02/50061 WO02/56882, WO02/96907, WO2003087098, WO2003010138, WO2003064421, WO2003064431, WO2004002992, WO2004002490, WO2004014361, WO2004041210, WO2004096982, WO2002050036, WO2004058144, WO2004087145, WO2003082835, WO2002026723, WO06002047 and W006014580.

As shown in Scheme 6, the hydroxy-aminomethylpyrrolidines of formula (XIII) (HA-NH(R²⁰), A is (ii), X is CR⁴R⁸, W¹ is a bond, W² and W³ are both CH₂, R⁴ and R⁷ are H and R⁸ is OH) can be prepared from doubly protected chiral intermediate (XVI), separated by preparative HPLC. The benzyloxycarbonyl protecting group is removed by hydrogenation to give (XV) and the amino function converted to a trifluoroacetamide (XIV). The t-butoxycarbonyl (Boc) protecting group is removed with HCl to give the pyrrolidine hydrochloride salt (III).

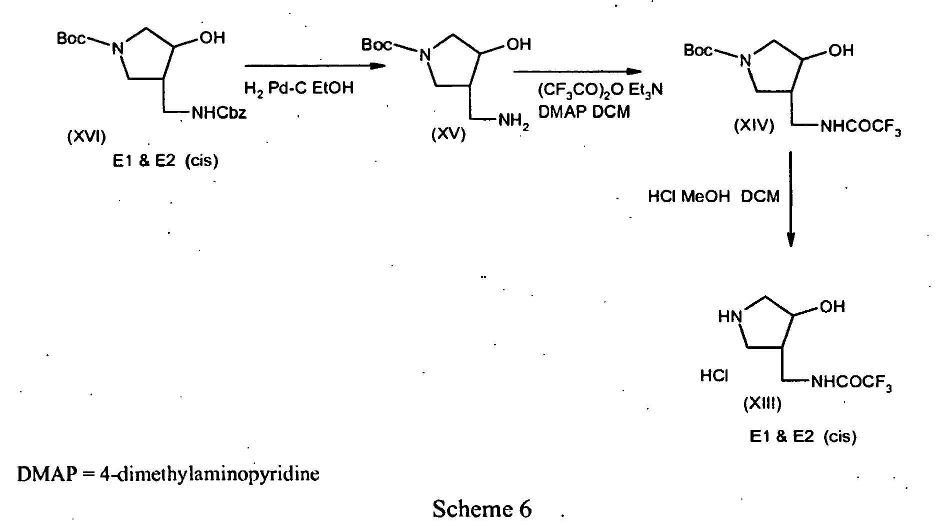

The intermediate (XVI) may be prepared by the general method of Scheme 7:

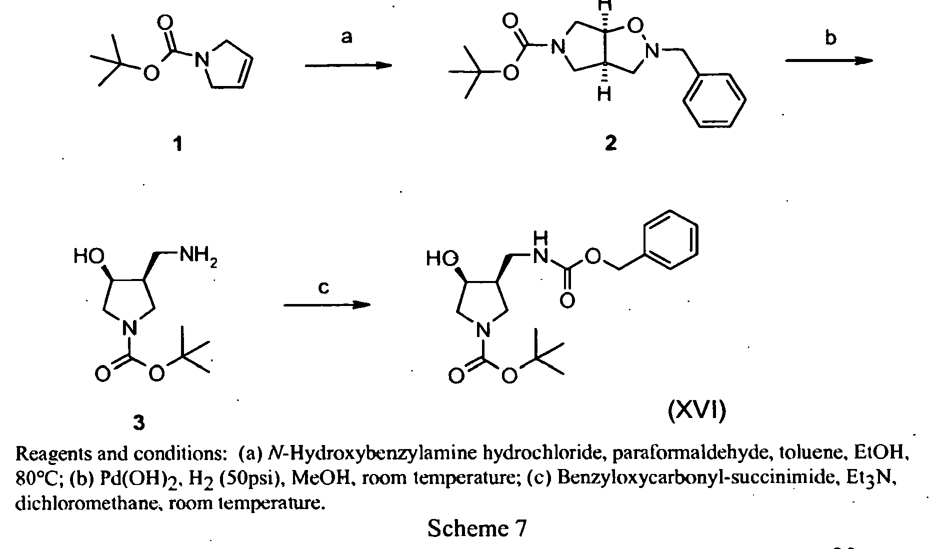

In Scheme 8 the aminomethylpyrrolidine of formula (XVII) (HA-NH(R²⁰), A is (ii), X is CR⁴R⁸, W¹ is a bond, W² and W³ are both CH₂, R⁴, R⁷ and R⁸ are all H) can be prepared from commercially available Boc-protected aminomethylpyrrolidine, and converted to the tri-fluoroacetamide.

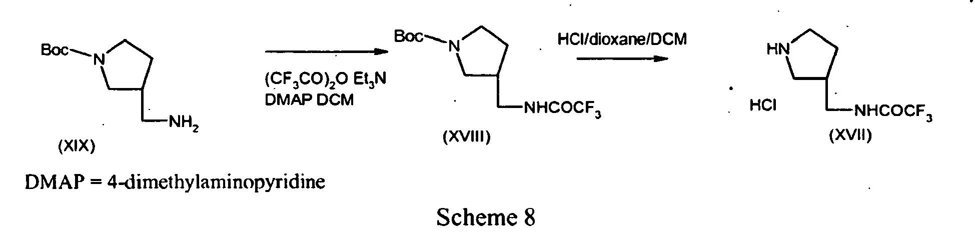

The aminomethylmorpholine intermediate of formula (XXI) (HA-NH(R²⁰), A is (ii), X is O, W¹, W² and W³ are each CH₂) may be prepared from a chiral dichlorobenzyl intermediate (XXIII) (WO2003082835) (Scheme 9) by first protecting the amino function with a Boc-protecting group (XXII), removing the dichlorobenzyl group by hydrogenation to give (XXI), protecting the morpholine N-atom with a benzyloxycarbonyl group (to allow purification by chromatography) (XX), and hydrogenation to afford the required morpholine derivative (XXI).

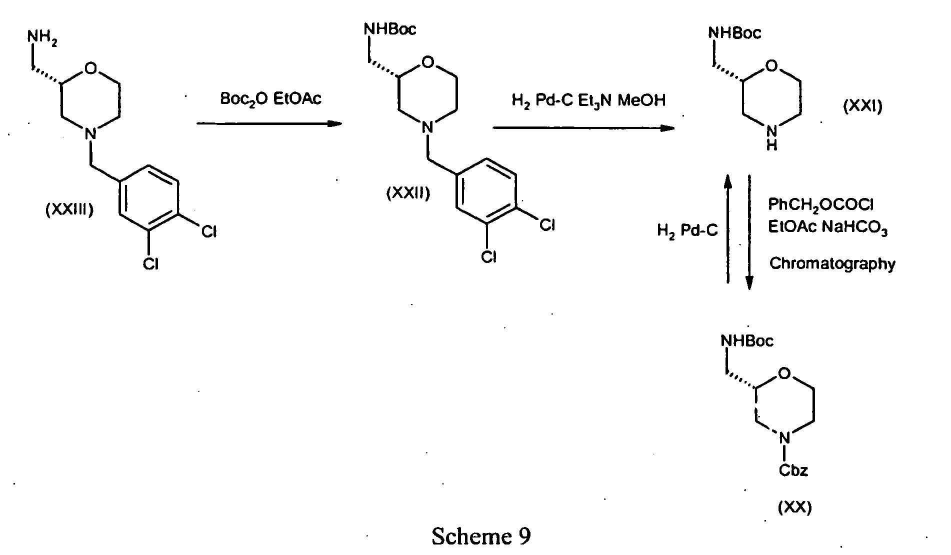

Further details for the preparation of compounds of formula (I) are found in the examples.

The antibacterial compounds according to the invention may be formulated for administration in any convenient way for use in human or veterinary medicine, by analogy with other antibacterials.

The pharmaceutical compositions of the invention include those in a form adapted for oral, topical or parenteral use and may be used for the treatment of bacterial infection in mammals including humans.

The composition may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then scaled in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-1000 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 30 mg/kg per day.

The compound of formula (1) may be the sole therapeutic agent in the compositions of the invention or a combination with other antibacterials. If the other antibacterial is a β-lactam then a β-lactamase inhibitor may also be employed.

Compounds of formula (I) may be used in the treatment of bacterial infections caused by a wide range of organisms including both Gram-negative and Gram-positive organisms. Some compounds of formula (I) may be active against more than one organism. This may be determined by test methods described herein.

All publications, including but not limited to patents and patent applications, cited in this specification are herein incorporated by reference as if each individual publication were specifically and individually indicated to be incorporated by reference herein as though fully set forth.

The following examples illustrate the preparation of certain compounds of formula (I) and the activity of certain compounds of formula (I) against various bacterial organisms.

### Examples and Experimental

### General

### Abbreviations in the examples:

rt = room temperature
MS = mass spectrum
ES = Electrospray mass spectroscopy
LCMS or LC-MS = Liquid chromatography mass spectroscopy
HPLC = High Performance Liquid Chromatography (Rt refers to retention time)
MDAP or Mass directed autoprep = mass directed preparative HPLC (using a ZQ mass spectrometer (Waters))

Certain reagents are also abbreviated herein. DMF refers to N,N-dimethylformamide, TFA refers to trifluoroacetic acid, THF refers to tetrahydrofuran, Pd/C refers to palladium on carbon catalyst, DCM refers to dichloromethane, Boc refers to tert-Butoxycarbonyl, MeOH refers to methanol.

Proton nuclear magnetic resonance (¹H NMR) spectra were recorded at 400 or 250 MHz, and chemical shifts are reported in parts per million (δ) downfield from the internal standard tetramethylsilane (TMS). Abbreviations for NMR data are as follows: s = singlet, d = doublet, t = triplet, q = quartet, m = multiplet, dd = doublet of doublets, dt = doublet of triplets, app = apparent, br = broad. CDCl₃ is deuteriochloroform, DMSO-d₆ is hexadeuteriodimethylsulfoxide, and CD₃OD is tetradeuteriomethanol. Mass spectra were obtained using electrospray (ES) ionization techniques. All temperatures are reported in degrees Celsius.

MP-carbonate refers to macroporous triethylammonium methylpolystyrene carbonate (Argonaut Technologies). Chiralpak AD and AD-H columns comprise silica for preparative columns (5um particle size AD-H and 10um particle size AD 21x250mm; 20 um particle size AD, 101.1x250mm) coated with amylose tris (3,5-dimethylphenylcarbamate) (Chiral Technologies USA). Chiralpak.AS-H column comprise of amylose tris [(S)-alpha-methylbenzylcarbamate) coated onto 5µm silica. Chiralpak IA column comprise of amylose tris (3,5- dimethylphenylcarbamate) immobilized onto 5µm silica. Luna™ C18 semi-prep reverse phase columns comprise of silca particles coated at high density with C18 alkyl chains and have good acid stability within a wide pH range (pH1.5 to pH10). The SCX (Strong Cation eXchange) column has benzene sulphonic acid covalently attached to a silica support and as such strongly retains high pKa·(ie basic) organic molecules such as amines, which can be subsequently liberated with excess ammonia in an appropriate solvent. Measured retention times are dependent on the precise conditions of the chromatographic procedures. Where quoted below in the Examples they are indicative of the order of elution.

Reactions involving metal hydrides including lithium hydride, lithium aluminium hydride, di-isobutylaluminium hydride, sodium hydride, sodium borohydride and sodium triacetoxyborohydride are carried out under argon.

### Differential Scanning Calorimetry (DSC)

### Method A

DSC is conducted on a TA Instrument model Q100 Differential Scanning Calorimeter. The sample is placed and weighed in a A1 DSC pan. The pan is scaled using the hand press supplied by the vendor. The sample is ramped from 25 °C to 300 °C at 15 °C/minute.

### Method B

DSC is conducted on a TA Instrument Q1000 Differential Scanning Calorimeter. The sample is weighed and placed in the DSC pan (sample weights are recorded on the DSC plot). The pan is scaled by applying pressure by hand and pushing each part the pan together (loose lid configuration). The sample is ramped from 25 °C to 350°C at 10 °C/minute.

### X-Ray Powder Diffraction (XRPD)

### Method A: PXRD General Area Detector Diffraction System

The sample is scanned using the following parameters:
Scan range: 2-40 degrees two-theta
Generator power: 40kV, 40mA
Radiation Source: Cu Ka
Scan type: Coupled scan
Number of frames: 3 frames
Time per frame: 5 min
Sample Oscillation: 0. 1-0.5mm oscillation depending on sample size
Detector Distance: 25cm
Filter/monochrometer: Single Goebel Mirror
Detector Type: General Area Detector Diffraction

### Method B: PXRD PANalytical X'Pert Pro MPD w/ Alpha-1 Monochrometer

The sample is scanned using the following parameters:
Scan range: 2-40 degrees two-theta
Generator power: 40kV, 45mA
Radiation Source: Cu Ka
Scan type: Continuous
Time per step: 30 seconds.
Step size: 0.017 degrees two-theta per step
Sample Rotation: 1s revolution time
Incident Beam optics: 0.04 radian soller slits, Automatic divergent slit, Diffracted Beam optics: Automatic slits (X'celerator module w/ Alpha-1 Monochrometer), 0.04 radian soller slits Detector Type: Philips X'Celerator

### Method C: PXRD PANalytical X'Pert Pro MPD w/ Alpha-1 Monochrometer

The sample is scanned using the following parameters:
Scan range: 2-40 degrees two-theta
Generator power: 40kV, 40mA
Radiation Source: Cu Ka
Scan type: Continuous
Time per step: 10 seconds
Step size: 0.017 degrees two-theta per step
Sample Rotation: 1s revolution time
Incident Beam optics: 0.04 radian soller slits, 0.25 degree divergent slit, 10mm beam mask, 0.5 degrees anti-scatter slit Diffracted Beam optics: fixed slits (X'celerator module), 0.04 radian soller slits Detector Type: Philips X'Celerator RTMS (Real Time Multi Strip)

As will be understood by the skilled chemist, references to preparations carried out in a similar manner to, or by the general method of, other preparations, may encompass variations in routine parameters such as time, temperature, workup conditions, minor changes in reagent amounts etc.

### Example 1 1-({4-[(2,3-Dihydro[1,4]dioxino[2,3-c]pyridin-7-ylmethyl)amino]-1-piperidinyl}methyl)-9-fluoro-1,2-dihydro-4H-pyrrolo[3,2,1-ij]quinolin-4-one, Enantiomer E2 Dihydrochloride

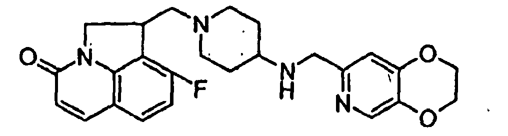

### (a) (2E)-N-(3-Fluoro-2-methylphenyl)-3-phenyl-2-propcnamide

A solution of cinnamyl chloride (100 g, 610 mmol) in ethyl acetate (400 ml) was added to a vigorously-stirred mixture of 3-fluoro-2-methylaniline (75 g, 600 mmol), saturated aqueous sodium bicarbonate (850 ml), ice (ca 100 g) and ethyl acetate (400 ml) over 2 min. After 0.25 hour the mixture was filtered, washing with water, more solids coming out of the filtrate and so refiltered. The resulting solid was dried *in vacuo* (∼160 g, 100%).
MS (+ve ion electrospray) m/z 256 (MH+).

### (b) 7-Fluoro-8-methyl-2(1H)-quinolinone

A slurry of (2E)-N-(3-fluoro-2-methylphenyl)-3-phenyl-2-propenamide (75 g, 305 mmol) in chlorobenzene (400 ml) was treated slowly with aluminium trichloride (163 g, 1.2 mol), with the temperature < 30°C. The reaction was stirred vigorously and heated to 65°C (internal temperature) for 1 hour then to 75°C (internal temperature) for 0.5 hour. The mixture was allowed to cool (ca 40°C), then added to excess ice with vigorous stirring. The resulting precipitate was isolated by filtration and washed with water. Drying *in vacuo* afforded the product (42.5g, 79%).
MS (+ve ion electrospray) m/z 178 (MH+).

### (c) 7-Fluoro-8-methyl-2-(methytoxy)quinoline

Crude 7-fluoro-8-methyl-2(1H)-quinolinone (46 g, 260 mmol) was suspended in DMSO (300 ml), warmed to 35°C, then treated with potassium t-butoxide (32g, 286 mmol), under argon (the internal temperature rose to 45°C)**.** After 15 minutes methyl iodide (21 min, 48 g, 338 mmol) was added over 2 minutes. (The internal temperature rose to 60°C). After 30 min the mixture was added to water (2 litres) and extracted with hexane (1.5 litres). The hexane extract was further washed with brine, dried over sodium sulphate, and filtered through a plug of silica, eluting with 1:1 hexane:dichloromethane (500 ml). Evaporation afforded the product (36.8g, 74%).
MS (+ve ion electrospray) m/z 192 (MH+).

### (d) 8-(Bromomethyl)-7-fluoro-2-(methyloxy)quinoline

A solution of 7-fluoro-8-methyl-2-(methyloxy)quinoline (36.7 g, 192 mmol) in trifluoromethylbenzene (500 ml) was treated with N-bromosuccinimide (37.6 g, 211 mmol) and benzoyl peroxide (243 mg, 1 mmol) and heated at 70°C (oil bath temperature) while irradiating'with a 120 Watt tungsten lamp for I hour. The cooled mixture was filtered, washed with dichloromethane, and the combined organic fractions were washed with saturated aqueous sodium bicarbonate solution then dried. The solution was filtered through a plug of silica and evaporated affording a pale yellow solid (51.4 g, 99%).
MS (+ve ion electrospray) m/z 271 (MH+).

### (e) [7-Fluoro-2-(methyloxy)-8-quinolinyl]acetonitrile

A solution 8-(bromomethyl)-7-fluoro-2-(mcthyloxy)quinoline (22.6 g, 84 mmol) in DMF (600 ml) was treated with potassium cyanide (25 g, 385 mmol) and heated at 70°C (oil bath temperature) overnight. The mixture was evaporated to dryness and the residue partitioned between ethyl acetate and water.The organic extract was washed with brine, dried and filtered through a plug of silica and evaporated affording a pale brown solid (17. 6g, 97%).
MS (+ve ion electrospray) m/z 217 (MH+).

### (f) Methyl [7-fluoro-2-(methyloxy)-8-quinolinyl]acetate

(i) A solution of [7-fluoro-2-(methyloxy)-8-quinolinyl]acetonitrile (50 g, 0.231 mol) in dry methanol (850 ml) was treated with trimethylsilyl chloride (100 ml; 0.78 mol) and heated at 79°C for 2.25 hours. The mixture was evaporated and then partitioned between ethyl acetate (1L) and water (700 ml). The mixture was filtered to remove methyl (7-fluoro-2-oxo-1,2-dihydro-8-quinolinyl)acetate and the aqueous layer was re-extracted with ethyl acetate (2 x 300 ml). The combined organic fraction was washed with 2N sodium hydroxide, water (x 2), dried (sodium sulphate), and evaporated. The reaction was repeated on the same scale, as above.

The combined reaction products were chromatographed on silica gel (1.5 kg); eluting with dichloromethane, to afford (83.3 g; 72%).
MS (+ve ion electrospray) m/z 250 (MH+)..

(ii) The recovered methyl (7-fluoro-2-oxo-1,2-dihydro-8-quinolinyl)acetate (11 g, 46.8 mmol) was suspended in methanol (20 ml), acetonitrile (200 ml) and triethylamine (8 ml, 57 mmol), with stirring together with 2M (trimethylsilyl)diazomethane in hexanes (30 ml, 60 mmol) and the mixture was stirred at room temperature for 3 hours. It was evaporated to dryness and chromatographed on silica gel, eluting with DCM, to afford an additional quantity of methyl [7-fluoro-2-(methyloxy)-8-quinolinyl]acetate (10.8 g).
[Total yield 81%]

### (g) Methyl 2-[7-fluoro-2-(methyloxy)-8-quinolinyl]-2-propenoate

A mixture of methyl [7-fluoro-2-(methyloxy)-8-quinolinyl]acetate (48 g; 0.193 mol), paraformaldehyde (41 g; 1.37 mol), potassium carbonate (41 g; 0.295 mol) and benzyltriethyl ammonium chloride (70 g; 0.307 mol) in cyclohexane (1.2 L) was heated at 86°C , with vigorous stirring for 5 hours. The mixture was cooled, water added and the mixture was extracted with ethyl acetate (x 3). The mixture was filtered and re-extracted with ethyl acetate (x 3). The combined organic fraction was washed with water (x 2), brine, and dried. The reaction was repeated on the same scale, as above, and the products combined and evaporated to give a solid (97.9 g; crude yield 97%), sufficiently pure (ca. 90% by NMR) for the next step. The other 10% of material is mainly starting material.
MS (+ve ion electrospray) m/z.262 (MH+).

### (h) Methyl 3-[4-({[(1,1-dimethylethyl)oxy]carbonyl}amino)-1-piperidinyl]-2-[7-fluoro-2-(methyloxy)-8-quinolinyl]propanoate

A solution of methyl 2-[7-fluoro-2-(methytoxy)-8-quinolinyl]-2-propenoate (90% pure; 106 g; equiv. to 0.367 mol), 1,1-dimethylethyl 4-piperidinylcarbamate (80.75 g; 0.404 mol) and 1,1,3,3-tetramethylguanidine (13 ml) in dry DMF (1.2 L) was heated at 80 °C for 2 hours, and then at 50°C overnight. The mixture was evaporated to dryness, azeotroped with toluene, and chromatographed on silica gel, eluting with hexane and then ethyl acetate-hexane (1:1), affording the product (155.4 g; 92%).
MS (+ve ion electrospray) m/z 462 (MH+).

### (i) 1,1-Dimethylethyl (1-{2-[7-fluoro-2-(methyloxy)-8-quinolinyl]-8-hydroxypropyl}-4-piperidinyl)carbamate

A solution of methyl 3-[4-({[(1,1-dimethylethyl)oxy]carbonyl}amino)-1-piperidinyl]-2-[7-fluoro-2-(methyloxy)-8-quinolinyl]propanoate (76 g, 0.165mol) in dry tetrahydrofuran (900 ml) at -70 °C was treated with a solution of lithium aluminium hydride in tetrahydrofuran (1M, 196 ml, 0.196mol) and stirred at this temperature for 1 hour, then at 0-10°C for 1 hour. Water (18 ml) was cautiously added followed by aqueous sodium hydroxide solution (2M, 33 ml, 0.196 mol), and water (38 ml). The mixture was stirred for 0.5 hour then ether and sodium sulphate were added and the mixture was stirred for 0.5 hour. It was filtered and evaporated, and the residue was recrystallised from ethyl acetate/hexane to give a white solid in two crops (57.7 g; 81%).
MS.(+ve ion electrospray) m/z 434 (MH+).

### (j) 1,1-Dimethylethyl {1-[(9-fluoro-4-oxo-1,2-dihydro-4H-pyrrolo[3,2,1-ij]quinolin-1-yl)methyl]-4-piperidinyl}carbamate

A solution of 1,1-dimethylethyl (1-{2-[7-fluoro-2-(methyloxy)-8-quinolinyl]-3-hydroxypropyl}-4-piperidinyl)carbamate (67.35 g, 0.156 mol) in chloroform (1L) was treated with diisopropylethylamine (60 ml, 0.34 mol) and methanesulphonic anhydride (32.6 g, 0.187 mol). The mixture was stirred at room temperature for 0.5 hour, then heated at 65°C for 3 hours, and then allowed to cool to room temperature. The mixture was washed with sodium bicarbonate solution (2 x 1 L), brine (1L), dried, and evaporated to give a solid (54.75 g; 88%). MS (+ve ion electrospray) m/z 402 (MH+).

### (k) 1-[(4-Amino-1-piperidinyl)methyl]-9-fluoro-1,2-dihydro-4H-pyrrolo[3,2,1-ij]quinolin-4-one enantiomers E1 and E2

### Method A

1,1-Dimethylethyl{1-[(9-fluoro-4-oxo-1,2-dihydro-4H-pyrrolo[3,2,1-ij]quinolin-1-yl)methyl]-4-piperidinyl}carbamate (54.75 g, 0.14 mmol) was dissolved in dichloromethane (300 ml) and trifluoroacetic acid (100 ml), stirred at room temperature for 3 hours, evaporated to dryness and azeotroped with toluene. The residue was triturated with ether to give a pink solid that was filtered off, washed with more ether and dried at 35°C under vacuum overnight to give a solid (62.35 g; 110% - contains excess TFA).
MS (+ve ion electrospray) m/z 302 (MH+).

Racemic material (as trifluoroacetate salt; 114 g) was separated by preparative chiral hplc into the two enantiomers, E1 and E2, using a 20 um Chiralpak AD column, eluting with 80:20:0.1- CH₃CN:CH₃OH:Isopropylamine with Rt E1 7.2 min and Rt E2 8.3 min.
The recovery was E1 29.3 g (97.4% ee) and E2 30.2 g (94.4 % ee).

### Method B

1,1-Dimethylethyl {1-[(9-fluoro-4-oxo-1,2-dihydro-4H-pyrrolo[3,2,1-ij]quinolin-1-yl)methyl]-4-piperidinyl}carbamate (92g, 229 mmol) was treated with concentrated hydrochloric acid (370 ml) and water (300 ml) with external cooling in ice bath. The mixture was stirred overnight, warming to room temperature. The mixture was then concentrated in vacuo at 50°C for 3 hours. The resultant amorphous gel was triturated with ethanol (1 litre) and stirred vigorously affording a fine white solid. This was isolated by filtration, washing with ether (3 x 500 ml). Drying in vacuo at 45°C afforded a white solid (70.56g, 82%).

1-[(4-Amino-1-piperidinyl)methyl]-9-fluoro-1,2-dihydro-4H-pyrrolo[3,2,1-ij]quinolin-4-one (racemic, dihydrochloride salt) (30g) was subjected to preparative hplc chromatography on a 20um Chiralpak AD column eluting with 80:20:0.1 acetonitrile:methanol:isopropylamine affording the E1 enantiomer (Rt 3.6 minutes) as an off-white solid (9.42g).

Triethylamine can be substituted for isopropylamine in the preparative hplc stage.

### (1) Title compound

A mixture of 1-[(4-amino-1-piperidinyl)methyl]-9-fluoro-1,2-dihydro-4H-pyrrolo[3,2,1-ij]quinolin-4-one (enantiomer E2, 67 mg, 0.22 mmol) and 2,3-dihydro[1,4]dioxino[2,3-c]pyridine-7-carboxaldehyde (for a synthesis see WO2004058144, Example 2(c)) (37 mg) in dichloromethane/methanol (3 ml/0.2 ml) was treated with sodium triacetoxyborohydride (141 mg, 0.66 mmol). After stirring overnight the mixture was partitioned between 5% methanol in dichloromethane and saturated aqueous sodium bicarbonate solution. The aqueous phase was extracted several times with 5% methanol in dichloromethane then the combined organic extracts were dried (magnesium sulphate) and evaporated under vacuum. The residue was chromatographed on silica gel, eluting with 0-20% methanol in DCM. then a 20 -50% gradient of methanol in ethyl acetate affording the free base of the title compound as a yellow oil (71 mg, 71%).
δH (CDCl₃, 250MHz) 1.40-1.55 (2H, m), 1.80-1.95 (2H, m), 2.08 (1H, dt), 2.22 (1H, dt), 2.45-2.55 (2H, m), 2.75-2.90 (2H, m), 2.95-3.05 (1H, bd), 3.78 (2H, s), 3.95-4.05 (1H, m), 4.28-4.35 (4H, m), 4.40-4.50 (2H, m), 6.60 (1H, d), 6.80-6.90 (2H, m), 7.40 (1H, dd), 7.65 (1H, d), 8.10 (1H, s).
MS (+ve ion electrospray) m/z 451 (MH+).

This material was converted into the title compound by adding excess hydrogen chloride in ether(86 mg).

### Example 2 1-({4-[(2,3-Dihydro[1,4]dioxino[2,3-c]pyridin-7-ylmethyl)amino]-1-piperidinyl}methyl)-9-fluoro-1,2-dihydro-4H-pyrrolo[3,2,1-ij]quinolin-4-one, Enantiomer E1 Dihydrochloride

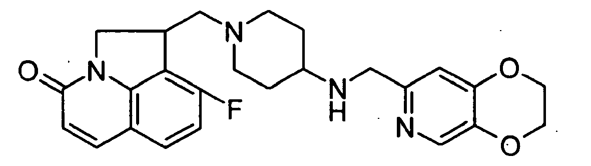

The E1 enantiomer free base (63 mg) was prepared from 1-[(4-amino-1-piperidinyl)methyl]-9-fluoro-1,2-dihydro-4H-pyrrolo[3,2,1-ij]quinolin-4-one (enantiomer E1) (64 mg) and 2,3-dihydro[1,4]dioxino[2,3-c]pyridine-7-carboxaldehyde (35 mg) by the general method of Example 1(1) (chromatographed on silica gel, eluting with 0-20% methanol in dichloromethane), and exhibited the same NMR and MS spectroscopic properties.

The free base in methanol, was converted to the dihydrochloride salt by adding an excess of I M hydrogen chloride in methanol, followed by evaporation to dryness and trituration with ether, to give a solid (61 mg).

### Example 3 1-({(3R,4S)-4-[(2,3-Dihydro[1,4]dioxino[2,3-c]pyridin-7-ylmethyl)amino]-3-hydroxy-1-piperidinyl}methyl)-9-fluoro-1,2-dihydro-4H-pyrrolo[3,2,1-ij]quinolin-4-one Diastereomer 1, Dihydrochloride

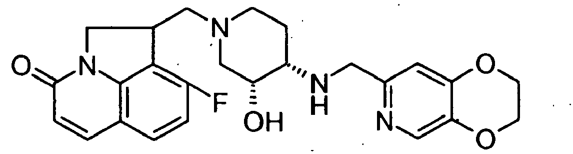

### (a) 1-{[(3R,4S)-4-amino-3-hydroxy-1-piperidinyl]methyl}-9-fluoro-1,2-dihydro-4H-pyrrolo[3,2,1-ij]quinolin-4-one

This was prepared from methyl 2-[7-fluoro-2-(methyloxy)-8-quinolinyl]-2-propenoate and 1,1-dimethylethyl[(3R,4,S)-3-hydroxy-4-piperidinyl]carbamate (for a synthesis see WO2004058144, Example 34(a) (cis Enantiomer 1)) according to the general method of Examples 1(h), 1(i), 1(j) and 1(k) affording the product as a white solid, racemic at the benzylic centre.
MS (+ve ion electrospray) m/z 318 (MH+).

The material (racemic at the benzylic centre) was separated by preparative chiral hplc into the two diastereomers D1 and D2 in a similar manner to Example 1(k). The stationary phase was 5µm Chiralpak AD-H, eluting with 50 :50:0.1-CH₃CN:CH₃OH:Isopropylamine, Rt D1 3.0 min and Rt D2 27 min.

The recovery was D1 222 mg (>99% de) and D2 123 mg (>99 % de) from 400 mg of diasteromeric amine.

### (b) Title compound

A solution of 1-{[(3R,4S)-4-amino-3-hydroxy-1-piperidinyl]methyl}-9-fluoro-1,2-dihydro-4H-pyrrolo[3,2,1-ij]quinolin-4-one (222 mg, 0.7 mmol, D1 diastereomer) and 2,3-dihydro[1,4]dioxino[2,3-c]pyridine-7-carboxaldehyde (for a synthesis see WO2004058144, Example 2(c)) (115 mg, 0.7 mmol) in N,N-dimethylformamide (3 ml) was treated with sodium triacetoxyborohydride (445 mg, 2.1 mmol). After 1 day the mixture was evaporated and the residue worked up and chromatographed in a similar manner to Example 1(1) affording the free base of the title compound as a colourless oil (202 mg, 62%).
δH (CDCl₃, 250MHz) 1.70-1.80 (2H, m), 2.20-2.30 (2H, m), 2.50-2.6.5 (2H, m), 2.70-2.95 (3H, m), 3.00-3.10 (1H, m), 3.85 (2H, s), 3.95-4.05 (1H, m), 4.25-4.35 (4H, m), 4.40-4.55 (2H, m), 6.62 (1H, d), 6.85 (1H, t), 6.85 (1H, s), 7.58 (1H, dd), 7.65 (1H, m), 8.10 (1H, s).

This material was converted into the title compound (220 mg) by dissolving in dichloromethane and then adding excess hydrogen chloride in ether in a similar manner to Example 1.
MS (+ve ion electrospray) m/z 467 (MH+).

### Example 4 1-({(3R,4S)-4-[(2,3-Dihydro[1,4]dioxino[2,3-c]pyridin-7-ylmethyl)amino]-3-hydroxy-1-piperidinyl}methyl)-9-fluoro-1,2-dihydro-4H-pyrrolo[3,2,1-ij]quinolin-4-one Diastereomer 2, Dihydrochloride

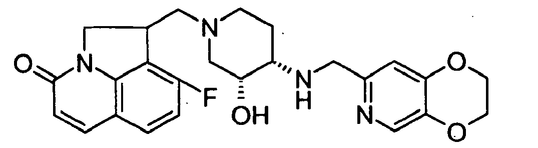

The free base of the title compound (93 mg) was prepared from 1-{[(3R,4S)-4-amino-3-hydroxy-1-piperidinyl]methyl}-9-fluoro-1,2-dihydro-4H-pyrrolo[3,2,1-ij]quinolin-4-one (D2 diastereomer) (122 mg) and 2,3-dihydro[1,4]dioxino[2,3-c]pyridine-7-carboxaldehyde (for a synthesis see WO2004058144, Example 2(c)) (64 mg) by the general method of Example 3 (chromatographed on silica gel, eluting with 0-30% methanol in dichloromethane), giving material with the same NMR and MS spectroscopic data as Example 3.

The free base in methanol, was converted to the dihydrochloride salt by adding an excess of 1M hydrogen chloride in methanol, followed by evaporation to dryness, to give a solid (87 mg).

### Example 5 9-Fluoro-1-({(3R,4S)-3-hydroxy-4-[([1,3]oxathiolo[5,4-c]pyridin-6-ylmethyl)amino]-1-piperidinyl}methyl)-1,2-dihydro-4H-pyrrolo[3,2,1-ij]quinolin-4-one Dihydrochloride

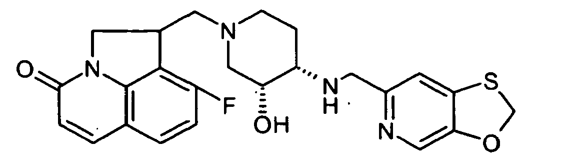

The free base of the title compound (41 mg) was prepared from diastereomeric 1-{[(3R,4S)-4-amino-3-hydroxy-1-piperidinyl]methyl}-9-fluoro-1,2-dihydro-4H-pyrrolo[3,2,1-ij]quinolin-4-one and [1,3]oxathiolo[5,4-c]pyridine-6-carbaldehyde (for a synthesis, see WO2004058144, Example 61) in 55% yield by the general method of Example 3(b).
δH (CDCl₃, 250MHz) 1.70-1.80 (2H, m), 2.20-2.60 (4H, m), 2.70-3.00 (3H, m), 3.00-3.10 (1H, m), 3.87 (2H, s), 3.95-4.05 (1H, m), 4.40-4.55 (2H, m), 5.75 (2H, s), 6.62 (1H, d), 6.85 (1H, t), 7.25 (1H,s), 7:58 (1H, dd), 7.65 (1H, m), 8.00 (1H, s).
MS (+ve ion electrospray) m/z 469 (MH+).

The free base was converted to the title dihydrochloride salt in a similar manner to Example 1.

### Example 6 9-Fluoro-1-[((3R,4S)-3-hydroxy-4-{[(6-oxo-6,7-dihydro-5H-pyridazino[3,4-b][1,4]thiazin-3-yl)methyl]amino}-1-piperidinyl)methyl]-1,2-dihydro-4H-pyrrolo[3,2,1-ij]quinolin-4-one Dihydrochloride

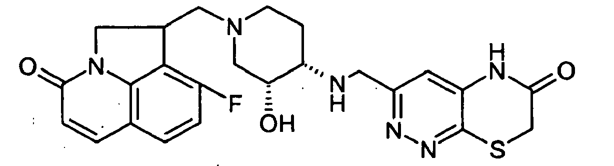

The free base of the title compound (12 mg) was prepared from diastereomeric 1-{[(3R,4S)-4-amino-3-hydroxy-1-piperidinyl]methyl}-9-fluoro-1,2-dihydro-4H-pyrrolo[3,2,1-ij]quinolin-4-one (122 mg) and 6-oxo-6,7-dihydro-5H-pyridazino[3,4-b][1,4]thiazine-3-carboxaldehyde (for a synthesis, see WO2004058144, Example 58(d)) (75 mg) in 6% yield by the general method of Example 3(b).
δH (CDCl₃, 250MHz) 1.70-1.80 (2H, m), 2.20-2.60 (4H, m), 2.70-3.00 (3H, m), 3.00-3.10 (1H, m), 3.78 (2H, s), 3.90-4.00 (1H, m), 4.05 (2H, s), 4.40-4.55 (2H, m), 6.62 (1H, d), 6.95 (1H, t), 7.16 (1H, s), 7.58 (1H, dd), 7.90 (1H, d).
MS (+ve ion electrospray) m/z 497 (MH+).

The free base was converted to the title dihydrochloride salt (9 mg) in a similar manner to Example 1

### Example 7 1-({(3R,4S)-4-[(2,3-Dihydro-1H-pyrido[3,4-b][1,4]oxazin-7-ylmethyl)amino]-3-hydroxy-1-piperidinyl}methyl)-9-fluoro-1,2-dihydro-4H-pyrrolo[3,2,1-ij]quinolin-4-one Dihydrochloride

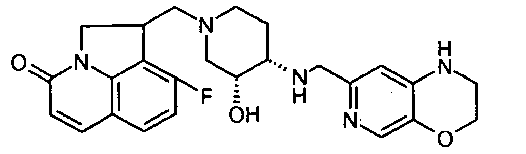

### (a) {5-({[4-(Methyloxy)phenyl]methyl}oxy)-4-[(phenylmethyl)oxy]-2-pyridinyl}methyl acetate

A solution of triphenylphosphine (39.3 g, 150 mmol) in tetrahydrofuran (600 ml) was treated at 0°C with bis(1-methylethyl) (E)-1,2-diazenedicarboxylate (30 ml, 152 mmol). After 10 minutes [5-({[4-(methoxy)phenyl]methyl}oxy)4-oxo-1,4-dihydro-2-pyridinyl]methyl acetate (33.5g, 110 mmol) (for a synthesis, see WO2004058144, Example 60(c)) was added. After 10 minutes benzyl alcohol (13 g, 120 mmol) was added and the mixture was stirred overnight. Evaporation and chromatography on silica eluting with 20-40% ethyl acetate in hexane afforded an oil (26.3 g, 67%) (containing some triphenylphosphine oxide as an impurity).
MS (+ve ion electrospray) m/z 394 (MH+).

### (b) {5-Hydroxy-4-[(phenylmethyl)oxy]-2-pyridinyl}methyl acetate, trifluoroacetate salt

A solution of {5-({[4-(methyloxy)phenyl]methyl}oxy)-4-[(phenylmethyl)oxy]-2-pyridinyl} methyl acetate (containing triphenylphosphine oxide as an impurity) (20 g, 50.8 mmol) in dichloromethane (500 ml) was treated with triethylsilane (10 ml, 62.6 mmol). A solution of trifluoroacetic acid (35 ml, 0.45 mol) in dichloromethane (200ml) was added over I hour. After 2 hours the mixture was evaporated and chromatographed on silica gel dating with 50-100% ethyl acetate-hexane, then 5-10% methanol-DCM affording a solid, the TFA salt in a 1:1 mixture with triphenylphosphine oxide (8.33 g). MS (+ve ion electrospray) m/z 274 (MH+).

### (c) (5-{[2-({[(1,1-Dimethylethyl)oxy]carbonyl}amino)ethyl]oxy}-4-{[(trifluoromethyl)sulfonyl]oxy}-2-pyridinyl)methyl acetate

A solution of triphenylphosphine (24.1 g, 92 mmol) in tetrahydrofuran (600 ml) was treated at 0°C with bis(1-methylethyl) (E)-1,2-diazenedicarboxylate (18.1 ml, 92 mmol). After 30 minutes a solution of 5-hydroxy-4-[(phenylmethyl)oxy]-2-pyridinyl}methyl acetate trifluoroacetate salt as a 1:1 mixture with triphenylphosphine oxide (23.8g, 61.3 mmol) and triethylamine (8.6ml, 61.3 mmol) in tetrahydrofuran (200 ml) was added. After 30 minutes the reaction was warmed to room temperature and left to stir for a further 30 minutes. 1,1-Dimethylethyl (2-hydroxyethyl)carbamate (9.5 ml, 61.3 mmol) was added and the mixture stirred overnight. Evaporation and chromatography on silica eluting with 0-100% ethyl acetate in petrol afforded an oil (40.2 g).The oil (40.2g) was dissolved in EtOH (300ml) and hydrogenated over 10% palladium on charcoal (20 g) for 16 hours. The mixture was filtered and evaporated to give a yellow oil (44.4 g).

A solution of the yellow oil (44.4 g) in dichloromethane (500 ml) was treated with triethylamine (9.41 ml) then 1,1,1-trifluoro-N-phenyl-N-[(trifluoromethyl)sulfonyl]methanesulfonamide (21.9 g). After 16 hours the mixture was washed with water, dried and evaporated. Chromatography on silica gel, eluting with 0-100% ethyl acetate in petrol afforded a colourless oil (19.7 g, 70%).
MS (+ve ion electrospray) m/z 459 (MH+).

### (d) 1,1-Dimethylethyl 7-[(acetyloxy)methyl]-2,3-dihydro-1H-pyrido[3,4-b][1,4]oxazine-1-carboxylate

A mixture of (5- {[2-({[(1,1-dimethylethyl)oxy]carbonyl}amino)ethyl]oxy}-4-{[(trifluoromethyl)sulfonyl]oxy}-2-pyridinyl)methyl acetate (1.58 g, 3.4 mmol), (±)-2,2'-bis(diphenylphosphino)-1,1'-binaphthalene (BINAP) (110 mg, 0.2 mmol), palladium(II) acetate (25 mg, 0.1 mmol) and cesium carbonate (1.57 g, 4.8 mmol) in toluene (20 ml) was heated to 100°C under argon for 16 hours then filtered and evaporated. (See S. L. Buchwald, Org Letts, 1999, 1, 35-37; for the procedure). The residue was chromatographed on silica gel, eluting with 0-100% ethyl acetate in petrol, to afford a white solid (0.84 g, 79%)
MS (+ve ion electrospray) m/z 309 (MH+).

### (e) 1,1-Dimethylethyl 7-(hydroxymethyl)-2,3-dihydro-1H-pyrido[3,4-b][1,4]oxazine-1-carboxylate

A solution of 1,1-dimethylethyl 7-[(acetyloxy)methyl]-2,3-dihydro-1H-pyrido[3,4-b][1,4]oxazine-1-carboxylate (0.84 g, 2.7 mmol) in dioxan (20 ml) and water (5 ml) was treated with 2M sodium hydroxide solution (2.72 ml, 5.4 mmol). After 0.5 hour the mixture was concentrated to a volume of 5 ml and then partitioned between ethyl acetate and water. The organic extract was dried and evaporated to afford a colourless oil (0.78 g, 105%).
MS (+ve ion electrospray) m/z 267(MH+).

### (f) 1,1-Dimethylethyl 7-formyl-2,3-dihydro-1H-pyrido[3,4-b][1,4]oxazine-1-carboxylate

A solution of 1,1-dimethylethyl 7-(hydroxymethyl)-2,3-dihydro-1H-pyrido[3,4-b][1,4]oxazine-1-carboxylate (0.78 g, 2.9 mmol) in dichloromethane (100 ml) was treated with manganese(IV) oxide (2.02 g, 23.3 mmol) and stirred overnight. Filtration and evaporation afforded a white solid (0.62 g, 81%).
MS (+ve ion electrospray) m/z 265(MH+).

### (g) 1,1-Dimethylethyl 7-[({(3R,4S)-1-[(9-fluoro-4-oxo-1,2-dihydro-4H-pyrrolo[3,2,1-ij]quinolin-1-yl)methyl]-3-hydroxy-4-piperidiny}amino)methyl]-2,3-dihydro-1H-pyrido[3,4-b][1,4]oxazine-1-carboxylate

A solution of diastereomeric 1-{[(3R,4S)-4-amino-3-hydroxy-1-piperidinyl]methyl}-9-fluoro-1,2-dihydro-4H-pyrrolo[3,2,1-ij]quinolin-4-one (50 mg) was reacted with 1,1-dimethylethyl 7-formyl-2,3-dihydro-1H-pyrido[3,4-b][1,4]oxazine-1-carboxylate (40 mg) by the general method of Example 3(b) affording, after chromatography, eluting with 0-20% methanol in dichloromethane, a white solid (62 mg, 69%).
MS (+ve ion electrospray) m/z 566(MH+).

### (h) Title compound

A solution of 1,1-dimethylethyl 7-[({(3R,4S)-1-[(9-fluoro-4-oxo-1,2-dihydro-4H-pyrrolo[3,2,1-ij]quinolin-1-yl)methyl]-3-hydroxy-4-piperidinyl}amino)methyl]-2,3-dihydro-1H-pyrido[3,4-b][1,4]oxazine-1-carboxylate (62 mg) in dichloromethane (1.5 ml) was treated with trifluoroacetic acid (1.5 ml). After 1 hour the mixture was evaporated and the residue was azeotroped with toluene. The residual trifluoroactetate salt was converted to the crude free base by stirring with an excess of MP-carbonate resin base until pH 7, filtering and evaporating to dryness. It was chromatographed on silica gel eluting with 10-40% 2M ammonia/methanol in dichloromethane, followed by further purification on a reverse-phase HPLC system with mass-directed collection (MDAP) (eluent acetonitrile/water/formic acid, monitoring for *m*/*z 466*), affording a white solid (35 mg, 62%).
δH (d-6 methanol, 250MHz) 1.70-1.90 (2H, m), 2.20-2.60 (4H, m), 2.70-3.00 (2H, m), 3.10-3.25(1H, m), 3.50 (2H, t), 4.00 (2H, s), 4.05 (2H, s), 4.20 (2H, t), 4.40-4.55 (2H, m), 6.60 (1H, d), 6.72 (1H, s), 7.00 (1H, t), 7.60 (1H, dd), 7.90 (1H, d), 7.95 (1H, d).
MS (+ve ion electrospray) m/z 466(MH+).

The free base was converted to the title dihydrochloride salt (22 mg) in a similar manner to Example 1.

### Example 8 1-({(3R,4S)-4-(2,3-Dihydrofuro[2,3-c]pyridin-5-ylmethyl)amino]-3-hydroxy-1-piperidinyl}methyl}-9-fluoro-1,2-dihydro-4H-pyrrolo[3,2,1-ij]quinolin-4-one Dihydrochloride

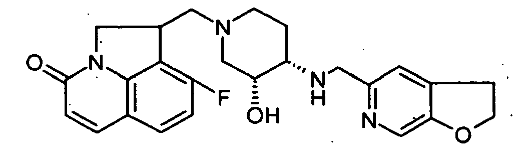

### (a) {5-({[4-(Methyloxy)phenyl]methyl}oxy)-4-[(trimethylsilyl)ethynyl]-2-pyridinyl}methyl acetate

(5-({[4-(Methoxy)phenyl]methyl}oxy)-4-{[(trifluoromethyl)sulfonyl]oxy}-2-pyridinyl)methyl acetate (for a synthesis, see WO2004058144 Example 60(d)) (10 g, 23 mmoles) was dissolved in acetonitrile (400 ml) and triethylamine (65 ml) and copper (I) iodide (0.44 g, 2.3 mmoles) were added. The mixture was degassed and placed under a blanket of argon. Trimethylsilylacetylene (10 ml, 69 mmoles) and bis(triphenylphosphine)palladium(II) dichloride (0.645 g, 0.9mmoles) were added and the mixture heated to 45°C for 18hrs. The mixture was then allowed to cool and filtered. The filtrate was evaporated to dryness and the residue partitioned between ethyl acetate and water. The organic layer was separated and dried (sodium sulphate).

Chromatography on silica gel, eluting with a gradient of 20 - 75% ethyl acetate in 40-60 petroleum ether, gave an oil (8.45 g, 96%).
MS (+ve ion electrospray) m/z 384 (MH+).

### (b) {5-Hydroxy-4-(trimethylsilyl)ethynyl]-2-pyridinyl} methyl acetate, trifluoroacetate

{5-({[4-(Methyloxy)phenyl]methy}oxy)-4-[(trimethylsilyl)ethynyl]-2-pyridinyl}methyl acetate (8.45 g, 22 mmoles) in dichloromethane (70 ml) was treated with trifluoroacetic acid (9.4 ml) and triethylsilane (3.33 ml) and stirred at ambient temperature for 18hrs. The mixture was evaporated to dryness and chromatographed on silica gel, eluting with a gradient of 2 - 8% methanol in dichloromethane. This gave an oil (10g, 100%).
MS (+ve ion electrospray) m/z 264 (MH+).

### (c) Furo[2,3-c]pyridin-5-ylmethyl acetate

{5-Hydroxy-4-[(trimethylsilyl)ethynyl]-2-pyridinyl}methyl acetate, trifluoroacetate) (10 g, 22 mmoles) was dissolved in pyridine (200 ml) and treated with copper(I) iodide (5.2 g, 27 mmoles) then heated under reflux for 18hrs. The mixture was allowed to cool, evaporated to dryness and the residue partitioned between ethyl acetate and water. This mixture was filtered through kieselguhr to remove copper residues. The organic layer was separated from the filtrate, dried and chromatographed on silica gel, eluting with a gradient of 10 - 60% ethyl acetate in 40-60 petroleum ether. This gave furo[2,3-c]pyridin-5-ylmethyl acetate (1.15 g, 27%) and a less polar product [2-(trimethylsilyl)furo[2,3-c]pyridin-5-yl]methyl acetate (1.3 g, 23%) as oils.
MS (+ve ion electrospray) m/z 192 (MH+) and MS (+ve ion electrospray) m/z 264 (MH+).

### (d) Furo[2,3-c]pyridin-5-ylmethanol

A solution of furo[2,3-c]pyridin-5-ylmethyl acetate (1.15 g) in 1,4-dioxane (30 ml) and water (10 ml) was treated with 2M sodium hydroxide (12 ml) then stirred at ambient temperature for 18hrs. The mixture was then partitioned between ethyl acetate and water. The organics were separated and dried then evaporated to dryness. This gave an oil (0.63 g, 70%).
MS (+ve ion electrospray) m/z 150 (MH+).

### (e) 2,3-Dihydrofuro[2,3-c]pyridin-5-ylmethanol

Furo[2,3-c]pyridin-5-ylmethanol (1.29 g, 8.7 mmoles) was dissolved in ethanol (50 ml) and hydrogenated at S.T.P (standard temperature and pressure) over 10% palladium on charcoal paste for 18hrs. The mixture was filtered through kieselguhr and the filtrate evaporated to dryness, to give (1.31 g, 100%).
MS (+ve ion electrospray) m/z 152 (MH+).

### (f) 2,3-Dihydrofuro[2,3-c]pyridine-5-carbaldehyde

2,3-Dihydrofuro[2,3-c]pyridin-5-ylmethanol (1.31 g, 8.7 mmoles) was dissolved in dichloromethane (100 ml), treated with manganese (IV)dioxide (6 g, 69 mmoles) and heated under reflux for 18hrs. Filtration through kieselguhr and evaporation of the filtrate to dryness gave an oil (0.9 g, 70%).
MS (+ve ion electrospray) m/z 150 (MH+).

### (g) Title compound

The free base of the title compound (65 mg) was prepared from diastereomeric 1-{[(3R,4S)-4-amino-3-hydroxy-1-piperidinyl]methy}-9-fluoro-1,2-dihydro-4H-pyrrolo[3,2,1-ij]quinolin-4-one (50 mg) and 2,3-dihydrofuro[2,3-c]pyridine-5-carbaldehyde (23 mg) in 91% yield by the general method of Example 3(b).
δH (d-6 methanol, 250MHz) 1.70-1.90 (2H, m), 2.20-2.60 (4H, m), 2.70-3.00 (2H, m), 3.10-3.25(1H, m), 3.50 (2H, t), 4.10-4.20 (2H, m), 4.30 (2H, s), 4.50 (2H, t), 4.60 (2H, t), 6.60 (1H, d), 7.00 (1H, t), 7.40 (1H, s), 7.60 (1H, dd), 7.95 (1H, d), 8.10 (1H, s).
MS (+ve ion electrospray) m/z 451 (MH+).

The free base was converted to the title dihydrochloride salt (46 mg) in a similar manner to Example 1

### Example 9 9-Fluoro-1-[((3R,4S)-3-hydroxy-4-{[(7-oxo-1,5,6,7-tetrahydro-1,8-naphthyridin-2-yl)methyl]amino}-1-piperidinyl)methyl]-1,2-dihydro-4H pyrrolo[3,2,1-ij]quinolin-4-one Dihydrochloride

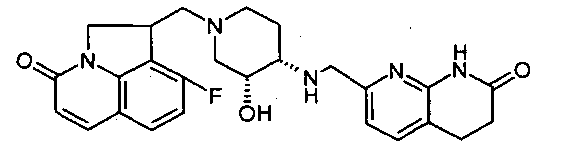

The free base of the title compound (12 mg) was prepared from diastereomeric 1-{[(3R,4S)-4-amino-3-hydroxy-1-piperidinyl]methyl}-9-fluoro-1,2-dihydro-4H-pyrrolo[3,2,1-ij]quinolin-4-one (50 mg) and 7-oxo-1,5,6,7-tetrahydro-1,8-naphthyridine-2-carboxaldehyde (for a synthesis, see WO2003087098, Example 307(f) (synonym 7-oxo-5,6,7,8-tetrahydro-[1,8]naphthyridine-2-carboxaldehyde)) (27 mg), in 16% yield by the general method of Example 3(b).
δH (CDCl₃, 250MHz) 1.70-1.90 (2H, m), 2.00-2.30 (4H, m), 2.40-2.75 (5H, m), 2.80-3.10 (3H, m), 3.95-4.10 (3H, m), 4.45 (2H, m), 6.65 (1H, d), 6.90 (1H, t), 7.00 (1H, dd), 7.35-7.45 (2H, m), 7.68 (1H, d), 8.30 (1H, bs).
MS (+ve ion electrospray) m/z 478 (MH+).

The free base was converted to the title dihydrochloride salt (8 mg) in a similar manner to Example 1

### Example 10A 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Dihydrochloride

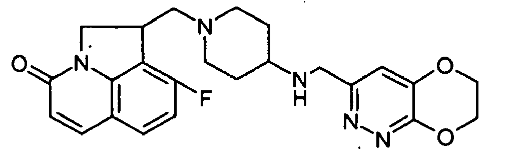

### (a) 3,4,6-Trichloropyridazine

This was prepared by a slight variation on the method of Kasnar et al, Nucleosides & Nucleotides (1994), 13(1-3), 459-79.

Hydrazine sulphate salt (51 g) was suspended in water (250ml), heated to reflux and bromomaleic anhydride (90.38 g) was added dropwise. The mixture was heated at reflux for 4 hours then cooled to room temperature. The reaction was repeated with 29 g hydrazine sulphate, 53 g bromomaleic anhydride and 130ml water. The precipitates were collected by filtration, washed with water and acetone and dried as a combined batch in vacuo to afford 4-bromo-1,2-dihydro-3,6-pyridazinedione as a white solid (113 g).

The solid in two batches was treated with phosphorus oxychloride (2 x 200 ml) and heated to reflux for 3.5 hours. The mixture was cooled, evaporated and azeotroped with toluene. The residue was partitioned between dichloromethane and saturated aqueous sodium bicarbonate solution and extracted with DCM twice more. The organic extracts were dried and evaporated. This residue was re-dissolved in dichloromethane, and chromatographed on silica gel (300 g) (DCM as eluent) to give a white solid (101.5 g, 87%).
(LC/MS analysis showed ca 20-30% impurity, isomers of bromo-dichloropyridazine). MS (+ve ion electrospray) m/z 184/185/186 (MH+), trichloropyridazine
MS (+ve ion electrospray) m/z 228/229/231 (MH+), bromo-dichloropyridazine;

### (b) 2-[(3,6-Dichloro-4-pyridazinyl)oxy]ethanol

A solution of ethylene glycol (55 ml) in tetrahydrofuran (200 ml) was treated at around 0°C (ice bath cooling) with sodium hydride (60% dispersion in oil, 5.9 g) over 40 minutes. After the addition was complete, 3,4,6-trichloropyridazine containing isomers of bromo-dichloropyridazine as impurity (27 g) was added portionwise and washed in with more dry THF (50ml) and the mixture was stirred at 0°C for I hour then at room temperature overnight. The mixture was concentrated (to 1/3 volume) then diluted with aqueous sodium bicarbonate solution and extracted with chloroform (5x) and ethyl acetate (3x). The combined organic extracts were washed with water, dried over sodium sulphate and evaporated and the solid filtered off and washed with CHCl₃ (x3) and dried in a vacuum over overnight at 40°C affording a white solid (25.5 g, 83%), containing some bromo-derivative (10-15%).
MS (+ve ion electrospray) m/z 209/211 (MH+).
MS (+ve ion electrospray) m/z 255/7 (MH+), bromo-derivative.

### (c) 3-Chloro-6,7-dihydro[1,4]dioxino[2,3-c]pyridazine

A solution of 2-[(3,6-dichloro-4-pyridazinyl)oxy]ethanol containing some bromo-derivative (15.46 g; 0.0703 mol) in dry dioxan (1.2 L) was treated with lithium hydride (2.3 g; 0.28 mol) in portions and stirred at room temperature for I hour under argon, then heated at 110 °C overnight. The reaction mixture was quenched with wet dioxan, then iced-water. The solution was evaporated to half volume, taken to pH 8 with 5M hydrochloric acid and evaporated to dryness. Water was added and the residue was extracted 5x with chloroform, dried (sodium sulphate) and evaporated to afford a white solid (12.4 g, ca.77%) (containing ca. 15% of a bromo species).
MS (+ve ion electrospray) m/z 173/5 (Cl MH+); 217/9 (Br MH+)

### (d) 3-Ethenyl-6,7-dihydro[1,4]dioxino[2,3-c]pyridazine

A solution of 3-chloro-6,7-dihydro[1,4]dioxino[2,3-c]pyridazine containing ca. 15% of a bromo species (13.6 g, 0.079 mol) in dimethoxyethane (400 ml) was degassed under argon for 10 min then tetrakis(triphenylphosphine)palladium (0) (2 g), potassium carbonate (10.33 g), 2,4,6-trivinylcyclotriboroxane pyridine complex (11.32 g) and water (55 ml) were added. The mixture was heated at 95 °C for 48 hours and cooled and evaporated to dryness. The mixture was treated with aqueous sodium bicarbonate solution and extracted (5x) with DCM. Extracts were dried (sodium sulphate), evaporated and the residue chromatographed on silica gel (500 g), eluting with 0-100% ethyl acetate - hexane, affording the product (6.43 g, 50%); [also some impure fractions (1.8 g)] MS (+ve ion electrospray) m/z 165 (MH+).

### (e) 6,7-Dihydro[1,4]dioxino[2,3-c]pyridazine-3-carbaldehyde

### Method A

A solution of 3-ethenyl-6,7-dihydro[1,4]dioxino[2,3-c]pyridazine (11.58 g) in dioxan/water (600 ml/180 ml), cooled in ice, was treated with an aqueous solution of osmium tetroxide (4% w/v, 25 ml) and sodium periodate (43 g). This mixture was allowed to warm to rt and after 7 hours under stirring the mixture was evaporated to dryness and azeotroped with dioxan. Silica gel, dioxan and chloroform were added and the mixture was evaporated to dryness overnight, then added to a silica column (400 g) and chromatographed, eluting with chloroform then 0-100% ethyl acetate in hexane, to afford a white solid (7.55 g, 64%).
MS (+ve ion electrospray) m/z 167 (MH+).

### Method B

### (i) Butyl 6,7-dihydro[1,4]dioxino[2,3-c]pyridazine-3-carboxylate

Carbon monoxide was bubbled through a mixture of 3-chloro-6,7-dihydro[1,4]dioxino[2,3*-c*]pyridazine (100 mg, 0.58 mmol) and *n*-butanol (2.5 ml) for 10 minutes then palladium(11)chloride (5 mg, 0.03 mmol), 1,3-bis(diphenylphosphino)propane (24 mg, 0.06 mmol) and 1,8-diazabicyclo[5.4.0]undec-7-ene (0.1 ml, 0.64 mmol) were added. The mixture was heated at 100°C for 5 hours under a stream of carbon monoxide, allowed to cool and then evaporated. Chromatography eluting with 75% ethyl acetate in hexane afforded slightly impure product (99 mg). This material was taken up in ethyl acetate and filtered removing a small amount of insoluble yellow solid. Evaporation of the filtrate afforded the product (90 mg, 65%).
MS (+ve ion electrospray) m/z 239 (MH+).

### (ii) 6,7-Dihydro[1,4]dioxino[2,3-c]pyridazine-3-carbaldehyde

A solution of butyl 6,7-dihydro[1,4]dioxino[2,3-c]pyridazine-3-carboxylate (570 mg, 2.39 mmol) in THF (10 ml) was cooled to approximately -50 to -40°C (solid carbon dioxide/acetonitrile cooling bath) and treated with a toluene solution of di-isobutylaluminium hydride (1M;4.6 ml, 4.6 mmol). After 2 hours aqueous sodium hydroxide solution (2M, 12 drops) were added followed by DCM (10 ml). The mixture was stirred for 15 minutes at -40°C then allowed to warm temperature. Sodium sulphate was added and the mixture stirred for 45 minutes. The mixture was filtered through Kieselguhr washing three times with 1:1 THF:DCM and the combined filtrates evaporated. Chromatography on 10g silica eluting with 0-20% methanol in DCM afforded the product (290 mg, 73%).
MS (+ve ion electrospray) m/z 167 (MH+).

An alternative work-up procedure comprises quenching with methanol instead of with sodium hydroxide and DCM. The reaction mixture is evaporated to dryness then dissolved in DCM and water added to form a gel-like precipitate. Conc. HCl is added and the mixture stirred and then the phases partitioned and separated. Salt is added to the aqueous phase followed by DCM, water and methanol. After stirring, the layers are separated, the aqueous extracted with DCM and all the combined organics dried over magnesium sulphate.

### Method C

### (i) 6,7-Dihydro[1,4]dioxino[2,3-c]pyridazine-3-carbonitrile

A solution of 3-chloro-6,7-dihydro[1,4]dioxino[2,3-c]pyridazine (2g, 11.6 mmol) in DMF (40 ml) was degassed under argon for 10 minutes then zinc(II)cyanide (0.82g, 7 mmol), tris(dibenzylideneacetone)palladium(0) (266 mg) and 1,1'-bis(diphenylphosphino)ferrocene (322 mg) were added. The mixture was heated at 120°C overnight then evaporated to dryness. The residue was partitioned between saturated aqueous sodium bicarbonate solution and DCM, extracting 3 times with DCM. The DCM phase was dried over sodium sulphate then filtered and evaporated. Chromatography on a 50g silica column eluting with 0-100% ethyl acetate in hexane afforded the product (1.5g, _ 79%).
MS (+ve ion electrospray) m/z 164 (MH+).

### (ii) Butyl 6,7-dihydro[1,4]dioxino[2,3-c]pyridazine-3-carboxylate

6,7-Dihydro[1,4]dioxino[2,3-c]pyridazine-3-carbonitrile (0.5g, 3.1 mmol) and caesium carbonate (1.5g, 4.6 mmol) were suspended in *n-*butanol (5 ml). After 5 hours stirring, hydrochloric acid (0.1M, 15 ml) then 2M hydrochloric acid was added until pH3-3.5 was attained. After 1 hour (more hydrochloric acid was added until pH no longer rose) the reaction mixture was quenched with saturated aqueous sodium bicarbonate solution and extracted three times with DCM. The combined DCM extracts were dried over sodium sulphate then evaporated to dryness. Chromatography on a 20g silica column eluting with 0-100% ethyl acetate in hexane afforded the product (0.55g, 77%) MS (+ve ion electrospray) m/z 239 (MH+).

### (iii) 6,7-Dihydro[1,4]dioxino[2,3-c]pyridazine-3-carbaldehyde

Butyl 6,7-dihydro[1,4]dioxino[2,3*-c*]pyridazine-3-carboxylate was reduced to the title compound in a similar manner to Method B (ii) above.

### Method D

3-Chloro-6,7-dihydro[1,4]dioxino[2,3-c]pyridazine may be converted to a morpholine amide by treatment with morpholine and CO, catalyzed by PdCl₂ and ligand (such as diphenylphosphinoferrocene) in a suitable solvent such as butyronitrile. The amide is then reduced to the carbaldehyde by reduction with di-isobutylaluminium hydride.

### (f) 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 (Method A)

A solution of 1-[(4-amino-1-piperidinyl)methyl]-9-fluoro-1,2-dihydro-4H-pyrrolo[3,2,1-ij]quinolin-4-one (enantiomer E1) (16.5 g, 55 mmol) and 6,7-dihydro[1,4]dioxino[2,3-c]pyridazine-3-carbaldehyde (10.0 g, 60.3 mmol) in dichloromethane/methanol (220 ml/60 ml) was cooled in an ice bath and treated with sodium triacetoxyborohydride (29 g, 135 mmol). The cooling bath was removed. After 3 hours, more 6,7-dihydro[1,4]dioxino[2,3-c]pyridazine-3-carbaldehyde (1.5 g, 9.1 mmol) was added and stirred overnight. More aldehyde (1.5 g, 9.1 mmol) was added and after 1 hour more sodium triacetoxyborohydride (2.5 g, 11.8 mmol) was added. After a further 2 hours the mixture was added slowly to a vigorously-stirred aqueous solution of sodium bicarbonate (250 ml). The phases were separated and the aqueous phase further extracted with with 15% methanol in dichloromethane (2 x 150 ml). The organic extracts were combined , evaporated, and chromatographed on silica gel, eluting with a gradient of 0-25% methanol in dichloromethane, affording the title compound (free base) as a yellow solid (18.1 g, 73%).
δH (d6-DMSO, 250MHz) 1.20-1.35 (2H, m), 1.75-1.85 (2H, m), 1.92 (1H, t), 2.10 (1H, t), 2.30-2.40 (1H, m), 2.52 (1H, m, partly obscured by solvent peak), 2.65 (1H, m), 2.75 (1H, m), 2.98 (1H, m), 3.85 (2H, s), 4.05 (1H, m), 4.20(1H, dd), 4.35 (1H, dd), 4.40 (2H, m), 4.48 (2H, in) 6.50 (1H, d), 7.00 (1H, t), 7.60 (1H, dd), 7.9 (1H, d).
MS (+ve ion electrospray) m/z 452 (MH+).

### (g) 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,-ij]quinolin-4-one Enantiomer E1 and E2 (Method B)

A solution of 1-[(4-amino-1-piperidinyl)methyl]-9-fluoro-1,2-dihydro-4H-pyrrolo[3,2,1-ij]quinolin-4-one (racemic free base with some TFA salt) (3.5 g, 11.63 mmol assumed all free base) and 6,7-dihydro[1,4]dioxino[2,3-c]pyridazine-3-carbaldehyde (1.93 g, 11.63 mmol) in DMF (50 ml) was cooled in an ice bath and treated with sodium triacetoxyborohydride (6.1 g, 29 mmol). The cooling bath was removed and the mixture was stirred at room temperature overnight. The mixture was treated with aqueous sodium bicarbonate (~ 20 ml) and water (200 ml), cooled to 0°C, and the solid was collected by filtration, and dried *in vacuo,* to give the free base of the racemate as a solid (3.6 g; 69%).

This was separated by preparative chiral hplc into the two enantiomers, E1 and E2, using a 20 um Chiralpak AD column, eluting with 80:20:0.1-CH₃CN:CH₃OH:Isopropylamine with Rt E1 10.2 min and Rt E2 12.4 min.
The recovery of E1, was 1.3 g (98 % ee), and E2 was 1.3 g (96 % ee).

### (h) Title compound

1-({14-[(6,7-Dihydro[1,4]dioxino[2,3-*c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 free base (~100 mg) was converted to the dihydrochloride salt (132 mg) in a similar manner to Example 1.

### Example 10B 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Monohydrochloride

### Method A

1-({4-[(6,7-Dihydro[1,4]dioxino[2,3*-c*]pyridazin-3-ylmethyl)amino]-1-piperidinyl}methyl)-9-fluoro-1,2-dihydro-4*H*-pyrrolo[3,2,1-*ij*]quinolin-4-one enantiomer E1, free base, (55.6 g; 0.123 mol) was slurried in absolute ethanol (700 ml), and heated to reflux to give a complete solution, then cooled to 67°C, and aqueous 6.0 N hydrochloric acid (20.5 ml; 0.123 mol) was added, in one portion. The solution was maintained for about 2 minutes, then crystallization began. The suspension was stirred at ambient temperature for 0.5 hours, then cooled to 3 °C and stirred for 2 hours. The solid was filtered off, washed with cold ethanol (100 ml), and dried at 50 °C, for 18 hours under high vacuum to give 57.2 g. 1M HCl in dioxane may be used in place of aqueous HCl.

### Method B

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one enantiomer E1, free base (3.9 g; 8.64 mmoles) was slurried for 30 minutes at 25°C in acetone (66 ml). To this suspension was added a few seed crystals of title compound, followed by a solution of 1.0 M HCl in dioxane (8.64 ml; 1.0 equiv.) Stirring was continued for 18 hours. The suspension was filtered and the filter cake washed with cold acetone. The resulting off white solid was dried at 0.5 mm, 5.0 °C, 4 hours to give 4.07 g (96%) title compound.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Monohydrochloride: melting onset 249°C.(DSC Method A)

### Example 10C 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Mono 4-methylbenzene sulphonate salt

(a) A slurry of crystalline 1-({4-[(6,7-dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one enantiomer E1, free base (244 mg, 0.54 mmol) and acetonitrile (4 mL) was stirred for 30 min and then treated with a solution of toluenesulphonic acid in THF (1M, 0.54 mL, 0.54 mmol) and kept overnight. The solid was filtered to yield 317 mg of the title compound. The solid may be washed with acetonitrile and dried at 50°C under a slow stream of nitrogen affording the title compound as a white solid.

(b) 1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one enantiomer E1, free base (1.0 g; 2.21 mmoles) was slurried for 30 minutes at 25°C in acetonitrile (15 ml). To this suspension was added a few seed crystals of title compound, followed by a solution of 1.0 M p-toluenesulfonic acid in THF (2.21 ml; 1.0 equiv.) Stirring was continued for 18 hours. The suspension was filtered and the filter cake washed with cold acetonitrile. The resulting white solid was dried at 0.5 mm, 50°C, for 4 hours. to give 1.14 g (83 %) title compound. Acetone may be used in place of acetonitrile and THF to give a similar yield of product.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono 4-methylbenzene sulphonate salt: melting onset 245°C. (DSC Method A)

### Example 10D 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Mono benzoate salt

Acetonitrile (10mL) was added to crystalline 1-({4-[(6,7-dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one enantiomer E1, free base 1 (458.0mg). To the slurry, benzoic acid (1.0 equivalent, 1.0 M solution in tetrahydrofuran) was added. The slurry was stirred for 24 hours at room temperature. The solids were then filtered and dried in a vacuum oven at 50°C overnight to give about 512.5mg title compound.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono benzoate salt: melting onset 154°C. (DSC Method A)

### Example 10E 1-({4-[(6,7-Dihydro(1,4(dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Mono (2E)-2-butenedioate salt

### (a) Anhydrate I

Ethanol (60 mL) was added to crystalline 1-({4-[(6,7-dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one enantiomer E1, free base (2.052 g). The slurry was heated to 50°C for 60 minutes, during which the crystalline free-base was dissolved completely in the solvent. To the solution, fumaric acid (1.0 equivalent, 527.5 mg) was added. The solution was again heated to 50°C for 10 hours and cooled back to room temperature. The solid was filtered, washed with ethanol and dried in a vacuum oven at 50°C with a slow nitrogen bleed. The yield of the crystalline fumarate salt anhydrate I was 94% (2.4275 g).

The fumaric acid may be dissolved in DMSO before addition, and if seed crystals are added to the solution of free base, these are preferably added immediately before the addition of fumaric acid.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono (2E)-2-butenedioate salt anhydrate I: melting onset 227°C, melting peak 228°C (DSC Method A).
XRPD peaks (values given in degrees two-theta): 7.9 ± 0.2 (2θ), 8.9 ± 0.2 (2θ), 9.5 ± 0.2 (2θ), 10.5 ± 0.2 (2θ), 11.7 ± 0.2 (2θ), 17.5 ± 0.2 (2θ), 17.8 ± 0.2 (2θ). (XRPD Method A).

### (b) Trihydrate

(i) Charged 2.224 g 1-({4-[(6,7-dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one enantiomer E1 mono (2E)-2-butenedioate salt to a 50 mL reactor. Charged 9 volumes of acetone and 3 volumes of water to the reactor. Heated slurry to 60°C. Charged an additional 4 volumes of acetone and 3.6 volumes of water. Dissolution was observed at 60°C. Cooled to 45°C. Held at 45°C for 1 hour. Cooled to 0°C. Isolated solid at 0°C. Rinsed solid with acetone. Dried under vacuum at 30°C.

(ii) Charged 65 g 1-({4-[(6,7-dihydro[1,4]dioxino[2,3*-c*]pyridazin-3-ylmethyl)amino]-1-piperidinyl}methyl)-9-fluoro-1,2-dihydro-4*H-*pyrrolo[3,2,1-ii]quinolin-4-one Enantiomer E1 Mono (2E)-2-butenedioate salt to a 1 L reactor. Charged 4 volumes of acetone and 4 volumes of water to the reactor. Heated solution to 55°C. Cooled to 50°C and seeded with 1% (wt/wt) of trihydrate seed. Held at 50°C for 1 hour. From 50°C, cooled to 40°C over 100 minutes. From 40°C, cooled to 25°C over 60 minutes. From 25°C, cooled to 0°C over 30 minutes. Charged 6 volumes of acetone to slurry, during which the slurry warmed to 2°C. Isolated solid at 2°C. Rinsed solid with 5 volumes of acetone. Dried under vacuum 35°C overnight. Final yield of 58 g of trihydrate.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E 1 Mono (2E)-2-butenedioate salt trihydrate: A broad melt/dehydration endotherm between 50-150 °C , followed by a sharp melting onset 222°C (DSC Method A).
XRPD peaks (values given in degrees two-theta): 5.7 ± 0.2 (2θ), 6.7 ± 0.2 (2θ), 8.1 ± 0.2 (2θ), 9.3 ± 0.2 (2θ), 9.9 ± 0.2 (2θ), 11.0 ± 0.2 (2θ), 11.5 ± 0.2 (2θ). (XRPD Method B).

### (c) Anhydrate II

1-({4-[(6,7-Dihydro[1,4]dioxino[2,3*-c*]pyridazin-3-yl)methyl)amino]-1-piperidinyl}methyl)-9-fluoro-1,2-dihydro-4*H-*pyrrolo[3,2,1-*ij*]quinolin-4-one Enantiomer E 1 Mono (2E)-2-butenedioate salt trihydrate was dried in a vacuum oven at 80°C to give title anhydrate 11.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono (2E)-2-butenedioate salt anhydrate II: melting onset 225°C, melting peak 227°C ΔH_{f} 137J/g. (DSC Method B).
XRPD peaks (values given in degrees two-theta): 6.1 ± 0.2 (2θ), 10.5 ± 0.2 (2θ), 10.9 ± 0.2 (2θ), 16.0 ± 0.2 (2θ), 18.3 ± 0.2 (2θ), 21.0 ± 0.2 (2θ). (XRPD Method C).

### (d) Dihydrate

Methanol:5 vol% Water (0.5 mL) was added to crystalline 1-({4-[(6,7-dihydro[1,4)dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1, free base (~30 mg). The resulting slurry was, under a vortex speed of 750rpm, held at 40°C for I h then was temperature-cycled from 0-40°C for ~48 hours (ramp at -1°C/min to 0°C, hold for 1h, +1°C/min to 40°C, hold for I h). Finally the product was ramped at -1°C/min to 23°C and held for I h at a vortex speed of 500rpm. The resulting solids and supernatant were separated by filtration at room temperature and put in a refrigerator and allowed to cool to ~4°C overnight. The supernatant was allowed to warm to room temperature and to evaporate slowly under ambient laboratory conditions thereby producing the dihydrate solid.

1-({4-[(6,7-Dihydro[1,4]dioxino[2,3*-c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono (2E)-2-butenedioate salt dihydrate: a broad melt/dehydration endotherm between 20~120 °C , followed by a sharp melting onset 173 °C (DSC Method A).
XRPD peaks (values given in degrees two-theta): 6.1 ± 0.2 (2θ), 6.9 ± 0.2 (2θ), 7.9 ± 0.2 (2θ), 10.6 ± 0.2 (2θ), 12.2 ± 0.2 (2θ), 12.9 ± 0.2 (2θ). (XRPD Method A).

### Example 11 1-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E2 Dihydrochloride

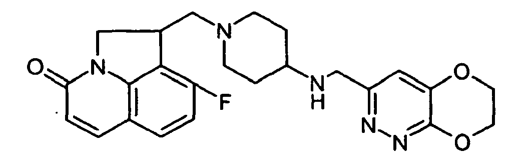

A solution of 1-[(4-amino-1-piperidinyl)methyl]-9-fluoro-1,2-dihydro-4H-pyrrolo[3,2,1-ij]quinolin-4-one (enantiomer E2) (100 mg, 0.33 mmol) and 6,7-dihydro[1,4]dioxino[2,3-c]pyridazine-3-carbaldehyde (56 mg, 0.332 mmol) in N,N-dimethylformamide (1.5 ml) was treated with sodium triacetoxyborohydride (216 mg, 0.997 mmol) for 16 hours at room temperature. The mixture was evaporated to dryness, treated with aqueous sodium bicarbonate, extracted with 10% methanol in dichloromethane and the combined organic extracts were dried (magnesium sulphate) and evaporated. The residue was chromatographed on silica gel, eluting with ethyl acetate followed by 0-30% methanol in dichloromethane, affording the free base as a white solid (101 mg, 66%).
MS (+ve ion electrospray) m/z 452 (MH+).

The free base in methanol, was converted to the dihydrochloride salt by adding an excess of 1 M hydrogen chloride in methanol, followed by evaporation to dryness, to give a solid (114 mg).

### Example 12 9-Fluoro-1-[(4-{[(3-oxo-3,4-dihydro-2H-pyrido[3,2-b][1,4]oxazin-6-yl)methyl]amino}-1-piperidinyl)methyl]-1,2-dihydro-4H-pyrrolo[3,2,1-ij]quinolin-4-one Enantiomer E1 Dihydrochloride

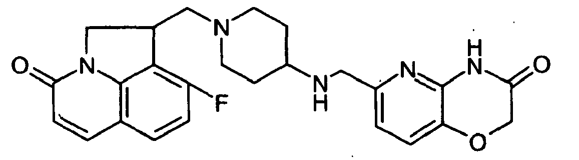

A solution of 1-[(4-amino-1-piperidinyl)methyl]-9-fluoro-1,2-dihydro-4H-pyrrolo[3,2,1-ij]quinolin-4-one (enantiomer E1) (50 mg; 0.17 mmol) and 3-oxo-3,4-dihydro-2*H-*pyrido[3,2*-b*][1,4]oxazine-6-carboxaldehyde (for a synthesis, see WO2004058144 Example 1(1)) (30 mg; 0.17 mmol) in dichloromethane/methanol (5 ml/0.5 ml) was treated with sodium triacetoxyborohydride (212 mg, 1.0 mmol) and stirred at room temperature. After 1 hour an aqueous solution of sodium bicarbonate (10 ml) was added and the mixture was evaporated to small volume. The resulting solid was collected and washed with water to give the free base (60 mg; 79%).
MS (+ve ion electrospray) m/z 464 (MH+).
δH (CDCl₃/CD₃OD, 250MHz) 1.52 (2H, m), 1.95 (2H, m), 2.10 (1H, t), 2.25 (1H, t), 2.48-2.71 (2H, m), 2.90 (2H, m), 3.10 (m, partly obscured by water peak), 3.85 (2H, s), 4.05 (1H, m), 4.48 (2H, m), 4.62 (2H, s), 6.63 (1H, d), 6.90 (2H, m), 7.22 (1H, d), 7.42 (1H, m), 7.75 (1H, d).

The free base in methanol/DCM, was converted to the dihydrochloride salt by adding an excess of 4N hydrogen chloride in dioxan, followed by evaporation to dryness, to give a solid (60 mg).

### Example 13 1-({4-[(2,3-dihydro[1,4]dioxino[2,3-c]pyridin-7-ylmethyl)amino]-1-piperidinyl}methyl)-4-oxo-1,2-dihydro-4H-pyrrolo[3,2,1-ij]quinoline-9-carbonitrile Enantiomer E1 Dihydrochloride

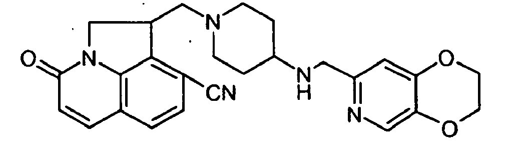

### (a) (2E)-N-(3-Bromo-2-methylphenyl)-3-phenyl-2-propenamide

A solution of cinnamyl chloride (89.6 g, 536 mmol) in ethyl acetate (400 ml) was added to a vigorously-stirred mixture of 3-bromo-2-methylaniline (99.8 g, 536 mmol), saturated aqueous sodium bicarbonate (850 ml), ice (ca 100 g) and ethyl acetate (400 ml). After I hour the mixture was concentrated (removing most of the ethyl acetate) and filtered. The residue was re-suspended in a 5% solution of methanol in water (500mL), stirred for 1 hour, filtered and dried in vacuo (170 g, 100%).MS (+ve ion electrospray) m/z 317 (MH+).

### (b) 7-Bromo-8-methyl-2(1H)-quinolinone

A suspension of (2E)*-N*-(3-bromo-2-methylphenyl)-3-phenyl-2-propenamide (50 g, 160 mmol) in chlorobenzene (206 ml) was treated slowly with aluminium trichloride 128 g, 960 mmol). The reaction was heated to 125°C for 0.5 hour, under argon. The mixture was allowed to cool to room temperature, then added to ice in water (ca. 2L). The mixture was filtered and the resulting solid was washed with water and dried *in vacuo* to afford an off white solid (59.2g, quant.).
MS (+ve ion electrospray) m/z 239 (MH+).

### (c) 7-Bromo-8-methyl-2-(methyloxy)quinoline

Crude 7-bromo-8-methyl-2(1H)-quinolinone (25 g, 105 mmol) was suspended in DMSO (138 ml), then treated with potassium t-butoxide (11.8 g, 115 mmol), under argon (the internal temperature was stable at 30°C). After 10 minutes methyl iodide (8.5 ml, 136 mmol) was added (the internal temperature rose to 40°C and settled at 35 °C after 10 minutes). The reaction mixture was stirred at 35 °C for 30 minutes The mixture was added to water (1 L) and extracted twice with hexane (2x300 ml). The hexane extracts were further washed with brine (300 ml), dried over magnesium sulphate, filtered and evaporated under vacuum to afford a pale yellow solid (19.3 g, 73%).
MS (+ve ion electrospray) m/z 253 (MH+).

### (d) 7-Bromo-8-bromomethyl-2-(methyloxy)quinoline

A solution of 7-bromo-8-methyl-2-(methyloxy)quinoline (19.3 g, 76.6 mmol) in trifluoromethylbenzene (292 ml) was treated with N-bromosuccinimide (27.3 g, 153.2 mmol) and benzoyl peroxide (117 mg) and heated at reflux while irradiating with a 100 Watt tungsten lamp for 2 hours. The cooled mixture was washed with saturated aqueous sodium bicarbonate solution and water then dried over magnesium sulphate and evaporated under vacuum. The residue was chromatographed on silica eluting with a 0-100% gradient of dichloromethane in petroleum ether affording a white solid (23.2 g, 91%).
MS (+ve ion electrospray) m/z 332 (MH+).

### (e) 7-Bromo-2-(methyloxy)-8-quinolinyl acetonitrile

A solution of 7-bromo-8-bromomethyl-2-(methyloxy)quinoline (19.3 g, 58.3 mmol) in DMF (345 ml) was treated with potassium cyanide (15.2 g, 233 mmol) and stirred at 25°C overnight. The mixture was evaporated to dryness and the dark residue partitioned between dichloromethane and water. The aqueous layer was extracted twice more with dichloromethane. The combined organic extracts were dried over magnesium sulphate filtered and evaporated under vacuum. The residue was chromatographed on silica eluting with a 0-100% dichloromethane in petroleum ether gradient affording a white solid (12.8 g, 79%).
MS (+ve ion electrospray) m/z 277 (MH+).

### (f) Methyl [7-bromo-2-(methyloxy)-8-quinolinyl]acetate

A solution of 7-bromo-2-(methyloxy)-8-quinolinyl acetonitrile (12.8 g, 46.2 mmol) in dry methanol (200 ml) was treated with trimethylsilyl chloride (20 ml; 157.1 mmol) and heated at 60°C for 3 hours. Methanol was partially evaporated under vacuum. Water (60 ml) was added then solid potassium carbonate (13 g). The aqueous layer was extracted twice with dichloromethane. The combined organic layers were dried over magnesium sulphate, filtered and evaporated under vacuum. The residue was chromatographed on silica eluting with dichloromethane affording a white solid (13.1 g, 91%).
MS (+ve ion electrospray) m/z 311 (MH+).

### (g) Methyl 2-[7-bromo-2-(methyloxy)-8-quinolinyl]-2-propenoate

A mixture of methyl [7-bromo-2-(mcthyloxy)-8-quinolinyl]acetate (13.1 g; 42.2 mmol), paraformaldehyde (8.8 g; 295 mmol), potassium carbonate (5.8 g; 63 mmol) and benzyltriethyl ammonium chloride (15.4 g; 67.6 mmol) in cyclohexane (275 ml) was heated at 85°C , with vigorous stirring for 18 hours. More paraformaldehyde (8.8 g; 295 mmol), potassium carbonate (2.9 g; 29.5 mmol) and benzyltriethyl ammonium chloride (7.7 g; 33.8 mmol) were added and the reaction mixture was stirred at 85°C for a further 5 hours and then at 90°C for 18 hours. The mixture was cooled, water (200 ml) added and the mixture was extracted with ethyl acetate (2x 200 ml). The combined organic layers were washed with brine (150 ml), dried over magnesium sulphate and evaporated under vacuum affording a white solid (12.4 g, 91%).
MS (+ve ion electrospray) m/z 323 (MH+).

### (h) Methyl 2-[7-bromo-2-(methyloxy)-8-quinolinyl]-3-[4-((2,3-dihydro[1,4]dioxino[2,3-c]pyridin-7-ylmethyl){[(1,1-dimethylethyl)oxy]carbonyl}amino)-1-piperidinyl]propanoate

A solution of methyl 2-[7-bromo-2-(methy)oxy)-8-quinolinyl]-2-propenoate(1 g, 3.1 mmol), 1,1-dimethylethyl (2,3-dihydro[1,4]dioxino[2,3*-c*]pyridin-7-ylmethyl)-4-piperidinylcarbamate (for a synthesis, see WO 2004058144 Example 99(h)) (2.2 g; 6.2 mmol) and 1,1,3,3-tetramethylguanidine (15 drops) in dry DMF (9.5 mL) was heated at 90°C for 24 hours then at 100°C for a further 23 hours. The mixture was evaporated to dryness, and chromatographed on silica gel, eluting with 0-20% methanol in dichloromethane affording a yellow oil (1.7 g; 81%).
MS (+ve ion electrospray) m/z 672 (MH+).

### (i) 1,1-Dimethylethyl (1-{2-[7-bromo-2-(methyloxy)-8-quinolinyl]-3-hydroxypropyl}-4-piperidinyl)(2,3-dihydro[1,4]dioxino[2,3-c]pyridin-7-ylmethyl)carbamate

A solution of methyl 2-[7-bromo-2-(methyloxy)-8-quinolinyl]-3-[4-((2,3-dihydro[1,4]dioxino[2,3*-c*]pyridin-7-ylmethyl){[(1,1-dimethylethyl)oxy]carbonyl}amino) -1-piperidinyl]propanoate(1.7 g, 2.5 mmol) in dry tetrahydrofuran (18.5 ml) at -78 °C was treated with a solution of lithium aluminium hydride in tetrahydrofuran (1M, 3 ml, 3 mmol) and stirred at this temperature for 1 hour, then allowed to reach room temperature over 30 minutes The reaction mixture was cooled again to -78 °C and a solution of lithium aluminium hydride in tetrahydrofuran (1M, 3 ml, 3 mmol) was added. The reaction mixture was allowed to reach room temperature and stirred for 1 hour. The reaction mixture was quenched with saturated sodium bicarbonate and filtered. The filtrate was evaporated under vacuum. The residue was redissolved in dichloromethane and chromatographed on silica gel, eluting with a 1-40% methanol in dichloromethane gradient, affording a yellow solid (835 mg, 51%).
MS (+vc ion electrospray) m/z 644 (MH+).

### (j) 1,1-Dimethylethyl{1-[(9-bromo-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate

A solution of 1,1-dimethylethyl(1-{2-[7-bromo-2-(methyloxy)-8-quinolinyl]-3-hydroxypropyl}-4-piperidinyl)(2,3-dihydro[1,4]dioxino[2,3*-c*]pyridin-7-ylmethyl)carbamate (835 mg, 1.29 mmol) in chloroform (17 ml), at 0°C, under argon, was treated with diisopropylethylamine (0.48 ml, 2.85 mmol) and methanesulphonic anhydride (271 mg, 1.55 mmol) in chloroform (3 ml). The mixture was heated at 60°C for 1 hour. The reaction mixture was concentrated to - 10 ml, and chromatographed on silica gel, eluting with a 0-30% methanol in ethyl acetate gradient, affording a colorless oil (641 mg, 58%).
MS (+ve ion electrospray) m/z 612 (MH+).

### (k) 1,1-dimethylethyl {1-[(9-cyano-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate

A mixture of 1,1-dimethylethyl {1-[(9-bromo-4-oxo-1,2-d ihydro-4*H-*pyrrolo[3,2,1-*ij*]quinolin-1-yl)methyl]-4-piperidinyl}(2,3-dihydro[1,4]dioxino[2,3-c]pyridin-7-ylmethyl)carbamate (461 mg, 0.75 mmol) and copper(I) cyanide (16 mg, 1.88 mmol) in N,N-dimethylformamide (4.2 ml) was heated at 140°C for 2 hours. The reaction mixture was cooled to room temperature and partitioned between dichloromethane/concentrated ammonia/brine. The aqueous layer was extracted twice with dichloromethane. The combined organic layers were dried over magnesium sulphate and evaporated under vacuum. The brown oil residue was chromatographed on silica gel, eluting with a 0-30% methanol in dichloromethane gradient, affording a yellow oil (203 mg, 48%).
MS (+ve ion electrospray) m/z 558 (MH+).

### (I) Title compound

A solution of 1,1-dimethylethyl {-[(9-cyano-4-oxo-1,2-dihydro-4*H-*pyrrolo[3,2,1-*ij*]quinolin-1-yl)methyl]-4-piperidinyl}(2,3-dihydro[1,4]dioxino[2,3-c]pyridin-7-ylmethyl)carbamate (203 mg, 0.36 mmol) in dichloromethane (9 ml) was treated with trifluoroacetic acid (9 ml). The reaction mixture was stirred at room temperature for 1 hour and evaporated to dryness. The residue was dissolved in a 1:1 mixture of methanol:dichloromethane (20 ml) and treated with MP-carbonate resin (3 mmol/g). After 30 minutes, the mixture was filtered under vacuum. The filtrate was evaporated to dryness affording the free base as a colorless oil (141 mg, 85%).
δH (CDCl₃, 250MHz) 1.40-1.55 (2H, m), 1.80-1.95 (2H, m), 2.1.-2.4 (2H, m), 2.5 (2H, dt), 2.8 (1H, m), 2.9-3.1 (2H, m), 3.79 (2H, s), 4.00-4.08 (1H, m), 4.25-4.35 (4H, m), 4.43-4.60 (2H, m), 6.84 (1H, d), 7.34-7.36 (2H, d), 7.47-7.51 (1H, d), 7.71-7.74 (1H, d), 8.10 (1H, s).
MS (+ve ion electrospray) m/z 458(MH+).

Racemic material (as free base; 200 mg) was separated by preparative chiral hplc into the two enantiomers, E1 and E2, using a 5um Chiralpak AD-H column, eluting with 50:50:0.1- CH₃CN:CH₃OH:Isopropylamine with Rt E1 7min and Rt E2 13.8 min. The recovery was E1 80 mg (>99.5% pure) and E2 86 mg (>99.4 % pure)

The E1 enantiomer was converted into the dihydrochloride salt by dissolving the free base in a small amount of methanol and excess of a 6N solution of hydrochloric acid. The solution was then evaporated under vacuum to give a solid.

### Example 14A 1-({4-[(2,3-Dihydro[1,4]dioxino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E2 Dihydrochloride

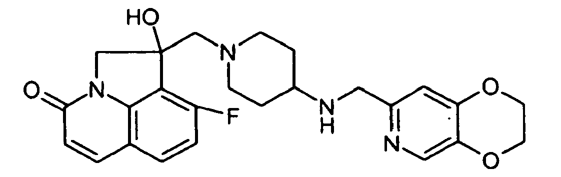

### (a) Methyl 2-[7-fluoro-2-(methyloxy)-8-quinolinyl]-2-oxiranecarboxylate

m-Chloroperbenzoic acid (50%; 6.95 g; 0.0201 mol) was added to a solution of a 1:1 mixture (5.251 g) of methyl. 2-[7-fluoro-2-(methyloxy)-8-quinolinyl]-2-propenoate (2.63 g; 0.0101 mol) and methyl [7-fluoro-2-(methyloxy)-8-quinolinyl]acetate in dichloromethane (60 ml) and the mixture was heated at 50°C for 6.5 hours and then 40°C until 16 hours. [Further m-chloroperbenzoic acid (3.5 g) was added at 2 hours]. The mixture was cooled, diluted with water and DCM and treated with excess sodium sulfite, followed by aqueous sodium bicarbonate to pH ∼8, and then extracted (3x more) with dichloromethane. The organic fraction was dried, evaporated and chromatographed on silica gel, eluting with 0-100% ethyl acetate-petroleum ether then 0-20% methanol-ethyl acetate to afford the product (2.614 g; 94% based on methyl 2-[7-fluoro-2-(methyloxy)-8-quinolinyl]-2-propenoate starting material).

### (b) 9-Fluoro-1-hydroxy-4-oxo-1,2-dihydro-4H-pyrrolo[3,2,1-ij]quinoline-1-carboxylic acid

A mixture of methyl 2-[7-fluoro-2-(methyloxy)-8-quinolinyl]-2-oxiranecarboxylate (3.105 g; 0.012 mol), and lithium perchlorate (2.38 g; 0.0224 mol) in acetonitrile (30 ml) and water (30 ml) was heated at 85°C for 120 hours, cooled, and evaporated to dryness. 10% Methanol in dichloromethane was added and the resulting solid was collected and dried to give (1.4 g; 51%).
MS (+ve ion electrospray) m/z 249 (MH+).

### (c) Methyl 9-fluoro-1-hydroxy-4-oxo-1,2-dihydro-4H-pyrrolo[3,2,1-ij]quinoline-1-carboxylate

A solution of 9-fluoro-1-hydroxy-4-oxo-1,2-dihydro-4*H*-pyrrolo[3,2,1-*ij*]quinoline-1-carboxylic acid (1.30.g) in methanol (52 ml) was treated with conc. sulphuric acid (0.52 ml) and stirred at room temperature for 1.5 hour. The solution was quenched by stirring with excess MP-carbonate resin until pH ∼7, filtered and evaporated to give a yellow solid (0.855 g; 62%).
MS (+ve ion electrospray) m/z 264 (MH+).

### (d) 9-Fluoro-1-hydroxy-1-(hydroxymethyl)-1,2-dihydro-4H-pyrrolo[3,2,1-ij]quinolin-4-one

A solution of methyl 9-fluoro-1-hydroxy-4-oxo-1,2-dihydro-4*H*-pyrrolo[3,2,1-*ij*]quinoline-1-carboxylate (0.855 g; 3.25 mmol) in methanol (85 ml) was cooled to 0°C and sodium borohydride (0.123 g; 3.25 mmol) added. The mixture was stirred at this temperature for 2 hours. It was quenched with ammonium chloride (5 ml), evaporated to dryness and the residue treated with methanol and then re-evaporated to dryness. Water and dichloromethane were added and the aqueous fraction was evaporated to dryness and again treated with methanol. The resulting solid was filtered off and dried, (0.765 g), sufficiently pure for the next reaction.
MS (+ve ion electrospray) m/z 236 (MH+).

### (e) 9-Fluoro-1-(hydroxymethyl)4-oxo-1,2-dihydro-4H-pyrrolo[3,2,1-ij]quinolin-1-yl 4-methylbenzenesulfonate

A mixture of 9-fluoro-1-hydroxy-1-(hydroxymethyl)-1,2-dihydro-4*H-*pyrrolo[3,2,1-*ij*]quinolin-4-one (0.765 g; 3.25 mmol), p-toluenesulfonyl chloride (0.62 g, 3.25 mmol) and di-n-butyl(oxo)stannane (40.5 mg; 0.1626 mmol) in dichloromethane (30 ml), tetrahydrofuran (30 ml), DMF (3 ml) and triethylamine (0.68 ml) were stirred at room temperature for 16 hours, then sodium bicarbonate solution was added and the mixture was extracted with 10% methanol-dichloromethane. The organic fraction was dried and evaporated to give a yellow oil that was chromatographed on silica gel, eluting with 0-100% ethyl acetate-petroleum ether followed by 0-20% methanol-ethyl acetate to give a yellow oil (0.968 g) (77% yield over 2 steps).
MS (+ve ion electrospray) m/z 390 (MH+).

### (f) 1,1-Dimethylethyl {1-[(9-fluoro-1-hydroxy-4-oxo-1,2-dihydro-4H-pyrrolo[3,2,1 - ij]quinolin-1-yl)methyl]-4-piperidinyl}carbamate

A mixture of 9-fluoro-1-(hydroxymethyl)-4-oxo-1,2-dihydro-4*H*-pyrrolo[3,2,1-*ij*]quinolin-1-yl 4-methylbenzenesulfonate (0.968 g; 2.49 mmol), 1,1-dimethylethyl 4-piperidinylcarbamate (0.47 g; 2.35 mmol), and anhydrous sodium carbonate (0.746 g; 7.04 mmol), in ethanol (100 ml), was stirred at room temperature for 16 hours. Water was added and the mixture was extracted with 10% methanol-dichloromethane. The organic extracts were dried and evaporated to give a yellow oil (1.038 g; 100%).
MS (+ve ion electrospray) m/z 418 (MH+).

### (g) 1-[(4-Amino-1-piperidinyl)methyl]-9-fluoro-1-hydroxy-1,2-dihydro-4H-pyrrolo[3,2,1-ij]quinolin-4-one

A solution of 1,1-dimethylethyl {1-[(9-fluoro-1-hydroxy-4-oxo-1,2-dihydro-4*H-*pyrrolo[3,2,1-*ij*]quinolin-1-yl)methyl]-4-piperidinyl}carbamate (1.038 g) in dichloromethane (5 ml) and trifluoroacetic acid (2.5 ml) was stirred at room temperature for 2 hours, during which a further 2 ml trifluoroacetic acid was added, and evaporated to dryness. The residue was dissolved in 1:1 dichloromethane/methanol and stirred with excess MP-carbonate resin until pH -8, filtered and evaporated to give a yellow oil (0.638 g; 81%).
MS (+ve ion electrospray) m/z 318 (MH+).

Racemic material (0.90 g) was separated by preparative chiral hplc into the two enantiomers, E1 and E2, using a Chiralpak AD 10um (21 x 250 mm) column, eluting with 80:20:0.1- CH₃CN:CH₃OH:Isopropylamine (20 ml/min) with Rt E1 5.5 min and Rt E2 7.0 min.

The recovery was E1 379 mg (>99% ee) and E2 395 mg (>99 % ee).

### (h) Title compound

A solution of 1-[(4-amino-1-piperidinyl)methyl]-9-fluoro-1-hydroxy-1,2-dihydro-4*H*-pyrrolo[3,2,1-*ij*]quinolin-one enantiomer E2 (40 mg, 0.13 mmol) and 2,3-dihydro[1,4]dioxino[2,3*-c*]pyridine-7-carboxaldehyde (for a synthesis see WO2004058144, Example 2(c)) (20 mg, 0.13 mmol) in DCM (0.5 ml) and methanol (0.10 ml) was stirred at room temperature with sodium triacetoxyborohydride (85 mg, 0.40 mmol) for 4 hours at room temperature. The mixture was treated with aqueous sodium bicarbonate (2 ml) and extracted with 5% methanol in dichloromethane (2 ml) and the organic phase was chromatographed on silica gel, eluting with 0-20% methanol in dichloromethane, affording the free base as a yellow oil (30 mg, 49%).
MS (+ve ion electrospray) m/z 467 (MH+).
δH(CD₃OD, 400MHz) 1.13-1.25 (1H, m), 1.40-1.50 (1H, m), 1.70-1.78 (1H, m), 1.85-1.93 (1H, m), 2.18-2.25 (1H, t), 2.28-2.35 (1H, t), 2.50-2.58 (1H, m), 2.65-2.70 (1H, m), 3.00 (2H, s), 3.10-3.18 (1H, m), 3.80 (2H, s), 4.20 (1H, d), 4.25-4.30 (2H, m), 4.32-4.38 (2H, m), 4.65 (1H, d), 6.62 (1H, d), 6.95 (1H, s), 7.05 (1H, t), 7.68-7.72 (1H, m), 7.95 (1H, d), 8.00 (1H, s).

The free base in methanol-DCM (0.5ml/0.5ml), was converted to the dihydrochloride salt by adding an excess of 1M hydrogen chloride in ether (2ml), followed by more ether (3ml), to precipitate a solid (34 mg).

### Example 14B 1-({4-[(2,3-Dihydro[1,4]dioxino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Dihydrochloride

The title compound was prepared from 9-fluoro-1-(hydroxymethyl)-4-oxo-1,2-dihydro-4*H*-pyrrolo[3,2,1-*ij*]quinolin-1-yl 4-methylbenzenesulfonate and 1,1-dimethylethyl (2,3-dihydro[1,4]dioxino[2,3-*c*]pyridin-7-ylmethyl)-4-piperidinylcarbamate (for a synthesis, see WO 2004058144 Example 99(h)) in a similar manner to procedures generally described herein.

### Example 14C 1-({4-[(2,3-Dihydro[1,4]dioxino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E2 Hydrochloride

The title compound was prepared from 9-fluoro-1-(hydroxymethyl)-4-oxo-1,2-dihydro-4*H*-pyrrolo[3,2,1-*ij*]quinolin-1-yl 4-methylbenzenesulfonate and 1,1-dimethylethyl (2,3-dihydro[1,4]dioxino[2,3-*c*]pyridin-7-ylmethyl)-4-piperidinylcarbamate (for a synthesis, see WO 2004058144 Example 99(h)) followed by separation of the enantiomer E2 and preparation of the hydrochloride salt, in a similar manner to procedures generally described herein.

### Example 15 1-({4-[(2,3-Dihydro[1,4]oxathiino[2,3-c]pyridin-7-ylmethyl)amino]piperidin-1-yl}methyl)-9-fluoro-1-hydroxy-1,2-dihydro-4H-pyrrolo[3,2,1-ij]quinolin-4-one Enantiomer E1 Dihydrochloride

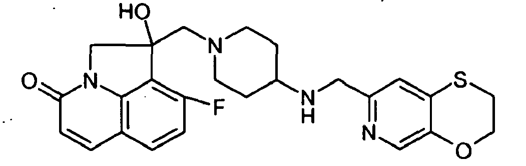

A solution of 1-[(4-amino-1-piperidinyl)methyl]-9-fluoro-1-hydroxy-1,2-dihydro-4*H*-pyrrolo[3,2,1-*ij*]quinolin-4-one enantiomer E1 (39 mg, 0.12 mmol) and 2,3-dihydro[1,4]oxathiino[2,3-*c*]pyridine-7-carbaldehyde (for a synthesis, see WO2004058144 Example 60(i)) (22 mg, 0.12 mmol) in N,N-dimethylformamide (1 ml) was treated with sodium triacetoxyborohydride (79 mg, 0.37 mmol) for 18 hours at 60°C. The mixture was evaporated to dryness, treated with aqueous sodium bicarbonate and extracted with 5% methanol in dichloromethane. The combined organic extracts were dried (magnesium sulphate) and evaporated. The residue was chromatographed, twice, on silica gel, eluting with 0-50% methanol in dichloromethane, affording the free base as a colorless oil (56 mg, 94%).
MS (+ve ion electrospray) m/z 483 (MH+).
δH (CDCl₃, 250MHz) Early signals partly obscured by a water peak, 1.40 -1.65 (4H, m), 1.95 (2H, m), 2.35 (1H, t), 2.55 (2H, m), 2.82 (1H, d), 3.0 (2H, m), 3.15 (2H, m), 3.35 (2H, d), 3.79 (2H, s), 4.40 (4H, m), 6.62 (1H, d), 6.88 (1H, t), 7.00 (1H, s), 7.49 (1H, dd), 7.69 (1H, d), 8.03 (1H, s).

The free base in methanol, was converted to the dihydrochloride salt by adding an excess of I M hydrogen chloride in ether, followed by evaporation to dryness, to give a solid (40 mg).

### Example 16 9-Fluoro-1-hydroxy-1-[(4-{[(3-oxo-3,4-dihydro-2H-pyrido[3,2-b][1,4]oxazin-6-yl)methyl]amino}-1-piperidinyl)methyl]-1,2-dihydro-4H-pyrrolo[3,2,1-ij]quinolin-4-one Dihydrochloride

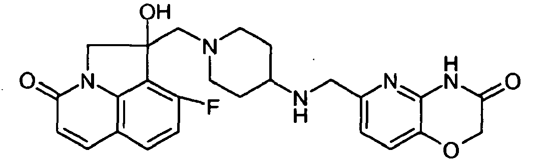

A solution of 1-[(4-amino-1-piperidinyl)methyl]-9-fluoro-1-hydroxy-1,2-dihydro-4*H*-pyrrolo[3,2,1-*ij*]quinolin-4-one (racemic) (44 mg, 0.138 mmol) and 3-oxo-3,4-dihydro-2*H*-pyrido[3,2-*b*][1,4]oxazine-6-carboxaldehyde (for a synthesis, see WO2003087098 Example 31 (e)) (25 mg, 0.138 mmol) in N,N-dimethylformamide (1ml) was treated with sodium triacetoxyborohydride (87 mg, 0.414 mmol) at room temperature for 16 hours. The mixture was evaporated to dryness, treated with aqueous sodium bicarbonate and extracted with 10% methanol in dichloromethane and the combined organic extracts were dried (magnesium sulphate) and evaporated. The residue was chromatographed on silica gel, eluting with 0-30% methanol in dichloromethane, affording the free base as a colorless oil (34 mg, 52%),

MS (+ve ion electrospray) m/z 480 (MH+).
δH (CD₃OD, 250MHz), 1.10-1.30 (1H, m), 1.35-1.55 (1H, m), 1.70-2.00 (4H, m), 2.15-2.40 (2H, m), 2.50-2.75 (2H, m), 3.0 (2H, m), 3.10-3.20 (2H, m), 3.30 (1H, m), 3.81 (2H, s), 4.20 (1H, d), 4.62 (2H, s), 6.61 (1H, d), 6.94 (1H, d), 7.04 (1H, t), 7.25 (1H, d), 7.69 (1H, dd), 7.95 (1H, d).

The free base in methanol, was converted to the dihydrochloride salt by adding an excess of 1M hydrogen chloride in methanol, followed by evaporation to dryness, to give a solid (42 mg).

### Example 17 1-({4-[(6,7-Dihydro[1,4]oxathiino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Dihydrochloride

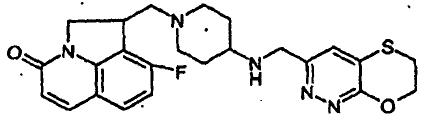

### (a) 2-[(3,6-Dichloro-4-pyridazinyl)thio]ethanol

A solution of 3,4,6-trichloropyridazine (25 g) in tetrahydrofuran (200 ml) and triethylamine (19 ml) was treated at 0°C (ice bath cooling) with 2-mercaptoethanol (8.33 ml) over 5 minutes. After the addition was complete, the mixture was stirred at room temperature for 72 hours: The mixture was stirred with aqueous sodium bicarbonate solution and dichloromethane and the solid was collected, washed with water, ether and pentane and dried *in vacuo,* giving (22.9 g). The combined aqueous and organic fraction was evaporated to half volume giving further solid, which was washed and dried as above (5.0 g). The total yield of solid (27.9 g; 91%) contained some bromo-analogue (5-10%) by NMR.

### (b) 3-Chloro-6,7-dihydro[1,4]dioxathiino[2,3-c]pyridazine

A solution of 2-[(3,6-dichloro-4-pyridazinyl)thio]ethanol(13g) (previously dried at 50°C *in vacuo)* in dry dioxan (250 ml) was treated with lithium hydride (3 g) in portions and heated at 105 - 110 °C for 24 hours. The reaction mixture was cooled and quenched with iced-water. The solution was taken to pH 10 - 11 with 5M hydrochloric acid and evaporated. Water was added and the mixture was extracted 4x with dichloromethane, dried (sodium sulphate), evaporated, and chromatographed on silica gel, eluting with 0-100% ethyl acetate-hexane, to afford a white solid (1.61 g) (containing ca. 10% of the bromo species).
MS (+ve ion electrospray) m/z 189/91 (Cl MH+); 233/5 (Br MH+)
δH (CDCl₃, 400MHz) 3.23 (2H, m), 4.67(2H, m), 7.26 (1H, s) (for major chloro-compound).

### (c) 3-Ethenyl-6,7-dihydro[1,4]oxathiino[2,3-c]pyridazine

A solution of 3-chloro-6,7-dihydro[1,4]oxathiino[2,3*-c*]pyridazine(1.0 g) in dimethoxyethane (25 ml) was degassed under argon then tetrakis(triphenylphosphine)palladium (0) (135 mg), potassium carbonate (0.695 g), 2,4,6-trivinylcyclotriboroxane pyridine complex (0.8 g) and water (3.7 ml) were added. The mixture was heated at 105 °C, overnight. More 2,4,6-trivinylcyclotriboroxane pyridine complex (0.4 g) and tetrakis(triphenylphosphine)palladium (0) (30 mg) were added and heating was continued for 24 hours. The mixture was cooled, treated with aqueous sodium bicarbonate solution, extracted (4x) with DCM, dried (sodium sulphate), evaporated and chromatographed on silica gel (70 g), eluting with 0-100% ethyl acetate - hexane, affording a solid (0.56 g) (87% pure by LC-MS).
MS (+ve ion electrospray) m/z 181 (MH+).

### (d) 6,7-Dihydro[1,4]oxathiino[2,3-c]pyridazine-3-carbaldehyde

A solution of 3-ethenyl-6,7-dihydro[1,4]oxathiino[2,3*-c*]pyridazine(320 mg) in dioxan/water (20 ml/5 ml) was treated with an aqueous solution of osmium tetroxide (4% w/v, 2 ml) and sodium periodate (1.08 g), initially stirred in an ice-bath, then allowed to warm to room temperature. After 2.5 hours the mixture was evaporated to dryness and dissolved in dioxan and chloroform. Silica gel was added and the mixture was evaporated to dryness, added to a silica column (50 g) and chromatographed, eluting with 0-100% ethyl acetate in hexane, to afford a white solid (116 mg, 36%).
MS (+ve ion electrospray) m/z 183 (MH+).

### (e) Title compound

A solution of 1-[(4-amino-1-piperidinyl)methyl]-9-fluoro-1,2-dihydro-4H-pyrrolo[3,2,1-ij]quinolin-4-one (enantiomer E1) (40 mg, 0.133 mmol) and 6,7-dihydro[1,4]oxathiino[2,3*-c*]pyridazine-3-carbaldehyde (24 mg, 0.133 mmol)in dichloromethane/methanol (1 ml/ 0.3 ml) was treated with sodium triacetoxyborohydride (93 mg, 0.44 mmol) at room temperature overnight. More 6,7-dihydro[1,4]oxathiino[2,3-c]pyridazine-3-carbaldehyde (8 mg) was added and the mixture was stirred for 1 hour. More sodium triacetoxyborohydride (93 mg) was added, and the mixture was stirred at room temperature overnight. Aqueous sodium bicarbonate was added and the mixture was extracted with 10% methanol in dichloromethane (4x). The organic extracts were combined , dried (sodium sulphate), evaporated, and chromatographed on silica gel, eluting with a gradient of 0-20% methanol in dichloromethane, affording the free base of the title compound as a solid.
δH (CDCl₃, 400MHz) 1.42 (2H, m), 1.85 (2H, t), 1.98 (2H, br.s), 2.05 (1H, t), 2.23 (1H, t), 2.45-2.55 (2H, m), 2.75 (1H, br.d), 2.85 (1H, dd) 3.00 (1H, br. d), 3.21 (2H, m), 4.00. (2H, m), 4.40-4.50 (2H, m), 4.65 (2H, m), 6.60 (1H, d), 6.86 (1H, t), 7.40 (2H, m), 7.67 (1H, d).
MS (+ve ion electrospray) m/z 468 (MH+).

The free base in methanol-chloroform was treated with an excess of 4M hydrogen chloride in dioxan, evaporated and triturated with ether to afford the title compound as a solid (43 mg).

### Example 17B 1-({4-[(6,7-Dihydro[1,4]oxathiino[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 Hydrochloride

A solution of 1-({4-[(6,7-dihydro[1,4]oxathiino[2,3*-c*]pyridazin-3-ylmethyl)amino]-1-piperidinyl}methyl)-9-fluoro-1,2-dihydro-4H-pyrrolo[3,2,1-ij]quinolin-4-one Enantiomer E1 (890 mg, 1.9 mmol) in methanol was treated with 5M hydrochloric acid (0.4 ml, 2 mmol) was evaporated to dryness and triturated with ether to give a white solid (950 mg).
MS (+ve ion electrospray) m/z 468 (MH+).

### Example 18 1-({4-[(6,7-Dihydro[1,4]dioxino[2,3-c]pyridazin-3-ylmethyl)amino]-1-piperidinyl}methyl)-9-fluoro-1-hydroxy-1,2-dihydro-4H-pyrrolo[3,2,1-ij]quinolin-4-one Dihydrochloride

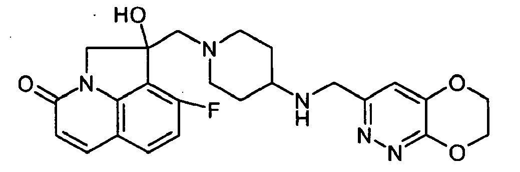

A solution of 1-[(4-amino-1-piperidinyl)methyl]-9-fluoro-1-hydroxy-1,2-dihydro-4*H*-pyrrolo[3,2,1-*ij*]quinolin-4-one (racemic) (44 mg, 0.138 mmol) and 6,7-dihydro[1,4]dioxino[2,3*-c*]pyridazine-3-carbaldehyde (23 mg, 0.138 mmol) in N,N-dimethylformamide (1 ml) was treated with sodium triacetoxyborohydride (87 mg, 0.414 mmol) at room temperature for 20 hours. More 6,7-dihydro[1,4]dioxino[2,3-c]pyridazine-3-carbaldehyde (12.5 mg) and sodium triacetoxyborohydride (43.5 mg) were added and the mixture was stirred at room temperature for a further 12 hours The mixture was evaporated to dryness, treated with aqueous sodium bicarbonate and extracted with 10% methanol in dichloromethane. The combined organic extracts were dried (magnesium sulphate) and evaporated. The residue was chromatographed, on silica gel, eluting with 0-30% methanol in dichloromethane, affording the free base as a colourless oil (25 mg, 40%),
MS (+ve ion electrospray) m/z 468 (MH+).
δH (CD₃OD, 250MHz), 1.10-1.30 (1H, m), 1.30-1.50 (1H, m), 1.60-1.95 (2H, m), 2.15-2.40(2H, m), 2.45-2.75 (2H, m), 3.06 (1H, s), 3.14 (1H, m), 3.31 (2H, m), 3.96 (2H, s), 4.20 (1H, d), 4.30-4.60 (4H, m), 4.66 (1H, d), 6.62 (1H, d), 7.02 (1H, d), 7.06 (1H, d), 7.69 (1H, dd), 7.95 (1H, d).

The free base in methanol, was converted to the dihydrochloride salt by adding an excess of 1M hydrogen chloride in methanol, followed by evaporation to dryness, to give a solid.

### Example 19 1-({4-[(2,3-dihydro-1H-pyrido[3,4-b][1,4]oxazin-7-ylmethyl)amino]-1-piperidinyl}methyl)-9-fluoro-1,2-dihydro-4H-pyrrolo[3,2,1-ij]quinolin-4-one Dihydrochloride

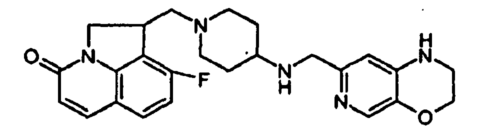

### (a) 1-[(4-Amino-1-piperidinyl)methyl]-9-fluoro-1,2-dihydro-4H-pyrrolo[3,2,1-ij]quinolin-4-one

1,1-Dimethylethyl {1-[(9-fluoro-4-oxo-1,2-dihydro-4H-pyrrolo[3,2,1-ij]quinolin-1-yl)methyl]-4-piperidinyl}carbamate (316 mg, 0.79 mmol) was dissolved in dichloromethane (2 ml) and trifluoroacetic acid (1 ml), stirred at room temperature for 3 hours then evaporated to dryness and azeotroped with chloroform. The residue was dissolved in DCM/methanol (1:1) and stirring with an excess of MP-carbonate resin until pH7-8. Filtration and evaporation afforded a yellow oil (238 mg, 100%).
MS (+ve ion electrospray) m/z 302 (MH+).

### (b) 1,1-Dimethylethyl 7-[({1-[(9-fluoro-4-oxo-1,2-dihydro-4H-pyrrolo[3,2,1-ij]quinolin-1-yl)methyl]-4-piperidinyl}amino)methyl]-2,3-dihydro-1H-pyrido[3,4-b][1,4]oxazine-1-carboxylate.

A solution of 1-[(4-amino-1-piperidinyl)methyl]-9-fluoro-1,2-dihydro-4*H-*pyrrolo[3,2,1-*ij*]quinolin-4-one (racemic) (40 mg, 0.132 mmol) and 1,1-dimethylethyl 7-formyl-2,3-dihydro-1H-pyrido[3,4-b][1,4]oxazine-1-carboxylate (35 mg, 0.132 mmol) in dichloromethane (1.5 ml) and methanol (0.1 ml) was treated with sodium triacetoxyborohydride (84 mg, 0.398 mmol) at room temperature overnight. The mixture was treated with aqueous sodium bicarbonate and extracted with 5% methanol in dichloromethane and the combined organic extracts were dried and evaporated. The residue was chromatographed, on silica gel, eluting with 0-30% methanol in dichloromethane, affording the free base (82 mg).
MS (+ve ion electrospray) m/z 550 (MH+).

### (c) Title compound

A solution of 1,1-dimethylethyl 7-[({1-[(9-fluoro-4-oxo-1,2-dihydro-4*H-*pyrrolo[3,2,1-*ij*]quinolin-1-yl)methyl]-4-piperidinyl}amino)methyl]-2,3-dihydro-1N-pyrido[3,4-*b*][1,4]oxazine-1-carboxylate (82 mg) in dichloromethane (2 ml) was treated with trifluoroacetic acid (1 ml). After 3 hours the mixture was evaporated and the residue was azeotroped with chloroform. The residual trifluoroactetate salt was converted to the crude free base by dissolving in DCM:MeOH (1:1), stirring with an excess of MP-carbonate resin base until pH 7-8, filtering and evaporating to dryness to afford a clear oil (ca. 44 mg).
δH (CDCl₃, 250MHz) 1.30-1.55 (2H, m), 1.70-2.00 (2H, m), 2.07 (1H, m), 2.23 (1H, m), 2.40-2.70 (2H, m), 2.70-2.90 (2H, m), 2.90-3.10 (1H, m), 3.45 (2H, m), 3.70 (2H, s),3.90-4.10 (1H, m), 4.15-4.30 (2H, m), 4.35-4.55 (2H, m), 4.61 (1H, s), 6.50 (1H, s), 6.62 (1H, d), 6.86 (1H, t), 7.38 (1H, dd), 7.66 (1H, d), 7.90 (1H, s).
MS (+ve ion electrospray) m/z 450(H+).

The free base in methanol, was converted to the dihydrochloride salt by adding an excess of 1M hydrogen chloride in methanol (0.3ml), followed by evaporation to dryness, to give a solid (44 mg).

### Example 20 1-({4-[(2,3-Dihydro[1,4]oxathiino[2,3-b]pyridin-7-ylmethyl)amino]-1-piperidinyl}methyl)-9-fluoro-1,2-dihydro-4H-pyrrolo[3,2,1-ij]quinolin-4-one Hydrochloride

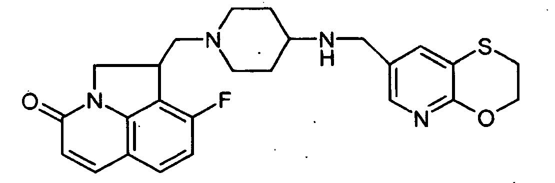

### (a) Ethyl 5-[(2-hydroxyethyl)thio]-6-oxo-1,6-dihydro-3-pyridinecarboxylate

Ethyl 5-iodo-6-oxo-1,6-dihydro-3-pyridinecarboxylate (0.59 g, 2.01 mmol) (prepared according to the method of 1. Houpis et al, Tet. Lett. 1994, 9355) with copper(I) iodide (20 mg, 0.105 mmol), potassium carbonate (0.55 g, 3.96 mmol), and 2-mercaptoethanol (1 ml, 14.3 mmol) in dry N,N-dimethylformamide (20 ml) was microwaved (150W) to reach a maximum internal temperature of 170°C, for 20 minutes. The reaction was cooled and combined with the reaction mixture from a second reaction carried out by identical means on the same scale. The solvent was evaporated and the residue partitioned between water and 10% methanol in dichloromethane. The layers were separated and the aqueous extracted with 10% methanol in dichloromethane (4x). The combined organics were dried over magnesium sulphate and evaporated. The residue was purified by chromatography on silica eluting with 0-10% ethyl acetate in hexane to give a white solid (0.86 g, 88%).
MS (-ve ion electrospray) m/z 242 (M-H⁻).

### (b) Ethyl 2,3-dihydro[1,4]oxathiino[2,3-b]pyridine-7-carboxylate

Triphenylphosphine (0.796 g, 3.03 mmol) was added to a solution of diisopropyl azodicarboxylate (0.60 ml, 3.05 mmol) in tetrahydrofuran (75 ml) at 0°C and stirred for 15 minutes. Ethyl 5-[(2-hydroxyethyl)thio]-6-oxo-1,6-dihydro-3-pyridinecarboxylate (0.52 g, 2.14 mmol) was then added and the mixture stirred at room temperature overnight. The mixture was evaporated and the residue chromatographed on silica eluting with 0-100% ethyl acetate in hexane to give a white solid (0.25 g, 52%).
MS (+ve ion electrospray) m/z 226 (MH+).

### (c) 2,3-Dihydro[1,4]oxathiino[2,3-b]pyridin-7-ylmethanol

Ethyl 2,3-dihydro[1,4]oxathiino[2,3-*b*]pyridine-7-carboxylate (0.25 g, 1.11 mmol) in dry tetrahydrofuran was cooled in ice/water and treated with 1.0M diisobutylaluminium hydride in tetrahydrofuran (3.75 ml). The mixture was stirred overnight and further diisobutylaluminium hydride solution (2 ml) was added at 0°C. After 1 hour the mixture was treated with an aqueous solution of potassium sodium tartrate (25ml), stirred for 1 hour and then evaporated. The residue was partitioned between water and ethyl acetate and the organic phase washed with brine and dried. The residue was chromatographed on silica eluting with 1-100% ethyl acetate in hexane to give a white solid (60 mg, 30%).
MS (+ve ion electrospray) m/z 184 (MH+).

### (d) 2,3-dihydro[1,4]oxathiino[2,3-b]pyridine-7-carbaldehyde

2,3-Dihydro[1,4]oxathiino[2,3-*b*]pyridin-7-ylmethanol (0.14 g, 0.765 mmol) in dichloromethane (20ml) was stirred overnight with manganese(IV) oxide (0.60 g, 3.8 mmol), filtered through kieselguhr and evaporated to give a white solid (100 mg, 72%). MS (+ve ion electrospray) m/z 182 (MH+).

### (e) Title compound

A solution of 1-[(4-amino-1-piperidinyl)methyl]-9-fluoro-1,2-dihydro-4*H-*pyrrolo[3,2,1-*ij*]quinolin-4-one (racemic) (150.5 mg, 0.5 mmol) and 2,3-dihydro[1,4]oxathiino[2,3-*b*]pyridine-7-carbaldehyde (100 mg, 0.55 mmol) in methanol (7 ml) and acetic acid (3 drops) was treated with (polystyrylmethyl)trimethylammonium cyanoborohydride (0.49 g, 2 mmol) with stirring at room temperature for 6 hours. After standing at room temperature for 6 days, the mixture was filtered and evaporated to dryness to give an orange oil (282 mg). The residue was chromatographed on silica gel, eluting with 0-10% 2M ammonia-methanol /dichloromethane, affording the free base as a colourless oil (149 mg; 64%).
MS (+ve ion electrospray) m/z 467(MH+).
δH (CDCl₃, 400 MHz) 1.40 (2H, m), 1.88 (2H, t), 2.08 (1H, t), 2.23 (1H, t), 2.50 (2H, m), 2.78 (1H, br. d), 2.85 (1H, dd), 3.02 (1H, br.d), 3.12 (2H, m), 3.70 (2H, s), 4.0 (1H, m), 4.45 (2H, m), 4.60 (2H, m), 6.60 (1H, d), 6.85 (1H, t), 7.38 (1H, m), 7.42 (1H, s), 7.65 (1H, d), 7.88 (1H, s).

The free base, in dichloromethane, was converted to the hydrochloride salt by adding one equivalent of a solution of 1M hydrogen chloride in ether, followed by evaporation to dryness, to give a solid (150 mg).

### Example 21 1-({4-[(2,3-Dihydro[1,4]dioxino[2,3-b]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E1 Dihydrochloride

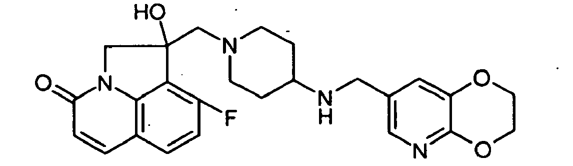

This was prepared from 1-[(4-amino-1-piperidinyl)methyl]-9-fluoro-1-hydroxy-1,2-dihydro-4*H*-pyrrolo[3,2,1-*ij*]quinolin-4-one enantiomer E1 (39 mg, 0.12 mmol) and 2,3-dihydro[1,4]dioxino[2,3-*b*]pyridine-7-carbaldehyde (for a synthesis, see WO2003087098 Example 20(e)) (20 mg, 0.12 mmol) by the general method of Example 15, to give the free base (22 mg; 38%).
MS (+ve ion electrospray) m/z 467(MH+).

The free base in methanol, was converted to the dihydrochloride salt by adding an excess of 1M hydrogen chloride in ether, followed by evaporation to dryness, to give a solid (21 mg).

### Example 22 1-({4-[(2,3-dihydro[1,4]dioxino[2,3-b]pyridin-7-ylmethyl)amino]-1-piperidinyl}methyl)-9-fluoro-1,2-dihydro-4H-pyrrolo[3,2,1-ij]quinolin-4-one Dihydrochloride

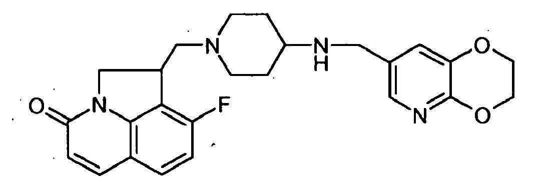

This was prepared from 1-[(4-amino-1-piperidinyl)methyl]-9-fluoro-1-hydroxy-1,2-dihydro-4*H*-pyrrolo[3,2,1-*ij*]quinolin-4-one (racemic) (200 mg, 0.66 mmol) and 2,3-dihydro[1,4]dioxino[2,3-*b*]pyridine-7-carbaldehyde (for a synthesis, see WO2003087098 Example 20(e)) (109.6 mg, 0.66 mmol) by the general method of Example 20(e) (except that the reaction mixture was filtered after stirring overnight), to give the free base as a solid (217 mg).
MS (+ve ion electrospray) m/z 451(MH+).
δH (CDCl₃, 400 MHz) 1.40 (2H, m), 1.88 (2H, t), 2.08 (1H, t), 2.23 (1H, t), 2.50 (2H, m), 2.78 (1H, br. d), 2.85 (1H, dd), 3.02 (1H, br.d), 3.72 (2H, s), 4.0 (1H, m), 4.23 (2H, m), 4.40 (4H, m), 6.60 (1H, d), 6.85 (1H, t), 7.21 (1H, s), 7.38 (1H, m), 7.68 (1H, d), 7.74 (1H, s).

The free base in dichloromethane, was converted to the dihydrochloride salt by adding a solution of 1M hydrogen chloride in ether, followed by evaporation to dryness, to give a solid (186 mg).

### Example 23 1-({4-[(1,2,3-bencothiadiazol-5-ylmethyl)amino]-1-piperidinyl}methyl)-9-fluoro-1-hydroxy-1,2-dihydro-4H-pyrrolo[3,2,1-ij]quinolin-4-one Dihydrochloride

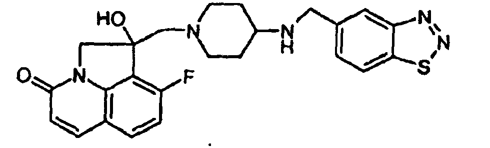

A solution of 1-[(4-amino-1-piperidinyl)methyl]-9-fluoro-1-hydroxy-1,2-dihydro-4*H*-pyrrolo[3,2,1-*ij*]quinolin-4-one (racemic) (48 mg, 0.15 mmol) and 1,2,3-benzothiadiazole-5-carbaldehyde (prepared by manganese (IV) oxide oxidation of benzo[1,2,3]thiadiazol-5-yl-methanol,(for a synthesis see WO2003087098 Example 6(a)), (25 mg, 0.15 mmol) in dichloromethane (3 ml) and methanol (1 ml) was treated with sodium triacetoxyborohydride (95 mg, 0.45 mmol) for 5 hours at room temperature. Excess sodium bicarbonate solution was added and the mixture evaporated to dryness. The residue was chromatographed on silica gel, eluting with 0-50% methanol in dichloromethane, affording the free base as an oil (25 mg, 36%).
MS (+ve ion electrospray) m/z 466 (MH+).
δH (CDCl₃, 250MHz) 1:51 (2H, m), 1.95-2.25 (m, signals partly obscured by a water peak), 2.38 (1H, t), 2.55 (1H, t), 2.65 (2H, m), 2.85 (1H, d), 3.0 (2H, br.d), 3.36 (1H, d), 4.05 (2H, s), 4.40.(2H, m), 6.62 (1H, d), 6.90 (1H, t), 7.50 (1H, q), 7.70 (2H, m), 8.05 (1H, d), 8.60 (1H, s).

The free base in methanol, was converted to the dihydrochloride salt by adding an excess of 1M hydrogen chloride in ether, followed by evaporation to dryness, to give a solid (30 mg).

### Example 24 1-({4-[(2,3-dihydro[1,4]dioxino[2,3-c]pyridin-7-ylmethyl)amino]-1-piperidinyl}methyl)-4-oxo-1,2-dihydro-4H-pyrrolo[3,2,1-ij]quinoline-9-carbonitrile Enantiomer E2 Dihydrochloride

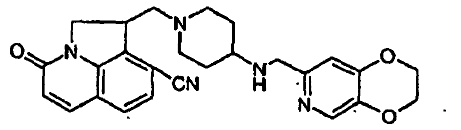

The E2 enantiomer from Example 13 was converted into the Dihydrochloride salt by dissolving the free base in a small amount of methanol and adding a 6N solution of hydrochloric acid. The solution was then evaporated under vacuum to give a solid.

### Example 25A 1-({4-[(2,3-Dihydro[1,4]dioxino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E1 Dihydrochloride

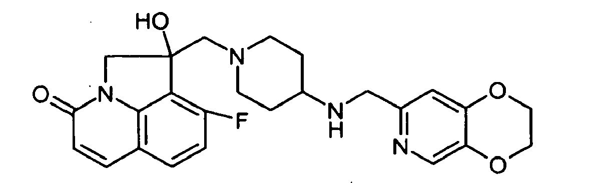

A solution of 1-[(4-amino-1-piperidinyl)methyl]-9-fluoro-1-hydroxy-1,2-dihydro-4*H*-pyrrolo[3,2,1-*ij*]quinolin-4-one enantiomer E1 (40 mg, 0.12 mmol) and 2,3-dihydro[1,4]dioxino[2,3*-c*]pyridine-7-carbaldehyde (for a synthesis see WO2004058144, Example 2(c)) (20 mg, 0.12 mmol) was reacted with sodium triacetoxyborohydride (85 mg, 0.40 mmol) by the general method described for enantiomer E2 (Example 14) affording the free base as a yellow oil (36 mg, 60%).
MS (+ve ion electrospray) m/z 467 (MH+).
δH (CD₃OD, 400MHz): identical NMR to the E2 enantiomer (Example 14).

The free base in methanol-DCM, was converted to the dihydrochloride salt by adding an excess of 1 M hydrogen chloride in ether, followed by more ether to precipitate a solid (52 mg).

### Example 25B 1-({4-[(2,3-Dihydro[1,4]dioxino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E1 Hydrochloride

The title compound was prepared from 9-fluoro-1-(hydroxymethyl)-4-oxo-1,2-dihydro-4*H*-pyrrolo[3,2,1-*ij*]quinolin-1-yl 4-methylbenzenesulfonate and 1,1-dimethylethyl (2,3-dihydro[1,4]dioxino[2,3*-c*]pyridin-7-ylmethyl)-4-piperidinylcarbamate (for a synthesis, see WO 2004058144 Example 99(h)) followed by separation of the enatiomer E1, in a similar manner to procedures generally described herein.

### Example 26 9-Fluoro-1-[(4-{[(5-oxo-1,2,3,5-tetrahydro-7-indolizinyl)methyl]amino}-1-piperidinyl)methyl]-1,2-dihydro-4H-pyrrolo[3,2,1-ij]quinolin-4-one Hydrochloride

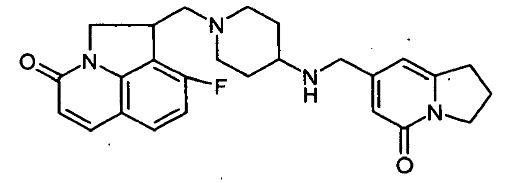

### (a) 2-Chloro-4-({[(1,1-dimethylethyl)(dimethyl)silyl]oxy}methyl)-6-(methyloxy)pyridine

A solution of [2-chloro-6-(methyloxy)-4-pyridinyl]methanol (for a synthesis, see Adamczyk, M.; Akireddy, S. R.; Reddy, Rajarathnam E. Tetrahedron 2002, 58(34), 6951)(8.02 g, 46.22 mmol) in dry dimethylformamide (100 ml) was treated with *tert*butyldimethylsilyl chloride (8.36 g, 55.46 mmol) and imidazole (3.77 g, 55.46 mmol) and stirred at room temperature for 2 hours. The reaction mixture was treated with water extracted 3x with dichloromethane, dried (magnesium sulphate), evaporated and chromatographed on silica gel (100 g), eluting with 1:4 ethyl acetate-hexane to give the desired product (12.38 g, 93%).
MS (+ve ion electrospray) m/z 288/290 (MH+).

### (b) Butyl (2E)-3-[4-({[(1,1-dimethylethyl)(dimethyl)silyl]oxy}methyl)-6-(methyloxy)-2-pyridinyl]-2-propenoate

A solution of 2-chloro-4-({[(1,1-dimethylethyl)(dimethyl)silyl]oxy}Methyl)-6-(methyloxy)pyridine (9.20 g, 32.01 mmol) in 1,4-dioxane (100 ml) was treated with bis(tri-t-butylphosphine)palladium(0) (327 mg, 0.64 mmol), tris(dibenzylideneacetone)dipalladium(0) (293 mg, 0.32 mmol), dicyclohexylmethylamine (7.53 ml, 35.21 mmol) and butyl acrylate (5.96 ml, 41.62 mmol). The reaction was heated at 120°C for 1h and was then treated with water extracted 3x with diethyl ether, dried (magnesium sulphate), evaporated and chromatographed on silica gel (250 g), eluting with 1:4 ethyl acetate-hexane to give the desired product (8.25 g, 68%).
MS (+ve ion electrospray) m/z 380 (MH+).

### (c) Butyl 3-[4-({[(1,1-dimethylethyl)(dimethyl)silyl]oxy}methyl)-6-(methyloxy)-2-pyridinyl]propanoate

A mixture of butyl (2*E*)-3-[4-({[(1,1-dimethylethyl)(dimethyl)silyl]oxy}methyl)-6-(methyloxy)-2-pyridinyl]-2-propenoate (4.84 g, 12.49 mmol) and 10% palladium on carbon in methanol (200 ml) was stirred at room temperature for 3 hours. The mixture was filtered through kieselguhr and evaporated to give the desired product (4.76 g, 98%). MS (+ve ion electrospray) m/z 382 (MH+).

### (d) 3-[4-({[(1,1-Dimethylethyl)(dimethyl)silyl]oxy}methyl)-6-(methyloxy)-2-pyridinyl]-1-propanol

A solution of butyl 3-[4-({[(1,1-dimethylethyl)(dimethyl)silyl]oxy}methyl)-6-(methyloxy)-2-pyridinyl]propanoate (4.76 g, 12.49 mmol) in THF (120 ml) was treated with LiAlH₄ solution (1 M in THF, 12,49 ml, 12.49 mmol) at -78°C. The reaction mixture was allowed warm to -20°C and after stirring at -20°C for 15 minutes, the mixture was treated with water (9 ml) and allowed to stir for I hour before being filtered and evaporated to give a slightly impure product (3.98 g, 102%).
MS (+ve ion electrospray) m/z 312 (MH+).

### (e) 7-({[(1,1-Dimethylethyl)(dimethyl)silyl]oxy}methyl)-2,3-dihydro-5(1H)-indolizinone

A solution of 3-[4-({[(1,1-dimethylethyl)(dimethyl)silyl]oxy}methyl)-6-(methyloxy)-2-pyridinyl]-1-propanol (5.16 g, 16.59 mmol) in dichloromethane (250 ml) was treated with pyridine (2.94 ml, 36.47 mmol) and trifluoromethanesulfonic anhydride (3.1ml, 19.88 mmol) and stirred at room temperature for 10 minutes before being treated with tetrabutylammonium iodide (30.61 g, 82.95 mmol) and stirred at room temperature for a further 4 hours. Water was then added and the mixture was extracted with diethyl ether (x3) and the combined organic extracts washed again with water. The organic extracts were dried with magnesium sulphate and evaporated. The residue was chromatographed on silica eluting with 0-10% methanol in dichloromethane to give the desired product (3.93 g, 14.09 mmol).
MS (+ve ion electrospray) m/z 280 (MH+).

### (f) 7-(Hydroxymethyl)-2,3-dihydro-5(1H)-indolizinone

A solution of 7-({[(1,1-dimethylethyl)(dimethyl)silyl]oxy}methyl)-2,3-dihydro-5(1*H*)-indolizinone (3.93 g, 14.09 mmol) in tetrahydrofuran (100 ml) was treated with acetic acid (1.61 ml, 28.17 mmol) and tetrabutylammonium fluoride (1M in THF, 21 ml, 21.13 mmol) and stirred at room temperature for 1 hour before being evaporated. The residue was chromatographed on silica eluting with 0-20% methanol in dichloromethane to give the desired product (1.87 g, 80%).
MS (+ve ion electrospray) m/z 166 (MH+).

### (g) 5-Oxo-1,2,3,5-tetrahydro-7-indolizinecarbaldehyde

A solution of 7-(hydroxymethyl)-2,3-dihydro-5(1*H*)-indolizinone (237 mg, 1.44 mmol) in acetone (12 ml) was treated with ortho-iodoxybenzoic acid (603 mg, 2.16 mmol) and heated at reflux for 1 hour. The mixture was then evaporated, dissolved in dichloromethane and filtered, redissolved in dichloromethane and filtered again to provide the desired product (238 mg, 101%).
MS (+ve ion electrospray) m/z 164 (MH+).

### (h) Title compound

A mixture of 1-[(4-amino-1-piperidinyl)methyl]-9-fluoro-1,2-dihydro-4*H-*pyrrolo[3,2,1-*ij*]quinolin-4-one (racemic) (97 mg, 0.322 mmol), 5-oxo-1,2,3,5-tetrahydro-7-indolizinecarbaldehyde (52 mg, 0.322 mmol) and 3A molecular sieves in chloroform (5 ml) and DMF (0.2 ml) was heated under reflux for 2 hours, cooled to room temperature, and then sodium triacetoxyborohydride (0.137 g g, 0.644 mmol) was added and the mixture was heated at 50°C for 1.5 hours. The mixture was cooled, filtered, evaporated and chromatographed on silica gel, eluting with 0-20% methanol-DCM to afford a white solid (66 mg, 46%).
MS (+ve ion electrospray) m/z 449 (MH+).
δH (CDCl₃, 400MHz) 1.35-1.50 (2H, m), 1.80-2.00 (2H, m), 2.05-2.10 (1H, m) 2.15-2.25 (3H, m), 2.45-2.55 (2H, m), 2.73-2.82 (1H, m), 2.83-2.89 (1H, m), 2.98-3.10 (3H, m), 3.57 (2H, s), 3.99-4.07 (1H, m), 4.10-4.17 (2H, t), 4.40-4.52 (2H, m), 6.21 (1H, s), 6.35 (1H, s), 6.62 (1H, d), 6.87 (1H, t), 7.79 (1H, m), 7.67 (1H, d).

The free base in methanol and chloroform was converted to the hydrochloride salt by adding one equivalent of a solution of 4M hydrogen chloride in dioxane, followed by evaporation to dryness, to give a solid (50 mg).

### Example 27 1-({4-[(2,3-Dihydro[1,4]oxathiino[2,3-c]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E2 Dihydrochloride

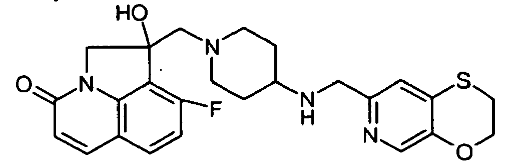

1-[(4-Amino-1-piperidinyl)methyl]-9-fluoro-1-hydroxy-1,2-dihydro-4*H-*pyrrolo[3,2,1-*ij*]quinolin-4-one enantiomer E2 (39 mg, 0.12 mmol) and 2,3-dihydro[1,4]oxathiino[2,3-*c*]pyridine-7-carbaldehyde (for a synthesis, see W02004058144 Example 60(i)) (22 mg, 0.12 mmol) in N,N-dimethylformamide (1 ml) was treated with sodium triacetoxyborohydride (79 mg, 0.37 mmol) at room temperature for 16 hours. The mixture was evaporated to dryness, treated with aqueous sodium bicarbonate and extracted with 10% methanol in dichloromethane. The combined organic extracts were dried (magnesium sulphate) and evaporated. The residue was chromatographed on silica gel, eluting with 0-30% methanol in dichloromethane, affording the free base as a colourless oil (48 mg).
MS (+ve ion electrospray) m/z 483 (MH+).
δH (CD₃OD, 250MHz) 1.12 (1H, m), 1.38 (1H, m), 1.65 (1H, br.d), 1. 80 (1H, br.d), 2.10-2.45 (3H, m) 2.62 (1H, br.d), 2.99 (2H, s), 3.08 (1H, d), 3.20 (2H, m), 3.65 (2H, s), 4.17 (1H, d), 4.37 (2H, m), 4.62 (1H, d), 6.60 (1H, d), 7.02 (1H, t), 7.08 (1H, s), 7.68 (1H, dd), 7.84 (1H, s), 7.92 (1H, d).

The free base in methanol, was converted to the dihydrochloride salt by adding an excess of 1M hydrogen chloride in methanol, followed by evaporation to dryness, to give a cream solid (51 mg; 86%).

### Example 28 1-({4-[(2,3-Dihydro[1;4]dioxino[12,3-b]pyridin-7-ylmethyl)amino]-1-piperidinyl}methyl)-9-fluoro-1-hydroxy-1,2-dihydro-4H-pyrrolo[3,2,1-ij]quinolin-4-one Enantiomer E2 Dihydrochloride

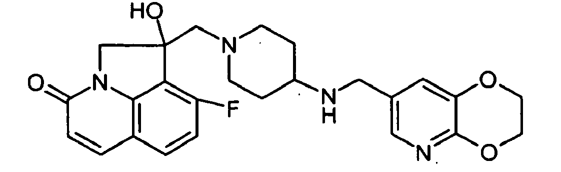

This was prepared from 1-[(4-amino-1-piperidinyl)methyl]-9-fluoro-1-hydroxy-1,2-dihydro-4*H*-pyrrolo[3,2,1-*ij*]quinolin-4-one enantiomer E2 (39 mg, 0.12 mmol) and 2,3-dihydro[1,4]dioxino[2,3-*b*]pyridine-7-carbaldehyde (for a synthesis, see WO2003087098 Example 20(e)) (20 mg; 0.12 mmol) by the general method of Example 27 to give, after chromatography, the free base as a yellow oil (40 mg)
MS (+ve ion electrospray) m/z 467 (MH+).
δH (CD₃OD, 250MHz) 1.20 (1H, m), 1.45 (1H, m), 1.75 (1H, br.d), 1. 91 (1H, br.d), 2.24 (2H, m) 2.65 (2H, m), 3.11 (2H, s), 3.18 (1H, d), 3.80 (2H, s), 4.22 (3H, m), 4.40 (2H, m), 4.64 (1H, d), 6.61 (1H, d), 7.03 (1H, t), 7.33 (1H, d), 7.70 (2H, m), 7.94 (1H, d).

The free base, in methanol, was converted to the dihydrochloride salt by adding an excess of 1M hydrogen chloride in methanol, followed by evaporation to dryness, to give a cream solid (41 mg).

### Example 29 1-({4-[(6,7-Dihydro[1,4]oxathiino[2,3-c]pyridazin-3-ylmethyl)amino]-1-piperidinyl}methyl)-9-fluoro-1-hydroxy-1,2-dihydro-4H-pyrrolo[3,2,1]quinolin-4-one Enantiomer E1 Dihydrochloride

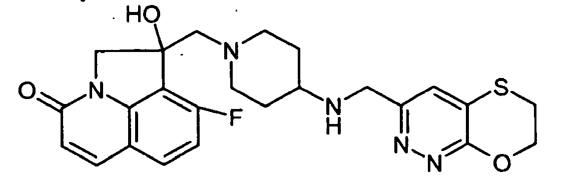

A solution of 1-[(4-amino-1-piperidinyl)methyl]-9-fluoro-1-hydroxy-1,2-dihydro-4*H*-pyrrolo[3,2,1-*ij*]quinolin-4-one enantiomer E1 (40 mg, 0.126 mmol) and 6,7-dihydro[1,4]oxathiino[2,3-*c*]pyridazine-3-carbaldehyde (27.5 mg, 0.151 mmol) in methanol (0.3 ml) and dichloromethane (1 ml) was treated with sodium triacetoxyborohydride (95 mg) at room temperature for 18 hours. Further 6,7-dihydro[1,4]oxathiino[2,3-*c*]pyridazine-3-carbaldehyde(10 mg) was added and the mixture was stirred for 1 hour, when more sodium triacetoxyborohydride (95 mg) was added. The mixture was stirred at room temperature overnight. The mixture was treated with aqueous sodium bicarbonate and extracted (3x) with 10% methanol in dichloromethane. The combined organic extracts were dried (sodium sulphate), evaporated and chromatographed on silica gel, eluting with 0-25% methanol in dichloromethane, affording the free base as a colourless oil.
MS (+ve ion electrospray) m/z 484 (MH+).
δH (CDCl₃, 400MHz) 1.48 (2H, m), 1.91 (2H, br.t), 2.36 (1H, t), 2.51 (1H, t), 2.58 (1H, m), 2.82 (1H, d), 2.95 (2H, m), 3.22 (2H, m), 3.32 (1H, d), 3.97 (2H, s), 4.39 (2H, q), 4.64 (2H, m), 6.60 (1H, d), 6.90 (1H, t), 7.33 (1H, s), 7.49 (1H, m), 7.69 (1H, d).

The free base in methanol, was converted to the dihydrochloride salt by adding an excess of 4M hydrogen chloride in dioxan, followed by evaporation to dryness, and trituration with ether to give an off-white solid (34 mg).

### Example 30 1-({4-[(6,7-Dihydro[1,4]oxathiino[3,2-c]pyridazin-3-ylmethyl)amino]-1-piperidinyl}methyl)-9-fluoro-1,2-dihydro-4H-pyrrolo[3,2,1-ij]quinolin-4-one Enantiomer E1 Dihydrochloride

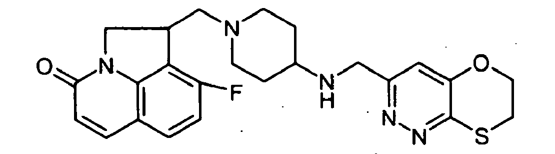

### (a) 3-Chloro-6,7-dihydro[1,4]oxathiino[3,2-c]pyridazine

A solution of 2-[(3,6-dichloro-4-pyridazinyl)thio]ethanol (34 g, 0.15 mol) in dry dioxane (700 ml) was treated with lithium hydride (1.52 g, 0.18 mol) and heated at reflux overnight. More lithium hydride (1.15 g) was added and the mixture was heated again at reflux overnight. The reaction mixture was cooled, quenched with ice-water and filtered. The filtrate was evaporated to a quarter of its volume. Water was added. The aqueous layer was acidified, extracted 4x with dichloromethane, dried (sodium sulphate), evaporated and chromatographed on silica gel eluting with 0-50% ethyl acetate in dichloromethane affording a yellow solid (170 mg, 0.5%), in the early fractions. Trituration with ethyl acetate-hexane gave the pure product (98 mg).
MS (+ve ion electrospray) m/z 189/91 (MH+).
δH (CDCl₃, 400MHz) 3.29 (2H, m), 4.51 (2H, m), 6.86 (1H, s).

[Later fractions gave the isomeric 3-chloro-6,7-dihydro[1,4]oxathiino[2,3-c]pyridazine (4.2 g) - see Example 17(b)]

### (b) 3-Ethenyl-6,7-dihydro[1,4]oxathiino[3,2-c]pyridazine

A solution of 3-chloro-6,7-dihydro[1,4]oxathiino[3,2-*c*]pyridazine(450 mg, 2.4 mmol) in dimethoxyethane (12 ml) was treated with tetrakis(triphenylphosphine)palladium (0) (61 mg), potassium carbonate (313 mg), 2,4,6-trivinylcyclotriboroxane pyridine complex (375 mg) and water (1.5 ml). The mixture was heated at 96°C, overnight. The mixture was evaporated to dryness, treated with aqueous sodium bicarbonate solution, extracted (4x) with DCM, dried (sodium sulphate), evaporated and chromatographed on silica gel (50 g), eluting with 1:1 ethyl acetate-hexane, affording a solid (200 mg, 46%), containing slightly impure product.
MS (+ve ion electrospray) m/z 181 (MH+).

### (c) 6,7-Dihydro[1,4]oxathiino[3,2-c]pyridazine-3-carbaldehyde

A solution of 3-ethenyl-6,7-dihydro[1,4]oxathiino[3,2-*c*]pyridazine(200mg, 1.11 mmol) in dioxan/water (10 ml/2 ml) was treated with an aqueous solution of osmium tetroxide (4% w/v, I ml) and sodium periodate (0.55 g), initially stirred in an ice-bath for 1.5 hours, then allowed to warm to room temperature. After 1.5 hours the mixture was treated with sodium bicarbonate solution, evaporated to dryness and dissolved in dioxan and chloroform. Silica gel was added and the mixture was evaporated to dryness, added to a silica column (20 g), and chromatographed, eluting with 0-100% ethyl acetate in hexane, to afford a pale yellow solid (63 mg, 31%).
MS (+ve ion electrospray) m/z 183 (MH+).

### (d) Title compound

A solution of 1-[(4-amino-1-piperidinyl)methyl]-9-fluoro-1,2-dihydro-4*H-*pyrrolo[3.2,1-*ij*]quinolin-4-one enantiomer E1 (53 mg, 0.176 mmol), 6,7-dihydro[1,4]oxathiino[3,2-*c*]pyridazine-3-carbaldehyde (31 mg, 0.17 mmol) in methanol (0.5 ml) and dichloromethane (3 ml) was stirred with 3A molecular sieves overnight at room temperature. Sodium triacetoxyborohydride (0.113 g, 0.53 mmol) was added and the mixture stirred at room temperature overnight. Dichloromethane and sodium carbonate were added and the mixture extracted with 10% methanol in dichloromethane (4x) The extracts were dried with sodium sulphate and evaporated. The residue was chromatographed on silica eluting with 0-20% methanol in dichloromethane to give the free base as an oil.
MS (+ve ion electrospray) m/z 468 (MH+).
δH (CDCl₃, 400MHz) 1.35-1.50 (2H, m), 1.85-2.05 (4H,m), 2.07 (1H, m), 2.22 (1H, m), 2.45-2.60 (2H, m), 2.77 (1H, d), 2.84 (1H, m), 3.02 (1H, d), 3.28 (2H, m), 4.02 (2H, s), 4.40-4.55 (4H, m), 6.62 (1H, d), 6.86 (1H, t), 6.91 (1H, s), 7.39 (1H, m), 7.67 (1H, d).

The free base in methanol and chloroform was converted to the dihydrochloride salt by adding an excess of 4M hydrogen chloride in dioxan, followed by evaporation to dryness, and trituration with ether to give an off-white solid (60 mg).

### Example 31 1-({4-[(6,7-Dihydro-5H-pyrano[2,3-c]pyridacin-3-ylmethyl)amino]-1-piperidinyl}methyl)-9-fluoro-1,2-dihydro-4H-pyrrolo[3,2,1-ij]quinolin-4-one Enantiomer E1 Dihydrochloride

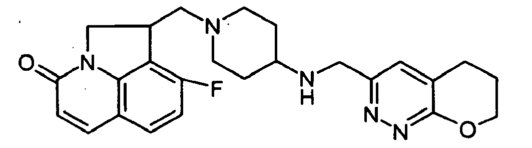

### (a) 4-Bromo-2-{[4-(methyloxy)phenyl]methyl}-6-({[4-(methyloxy)phenyl]methyl}oxy)-3(2H)-pyridazinone and 5-bromo-2-{[4-(methyloxy)phenyl]methyl}-6-({[4-(methyloxy)phenyl]methyl}oxy)-3(2H)-pyridazinone

A solution of 4-methoxybenzyl alcohol (6.2ml, 50 mmol) in dry ether (120 ml) was treated dropwise with phosphorus tribromide (2.07 ml, 22 mmol), refluxed for 1 hour, cooled, washed twice with water, dried over magnesium sulfate and the solvent evaporated. The 4-methoxybenzyl bromide thus produced was added to a mixture of 4-bromo- 1,2-dihydro-3,6-pyridazinedione (for a preparation, see Example 10A(a)) (4 g, 21 mmol) and potassium carbonate (8.28 g, 60 mmol) in dry DMF (60 ml) and stirred overnight at room temperature. The mixture was diluted with ethyl acetate, washed 3 times with water, dried over magnesium sulfate and evaporated to low volume. Some solid was filtered off and washed with ethyl acetate. The filtrate was evaporated to dryness and the residue chromatographed on silica gel, eluting with 20% ethyl acetate/hexane. This gave the less polar of the 2 desired products (3.233 g), the more polar of the 2 desired products (1.626 g) and a mixture of these (1.351 g). Total yield 6.30g, 70%.
Less polar product MS (+ve ion electrospray) m/z 431 and 433 (MH⁺, 15%), 121 (100%). More polar product MS (+ve ion electrospray) m/z 431 and 433 (MH⁺, 15%), 121 (100%).

### (b) Butyl (2E)-3-[2-{[4-(methyloxy)phenyl]methyl}-6-({[4-(methyloxy)phenyl]methyl} oxy)-3-oxo-2,3-dihydro-4-pyridazinyl]-2-propenoate and butyl (2E)-3-[2-{[4-(methytoxy)phenyl]methyl}-6-({[4-(methyloxy)phenyl]methyl}oxy)-3-oxo-2,3-dihydro-5-pyridazinyl]-2-propenoate

Argon was bubbled through a mixture of 4-bromo-2-{[4-(methyloxy)phenyl] methyl}-6-({[4-(methyloxy)phenyl]methyl}oxy)-3(2*H*)-pyridazinone and 5-bromo-2-{[4-(methyloxy)phenyl]methyl}-6-({[4-(methyloxy)phenyl]methyl}oxy)-3(2*H*)-pyridazinone (1.35g, 3.14 mmol) in dry dioxan (7.5 ml) for 20 minutes. The solution was then treated with bis(tri-t-butylphosphine)palladium(0) (32 mg, 0.0628 mmol), tris(dibenzylideneacetone)dipalladium(0) (29 mg, 0.03.14 mmol), dicyclohexylmethylamine (0.74 ml, 3.45 mmol) and n-butyl acrylate (0.543 ml, 3.78 mmol), stirred under argon at room temperature for 1 hour and heated at 95°C overnight. The mixture was cooled and partitioned between ethyl acetate and water, separated, and the aqueous re-extracted with ethyl acetate. The combined organic solution was dried and evaporated and the residue chromatographed, eluting with 15% ethyl acetate/hexane to obtain the less polar product and 35% ethyl acetate/hexane for the more polar.
Less polar product (butyl (2*E*)-3-[2-{[4-(methyloxy)phenyl]methyl}-6-({[4-(methyloxy)phenyl]methyl}oxy)-3-oxo-2,3-dihydro-4-pyridazinyl]-2-propenoate) (838 mg, 55%).
MS (+ve ion electrospray) m/z 479 (MH⁺, 70%), 121 (100%).
More polar product (butyl (2*E*)-3-[2-{[4-(methyloxy)phenyl]methyl}-6-({[4-(methyloxy)phenyl]methyl}oxy)-3-oxo-2,3-dihydro-5-pyridazinyl]-2-propenoate) (580 mg, 39%).
MS (+ve ion electrospray) m/z 479 (MH⁺, 70%), 121 (100%).

### (c) Butyl 3-(2-{[4-(methyloxy)phenyl]methyl}-3,6-dioxo-1,2,3,6-tetrahydro-4-pyridazinyl)propanoate

A solution of butyl (2*E*)-3-[2-{[4-(methyloxy)phenyl]methyl}-6-({[4-(methyloxy)phenyl]methyl}oxy)-3-oxo-2,3-dihydro-4-pyridazinyl]-2-propenoate) (838 mg) in ethanol (15 ml)/dioxan (10 ml) was treated with 10% Pd/C (400 mg) and stirred under hydrogen at atmospheric pressure and room temperature for 2 hours. The catalyst was filtered off using kieselguhr and the filtrate evaporated to give the product (0.56 g, 89%).
MS (+ve ion electrospray) m/z 361 (MH⁺, 60%), 121 (100%).

### (d) 5-(3-Hydroxypropyl)-1-{[4-(methyloxy)phenyl]methyl}-1,2-dihydro-3,6-pyridazinedione

Butyl 3-(2-{[4-(methyloxy)phenyl]methyl}-3,6-dioxo-1,2,3,6-tetrahydro-4-pyridazinyl)propanoate (0.56 g, 1.56 mmol) was dissolved in dioxan and the solution evaporated to dryness, then redissolved in dry THF (30 ml). The solution, under argon, was cooled to -30°C, and treated dropwise with a 1M solution of lithium aluminium hydride in THF (1.8 ml, 1.8 mmol), allowed to warm gradually to 0°C and stirred in an ice bath for 30 minutes. 2M hydrochloric acid was added until the pH was 3 and the mixture was partitioned between water and ethyl acetate. The aqueous was re-extracted with ethyl acetate and the combined organic solution dried and evaporated. Chromatography of the residue, eluting with ethyl acetate, gave the product (300 mg, 67%).
MS (+ve ion electrospray) m/z 291 (MH⁺, 30%), 121 (100%).

### (e) 4-(3-Hydroxypropyl)-1,2-dihydro-3,6-pyridazinedione

5-(3-Hydroxypropyl)-1-{[4-(methyloxy)phenyl]methyl}-1,2-dihydro-3,6-pyridazinedione (2.734 g) was treated with anisole (10 ml) and TFA (100 ml) and stirred at 40°C overnight. The solution was cooled, evaporated to dryness and kept under high vacuum for 30 minutes. The residue was taken up in methanol (150 ml), refluxed for 12 hours, cooled and evaporated. The residue was kept 1 hour under high vacuum, triturated under ether and the solid filtered off and ether-washed. Drying under vacuum gave the product as a solid (1.48 g, 92%).
MS (+ve ion electrospray) m/z 171 (MH⁺, 100%).

### (f) 6,7-Dihydro-2H-pyrano[2,3-c]pyridazin-3(5H)-one

A suspension of 4-(3-hydroxypropyl)-1,2-dihydro-3,6-pyridazinedione (1.48 g, 8.7 mmol) in THF (105ml) was held in an ultrasound bath for 5 minutes, then cooled under argon in an ice bath. Triphenylphosphine (3.67 g, 14 mmol) was added, followed by diisopropyl azodicarboxylate (2.76 ml, 14 mmol). After 30 minutes the solvent was evaporated and the residue kept under high vacuum overnight. Chromatography, eluting first with 2.5% methanol/dichloromethane until triphenylphosphine oxide was removed and then with 5% methanol/dichloromethane, gave the product as an off-white solid (1.049 g, 79%).
MS (+ve ion electrospray) m/z 153 (MH⁺, 100%)

### (g) Butyl 3-(1-{[4-(methyloxy)phenyl]methyl}-3,6-dioxo-1,2,3,6-tetrahydro-4-pyridazinyl)propanoate

A solution of butyl (2*E*)-3-[2-{[4-(methyloxy)phenyl]methyl}-6-({[4-(methyloxy)phenyl]methyl}oxy)-3-oxo-2,3-dihydro-5-pyridazinyl]-2-propenoate) (580 mg) in ethanol (15 ml)/dioxan (5 ml) was treated with 10% Pd/C (400 mg) and stirred under hydrogen at atmospheric pressure and room temperature for 2 hours. The catalyst was filtered off using kieselguhr and the filtrate evaporated to give the product (0.43 g, 98%).
MS (+ve ion electrospray) m/z 361 (MH⁺, 50%), 121 (100%).

### (h) 4-(3-Hydroxypropyl)-1-{[4-(methyloxy)phenyl]methyl}-1,2-dihydro-3,6-pyridazinedione

Butyl 3-(1-{[4-(methyloxy)phenyl]methyl}-3,6-dioxo-1,2,3,6-tetrahydro-4-pyridazinyl)propanoate (0.43 g, 1.19 mmol) was dissolved in dioxan and the solution evaporated to dryness, then redissolved in dry THF (20 ml). The solution under argon was cooled to -30°C, treated dropwise with a 1M solution of lithium aluminium hydride in THF (1.4 ml, 1.4 mmol), allowed to warm gradually to 0°C and stirred in an ice bath for 30 minutes. 2M Hydrochloric acid was added until the pH was 3 and the mixture was partitioned between water and ethyl acetate. The aqueous was re-extracted with ethyl acetate and the combined organic solution dried and evaporated. The resulting solid was triturated under ethyl acetate, filtered off, washed with ethyl acetate and dried under vacuum to give the product (241 mg, 70%).
MS (+ve ion electrospray) m/z 291 (MH⁺, 10%), 121 (100%).

### (i) 2-{[4-(Methyloxy)phenyl]methyl}-6,7-dihydro-2H-pyrano[2,3-c]pyridazin-3(5H)-one

A suspension of 4-(3-hydroxypropyl)-1-{[4-(methyloxy)phenyl]methyl}-1,2-dihydro-3,6-pyridazinedione (2.624 g, 9.1 mmol) in THF (100 ml) was held in an ultrasound bath for 15 minutes, then cooled under argon to -10°C. Triphenylphosphine (3.57 g, 13.6 mmol) was added, followed by diisopropyl azodicarboxylate (2.68 ml, 13.6 mmol) and the mixture allowed to warm gradually to room temperature. After 1 hour the solvent was evaporated. Chromatography on silica gel, eluting first with ethyl acetate to remove byproducts and then with 10% ethanol/ethyl acetate, gave the product contaminated with a little triphenylphosphine oxide (2.55 g).
MS (+ve ion electrospray) m/z 273 (MH⁺, 50%), 121 (100%).

### (j) 6,7-Dihydro-2H-pyrano[2,3-c]pyridazin-3(5H)-one

2-{[4-(Methyloxy)phenyl]methyl}-6,7-dihydro-2*H*-pyrano[2,3-*c*]pyridazin-3(5*H*)-one (2.75 g, 10.1 mmol) was treated with anisole (10 ml) and TFA (100 ml) and heated at 70°C for 24 hours. The solution was cooled and evaporated and the residue taken up in 2.5% methanol/dichloromethane. This was applied to a silica gel column, and then elution with this solvent mixture followed by 5% methanol/dichloromethane gave the product (1.36 g, 88%).
MS (+ve ion electrospray) m/z 153 (MH⁺, 100%).

### (k) 6,7-Dihydro-5H-pyrano[2,3-c]pyridazin-3-yl trifluoromethanesulfonate

A solution of 6,7-dihydro-2*H*-pyrano[2,3-*c*]pyridazin-3(5*H*)-one (152 mg, 1 mmol) in DMF (2.5 ml) under argon was ice-cooled, treated with sodium hydride (60 mg of a 60% dispersion in oil, 1.5 mmol) and stirred for 1 hour, allowing to warm to room temperature. N-Phenyl-bis(trifluoromethanesulfonirnide) (505 mg, 1.4 mmol) was added and stirring was continued for 2 hours. The mixture was diluted with ethyl acetate, washed with saturated aqueous sodium bicarbonate solution and water (twice), dried over magnesium sulfate and evaporated. Chromatography on silica gel, eluting with 40% ethyl acetate/hexane, gave the product as a white solid (228mg, 80%).
MS (+ve ion electrospray) m/z 285 (MH⁺, 100%).

### (l) 3-Ethenyl-6,7-dihydro-5H-pyrano[2,3-c]pyridazine

Argon was bubbled for 15 minutes through a solution of 6,7-dihydro-5*H-*pyrano[2,3-*c*]pyridazin-3-yl trifluoromethanesulfonate (228 mg, 0.8 mmol) in 1,2-dimethoxyethane (6.5 ml), Tetrakis(triphenylphosphine)palladium(0) (50 mg, 0.0475 mmol) was added and the solution stirred for 20 minutes under argon. The mixture was then treated with potassium carbonate (111 mg, 0.8 mmol), water (1.9 ml) and 2,4,6-trivinylcyclotriboroxane:pyridine complex (180 mg, 0.75 mmol) (for a preparation of this reagent see F. Kerins and D. F. O'Shea, J. Org. Chem. 2002, 67, 4968-4971). After stirring for 2 hours at 80°C, the mixture was cooled and partitioned between dichloromethane and saturated aqueous sodium bicarbonate solution.The layers were separated and the aqueous fraction was extracted twice with 20% methanol/dichloromethane. The combined organic solution was dried over magnesium sulfate, evaporated and the residue chromatographed on silica gel, eluting with ethyl acetate to give product as a white solid (100 mg, 77%).
MS (+ve ion electrospray) m/z 163 (MH⁺, 100%).

### (m) 6,7-Dihydro-5H-pyrano[2,3-c]pyridazine-3-carbaldehyde

A solution of 3-ethenyl-6,7-dihydro-5*H*-pyrano[2,3-*c*]pyridazine (100 mg, 0.617 mmol) in dioxan (5.5 ml)/water (1.1 ml) was cooled in ice/water and treated with sodium periodate (306 mg, 1.43 mmol) and a 4% aqueous solution of osmium tetroxide (0.55 ml). The mixture was allowed to warm to room temperature after an hour, and after a total of 4.75 hours stirring, the solvent was evaporated. Dioxan was added and evaporated, followed by dichloromethane and the mixture briefly held in an ultrasonic bath. The whole mixture was applied to a silica gel column and eluted with ethyl acetate to give product (55 mg, 54%).
MS (+ve ion electrospray) m/z 165 (MH⁺, 100%)

### (n) Title compound

A solution of 1-[(4-amino-1-piperidinyl)methyl]-9-fluoro-1,2-dihydro-4H-pyrrolo[3,2,1-ij]quinolin-4-one (enantiomer E1) (82 mg, 0.272 mmol) and 6,7-dihydro-5*H*-pyrano[2,3-*c*]pyridazine-3-carbaldehyde (50 mg, 0.305 mmol) in chloroform/methanol (1.6 ml/ 1.6 ml) was heated with 3A molecular sieves at 65°C for 5 hours. The mixture was cooled, and treated with sodium triacetoxyborohydride (115 mg, 0.544 mmol), and stirred at room temperature overnight. It was filtered and partitioned between sodium bicarbonate and 20% methanol-DCM (x3). The organic phase was dried, evaporated and chromatographed on silica gel, eluting with DCM/methanol/0.88 ammonia (95:5:0.5) to afford a white foam (92 mg; 75%)
MS (+ve ion electrospray) m/z 450 (MH⁺, 20%), 226 (100%).
8H (CDCl₃, 400MHz) 1.35-1.50 (2H, m), 1.85-2.00 (2H,m), 2.00-2.15 (3H, m), 2.15-2.30 (1H, m), 2.45-2.65 (2H, m), 2.67 (1H, d), 2.80-2.90 (3H, m), 3.02 (1H, d), 3.95-4.15 (3H, m), 4.35-4.55 (4H, m), 6.62 (1H, d), 6.86 (1H, t), 7.30 (1H, s), 7.39 (1H, dd), 7.67 (1H, d).

The free base in DCM was converted to the dihydrochloride salt by adding an excess of 1M hydrogen chloride in ether followed by evaporation to dryness, to give a pale yellow solid (110 mg).

1-({4-[(6,7-Dihydro-5H-pyrano[2,3-*c*]pyridazin-3-ylmethyl)amino]-1-piperidinyl}methyl)-9-fluoro-1,2-dihydro-4*H*-pyrrolo[3,2,1-ij]quinolin-4-one was converted to the hydrochloride salt in a similar manner to procedures described herein.

### Example 32 9-Fluoro-1-[((3R)-3-{[([1,3]oxathiolo[5,4-c]pyridin-6-ylmethyl)amino]methyl}-1-pyrrolidinyl)methyl]-1,2-dihydro-4H-pyrrolo[3,2,1-ij]quinolin-4-one Dihydrochloride

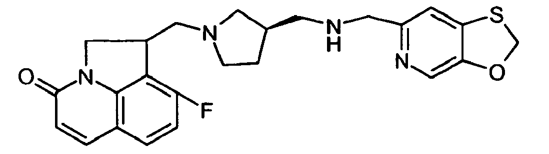

### (a) 1,1-Dimethylethyl (3R)-3-1{[(trifluoroacetyl)amino]methyl}-1-pyrrolidinecarboxylate

To a solution of 1,1-dimethylethyl (3R)-3-(aminomethyl)-1-pyrrolidinecarboxylate (2 g, 10 mmol), triethylamine (2.9 ml, 21mmol) and dimethylaminopyridine (0.13 g, 1 mmol) in DCM (100 ml) was added trifluoroacetic anhydride (1.5 ml, 10.5 mmol) under argon at room temperature. After 2 hours the mixture was treated with water (150 ml) and extracted with 10% methanol in DCM (3x100ml), dried, and the solvent evaporated. The residue was subjected to chromatography on silica gel using 0% - 20% methanol-DCM gradient to provide the desired compound (3.12g, 105%).
δH (CDCl₃, 400MHz) 1.5 (9H, s), 1.64 (2H, d), 2.04 (1H, m), 2.48 (1H, m), 3.01 (0.5H, m), 3.10 (0.5H, m), 3.20-3.60 (4H, m), 6.50 (0.5H, bs), 6.80 (0.5H, bs).

### (b) 2,2,2-Trifluoro-N-[(3S)-3-pyrrolidinylmethyl]acetamide hydrochloride

A solution of 1,1-dimethylethyl (3*R*)-3-{[(triftuoroacetyl)amino]methyl}-1-pyrrolidinecarboxylate (3.12 g,10 mmol) in DCM (50 ml) was treated slowly with a 4M solution of HCl in dioxane (25 ml). The reaction was stirred at room temperature for 3 hours. The solvent was then removed to afford a pale yellow oil (2.6 g, 112%).
δH (MeOD, 400MHz) 1.77 (1H, m), 2.18 (1H, m), 2.64 (1H, m), 2.99 (1H, m), 3.30 (1H, m), 3.70 (5H, m), 9.5 (1H, bs).

### (c) Methyl 2-[7-fluoro-2-(methyloxy)-8-quinolinyl]-3-((3R)-3-{[(trifluoroacetyl)amino]methyl}-1-pyrrolidinyl)propanoate

A solution of methyl 2-[7-fluoro-2-(methyloxy)-8-quinolinyl]-2-propenoate (2.4 g, 9.2 mmol), 2,2,2-trifluoro-*N*-[(3*S*)-3-pyrrolidinylmethyl]acetamide hydrochloride (2.4 g, 10.12 mmol) and triethylamine (3.4 ml, 23 mmol) in DMF (30 ml) was stirred and heated at 60 °C overnight. The solvent was removed *in vacuo* and the residue was subjected to chromatography on silica gel using a 0%-10% methanol-DCM gradient to give a brown oil (4.2 g, 100%).
MS (+ve ion electrospray) m/z 458 (MH+).

### (d) Methyl 3-[(3R)-3-(aminomethyl)-1-pyrrolidinyl]-2-[7-fluoro-2-(methyloxy)-8-quinolinyl]propanoate

Methyl 2-[7-fluoro-2-(methyloxy)-8-quinolinyl]-3-((3*R*)-3-{[(trifluoroacetyl)amino]methyl}-1-pyrrolidinyl)propanoate (3.4 g, 7.4 mmol) was treated with a 7% solution of potassium carbonate in 2:5 water:methanol (119 ml) for 4 hours. The solvents were then evaporated and the residue redissolved in 20% methanol in DCM. The organic phase was dried over magnesium sulphate and the solvent was removed under reduced pressure. The residue was subjected to chromatography on silica gel using a gradient of 0-20% 2M ammonia-methanol in DCM to provide the desired compound (2.2 g, 82%).
MS (+ve ion electrospray) m/z 362 (MH+).

### (e) Methyl 3-{(3R)-3-[({[(1,1-dimethylethyl)oxy]carbonyl}amino)methyl]-1-pyrrolidinyl}-2-[7-fluoro-2-(methyloxy)-8-quinolinyl]propanoate

A solution of methyl 3-[(3*R*)-3-(aminomethyl)-1-pyrrolidinyl]-2-[7-fluoro-2-(methyloxy)-8-quinolinyl]propanoate (2.2 g, 6.1 mmol) and triethylamine (0.86 ml, 6.1 mmol) in DCM (30 ml) was treated with a solution of di-tert-butyl dicarbonate (1.3 g, 6.1 mmol) in DCM at 0 °C. After stirring the mixture at room temperature for 1 hour, water (50 ml) was added and the aqueous fraction was extracted with 20% methanol in DCM (3x200 ml). The organic phase was dried and the solvent was evaporated. The residue was subjected to chromatography on silica gel using a 0-10% methanol-DCM gradient to provide the desired compound (2.56 g, 87%).
MS (+ve ion electrospray) m/z 462 (MH+).

### (f) 1,1-Dimethylethyl [((3R)-1-{2-[7-fluoro-2-(methyloxy)-8-quinolinyl]-3-hydroxypropyl}-3-pyrrolidinyl)methyl]carbamate

A solution of methyl3-{(3*R*)-3-[({[(1,1-dimethylethyl)oxy]carbonyl}amino) methyl]-1-pyrrolidinyl}-2-[7-fluoro-2-(methyloxy)-8-quinolinyl]propanoate (2.56 g, 5.55 mol) in dry tetrahydrofuran (60 ml) at -78°C under argon was treated with a solution of lithium aluminium hydride in tetrahydrofuran (1 M, 7.2 ml, 7.2 mmol) and then slowly allowed to warm to room temperature. After 0.5 hour, water (0.5 ml) was added followed by aqueous sodium hydroxide solution (2 M, 0.9 ml) and water (1 ml). The mixture was stirred at ambient temperature for 1 hour. It was filtered and evaporated, and the residue was subjected to chromatography on silica gel using a 0-20% methanol-DCM gradient to provide the desired compound (1.88 g, 78%).
MS (+ve ion electrospray) m/z 434 (MH+).

### (g) 1,1-Dimethylethyl ({(3R)-1-[(9-fluoro-4-oxo-1,2-dihydro-4H-pyrrolo[3,2,1-ij]quinolin-1-yl)methyl]-3-pyrrolidinyl}methyl)carbamate

A solution of 1,1-dimethylethyl [((3*R*)-1-{2-[7-fluoro-2-(methyloxy)-8-quinolinyl]-3-hydroxypropyl}-3-pyrrolidinyl)methyl]carbamate (1.88 g, 4.4 mmol) in chloroform (20 ml) was treated with diisopropylethylamine (1.2 ml, 7.04 mmol) and methanesulphonyl chloride (0.45 ml, 5.5 mmol) at 0°C under argon. The mixture was stirred at 0°C for 0.5 hour, warmed to rt and stirred for 1 hour then heated at 45°C overnight, and allowed to cool to room temperature. The mixture was diluted with DCM and washed with sodium bicarbonate solution. The aqueous phase was extracted with 10% methanol in DCM (3 x 80 ml). The organic phase was dried and the solvent evaporated. The residue was subjected to chromatography on silica gel using a 0-10% methanol-DCM gradient to provide the desired compound (1.47 g, 84%).
MS (+ve ion electrospray) m/z 402 (MH+).

### (h) 1-{[(3R)-3-(Aminomethyl)-1-pyrrolidinyl]methyl}-9-fluoro-1,2-dihydro-4H-pyrrolo[3,2,1-ij]quinolin-4-one

1,1-Dimethylethyl({(3*R*)-1-[(9-fluoro-4-oxo-1,2-dihydro-4*H*-pyrrolo[3,2,1-*ij*]quinolin-1-yl)methyl]-3-pyrrolidinyl}methyl)carbamate (1.47 g, 3.7 mmol) was dissolved in dichloromethane (15 ml) and trifluoroacetic acid (15 ml) and stirred at room temperature for 30 minutes, then evaporated to dryness. The residue was redissolved in methanol and stirred with excess Amberlyst® A21 ion-exchange resin (Aldrich: a weakly basic, macroreticular resin with alkyl amine functionality) for 1 hour and then filtered. The solvent was removed under reduced pressure and the residue was subjected to chromatography on silica gel using a 0-20% 2M ammonia in methanol-DCM gradient to provide the desired compound (0.75 g, 68%)
MS (+ve ion electrospray) m/z 302 (MH+).

### (i) Title compound

A solution of 1-{[(3*R*)-3-(aminomethyl)-1-pyrrolidinyl]methyl}-9-fluoro-1,2-dihydro-4*H*-pyrrolo[3,2,1-*ij*]quinolin-4-one (100 mg) and [1,3]oxathiolo[5,4-c]pyridine-6-carbaldehyde (for a synthesis, see WO2004058144, Example 61) (55 mg) in methanol (4 ml) and chloroform (4 ml) was stirred at room temperature for 2 hours. Sodium triacetoxyborohydride (210 mg) was added and the reaction was stirred at room temperature. The solvent was evaporated and the residue was subjected to chromatography on silica gel using a 0-20% methanol-DCM gradient to provide the desired compound (137 mg) as an acetate salt.
MS (+ve ion electrospray) m/z 453 (MH+).
δH (CDCl₃, 400MHz) 1.53 (1H, m), 2.00 (2H, m), 2.30-3.00 (8H, m), 3.80-4.10 (3H, m), 4.50 (2H, m), 4.88 (2H, bs), 5.75 (2H, s), 6.61 (1H, d), 6.86 (1H, t), 7.19 (1H, s), 7.38 (1H, m), 7.66 (1H, d), 8.03 (1H, d).

The acetate salt in DCM, was converted to the dihydrochloride salt by adding an excess of 4M hydrogen chloride in dioxan, followed by evaporation to dryness, and trituration with ether to give a solid.

### Example 33 1-{[(3R)3-({[(7-Chloro-3-oxo-3,4-dihydro-2H-pyrido[3,2-b][1,4]oxazin-6-yl)methyl]amino}methyl)-1-pyrrolidinyl]methyl}-9-fluoro-1,2-dihydro-4H-pyrrolo[3,2,1-ij]quinolin-4-one Dihydrochloride

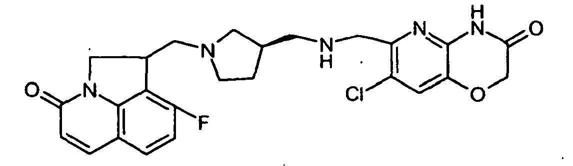

This was prepared from 1-{[(3*R*)-3-(aminomethyl)-1-pyrrolidinyl]methyl}-9-fluoro-1,2-dihydro-4*H*-pyrrolo[3,2,1-*ij*]quinolin-4-one (50 mg) and 7-chloro-3-oxo-3,4-dihydro-2*H*-pyrido[3,2-*b*][1,4]oxazine-6-carboxaldehyde (for a synthesis, see WO2003064421 Example 15(c)) (35.2 mg) by the general method of Example 32(i). The product was chromatographed on silica gel using a 0-15% methanol-DCM gradient to provide the desired compound (66 mg) as an acetate salt.
MS (+ve ion electrospray) m/z 498 (MH+).
δH (CDCl₃, 100MHz) 1.55 (1H, m), 2.00 (2H, m), 2.30-3.00 (8H, m), 4.00 (3H, m), 4.50 (2H, m), 4.6 (2H, s), 5.79 (2H, bs), 6.62 (1H, d), 6.85 (1H, m), 7.23 (1H, s), 7.38 (1H, m), 7.66 (1H, m).

The acetate salt in DCM, was converted to the dihydrochloride salt by adding an excess of 4M hydrogen chloride in dioxan, followed by evaporation to dryness, and trituration with ether to give a solid.

### Example 34 9-Fluoro-1-{[(3R)-3-({[(3-oxo-3,4-dihydro-2H-pyrido[3,2-b][1,4]thiazin-6-yl)methyl]amino}methyl)1-pyrrolidinyl]methyl}-1,2-dihydro-4H-pyrrolo[3,2,1-ij]quinolin-4-one Dihydrochloride

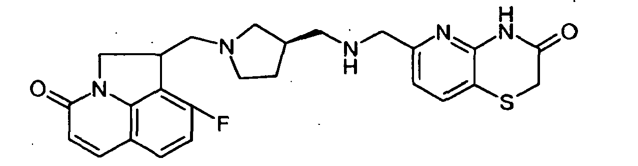

This was prepared from 1-{[(3*R*)-3-(aminomethyl)-1-pyrrolidinyl]methyl}-9-fluoro-1,2-dihydro-4*H*-pyrrolo[3,2,1-*ij*]quinolin-4-one (100 mg) and 3-oxo-3,4-dihydro-2*H*-pyrido[3,2-*b*][1,4]thiazine-6-carboxaldehyde (for a synthesis, see WO 2004058144A2 Example 7(d)) (64.5 mg) by the general method of Example 32(i). The product was chromatographed on silica gel using a 0-20% methanol-DCM gradient to provide the desired compound (127 mg) as an acetate salt.
MS (+ve ion electrospray) m/z 480 (MH+).
δH (CDCl₃, 400MHz) 1.53 (1H, m), 2.00 (2H, m), 2.30-3.00 (8H, m), 3.15 (1H, bs), 3.86 (2H, s), 4.00 ( 1H, m), 4.49 (2H, m), 6.63 (1H, m), 6.87 (1H, m), 6.97 (1H, d), 7.39 (1H, m), 7.59 (1H, d), 7.67 (1H, d).

The acetate salt in DCM, was converted to the dihydrochloride salt by adding an excess of 4M hydrogen chloride in dioxan, followed by evaporation to dryness, and trituration with ether to give a solid.

### Example 35 9-Fluoro-1-{[(3R)-3-({[(3-oxo-3,4-dihydro-2H-pyrido[3,2-b][1,4]oxazin-6-yl)methyl]amino}methyl)-1-pyrrolidinyl]methyl}-1,2-dihydro-4H-pyrrolo[3,2,1-ij]quinolin-4-one Dihydrochloride

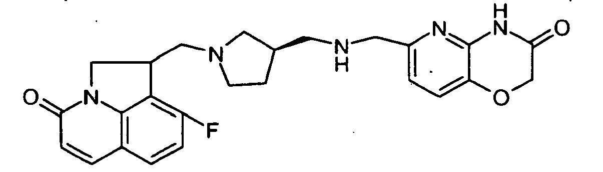

This was prepared from 1-{[(3*R*)-3-(aminomethyl)-1-pyrrolidinyl-1-methyl}-9-fluoro-1,2-dihydro-4*H*-pyrrolo[3,2,1-*ij*]quinolin-4-one (50 mg) and 3-oxo-3,4-dihydro-2*H*-pyrido[3,2-*b*][1,4]oxazine-6-carboxaldehyde (for a synthesis, see WO2004058144 Example 1) (30 mg) by the general method of Example 32(i). The product was chromatographed on silica gel using a 0-20% methanol-DCM gradient to provide the desired compound (63 mg) as an acetate salt.
MS (+ve ion electrospray) m/z 464 (MH+).

The acetate salt in DCM, was converted to the dihydrochloride salt by adding an excess of 4M hydrogen chloride in dioxan, followed by evaporation to dryness, and trituration with ether to give a solid.

### Example 36 1-[((3R)-3-{[(2,3-Dihydro[1,4]dioxino[2,3-c]pyridin-7-ylmethyl)amino]methyl}-1-pyrrolidinyl)methyl]-9-fluoro-1,2-dihydro-4H-pyrrolo[3,2,1-ij]quinolin4-one Dihydrochloride

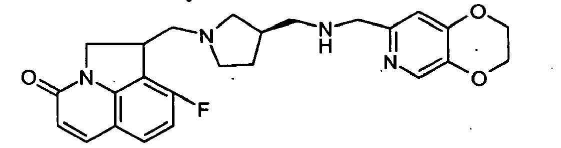

This was prepared from 1-{[(3*R*)-3-(aminomethyl)-1-pyrrolidinyl]methyl}-9-fluoro-1,2-dihydro-4*H*-pyrrolo[3,2,1-*ij*]quinolin-4-one (100 mg) and 2,3-dihydro[1,4]dioxino[2,3-*c*]pyridine-7-carbaldehyde (for a synthesis, see WO2003087098 Example 20(e)) (54.8 mg) by the general method of Example 32(i). The product was chromatographed on silica gel using a 0-20% methanol-DCM gradient to provide the desired compound (140 mg) as an acetate salt.
MS (+ve ion electrospray) m/z 451 (MH+).

The acetate salt in DCM, was converted to the dihydrochloride salt by adding an excess of 4M hydrogen chloride in dioxan, followed by evaporation to dryness, and trituration with ether to give a solid.

### Example 37 9-Fluoro-1-[(3-{[([1,3]oxathiolo[5,4-c]pyridin-6-ylmethyl)amino]methyl}-1-pyrrolidinyl)methyl]-1,2-dihydro-4H-pyrrolo[3,2,1-ij]quinolin-4-one Dihydrochloride

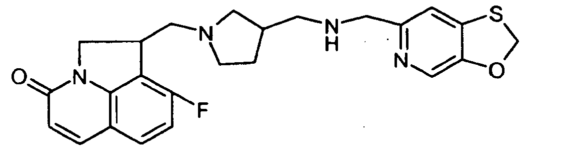

This was prepared from 1-{[3-(aminomethyl)-1-pyrrolidinyl]methyl}-9-fluoro-1,2-dihydro-4*H*-pyrrolo[3,2,1-*ij*]quinolin-4-one (prepared from 1,1-dimethylethyl 3-(aminomethyl)-1-pyrrolidinecarboxylate by the general method described for the (R)-enantiomer in Example 32) (100 mg) and [1,3]oxathiolo[5,4-*c*]pyridine-6-carbaldehyde (for a synthesis, see WO2004058144, Example 61) (55 mg) by the general method of Example 32(i). The product was chromatographed on silica gel using a 0-20% methanol-DCM gradient to provide the desired compound (110 mg) as an acetate salt.
MS (+ve ion electrospray) m/z 453 (MH+).

The acetate salt in DCM, was converted to the dihydrochloride salt by adding an excess of 4M hydrogen chloride in dioxan, followed by evaporation to dryness, and trituration with ether to give a solid.

### Example 38A 1-{[3-({[(7-Chloro-3-oxo-3,4-dihydro-2H-pyrido[3,2-b][1,4]oxazin-6-yl)methyl]amino}methyl)-1-pyrrolidinyl]methyl}-9-fluoro-1,2-dihydro-4H-pyrrolo[3,2,1-ij]quinolin-4-one Dihydrochloride

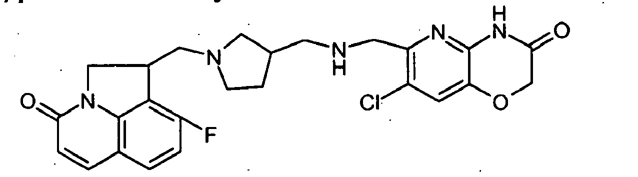

This was prepared from 1-{[3-(aminomethyl)-1-pyrrolidinyl]methyl}-9-fluoro-1,2-dihydro-4*H*-pyrrolo[3,2,1-*ij*]quinolin-4-one (100 mg) and 7-chloro-3-oxo-3,4-dihydro-2*H*-pyrido[3,2-*b*][1,4]oxazine-6-carboxaldehyde (for a synthesis, see WO2003064421 Example 15(c)) (58 mg) by the general method of Example 32(i). The product was chromatographed on silica gel using a 0-20% methanol-DCM gradient to provide the desired compound (105 mg) as an acetate salt.
MS (+ve ion electrospray) m/z 498 (MH+).

The acetate salt in DCM, was converted to the dihydrochloride salt by adding an excess of 4M hydrogen chloride in dioxan, followed by evaporation to dryness, and trituration with ether to give a solid.

### Example 38B 1-{[3-({[(7-Chloro-3-oxo-3,4-dihydro-2H-pyrido[3,2-b][1,4]oxazin-6-yl)methyl]amino}methyl)-1-pyrrolidinyl]methyl}-9-fluoro-1,2-dihydro-4H-pyrrolo[3,2,1-ij]quinolin-4-one Isomers 1, 2, 3 and 4, Hydrochloride

1 - {[3-({[(7-Chloro-3-oxo-3,4-dihydro-2*H*-pyrido[3,2-*b*][1,4]oxazin-6-yl)methyl]amino}methyl)-1-pyrrolidinyl]methyl}-9-fluoro-1,2-dihydro-4*H*-pyrrolo[3,2,1-*ij*]quinolin-4-one dihydrochloride (40 mg) was subjected to preparative chiral hplc purification on a 5um Chiralpak AD-H column eluting with 80:20:0.1 acetonitrile:methanol:isopropylamine affording Isomer 1 (6.0 mg), Isomer 2 (10.0 mg), Isomer 3 (9.0 mg) and Isomer 4 (9.2 mg), all with >99.5% purity. These free bases were then converted to the hydrochloride salts.

### Example 39 1-[(4-{[(3-Oxo-3,4-dihydro-2H-pyrido[3,2-b][1,4]thiazin-6-yl)methyl]amino}-1-piperidinyl)methyl]-1,2-dihydro-4H-pyrrolo[3,2,1-ij]quinolin-4-one Hydrochloride

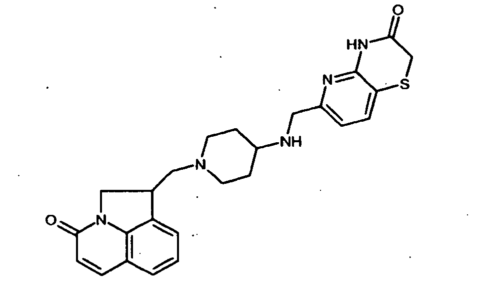

### (a) (2E)-N-(2-Bromophenyl)-3-phenyl-2-propenamide

To a solution of 2-bromoaniline (22.27 g, 0.13 mol) and potassium carbonate (26.8g, 0.13 mol) in acetone (50 ml) and water (65 ml) at 0°C was added cinnamoyl chloride (21.57 g, 0.13 mol) portionwise over 15 minutes. Another 150 ml of both acetone and water was then added to facilitate stirring. The reaction was stirred for 2 hours at 0°C before being added to ice water (400 ml). The resultant solid was filtered, washed with water (500 ml) and dried *in vacuo.* The resultant solid was triturated with hot hexane and dried *in vacuo* to provide the desired compound as a white solid (29.50 g, 75%).
MS (ES+) m/z 303 (MH⁺, 100%).

### (b) 8-Bromo-2(1H)-quinolinone

To a suspension of (2*E*)-*N*-(2-bromophenyl)-3-phenyl-2-propenamide (22.9 g, 76.0 mol) in chlorobenzene (100 ml) under an argon atmosphere at room temperature was added aluminium trichloride (60.78 g, 133.34 mmol). The reaction was heated for 2 hours at 125°C after which time the reaction mixture was cooled to 50°C before being carefully added to ice water (3 L). The resultant solid was filtered and then washed with water (500 ml), then triturated with hot ethanol, filtered and dried *in vacuo* to provide the desired compound as a white solid (7.39 g, 75%).
MS (ES+) m/z 225 (MH+, 100%).

### (c) 8-Bromo-2-(methyloxy)quinoline

To a suspension of 8-bromo-2(1*H*)-quinolinone (2.76 g, 12.32 mmol) in N,N-dimethylformamide (40 ml) under an argon atmosphere at 0°C was added potassium carbonate (3.4 g, 24.63 mmol). The reaction was then stirred for 15 minutes before methyl iodide (0.91 ml, 14.78 mmol) was added. The reaction was allowed to warm to room temperature and then stirred for 3 hours. The reaction mixture was then evaporated and the residue treated with dichloromethane and water. The aqueous fraction was re-extracted with dichloromethane. The combined organic fractions were then dried (MgSO₄), the solvent was removed under reduced pressure and then the residue was subjected to chromatography on silica gel using a methanol-dichloromethane gradient. This provided the desired compound as a yellow solid (2.16 g, 74%).
MS (ES+) m/z 239 (MH⁺, 100%).

### (d) [2-(Methyloxy)-8-quinolinyl]boronic acid

According to the literature procedure (Li, W.; Nelson, D.; Jensen, M.; Hoerrner, R.; Cai, D.; Larsen, R.; Reider, P J. Org. Chem. (2002), 67(15), 5394) a solution of 8-bromo-2-(methyloxy)quinoline (1.95 g, 8.19 mmol) and triisopropylborate (2.30 ml, 9.83 mmol) in toluene (20 ml) and tetrahydrofuran (5 ml) under an argon atmosphere was cooled to -78°C. A solution of n-butyl lithium (2.5M in hexanes, 3.9 ml, 9.83 mmol) was then added dropwise over 20 minutes. The reaction was stirred at -78°C for 2 hours and then warmed to -20°C. The reaction was then quenched with 2M HCl solution (10ml) and treated with dichloromethane. The aqueous fraction was re-extracted with dichloromethane. The combined organic fractions were then dried (MgSO₄) and the solvent removed under reduced pressure. The residue was triturated with hexane to give the desired compound as a yellow solid (453 mg, 40%).
MS (ES+) m/z 204 (MH⁺, 100%).

### (e) Methyl 2-[2-(Methyloxy)-8-quinolinyl]-2-propenoate

To a solution of methyl 2-bromo-2-propenoate (452 mg, 2.74 mmol) (for a synthesis see Rachon, J.; Goedken, V.; Walborsky, H. J. Org. Chem. (1989), 54(5), 1006) in degassed tetrahydrofuran (10 ml) under an argon atmosphere was added [2-(methyloxy)-8-quinolinyl]boronic acid (506 mg, 2.49 mmol), bis(tri-*t-*butylphosphine)palladium (0) (25 mg, 0.05 mmol), bis(dibenzylideneacetone)palladium(0) (23 mg, 0.025 mmol) and potassium fluoride (477 mg, 8.217 mmol). The reaction was heated at 70°C for 24 hours and then treated with water and dichloromethane. The aqueous fraction was re-extracted with dichloromethane. The combined organic fractions were then dried (MgSO₄) and the solvent removed under reduced pressure. The residue was subjected to chromatography on silica gel using a ethyl acetate-hexane gradient. This provided the desired compound as a yellow solid (381 mg, 63%).
MS (ES+) m/z 244 (MH⁺, 100%), 212 (80%).

### (f) Methyl 3-[4-({[(1,1-dimethylethyl)oxy]carbonyl}amino)-1-piperidinyl]-2-[2-(mehyloxy)-8-quinolinyl]propanoate

To a solution of methyl 2-[2-(methyloxy)-8-quinolinyl]-2-propenoate (381 mg, 1.57 mmol) in N,N'-dimethylformamide (5 ml) and tetramethylguanidine (0.05 ml) was added 1,1-dimethylethyl 4-piperidinylcarbamate (345 mg, 1.73 mmol). The reaction mixture was stirred for 12 hour at 60°C after which time the solvent was removed under reduced pressure. The residue was subjected to chromatography on silica gel using a methanol-dichloromethane gradient. This provided the desired compound as a yellow solid (546 mg, 79%).
MS (ES+) m/z 444 (MH⁺, 100%).

### (g) 1,1-Dimethylcthyl (1-{3-hydroxy-2-[2-(methyloxy)-8-quinolinyl]propyl}-4-piperidinyl)carbamate

To a solution of methyl 3-[4-({[(1,1-dimethylethyloxy]carbonyl}amino)-1-piperidinyl]-2-[2-(methyloxy)-8-quinolinyl]propanoate (546 mg, 1.23 mmol) in tetrahydrofuran (20 ml) at -78°C was added lithium aluminium hydride (1M in tetrahydrofuran, 1.50 ml, 1.48 mmol). The reaction was then stirred at -78°C for 0.5 hours before water (0.2 ml) and then 2M NaOH solution (0.4 ml) was added and the mixture warmed to 25°C: The mixture was then filtered, dried (MgSO₄) and the solvent was removed under reduced pressure. The residue was subjected to chromatography on silica gel using a methanol-dichloromethane gradient. This provided the desired compound as a white solid (370mg, 72%).
MS (ES+) m/z 416 (MH⁺, 100%).

### (h) 1,1-Dimethylethyl {1-[(4-oxo-1,2-dihydro-4H-pyrrolo[3,2,1-ij]quinolin-1-yl)methyl]-4-piperidinyl}carbamate

To a solution of 1,1-dimethylethyl (1-{3-hydroxy-2-[2-(methyloxy)-8, quinolinyl]propyl}-4-piperidinyl)carbamate (370 mg, 0.892 mmol) in chloroform (20 ml) at 0°C was added diisopropylethylamine (0.33 ml, 1.96 mmol) and methanesulfonic anhydride (0.186 g, 1.07 mmol). The reaction was then heated at 70°C for 5 hours and then treated with dichloromethane and water. The aqueous phase was extracted twice with dichloromethane and the combined organic phases were dried (MgSO₄) and the solvent was removed under reduced pressure. The residue was subjected to chromatography on silica gel using a methanol-dichloromethane gradient to provide the desired compound (0.276 g, 81%).
MS (ES+) m/z 384 (MH⁺, 10%), 284 (100%).

### (i) 1-[(4-Amino-1-piperidinyl)methyl]-1,2-dihydro-4H-pyrrolo[3,2,1-ij]quinolin-4-one dihydrochloride

A solution of 1,1-dimethylethyl {1-[(4-oxo-1,2-dihydro4*H*-pyrrolo[3,2,1-ij]quinolin-1-yl)methyl]-4-piperidinyl} carbamate (276 mg, 0.721 mmol) in chloroform (5 ml) and MeOH (5 ml) was treated with 4M HCl in dioxane (10 ml) and stirred at room temperature for 2 hours. The reaction mixture was evaporated to provide the desired compound (0.283 g, 110%) as the slightly impure dihydrochloride salt which was used without further purification.
MS (ES+) m/z 306 (M +Na, 10%), 284 (MH+, 100%).

### (j) Title compound

To a solution of the dihydrochloride salt of 1-[(4-amino-1-piperidinyl)methyl]-1,2-dihydro-4*H*-pyrrolo[3,2,1-*ij*]quinolin-4-one (32 mg, 0.089mmol) in methanol (0.1 ml) and dichloromethane (1 ml) was added triethylamine (24 µl, 0.178 mmol) and 3-oxo-3,4-dihydro-2*H*-pyrido[3,2-*b*][1,4]thiazine-6-carboxaldehyde (for a synthesis, see WO2003087098, Example 301 (d)) (17 mg, 0.089 mmol). This mixture was stirred for 1 hour at room temperature before sodium triacetoxyborohydride (57mg, 0.178mmol) was added and the reaction stirred for a further 1 hour. The solvent was removed under reduced pressure. The residue was subjected to chromatography on silica gel using a methanol-dichloromethane gradient. This provided the title compound as a yellow solid (39 mg, 95%).
MS (ES+) m/z 462 (MH⁺, 100%).
δH (CDCl₃, 400MHz) 1.66-1.75 (2H, m), 2.03-2.22 (3H, m), 2.55 (1H, dd), 2.79 (1H, dd), 2.81-2.89 (1H, m), 2.96-3.11 (3H, m), 3.47 (2H, s), 3.84-3.89 (1H, m), 3.91 (2H, s), 4.28 (1H, dd), 4.50 (1H, dd), 6.69 (1H, d), 7.01 (1H, d), 7.16 (1H, t), 7.41 (2H, d,), 7.50 (1H, br s), 7.60 (1H, d), 7.72 (1H, d).

This material was converted to the hydrochloride by dissolving in dichloromethane/methanol and adding 1 equivalent of 1M HCl/diethyl ether then evaporating to dryness.

### Example 40 1-({4-[(2,3-Dihydro[1,4]dioxino[2,3-c]pyridin-7-ylmethyl)amino]-1-piperidinyl}methyl)-1,2-dihydro-4H-pyrrolo[3,2,1-ij]quinolin-4-one Hydrochloride

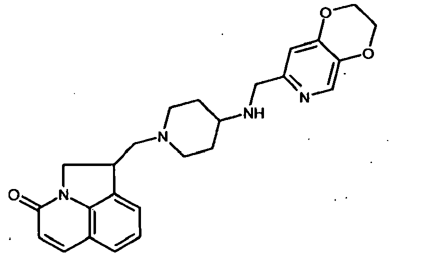

The title compound was synthesised from the dihydrochloride salt of 1-[(4-amino-1-piperidinyl)methyl]-1,2-dihydro-4H-pyrrolo[3,2,1-ij]quinolin-4-one (58 mg, 0.163 mmol) and 2,3-dihydro[1,4]dioxino[2,3-c]pyridine-7-carboxaldehyde (24 mg, 0.148 mmol) (for a synthesis, see WO2004058144, Example 2(c)) by the general method of Example 39(j), to give the desired compound (69 mg, 98% yield).
MS (ES+) m/z 433 (MH⁺, 100%), 284 (30%)
δH (CDCl₃, 400MHz) 1.64-1.75 (2H, m), 2.02-2.18 (3H, m), 2.56 (1H, dd), 2.73 (1H, dd), 2.78-2.84 (1H, m), 2.96-3.11 (4H, m), 3.85-3.95 (1H, m), 4.02 (2H, s), 4.25-4.35 (4H, m), 4.47-4.53 (1H, m), 6.68 (1H, d), 6.94 (1H, s), 7.16 (1H, t), 7.41-7.44 (2H, m), 7.97 (1H, d), 8.10 (1H, s)

This material was converted to the hydrochloride salt by dissolving in dichloromethane/methanol and adding I equivalent of 1M HCl/diethyl ether then evaporating to dryness.

### Example 41 1-({4-[([1,3]Oxathiolo[5,4-c]pyridin-6-ylmethyl)amino]-1-piperidinyl}methyl)-1,2-dihydro-4H-pyrrolo[3,2,1-ij]quinolin-4-one Hydrochloride

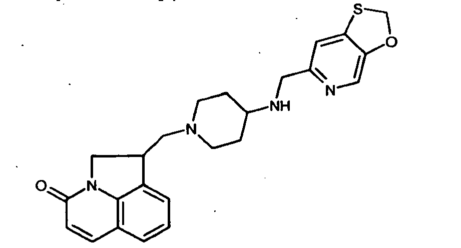

The title compound was synthesised from the dihydrochloride salt of 1-[(4-amino-1-piperidinyl)methyl]-1,2-dihydro-4*H*-pyrrolo[3,2,1-ij]quinolin-4-one and [1,3]oxathiolo[5,4-c]pyridine-6-carbaldehyde (for a synthesis, see WO2004058144, Example 61) according to the general method of Example 39(j), in 76% yield.
MS (ES+) m/z 435 (MH⁺, 100%), 284 (40%)
δH (CDCl₃, 400MHz) 1.64-1.73 (2H, m), 2.00-2.03 (2H, m), 2.11-2.21 (2H, m), 2.55 (1H, dd), 2.70-2.82 (2H, m), 2.96-3.11 (2H, m), 3.85-3.89 (1H, m), 4.00 (2H, s), 4.29 (1H, dd), 4.52 (1H, dd), 5.76 (2H, s), 6.69 (1H, d), 7.16 (1H, t), 7.29 (1H, s), 7.41-7.43 (2H, m), 7.71 (1H, d), 8.00 (1H, s)

This material was converted to the hydrochloride by dissolving in dichloromethane/methanol and adding 1 equivalent of 1M HCl/diethyl ether then evaporating to dryness.

### Example 42 1-[(4-{[(7-Chloro-3-oxo-3,4-dihydro-2H-pyrido[3,2-b] [1,4]oxazin-6-yl)methyl]amino}-1-piperidinyl)methyl]-1,2-dihydro-4H-pyrrolo[3,2,1-ij]quinolin4-one Hydrochloride

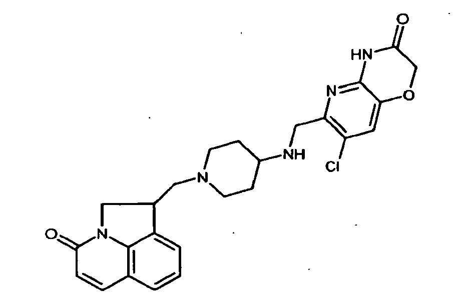

The title compound was synthesised from the dihydrochloride salt of 1-[(4-amino-1-piperidinyl)methyl]-1,2-dihydro-4*H*-pyrrolo[3,2,1-*ij*]quinolin-4-one and 7-chloro-3-oxo-3,4-dihydro-2*H*-pyrido[3,2-*b*] [1,4]oxazine-6-carboxaldehyde (for a synthesis, see WO2003064421, Example 15(c)) by the general method of Example 39(j), in 96% yield. MS (ES+) m/z 479 (MH⁺, 100%)
δH (CDCl₃, 400MHz) 1.88-2.27 (5H, m), 2.57 (1H, dd), 2.78 (1H, dd), 3.01-3.14 (4H, m), 3.85-3.92 (1H, m), 4.19 (2H, s), 4.30 (1H, dd), 4.51 (1H, dd), 4.59 (2H, s), 6.67 (1H, d), 7.16 (1H, t), 7.24 (1H, s), 7.40-7.43 (2H, m), 7.71 (1H, d)

This material was converted to the hydrochloride by dissolving in dichloromethane/methanol and adding 1 equivalent of 1M HCl/diethyl ether then evaporating to dryness.

### Example 43 1-({4-[(3,4-Dihydro-2H-pyrano[2,3-c]pyridin-6-ylmethyl)amino]-1-piperidinyl}methyl)-1,2-dihydro-4H-pyrrolo[3,2,1-ij][quinolin-4-one Hydrochloride

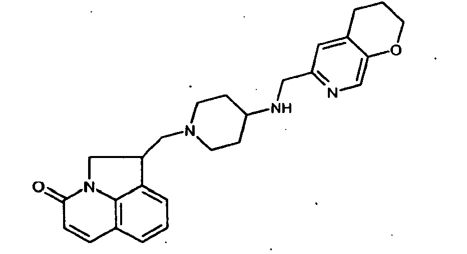

The title compound was synthesised from the dihydrochloride salt of 1-[(4-amino-1-piperidinyl)methyl]-1,2-dihydro-4*H*-pyrrolo[3,2,1-*ij*]quinolin-4-one and 3,4-dihydro-2H-pyrano[2,3-c]pyridine-6-carbaldehyde (for a synthesis, see W02004058144, Example 126(e)) according to the general method of Example 39(j), in 100% yield.
MS (ES+) m/z 431 (MH⁺, 100%)
δH (CDCl₃, 400MHz) 1.85-1.88 (2H, m), 1.90-2.21 (4H, m), 2.55 (1H, dd), 2.72 (1H, d), 2.80 (2H, t), 2.94-3.15 (5H, m), 3.84-3.87 (1H, m), 4.02-4.29 (5H, m), 4.46 (1H, dd), 6.67 (1H, d), 7.16 (1H, t), 7.32 (1H, s), 7.41-7.44 (2H, m), 7.72 (1H, d), 8.06 (1H, s)

This,material was converted to the hydrochloride by dissolving in dichloromethane/methanol and adding 1 equivalent of 1 M HCl/diethyl ether then evaporating to dryness.

### Example 44 1-[(3-{[(2,3-Dihydro[1,4]dioxino[2,3-c]pyridin-7-ylmethyl)amino]methyl}-1-pyrrolidinyl)methyl]-9-fluoro-1,2-dihydro-4H-pyrrolo[3,2,1-ij]quinolin4-one Dihydrochloride

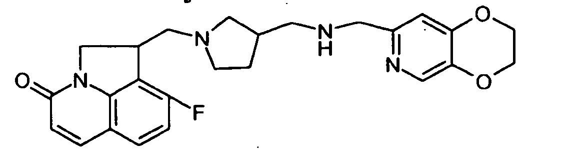

This was prepared from 1-{[3-(aminomethyl)-1-pyrrolidinyl]methyl]-9-fluoro-1,2-dihydro-4*H*-pyrrolo[3,2,1-*ij*]quinolin-4-one and 2,3-dihydro[1,4]dioxino[2,3-c]pyridine-7-carbaldehyde (for a synthesis, see WO2003087098 Example 20(e)) by the general method of Example 36.

### Example 45 1-({4-[(6,7-Dihydro[1,4]oxathiino[2,3-c]pyridazin-3-ylmethyl)amino]-1-piperidinyl}methyl)-9-fluoro-1-hydroxy-1,2-dihydro-4H-pyrrolo[3,2,1-ij]quinolin-4-one Enantiomer E2 Dihydrochloride

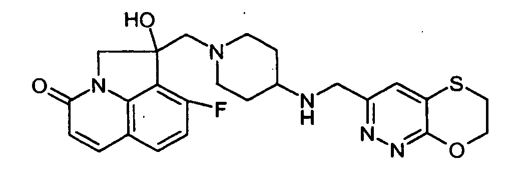

This was prepared from 1-[(4-amino-1-piperidinyl)methyl]-9-fluoro-1-hydroxy-1,2-dihydro-4*H*-pyrrolo[3,2,1-*ij*]quinolin-4-one enantiomer E2 and 6,7-dihydro[1,4]oxathiino[2,3-*c*]pyridazine-3-carbaldehyde by the general method of Example 29.

### Example 46 9-Fluoro-1-{[3-({[(3-oxo-3,4-dihydro-2H-pyrido[3,2-b] [1,4]thiazin-6-yl)methyl]amino}methyl)-1-pyrrolidinyl]methyl}-1,2-dihydro-4H-pyrrolo[3,2,1-ij]quinolin-4-one Dihydrochloride

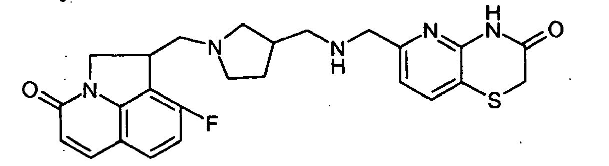

This was prepared from 1-{[3-(aminomethyl)-1-pyrrolidinyl]methyl}-9-fluoro-1,2-dihydro-4*H*-pyrrolo[3,2,1-*ij*]quinolin-4-one and 3-oxo-3,4-dihydro-2*H*-pyrido[3,2-*b*][1,4]thiazine-6-carbaldehyde (for a synthesis, see WO2004058144A2 Example 7(d)) by the general method of Example 34.

### Example 47 1-({4-[(6,7-Dihydro[1,4]dioxino[2,3-c]pyridazin-3-ylmethyl)amino]-1-piperidinyl}methyl)-4-oxo-1,2-dihydro-4H-pyrrolo[3,2,1-ij]quinoline-9-carbonitrile (Enantiomer E1) hydrochloride

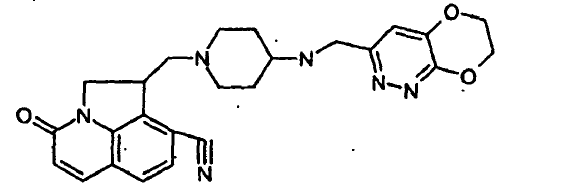

### (a) Methyl 2-[7-bromo-2-(methyloxy)-8-quinolinyl]-3-[4-({[(1,1-dimethylethyl)oxy]carbonyl}amino)-1-piperidinyl]propanoate

A solution of methyl 2-[7-bromo-2-(methyloxy)-8-quinolinyl]-2-propenoate (12.4g, 38.5 mmol), 1,1-dimethylethyl 4-piperidinylcarbamate (8.5g, 42.3 mmol) and 1,1,3,3-tetramethylguanidine (10 drops) in dry DMF (120 mL) was heated at 70°C for 3 days. More 1,1-dimethylethyl 4-piperidinylcarbamate (1.5g) was added and the mixture heated at 100°C for a further day. The mixture was evaporated and the residue chromatographed on silica eluting with 2% methanol in dichloromethane affording a pale yellow solid (17.1 g, 85%).
MS (+ve ion electrospray) m/z 523 (MH+).

### (b) 1,1-Dimethylethyl (1-{2-[7-bromo-2-(methyloxy)-8-quinolinyl]-3-hydroxypropyl}-4-piperidinyl)carbamate

A solution of methyl 2-[7-bromo-2-(methyloxy)-8-quinolinyl]-3-[4-({[(1,1-dimethylethyl)oxy]carbonyl}amino)-1-piperidinyl]propanoate (17g, 32.5 mmol) in THF (300 mL) at -78 °C under argon was treated with a solution of lithium aluminium hydride in THF (1M, 39 mL, 39 mmol). The reaction was stirred at -78 °C for I hour then allowed to stir at room temperature for 2 hours. Water (18 mL) was added followed by aqueous sodium hydroxide solution (2M, 40 mL) and more water (20 mL). Filtration and evaporation afforded a solid. This was chromatographed eluting with 0-20% methanol in dichloromethane affording a yellow solid (9.9g, 61%).
MS (+ve ion electrospray) m/z 495 (MH+).

### (c) 1,1-Dimethylethyl {1-[(9-bromo-4-oxo-1,2-dihydro-4H-pyrrolo[3,2,1-ij]quinolin-1-yl)methyl]-4-piperidinyl}carbamate

A solution of 1,1-dimethylethyl (1-{2-[7-bromo-2-(methyloxy)-8-quinolinyl]-3-hydroxypnopyl}-4-piperidinyl)carbamate (9.9g, 20 mmol), methanesulphonic anhydride (4.2g, 24 mmol) and diisopropylethylamine (7.7 mL, 44 mmol) in chloroform (260 mL) was heated at 60 °C (oil bath temperature) for 1 hour, then heated to reflux for 1.5 hours. The mixture was evaporated and the residue chromatographed eluting with 0-30% methanol in ethyl acetate affording a white solid (4.7g, 51%).
MS (+ve ion electrospray) m/z 463 (MH+).

### (d) 1,1-Dimethylethyl {1-[(9-cyano-4-oxo-1,2-dihydro-4H-pyrrolo[3,2,1-ij]quinolin-1-yl)methyl]-4-piperidinyl}carbamate

A mixture of 1,1-dimethylethyl {1-[(9-bromo-4-oxo-1,2-dihydro-4H-pyrrolo[3,2,1-ij]quinolin-1-yl)methyl]-4-piperidinyl}carbamate (4.7g, 10.2 mmol), copper(I) cyanide (3.3g, 36.6 mmol) and DMF (60 mL) was heated at 135 °C for 2 hours. The mixture was evaporated to dryness and the residue partitioned between saturated aqueous ammonia and dichloromethane. The aqueous phase was further extracted with dichloromethane and the combined organic extracts dried and evaporated (3.2g). The aqueous phase was further extracted twice with ethyl acetate and these extracts were combined, dried and evaporated (0.5g). The residues (3.7g in total) were combined and chromatographed eluting with 0-15% methanol in ethyl acetate affording a white solid (2.7g, 65%).
MS (+ve ion electrospray) m/z 409 (MH+).

### (e) 1-[(4-Amino-1-piperidinyl)methyl]-4-oxo-1,2-dihydro-4H-pyrrolo[3,2,1-ij]quinoline-9-carbonitrile

A solution of 1,1-dimethylethyl {1-[(9-cyano-4-oxo-1,2-dihydro-4H-pyrrolo[3,2,1-ij]quinolin-1-yl)methyl]-4-piperidinyl}carbamate (2.65g, 6.5 mmol) in dichloromethane (50 mL), was treated with TFA (50 mL). After 30 minutes the mixture was evaporated and the residue was twice azeotroped with chloroform then triturated with ether (three times). The resulting solid was redissolved in dichloromethane/methanol (60 mL/120 mL) and treated with MP-carbonate resin (3 mmol of carbonate per gramme, 22g, 66 mmol). The resin was removed by filtration, washing with dichloromethane and methanol. Evaporation of the filtrate afforded a white solid (2g) MS (+ve ion electrospray) m/z 309 (MH+).

### (f) 1-({4-[(6,7-Dihydro[1,4]dioxino[2,3-c]pyridazin-3-ylmethyl)amino]-1-piperidinyl}methyl)-4-oxo-1,2-dihydro-4H-pyrrolo[3,2,1-ij]quinoline-9-carbonitrile (Enantiomer E 1) hydrochloride

A solution of 1-[(4-amino-1-piperidinyl)methyl]-4-oxo-1,2-dihydro-4H-pyrrolo[3,2,1-ij]quinoline-9-carbonitrile (200 mg) and 6,7-dihydro[1,4]dioxino[2,3-c]pyridazine-3-carbaldehyde (100 mg) in dichloromethane/methanol (4 mL/1mL) was treated with sodium triacetoxyborohydride (400 mg). More 6,7-dihydro[1,4]dioxino[2,3-c]pyridazine-3-carbaldehyde (100 mg) and more sodium triacetoxyborohydride (200 mg) were added portionwise over 6 hours. The mixture was treated with saturated aqueous sodium bicarbonate solution. A solid was isolated by filtration which was then chromatographed eluting with 0-40% methanol in dichloromethane affording a white solid (110 mg).
δH (CDCl₃, 250MHz) 1.38-1.50 (2H, m), 1.85-2.00 (2H, m), 2.10-2.22 (1H, dt), 2.22-2.35 (1H, dt), 2.50-2.60 (1H, m), 2.75-2.85 (1H, m), 2.90-3.10 (2H, m), 4.00 (2H, s), 4.00-4.08 (1H, m), 4.35-4.40 (2H, m), 4.45-4.60 (4H, m), 6.82 (1H, d), 7.08 (1H, s), 7.38 (1H, d), 7.55 (1H, d), 7.78 (1H, d).

This was separated by preparative chiral hplc into the two enantiomers, E1 and E2, using a 5umChiralpak AD-H column, eluting with 80:20:0.1-CH₃CN:CH₃OH:Isopropylamine. The faster-running enantiomer (designated E1) was converted to the title compound by treatment with 1 equivalent of hydrochloric acid affording a solid (50 mg). >98% e.e.
MS (+ve ion electrospray) m/z 459 (MH+).

### Example 48 1-({4-[(6,7-Dihydro[1,4]dioxino[2,3-c]pyridazin-3-ylmethyl)amino]-1-piperidinyl}methyl)-4-oxo-1,2-dihydro-4H-pyrrolo[3,2,1-ij]quinoline-9-carbonitrile (Enantiomer E2) hydrochloride

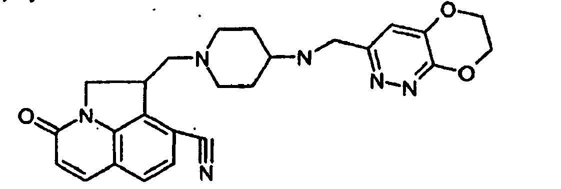

The free base of the title compound was prepared by preparative chiral hplc of the racemic material (slower-running enantiomer, see Example 47). This material was converted to the title compound with I equivalent of hydrochloric acid affording a solid (54 mg), >98% e.e.
MS (+ve ion electrospray) m/z 459 (MH+).

### Example 49 1-(R/S)-[(4-{[(3S)-2,3-Dihydro[1,4]dioxino[2,3-b]pyridin-3-ylmethyl]amino}-1-piperidinyl)methyl]-9-fluoro-1,2-dihydro-4H-pyrrolo[3,2,1-ij]quinolin-4-one Dihydrochloride

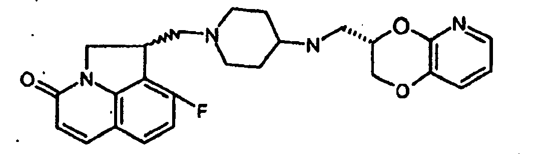

### (a) Methyl 3-(1,4-dioxa-8-azaspiro[4.5]dec-8-yl)-2-(R/S)-[7-fluoro-2-(methyloxy)-8-quinolinyl]propanoate

A mixture of methyl 2-[7-fluoro-2-(methyloxy)-8-quinolinyl]-2-propenoate (10.55 g, 40 mmol), 1,4-dioxa-8-azaspiro[4.5]decane (6.28 g, 44 mmol) and 1,1,3,3-tetramethylguanidine (2.4 mL) in dimethylformamide (200 mL) was heated under reflux overnight. The solvent was evaporated and the residue was dissolved in ethyl acetate and water. The aqueous phase was extracted again with ethyl acetate and the organic fractions were dried and evaporated. Chromatography on silica, eluting with 0-10% methanol/dichloromethane gave the product (11.41 g, 71%).
MS (+ve ion electrospray) m/z 405 (MH+).

### (b) 3-(1,4-Dioxa-8-azaspiro[4.5]dec-8-yl)-2-(R/S)-[7-fluoro-2-(methyloxy)-8-quinolinyl]-1-propanol

To a solution of methyl 3-(1,4-dioxa-8-azaspiro[4.5]dec-8-yl)-2-(R/S)-[7-fluoro-2-(methyloxy)-8-quinolinyl]propanoate (10.85 g, 27 mmol) in anhydrous THF (130 mL) at -70°C was added dropwise a solution of lithium aluminium hydride (2M in THF, 14 mL). The mixture was stirred for 5h while allowing to warm to -10°C. Water (5.5 mL) was added cautiously, followed by sodium hydroxide (2M, 6.5 mL), ether (87 mL) and sodium sulphate. After stirring at room temperature, the mixture was filtered through kieselguhr, washed through with ethyl acetate, and the filtrate was evaporated to give the crude alcohol (11.25 g).
MS (+ve ion electrospray) m/z 377 (MH+).

### (c) 1-(R/S)-(1,4-Dioxa-8-azaspiro[4.5]dec-8-ylmethyl)-9-fluoro-1,2-dihydro-4H-pyrrolo[3,2,1-ij]quinolin-4-one

A crude sample of 3-(1,4-dioxa-8-azaspiro[4.5]dec-8-yl)-2-(R/S)-[7-fluoro-2-(methyloxy)-8-quinolinyl]-1-propanol (11.25 g) was stirred with methanesulfonic anhydride (5.92 g, 34 mmol) and di-isopropylethylamine (11.4 mL, 67 mmol) in dry chloroform (130 mL) at 70°C for three days. The mixture was washed with aqueous sodium bicarbonate, the aqueous phase was extracted with dichloromethane, and the organic fractions were dried and evaporated to give a brown solid (7.70 g).
MS (+ve ion electrospray) m/z 345 (MH+).

### (d) 9-Fluoro-1-(R/S)-[(4-oxo-1-piperidinyl)methyl]-1,2-dihydro-4H-pyrrolo[3,2,1-ij]quinolin-4-one

1-(R/S)-(1,4-Dioxa-8-azaspiro[4.5]dec-8-ylmethyl)-9-fluoro-1,2-dihydro-4*H-*pyrrolo[3,2,1-*ij*]quinolin-4-one (7.70 g , 22 mmol) in acetone (600 mL) and 5M hydrochloric acid (300 mL) was heated overnight at 60°C. The mixture was basified with sodium bicarbonate and extracted with dichloromethane. The organic extracts were dried and evaporated. Chromatography on silica, eluting with 0-10% methanol/dichloromethane, gave a yellow solid (4.44g, 67%).
MS (+ve ion electrospray) m/z 301 (MH+).

### (e) Title compound

9-Fluoro-1-(R/S)-[(4-oxo-1-piperidinyl)methyl]-1,2-dihydro-4H-pyrrolo[3,2,1-ij]quinolin-4-one (0.10g, 0.33 mmol) and [(3*S*)-2,3-dihydro[1,4]dioxino[2,3-*b*]pyridin-3-ylmethyl]amine (for a preparation see EP0559285A1, Ex.5) (0.055g, 0.33 mmol) were stirred in dry dichloromethane and methanol (5 mL each) with glacial acetic acid (10 drops) and 3A molecular sieves at room temperature for I h. Sodium triacetoxyborohydride (0.084g, 1.33 mmol) was added and the mixture was stirred overnight. Aqueous sodium bicarbonate was added to basify and the phases were separated. The aqueous phase was extracted with 10% methanol/dichloromethane and the organic fractions were dried and evaporated. Chromatography on silica, eluting with 0-20% methanol/dichloromethane gave the free base of the title compound (0.12 g).
δH (CDCl₃, 250 MHz) 1.40 (2H, m), 1.86 (2H, m), 2.09 (1H, t), 2.23 (1H, t), 2.50 (2H, m), 2.78 (1H, m), 2.85 (1H, dd), 2.97 (2H, m), 3.00 (1H, m), 4.02 (1H, m), 4.05 (1H, dd), 4.30 (1H, dd), 4.45 (3H, m), 6.62 (1H, d), 6.87 (2H, m), 7.20 (1H, dd), 7.39 (1H, dd), 7.67 (1H, d), 7.82 (1H, dd).
MS (+ve ion electrospray) m/z 451 (MH+).

The free base was treated with hydrogen chloride in 1,4-dioxane (0.4M, 1.33 mL), evaporated and dried under vacuum to give the dihydrochloride salt (0.12 g).

### Example 50 1-({4-[(6,7-Dihydro-5H-pyrano[2,3-c]pyridazin-3-ylmethyl)amino]-1-piperidinyl}methyl)-4-oxo-1,2-dihydro-4H-pyrrolo [3,2,1-ij]quinoline-9-carbonitrile Enantiomer E1 Dihydrochloride

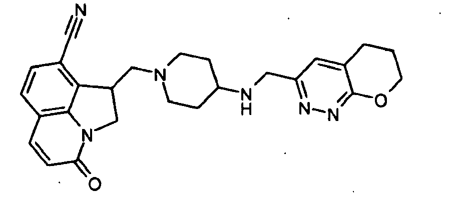

### (a) 1-[(4-Amino-1-piperidinyl)methyl]-4-oxo-1,2-dihydro-4H-pyrrolo[3,2,1-ij]quinoline-9-carbonitrile, Enantiomers 1 and 2

Racemic 1-[(4-amino-1-piperidinyl)methyl]-4-oxo-1,2-dihydro-4H-pyrrolo[3,2,1-ij]quinoline-9-carbonitrile (2.1g) was separated by chiral chromatography on a 5um Chiralpak AD-H column eluting with 95:5:0.1 acetonitrile:methanol:isopropylamine affording Enantiomer E1 750 mg, >98% ee, then Enantiomer E2, 760 mg, 98% ee.

### (b) Title compound

A solution of 1-[(4-amino-1-piperidinyl)methyl]-4-oxo-1,2-dihydro-4*H-*pyrrolo[3,2,1-*ij*]quinoline-9-carbonitrile, Enantiomer 1 (40 mg; 0.13 mmol) and 6,7-dihydro-5*H*-pyrano[2,3-c]pyridazine-3-carbaldehyde (26.7mg, 0.167 mmol) in MeOH (1ml), chloroform (1 ml) with 3A sieves was heated at 65°C under Ar for 5h. It was cooled and sodium triacetoxyborohydride (55mg; 0.26 mmol) was added and the mixture was stirred at rt overnight. The reaction was then filtered through kieselguhr, washing through with 1:1 MeOH/DCM. The solvents were evaporated and the residue partitioned between saturated aqueous NaHCO₃ and 20% MeOH in DCM. The aqueous was extracted twice more with 20% MeOH in DCM and then the combined organics were dried and evaporated. The residue was subjected to column chromatography on silica gel using a DCM, MeOH and aqueous ammonia gradient to provide the free base of the title compound (39mg, 66%).
MS (ES+) m/z 457(MH⁺).
¹HNMR (400MHz) δ(CDCl₃) 1.38-1.52 (2H, m), 1.83-1.99 (2H, m), 2.01-2.11 (2H, m), 2.11-2.2 (1H, m), 2.22-2.32 (1H, m), 2.50-2.61 (2H, m), 2.72-2.82 (1H, m), 2.85-2.91 (2H, m), 2.96 (1H, m), 3.01-3.08 (1H, s), 3.92-4.07 (3H, m), 4.24-4.07 (m, 3H), 4.51-4.61 (1H, m), 6.81 (1H, d, J), 7.30 (1H, s), 7.50 (1H, d,), 7.49 (1H, d), 7.73 (1H, d).

This material was converted to the dihydrochloride by dissolving in DCM and adding 1M HCl/diethyl ether then evaporating to dryness.

### Example 51 1-({4-[(6,7-dihydro-5H-pyrano[2,3-c]pyridazin-3-ylmethyl)amino]-1-piperidinyl}methyl)-9-fluoro-1-hydroxy-1,2-dihydro-4H-pyrrolo[3,2,1-ij]quinolin-4-one Enantiomer E1 Dihydrochloride

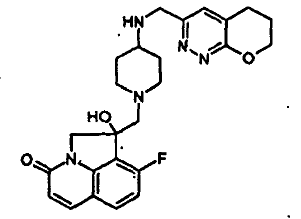

A solution of 1-[(4-amino-1-piperidinyl)methyl]-9-fluoro-1-hydroxy-1,2-dihydro-4*H*-pyrrolo[3,2,1-ij]quinolin-4-one enantiomer E1 (40 mg; 0.13 mmol) and 6,7-dihydro-5H-pyrano[2,3-*c*]pyridazine-3-carbaldehyde (25.7mg, 0.157 mmol) in MeOH (1 ml), chloroform (1ml) with 3A sieves was heated at 65°C under Ar for 5h. It was cooled and sodium triacetoxyborohydridc (55mg; 0.26 mmol) was added and the mixture was stirred at rt overnight. The reaction was then filtered through kieselguhr, washing through with 1:1 MeOH/DCM. The solvents were evaporated and the residue partitioned between saturated aqueous NaHCO₃ and 20% MeOH in DCM. The aqueous phase was extracted twice more with 20% MeOH in DCM and then the combined organics were dried and evaporated. The residue was subjected to column chromatography on silica gel using a DCM, MeOH and aqueous ammonia gradient to provide the free base of the title compound (32mg, 55%).
MS (ES+) m/z 466(MH⁺).
¹H NMR (400MHz) δ(CDCl₃) 1.41-1.91 (3H, m), 1.91-2.02 (2H, m), 2.03-2.11 (2H, m), 2.31-2.41 (1H, m), 2.51-2.65 (2H, m), 2.78-2.90 (3H, m), 2.92-3.05 (2H, m), 3.56 (1H, d), 4.01 (2H, s), 4.34-4.49 (4H, m), 6.63 (1H, d, J), 6.89-6.93 (1H, m), 7.28 (1H), 7.48-7.51 (1H, m), 7.69 (1H, d J).

This material was converted to the dihydrochloride by dissolving in DCM and adding 1M HCl/diethyl ether then evaporating to dryness.

### Example 52 9-Fluoro-1-[(4-{[(6-oxo-6,7-dihydro-5H-pyridazino[3,4-b][1,4]thiazin-3-yl)methyl]amino}-1-piperidinyl)methyl]-1,2-dihydro-4H-pyrrolo[3,2,1-ij]quinolin-4-one Enantiomer E1 Dihydrochloride

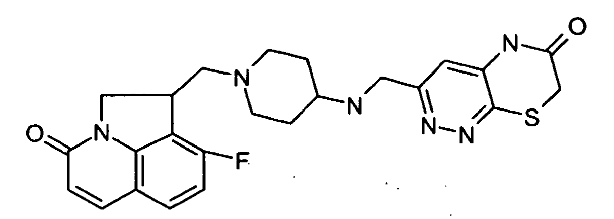

A solution of 1-[(4-amino-1-piperidinyl)methyl]-9-fluoro-1,2-dihydro-4H-pyrrolo[3,2,1-ij]quinolin-4-one (enantiomer E1, 45 mg, 0.15 mmol) and 6-oxo-6,7-dihydro-5H-pyridazino[3,4-b][1,4]thiazine-3-carboxaldehyde (for a synthesis see WO 2003087098, Example 312(d)) (19 mg, 0.1 mmol) in chloroform/methanol (3 ml/3 ml) was stirred for 6 hours then treated with sodium triacetoxyborohydride. After 72 hours the mixture was partitioned between water, sodium carbonate and 10% methanol in chloroform. The aqueous phase was extracted a further 3 times with 10% methanol in chloroform then the combined organic extracts were dried (sodium sulphate) and evaporated. The residue was chromatographed on silica gel, eluting with a 0-15% gradient of methanol in dichloromethane affording the free base of the title compound as a solid.
δH (d-6 methanol, 400 MHz) 2.02-2.15 (2H, m), 2.55 (2H, t), 2.70 (1H, t), 2.85 (1H, t), 3.15 (2H, t), 3.35-3.55 (3H, m), 3.70 (1H, m), 4.30 (2H, s), 4.60 (2H, s), 4.70 (2H, m), 7.20 (1H, d), 7.55 (1H, t), 7.65 (1H, s), 8.10 (1H, m), 8.45 (1H, d).
MS (ES+) m/z 481 (MH+).

This material was dissolved in methanol/dichloromethane and treated with 4M hydrochloric acid in dioxan. Evaporation afforded the title compound as a gelatinous white solid (28 mg).

### Examples 53 and 54 1-({4-[(2,3-dihydro[1,4]dioxino[2,3-c]pyridin-7-ylmethyl)amino]-1-piperidinyl}methyl)-1-hydroxy-4-oxo-1,2-dihydro-4H-pyrrolo[3,2,1-ij]quinoline-9-carbonitrile Enantiomers E1 and E2 Hydrochloride

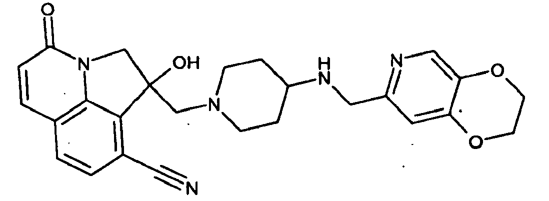

### (a) Methyl 2-[7-bromo-2-(methyloxy)-8-quinolinyl]-2-oxiranecarboxylate

A solution of methyl 2-[7-bromo-2-(methyloxy)-8-quinolinyl]-2-propenoate (4.9g, 15.2 mmol) in DCM (100 ml) was treated with meta-chloroperbenzoic acid (5.24g) and heated at 45°C for 21 hours. A further 1 equivalent of meta-chloroperbenzoic acid was added and heating continued for 4 hours. A further 1 equivalent of meta-chloroperbenzoic acid was added and heating continued for 17 hours. Water and DCM were added followed by sodium sulphite then sodium bicarbonate. The phases were separated and the aquoues phase further extracted (three times) with 10% methanol in DCM. The combined organic extracts were dried and evaporated to give a yellow oil. This was chromatographed on silica gel, eluting with a 0-100% gradient of ethyl acetate in hexane affording a pale yellow solid (4.3g, 84%).
MS (ES+) m/z 339 (MH⁺).

### (b) Methyl 9-bromo-1-hydroxy-4-oxo-1,2-dihydro-4H-pyrrolo[3,2,1-ij]quinoline-1-carboxylate '

A mixture of methyl 2-[7-bromo-2-(methyloxy)-8-quinolinyl]-2-oxiranecarboxylate (4.3g, 12.7 mmol), lithium perchlorate (4.06g, 38 mmol, 3 equivalents), acetonitrile (43 ml) and water (43 ml) was stirred at 85°C for 17 hours. More lithium perchlorate (2 equivalents) was added and the mixture heated at 85°C for 7 hours. More lithium perchlorate (2 equivalents) was added and the mixture heated at 85°C for 17 hours. The reaction mixture was allowed to coot to room temperature and treated with 10% methanol in DCM. The aqueous phase was further extracted with DCM and the combined organic extracts dried (MgSO4) and evaporated to give a yellow solid (4.2g). Chromatography on silica afforded a pale yellow solid (3.07g, 74%).
MS (ES+) m/z 327 (MH⁺).

### (c) 1,1-Dimethylethyl 1-[(9-bromo-1-hydroxy-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate

A solution of methyl 9-bromo-1-hydroxy-4-oxo-1,2-dihydro-4H-pyrrolo[3,2,1-ij]quinoline-1-carboxylate (1.56g, 4.6 mmol) in methanol (50 ml) was treated at 0°C with sodium borohydride (528 mg, 13.9 mmol). After 1 hour the mixture was allowed to warm to room temperature. After 1 hour at room temperature the mixture was heated to 40°C for I hour. The mixture was allowed to cool to room temperature and more sodium borohydride (528 mg, 13.9 mmol) was added. The mixture was stirred at room temperature overnight, then quenched with aqueous ammonium chloride. The mixture was filtered and the filtrate dried over magnesium sulphate. The mixture was filtered and evaporated. The residue was chromatographed on silica gel, eluting with a 10-30% gradient of methanol in DCM affording a solid (ca 10g). This mixture was suspended in 10% methanol in DCM (100 ml) and stirred overnight. Filtration and evaporation afforded a solid (4.2g) consistent with a mixture of 9-bromo-1-hydroxy-1-(hydroxymethyl)1,2-dihydro-4H-pyrrolo[3,2,1-ij]quinolin-4-one and inorganics. MS (ES+) m/z 297 (MH⁺).

A portion of this material (assume 1.5 mmol of 9-bromo-1-hydroxy-1-(hydroxymethyl)-1,2-dihydro-4H-pyrrolo[3,2,1-ij]quinolin-4-one) was suspended in DCM (17 ml), THF (17 ml) and DMF (1.7 ml) then treated with dibutyl(oxo)stannane (19 mg, 0.07 mmol), 4-methylbenzenesulfonyl chloride (293 mg, 1.5 mmol) and triethylamine (0.3 ml, 2.3 mmol). After 41 hours chloroform (25 ml), 4-methylbenzenesulfonyl chloride (95 mg) and dibutyl(oxo)stannane (25 mg) were added. After 2 hours the mixture was evaporated onto silica and the mixture subjected to chromatography eluting with a 0-10% gradient of methanol in ethyl acetate affording a white solid (350 mg). This material was consistent with a 2:1 mixture of (9-bromo-1-hydroxy-4-oxo-1,2-dihydro-4H-pyrrolo[3,2,1-ij]quinolin-1-yl)methyl 4-methylbenzenesulfonate [MS (ES+) m/z 451 (MH⁺)] and 9-bromo-1-(chloromethyl)-1-hydroxy-1,2-dihydro-4H-pyrrolo[3,2,1-ij]quinolin-4-one [MS (ES+) m/z 315 (MH⁺)].

This material (350 mg, estimated 0.9 mmol) was treated with 1,1-dimethylethyl (2,3-dihydro[1,4]dioxino[2,3-c]pyridin-7-ylmethyl)-4-piperidinylcarbamate (for a synthesis, see WO 2004058144 Example 99(h)) (453 mg, 1.3 mmol), sodium carbonate (318 mg, 3 mmol) and ethanol (10 ml) and heated at 38°C for 40 hours. The mixture was evaporated and the residue partitioned between DCM and dilute brine. The organic phase was added to the top of a silica column, eluting with 0-20% gradient of methanol in DCM affording a white foam (400 mg).
MS (ES+) m/z 629 (MH⁺).

### (d) 1,1-Dimethylethyl {1-[(9-cyano-1-hydroxy-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate

A mixture of 1,1-dimethylethyl {1-[(9-bromo-1-hydroxy-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate (400 mg, 0.64 mmol), copper(I)cyanide (200 mg, 2.3 mmol) and DMF (6 ml) was heated at 130°C for 16 hours then evaporated to dryness. The residue was partitioned between ethyl acetate/brine/concentrated aqueous ammonia solution. The organic extract was dried and evaporated to give a brown foam. Chromatography eluting with 0-30% gradient of methanol in DCM afforded the product (220 mg, 60%).
MS (+ve ion electrospray) m/z 574 (MH⁺).

### (e) Title compounds

A solution of 1,1-dimethylethyl {1-[(9-cyano-1-hydroxy-4-oxo-1,2-dihydro-4H-pyrrolo[3,2,1-ij]quinolin-1-yl)methyl]-4-piperidinyl}(2,3-dihydro[1,4]dioxino[2,3-c]pyridin-7-ylmethyl)carbamate (220 mg, 0.38 mmol) in TFA/DCM (5 ml/5 ml) was stirred for 45 minutes then evaporated, azeotroping with chloroform then dried *in vacuo.* The residue was dissolved in DMF/methanol (10 ml/10 ml) and treated with MP-carbonate resin (3g; 3 mmol of carbonate per gramme, 9 mmol). After 30 minutes the mixture was filtered and evaporated affording a brown oil which was subjected to chromatography eluting with 0-30% gradient of methanol in DCM afforded the free base of the title compounds as a pale yellow oil (110 mg, 61%).
¹H NMR (250 MHz) δ(CDCl₃) 1.40-1.65 (2H, m), 1.90-2.05 (2H, m), 2.35-2.70 (3H, m), 2.85 (1H, d), 2.92-3.10 (2H, m), 3.35 (1H, d), 3.80 (2H, s), 4.25-4.35 (4H, m), 4.40-4.50 (2H, m), 6.80-6.88 (2H, m), 7.45 (1H, d), 7.62 (1H, d), 7.78 (1H, d), 8.10 (1H, s). MS (+ve ion electrospray) m/z 474 (MH⁺).

A portion of this material (90 mg) was first partially purified by preparative C 18 HPLC using a 1 inch Luna C 18 semi-prep column eluting with a solvent system of 50 mmolar aq.ammonium formate pH 4.0 and acetonitrile. Then the pure (>99%) racemate was resolved into its two enantiomers by preparative chiral HPLC using a 5um Chiralpak 1A column eluting with 90:10:0.1- CH₃CN:CH₃OH:Isopropylamine, Rt 2.6 minutes for E1 and 3.3 minutes for E2. Both enantiomers were converted to their monohydrochloride salts, Enantiomer E 1 (40 mg) and Enantiomer E2 (39 mg).

### Example 55 1-[(4-{[(7-Chloro-3-oxo-3,4-dihydro-2H-pyrido[3,2-b][1,4]oxazin-6-yl)methyl]amino}-1-piperidinyl)methyl]-9-fluoro-1,2-dihydro-4H-pyrrolo[3,2,1-ij]quinolin-4-one Enantiomer E1 Dihydrochloride

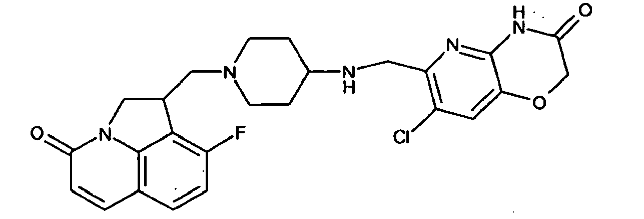

A solution of 1-[(4-amino-1-piperidinyl)methyl]-9-fluoro-1,2-dihydro-4H-pyrrolo[3,2,1-ij]quinolin-4-one (Enantiomer E1, 70 mg, 0.23 mmol) and 7-chloro-3-oxo-3,4-dihydro-2H-pyrido[3,2-b][1,4]oxazine-6-carbaldehyde (for a synthesis see WO 2006010040, Preparation 6(h)) (49 mg, 0.23 mmol) in chloroform/methanol (3 ml/3 ml) was stirred for 2 hours then treated with sodium triacetoxyborohydride (146 mg, 0.65 mmol). After 2 hours the solvents were removed. The residue was chromatographed on silica gel, eluting with a 0-10% gradient of methanol in dichloromethane affording the acetate salt of the free base of the title compound (105 mg).
¹H NMR (400 MHz) δ(CDCl₃) 1.55-1.70 (2H, m), 1.95-2.05 (5H, m), 2.15 (1H, t), 2.25 (1H, t), 2.55 (1H, t), 2.70 (1H, m), 2.80-2.90 (2H, m), 3.10 (1H, m), 4.00-4.05 (3H, m),' 4.45-4.50 (2H, m), 4.65 (2H, s), 6.65 (2H, m), 6.85 (1H, t), 7.38 (1H, m), 7.68 (1H, d). MS (+ve ion electrospray) m/z 498 (MH⁺).

This material was converted to the title dihydrochloride salt.

### Examples 56 and 57 1-({4-[(2,3-Dihydro[1,4]dioxino[2,3-c]pyridin-7-ylmethyl)amino]-1-piperidinyl}methyl)-9-(methyloxy)-1,2-dihydro-4H-pyrrolo[3,2,1-ij]quinolin-4-one, Enantiomers E1 and E2 , Hydrochloride, and 1-({4-[(2,3-Dihydro[1,4]dioxino[2,3-c]pyridin-7-ylmethyl)amino]-1-piperidinyl}methyl)-9-(methyloxy)-1,2-dihydro-4H-pyrrolo[3,2,1-ij]quinolin-4-one, Enantiomer E1 Dihydrochloride

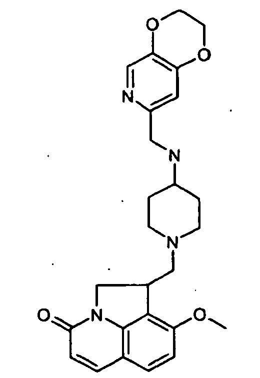

### (a) 1-[(4-Amino-1-piperidinyl)methyl]-9-(methyloxy)-)1,2-dihydro-4H-pyrrolo[3,2,1-ij]quinolin-4-one

A solution of 1-[(4-amino-1-piperidinyl)methyl]-9-fluoro-1,2-dihydro-4H-pyrrolo[3,2,1-ij]quinolin-4-one dihydrochloride (1.74g, 4.64mmol) in methanol (17mL)) at room temperature under argon, was treated with 25% sodium methoxide in methanol (2.5 ml, 11.5mmol). The reaction was then heated to 85°C for 2 hours, then a further addition of 25% sodium methoxide in methanol (5ml) was added. The reaction was left at 85°C overnight (16 hours). A further addition of 25% sodium methoxide in methanol (5ml) was required in the morning and reaction was left at 85°C for most of the day. The reaction mixture was treated with ammonium chloride (saturated) until the pH reached 8, where the solvent was removed under vacuum. The residue was re-dissolved in 10%methanol in DCM and stirred at room temperature for 1 hour with sodium sulphate. This mixture was then filtered and the solvent removed to give a yellow solid (3.81 g). This was purified on a 10g SCX column eluting with methanol then 2M ammonia in methanol to give a yellow oily solid (0.832, 57%).
MS (ES+) m/z 314 (MH⁺).

### (b) Title compound

1-[(4-amino-1-piperidinyl)methyl]-9-(methyloxy)-1,2-dihydro-4H-pyrrolo[3,2,1-ij]quinolin-4-one (0.108g, 0.345mmol) and 2,3-dihydro[1,4]dioxino[2,3-c]pyridine-7-carbaldehyde (see WO2004058144, Example 2(c)) (0.057g, 0.345mmol) was dissolved in chloroform (2.5ml) and methanol (0.25ml) at room temperature under argon. Sodium triacetoxyborohydride (0.219g, 1.03mmol) was then added and the reaction was allowed to stir at room temperature for 1 hour. After which it was purified by chromatography on silica gel (20g) using a 0-30% methanol in dichloromethane gradient to give the acetate salt of the free base of the title compound as a clear oil (0.175g, 100%).
MS (ES+) m/z 463 (MH⁺).
¹H NMR (250MHz) δ(MeOD) 1.55-1.97 (2H, m), 2.00 (3H, s), 2.05-2..38 (4H, m), 2.49 (1H, t), 2.90-3.30 (5H, m), 3.94 (3H, s), 4.19 (2H,s), 4.30-4.38 (6H, m), 6.43 (1H, d), 6.98 (1H, d), 7.03 (1H, s), 7.52 (1H, d), 7.81 (1H, d), 8.11 (1H, s).

A portion of this material (60 mg) was purified firstly on a 5um Chiralpak AD-H column eluting with 80:20:0.1 CH₃CN:CH₃OH:Isopropylamine then finally on a 5um Chiralpak AS-H column eluting with 90:10:0.1 CH₃CN:CH₃OH:Isopropylamine affording the E1 enantiomer free base (approximately 10 mg) (Rt 4.8 minutes, 100% ee, 99.5 % chemical purity) then the E2 enantiomer free base (approximately 10 mg, Rt 6.9 minutes, 100% ee, 98.5% purity).

Each enantiomer was separately converted to the corresponding hydrochloride salt, by dissolving the free base in methanol and the appropriate amount of 6N HCl was added. The reaction was stirred for approximately 1 hour and the methanol was removed to leave the remaining mono HCl salts. Enantiomer E1 was also converted to the Dihydrochloride salt.

### Example 58 1({4-[(6,7-Dihydro[1,4]dioxino[2,3-c]pyridazin-3-ylmethyl)amino]-1-piperidinyl}methyl)-9-(methyloxy)-1,2.dihydro-4H-pyrrolo[3,2,1-ij]quinolin-4-one, Enantiomer 1 Dihydrochloride

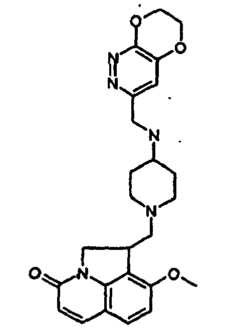

A solution of 1-[(4-amino-1-piperidinyl)methyl]-9-fluoro-1,2-dihydro-4H-pyrrolo[3,2,1-ij]quinolin-4-one, enantiomers E1 (0.301 g, 1.0mmol) in methanol (3mL) at room temperature under argon, was treated with 25% sodium methoxide in methanol (0.432ml, 2.0mmol. The reaction was then heated to 85°C for 2 hours, where a further addition of 25% sodium methoxide in methanol (0.432ml) was added and the reaction was left at 85°C overnight (16 hours). Again, further addition of 25% sodium methoxide in methanol (1.73ml) was required and the reaction was left at 85°C for a couple of hours. The reaction was cooled to room temperature. Ammonium chloride (saturated) was then added until the pH was at 8, then the solvent was removed under vacuum. The residue was re-dissolved in 10%MeOH in DCM and stirred at room temperature for 1 hour, whereupon sodium sulphate was added. This mixture was then filtered and the solvent removed to give a yellow solid (2.49g). This was purified on a 10g SCX column eluting with methanol then 2M ammonia in methanol to give an oily solid.

This material was consistent with a mixture of 1-[(4-amino-1-piperidinyl)methyl]-9-(methyloxy)-1,2-dihydro-4H-pyrrolo[3,2,1-ij]quinolin-4-one and inorganic material.
MS (ES+) m/z 314 (MH⁺).

A portion of this material (108 mg) and 6,7-dihydro[1,4]dioxino[2,3-c]pyridazine-3-carbaldehyde (0.057g, 0.345mmol) was dissolved in chloroform (2.5ml) and methanol (0.25ml) at room temperature under argon. Sodium triacetoxyborohydride (0.219g, 1.03 mmol) was then added and the reaction was allowed to stir at room temperature for 16 hours. The reaction was then diluted with 5ml sodium bicarbonate (saturated), and the aqueous phase was then separated then further extracted with 10% methanol in DCM (3 x 5ml). The organics were combined, dried (Na₂SO₄), filtered and the solvent was removed giving a yellow solid (0.120g). This was then purified by chromatography on silica gel (20g) using a.0-30% methanol in dichloromethane gradient to give the free base of the title compound as a clear oil (54 mg).
¹H NMR (250MHz) δ(MeOD) 1.46-1.58 (2H, m), 1.82-2.26 (5H, m); 2.42 (1H, t), 2.49-2.62 (1H, m), 2.80 (1H, br d), 2.95 (1H, dd), 3.19 (1H, br d), 3.93 (3H, s), 3.97 (1H, s), 4.28-4.38 (2H, m), 4.39-4.47(2H, m), 4.51-4.62 (2H, m), 6.42 (1H, d), 6.96(1H, d), 7.24 (1H, s), 7.50 (1H, d), 7.79 (1H, d).
MS (ES+) m/z 464 (MH⁺).

This material was converted to the dihydrochloride salt by treating a solution in methanol (1ml) with excess 1M hydrochloric acid in methanol (0.1ml) followed by evaporation to dryness.

### Example 59 9-Fluoro-1-{[4-({[(7R/S)-7-(hydroxymethyl)-6,7-dihydro[1,4]dioxino[2,3-c]pyridazin-3-yl]methyl}amino)-1-piperidinyl]methyl}-1,2-dihydro-4H-pyrrolo[3,2,1-ij]quinolin-4-one Diastereomer D1 Dihydrochloride

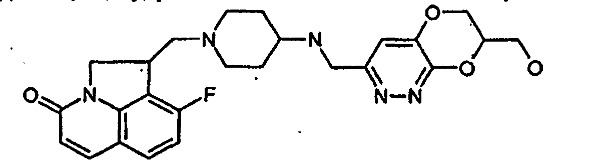

### (a) 3,6-Dichloro-4-({[(4R/S)-2,2-dimethyl-1,3-dioxolan-4-yl]methyl}oxy)pyridazine

[(4R/S)-2,2-Dimethyl-1,3-dioxolan-4-yl]methanol (3.3ml) in THF was cooled in ice and sodium hydride (1.2g, 60% dispersion in oil) was added below 10°C internal temperature. After 15 mins 3,4,6-trichloropyridazine (5g, 27.2mmol) was added and the mixture stirred overnight. Ice/water was added and the mixture evaporated. The residue was diluted with water and extracted with DCM. The extracts were dried and evaporated to give the product (4.78g,63% yield).
MS (ES+) m/z 280 (MH⁺).

### (b) (2R/S)-3-[(3,6-Dichloro-4-pyridazinyl)oxy]-1,2-propanediol

A solution of 3,6-dichloro-4-({[(4R/S)-2,2-dimethyl-1,3-dioxolan-4-yl]methyl}oxy)pyridazine (4.78g 17.2mmol) in methanol (100ml) and 4M hydrogen chloride in dioxan (100ml) was stirred for 3h. The reaction mixture was evaporated and azeotroped with toluene. The residue was dissolved in 10% methanol in DCM (100ml) and treated with MP-carbonate (50g). Further MP-carbonate (50g and 25g) was added after 2h and overnight stirring. After a further 2h the mixture was filtered and evaporated. Chromatography on silica gel eluting with i0-20% methanol in DCM gave the product (1.89g, 46%).
MS (ES+) m/z 240 (MH⁺).

### (c) [(7R/S)-3-Chloro-6,7-dihydro[1,4]dioxino[2,3-c]pyridazin-7-yl}methanol

(2R/S)-3-[(3,6-Dichloro-4-pyridazinyl)oxy]-1,2-propanediol (1.89g,) was azeotroped twice with dioxan (20ml), then dissolved in dioxan and stirred with lithium hydride for 4 days at 104 °C The mixture was quenched with ice and acidified to pH 7-8 with 2M hydrochloric acid then evaporated. The residue was extracted with chloroform and the extracts were dried and evaporated. Chromatography on silica gel eluting with 1-3% methanol in DCM gave the product (147mg).
MS (ES+) m/z 203 (MH⁺).

### (d) [(7R/S)-3-Ethenyl-6,7-dihydro[1,4]dioxino[2,3-c]pyridazin-7-yl]methanol

[(7R/S)-3-Chloro-6,7-dihydro[I,4]dioxino[2,3-c]pyridazin-7-yl]methanol (147mg, 0.72mmol) in 1,2-dimethoxyethane was degassed with a stream of argon. Tetrakis(triphenylphosphine)palladium(0) (18mg), triethenylboroxin.pyridine (110mg) potassium carbonate (100mg) and water (1.5ml) were added and the mixture heated at **100°C** overnight. The mixture was evaporated and chromatography on silica gel eluting with ethyl acetate gave the title compound (94mg, 67%yield).
MS (ES+) m/z 195 (MH⁺).

### (e) (7R/S)-7-(Hydroxymethyl)-6,7-dihydro[1,4]dioxino[2,3-c]pyridazine-3-carbaldehyde

[(7R/S)-3-Ethenyl-6,7-dihydro[1,4]dioxino[2,3-c]pyridazin-7-yl]methanol (94mg) in dioxan (4.4ml) and water (0.85ml) was stirred with 4M aqueous osmium tetroxide (0.43ml) and sodium periodate (238mg) for 7h. The mixture was evaporated and diluted with methanol, ethyl acetate and DCM. Silica was added and the mixture evaporated and chromatographed on silica eluting with 0-40% methanol in ethyl acetate to give the product (19mg, 20% yield).
MS (ES+) m/z 197 (MH⁺).

### (f) Title compound

(7R/S)-7-(Hydroxymethyl)-6,7-dihydro[1,4]dioxino[2,3-c]pyridazine-3-carbaldehyde (19mg, 0.097mmol) and 1-[(4-amino-1-piperidinyl)methyl]-9-fluoro-1,2-dihydro-4*H-*pyrrolo[3,2,1-*ij*]quinolin-4-one enantiomer E1 (35mg, 0.116mmol) in methanol (0.5ml) and chloroform (3ml) was stirred with 3A sieves for 24h. Sodium triacetoxy borohydride (62mg) was added and the mixture stirred for 72h with addition of further sodium triacetoxyborohydride after 8h. Saturated sodium carbonate (5 drops) was added followed by silica. Then the mixture was evaporated and chromatographed on silica eluting with 0-20% methanol in dichloromethane to give the free base of the title compound.
¹H NMR (400 MHz, CDCl₃ + CD₃OD) δ 7.82 (1H, s), 7.51 (1H, m), 7.21 (1H, m), 6.97 (1H, t), 6.63 (1H, d), 4.60-4.40 (3H, m), 4.29 (1H, m), 4.73 (3H, m), 3.92 (2H, m), 3.36 (1H, m), 3.13 (1H, m), 2.91 (2H, m), 2.70 (1H, m), 2.58 (1H, t), 2.27 (1H, t), 2.14 (1H, t) 1.56 (2H, m).

The free base was dissolved in methanol and DCM, 4M hydrogen chloride in dioxan (0.4ml) was added and the precipitate triturated with ether and dried to give the title compound (32mg)
LC/MS (+vc ion electrospray): *m*/*z* 482 (MH⁺).

### Example 60 1-({4-[(5,6-Dihydrofuro[2,3-c]pyridazin-3-ylmethyl)amino]-1-piperidinyl}methyl)-9-fluoro-1,2-dihydro-4H-pyrrolo[3,2,1-ij]quinolin-4-one Enantiomer E1 hydrochloride

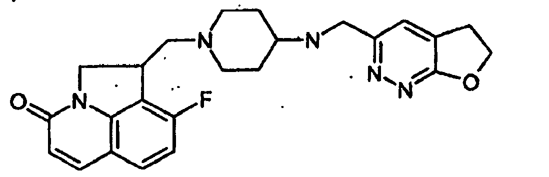

### (a),(3,6-Dioxo-1,2,3,6-tetrahydro-4-pyridazinyl)acetic acid

A mixture of (2,5-dioxo-2,5-dihydro-3-furanyl)acetic acid (9g) and hydrazine sulphate (7.2g) in water was heated to reflux for 4 hours then allowed to cool to ambient temperature. The precipitate was filtered, washing with water then acetone. Drying *in vacuo* afforded a white solid (8.04g, 82%).
MS (ES+) m/z 171 (MH⁺).

### (b) Methyl (3,6-dioxo-1,2,3,6-tetrahydro-4-pyridazinyl)acetate

A mixture of (3,6-dioxo-1,2,3,6-tetrahydro-4-pyridazinyl)acetic acid, (5.0g), methanol (75 ml) and 4M hydrochloric acid in dioxan (20 ml) was stirred overnight. Evaporation afforded a white solid.
MS (ES+) m/z 185 (MH⁺).

### (c) 4-(2-Hydroxyethyl)-1,2-dihydro-3,6-pyridazinedione

A suspension of methyl (3,6-dioxo-1,2,3,6-tetrahydro-4-pyridazinyl)acetate (11.1g, 60.3 mmol) in THF (2 litres) was sonicated to give a fine dispersion. The mixture was cooled to -15°C and treated dropwise with a solution of lithium aluminium hydride in THF (1M; 90 ml, 90 mmol). The mixture was stirred at 0°C for 2 hours. Sodium hydroxide (2M; 15 ml, 30 mmol) was added, then the mixture was acidified with 5M hydrochloric acid to around pH4-5. The supernatant was decanted off and discarded. The oily residue was extracted with water/methanol (500 ml/l litre). This extract was decanted from the remaining residue, treated with silica and evaporated. The silica residue was added to the top of a column, eluting with 10-30% methanol in DCM affording a pale yellow oil (2.7g).
MS (ES+) m/z 157 (MH⁺).

### (d) 5,6-Dihydrofuro[2,3-c]pyridazin-3(2H)-one

A mixture of 4-(2-hydroxyethyl)-1,2-dihydro-3,6-pyridazinedione (2.7g) in THF (200 ml) was treated with triphenylphosphine (6.6g) and bis(1-methylethyl) (E)-1,2-diazenedicarboxylate (5.0 ml) was warmed to 40°C. After 3 hours the mixture was evaporated and chromatographed onto silica which was added to the top of a column. Chromatography eluting with 0-10% methanol in DCM afforded impure product (420 mg) which was further purified by chromatography in a similar manner affording the product (390 mg).
MS (ES+) m/z 139 (MH⁺).

### (e) 5,6-Dihydrofuro[2,3-c]pyridazin-3-yl trifluoromethanesulfonate

A solution of 5,6-dihydrofuro[2,3-c]pyridazin-3(2H)-one (780 mg) in DMF (15 ml) was treated with sodium hydride (435 mg) then after 2 hours with N-phenyltrifluoromethanesulphonimide (3.62g). After 2 hours the mixture was diluted with ethyl acetate and washed with saturated aqueous sodium bicarbonate solution. The aqueous phase was further extracted (twice) with ethyl acetate and the combined organic extracts dried and evaporated affording the product (937 mg).
MS (ES+) m/z 271 (MH⁺).

### (f) 3-Ethenyl-5,6-dihydrofuro[2,3-c]pyridazine

A solution of 5,6-dihydrofuro[2,3-c]pyridazin-3-yl trifluoromethanesulfonate (500 mg, 1.85 mmol) in dimethoxyethane (20 ml) was degassed then treated with tetrakis(triphenylphosphine)palladium (0) (116 mg), potassium carbonate (257 mg), 2,4,6-trivinylcyclotriboroxane pyridine complex (416 mg) and water (3.6 ml).The mixture was stirred at 80°C for 2 hours then partitioned between DCM and saturated aqueous sodium bicarbonate solution. The aqueous phase was extracted with 10% methanol in DCM then the combined organic extracts dried and evaporated. The residue was chromatographed eluting with ethyl acetate affording a white solid (111 mg, 36%). MS (ES+) m/z 149 (MH⁺).

### (g) 5,6-Dihydrofuro[2,3-c]pyridazirie-3-carbaldehyde

A mixture of 3-ethenyl-5,6-dihydrofuro[2,3-c]pyridazine (110 mg, 0.74 mmol), 4% osmium tetroxide in water (0.66 ml), sodium periodate (367 mg), dioxan (6.6 ml) and water (1.3 ml) was stirred for 3 hours. The mixture was evaporated and the residue treated with chloroform and added to the top of a column. Elution with ethyl acetate afforded the product (23 mg).
MS (ES+) m/z 151 (MH⁺).

### (h) Title compound

A solution of 1-[(4-amino-1-piperidinyl)methyl]-9-fluoro-1,2-dihydro-4*H-*pyrrolo[3,2,1-ij]quinolin-4-one Enantiomer E1 (52mg, 0.173 mmol) and 5,6-dihydrofuro[2,3-c]pyridazine-3-carbaldehyde (23 mg, 0.153 mmol) in DCM/methanol (3 ml/0.5 ml) was stirred overnight then treated with sodium triacetoxyborohydride (97 mg). After 8 hours more sodium triacetoxyborohydride (97 mg) was added and the mixture stirred overnight. The mixture was partitioned between saturated aqueous sodium bicarbonate solution and 10% methanol in DCM. The extract was dried and evaporated then the residue chromatographed eluting with 0-20% methanol in DCM affording the free base of the title compound (34 mg).
δH (CDCl₃, 250MHz) 1.35-1.55 (2H, m), 1.80-2.10 (4H, m) 2.20 (1H, t), 2.40-2.65(2H, m), 2.75-2.90 (2H, m), 2.98-3.08 (1H, m), 3.32 (2H, t), 3.95-4.05 (3H, m), 4.404.55 (2H, m), 4.70 (2H, t), 6.65 (1H, d), 6.85 (1H, t), 7.35-7.42 (1H, m), 7.48 (1H, s), 7.78 (1H, d). MS (ES+) m/z 436 (MH⁺).

The free base was dissolved in methanol (2 ml) and treated with 0.1 M hydrochloric acid (0.8 ml) then evaporated. The residue was dissolved in methanol and added to ether. The resulting solid was isolated by centrifugation and dried in vacuo (26 mg).

### Example 61 1-({4-[(5,6-dihydrofuro[2,3-c]pyridacin-3-ylmethyl)amino]-1-piperidinyl}methyl)-9-fluoro-1-hydroxy-1,2-dihydro-4H-pyrrolo[3,2,1-ij]quinolin-4-one Enantiomer E1 Hydrochloride

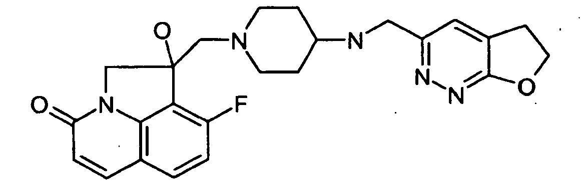

A solution of 1-[(4-amino-1-piperidinyl)methyl]-9-fluoro-1-hydroxy-1,2-dihydro-4*H-*pyrrolo[3,2,1-ij]quinolin-4-one enantiomer E1 (48 mg,) and 5,6-dihydrofuro[2,3-c]pyridazine-3-carbaldehyde (20 mg, 0.133 mmol) in DCM/methanol (3 ml/0.5 ml) was stirred overnight then treated with sodium triacetoxyborohydride (85 mg). After 7 hours a further portion of sodium triacetoxyborohydride (85 mg) was added. After 17 hours the mixture was partitioned between saturated aqueous sodium bicarbonate solution and 10% methanol in DCM. The extract was dried and evaporated then the residue chromatographed eluting with 0-20% methanol in DCM affording the free base of the title compound (8 mg).
δH (CDCl₃, 250MHz) 1.40-1.65 (2H, m), 1.90-2.70 (5H, m) 2.80 (1H, d), 2.90-3.05 (2H, m), 3.30-3.40 (3H, m), 4.05 (2H, s), 4.30-4.45 (2H, m), 4.70 (2H, t), 6.62 (1H, d), 6.90 (1H, t), 7.45 (1H, s), 7.50 (1H, m), 7.70 (1H, d).
MS (ES+) m/z 452 (MH⁺).

This material was converted to the title compound by treating a methanolic solution of hydrochloric acid and evaporating, followed by dissolving the residue in 20% methanol in DCM then precipitating with ether and isolating by centrifugation, giving a white solid (4.5 mg).

### Example 62 (1R/S)-1-[(4-{[(7S)-6,7-dihydro[1,4]dioxino[2,3-c]pyridazin-7-ylmethyl]amino}-1-piperidinyl)methyl]-9-fluoro-1,2-dihydro-4H-pyrrolo[3,2,1-ij]quinolin-4-one fumarate

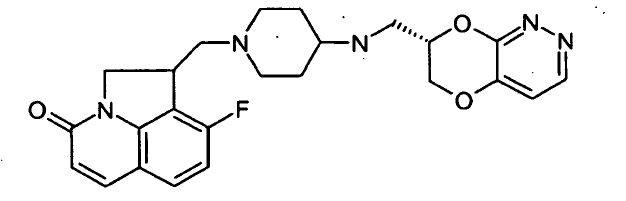

### (a) 3,6-Dichloro-4-{[(2S)-2-oxiranylmethyl]oxy}pyridazine

A solution of (2R)-2-oxiranylmethanol (400 mg) in THF (50 ml) was cooled in an ice bath and treated with sodium hydride (60% dispersion, 230 mg). After the addition was complete 3,4,6-trichloropyridazine (1.0g) was added portionwise and the mixture was stirred at room temperature overnight. The solvent was removed by evaporation and the residue partitioned between DCM and water. The aqueous phase was further twice extracted with DCM then the combined residues dried and evaporated affording the product (1.1g).
MS (+ve ion electrospray) m/z 221 (MH+).

### (b) (2S)-1-Azido-3-[(3,6-dichloro-4-pyridazinyl)oxy]-2-propanol

A mixture of 3,6-dichloro-4-{[(2S)-2-oxiranylmethyl]oxy}pyridazine (1.1g) and sodium azide (600 mg) in dioxan/water (25 ml/5 ml) was heated to reflux for 7 hours then concentrated, diluted with ethyl acetate, washed with brine, dried and evaporated. The residue was chromatographed eluting with hexane then 5% methanol in DCM affording the product (400 mg).
MS (+ve ion electrospray) m/z 264 (MH+).

### (c) (7S)-7-(Azidomethyl)-3-chloro-6,7-dihydro[1,4]dioxino[2,3-c]pyridazine

A mixture of (2S)-1-azido-3-[(3,6-dichloro-4-pyridazinyl)oxy]-2-propanol (400 mg) and lithium hydride (200 mg) in dioxan (50 ml) were heated under reflux over the weekend. The mixture was treated with ice then taken to approximately pH7.5 with 5M hydrochloric acid then concentrated to a low volume. The mixture was partitioned between water and DCM. The aqueous phase was extracted a further three times with DCM and the combined extracts dried and evaporated. Chromatography eluting with 0-10% methanol in DCM afforded the product (250 mg).
MS (+ve ion electrospray) m/z 228 (MH+).

### (d) [(7S)-6,7-Dihydro[1,4]dioxino[2,3-c]pyridazin-7-ylmethyl]amine hydrochloride

A solution of (7S)-7-(azidomethyl)-3-chloro-6,7-dihydro[1,4]dioxino[2,3-c]pyridazine (250 mg) in ethanol (15 ml) was hydrogenated over 10% palladium on charcoal (120 mg) for 18 hours. The mixture was filtered and evaporated affording the product (220 mg).

### (e) Title compound

A mixture of [(7S)-6,7-dihydro[1,4]dioxino[2,3-c]pyridazin-7-ylmethyl]amine hydrochloride (220 mg), (1R/S)-9-fluoro-1-[(4-oxo-1-piperidinyl)methyl]-1,2-dihydro-4H-pyrrolo[3,2,1-ij]quinolin-4-one (330 mg), sodium acetate (540 mg), acetic acid (30 drops) and 3A molecular sieves in methanol/DCM (15 ml/15 ml) was stirred for 2 hours then treated with sodium cyanoborohydride (280 mg) and stirred overnight. The mixture was basified and extracted with 10% methanol in DCM followed by drying and evaporation. The residue was chromatographed eluting with 0-50% methanol/DCM affording the free base of the title compound (29 mg).
δH (CDCl3, 400MHz), 1.35-1.45 (2H, m), 1.85-1.95 (2H, m), 2.10-2.30 (2H, m), 2.45-2.55 (2H, m), 2.75-2.80 (1H, m), 2.88 (1H, dd), 2.95-3.05 (3H, m), 4.00-4.08 (1H, m), 4.20-4.55 (5H, m), 6.65 (1H, d), 6.90 (1H, t), 6.95 (1H, d), 7.45 (1H, m), 7.70 (1H, d), 8.68 (1H, d).
MS (+ve ion electrospray) m/z 452 (MH+).

This material (29 mg) was dissolved in methano/DCM and treated with a solution of fumaric acid in methanol followed by ether. The resultant precipitate was isolated by centrifugation and then dried *in vacuo* (13 mg).

### Biological Activity

### Antimicrobial Activity Assay:

Whole-cell antimicrobial activity was determined by broth microdilution using the Clinical and Laboratory Standards Institute (CLSI) recommended procedure, Document M7-A7, "Methods for Dilution Susceptibility Tests for Bacteria that Grow Aerobically". The compounds were tested in serial two-fold dilutions ranging from 0.016 to 16 mcg/mL.

Compounds were evaluated against a panel of Gram-positive organisms, including *Staphylococcus aureus, Streptococcus pneumoniae, Streptococcus pyogenes, Enterococcus faecalis* and *Enterococcus faecium.*

In addition, compounds were evaluated against a panel of Gram-negative strains including *Haemophilus influenzae, Moraxella catarrhalis, Escherichia coli, Pseudomonas aeruginosa, Proteus mirabilis, Legionella pneumophila, Chlamydia pneumoniae, Enterobacter cloacae, Enterobacter aerogenes, Klebsiella pneumoniae* and *Stenotrophomonas maltophilia.*

The minimum inhibitory concentration (MIC) was determined as the lowest concentration of compound that inhibited visible growth. A mirror reader was used to assist in determining the MIC endpoint.

Each of Examples 1-61, as identified in the present application, tested in at least one exemplified salt form, had a MIC ≤2µg/ml against a strain of at least one of the organisms listed above. Example 62 had an MIC ≤4µg/ml against a strain of at least one of the organisms listed above.

## Claims

1. A compound of formula (I) or a pharmaceutically acceptable salt and/or solvate thereof: 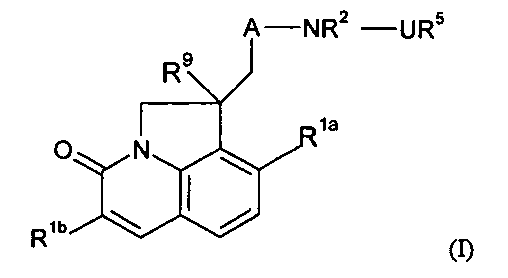 wherein:
R^{1a} and R^{1b} are independently selected from hydrogen; halogen; cyano; (C₁₋₆)alkyl; (C₁₋₆)alkylthio; trifluoromethyl; trifluoromethoxy; carboxy ; hydroxy optionally substituted with (C₁₋₆)alkyl or (C₁₋₆)alkoxy-substituted (C₁₋₆)alkyl; (C₁₋₆)alkoxy-substituted (C₁₋₆)alkyl; hydroxy (C₁₋₆)alkyl; an amino group optionally N-substituted by one or two (C₁₋₆)alkyl, formyl, (C₁₋₆)alkylcarbonyl or (C₁₋₆)alkylsulphonyl groups; or aminocarbonyl wherein the amino group is optionally substituted by (C₁₋₄)alkyl;
R² is hydrogen, or (C₁₋₄)alkyl, or together with R⁶ forms Y as defined below;
A is a group (ia) or (ib): 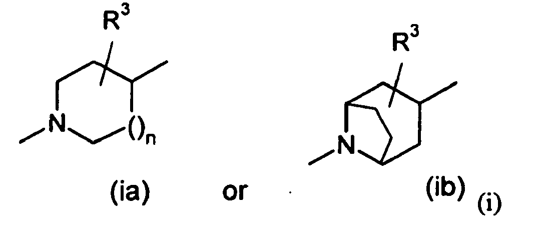 in which: R³ is as defined for R^{1a} or R^{1b} or is oxo and n is 1 or 2;
or A is a group (ii) 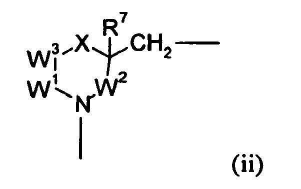 wherein:
W¹, W² and W³ are CR⁴R⁸;
or W² and W³ are CR⁴R⁸ and W¹ represents a bond between W³ and N;
X is O, CR⁴R⁸, or NR⁶;
one R⁴ is as defined for R^{1a} and R^{1b} and the remainder and R⁸ are hydrogen or one R⁴ and R⁸ are together oxo and the remainder are hydrogen;
R⁶ is hydrogen or (C₁₋₆)alkyl or together with R² forms Y;
R⁷ is hydrogen; halogen; hydroxy optionally substituted with (C₁₋₆)alkyl; or (C₁₋₆)alkyl;
Y is CR⁴R⁸CH₂; CH₂CR⁴R⁸; (C=O); CR⁴R⁸; CR⁴R⁸(C=O); or (C=O)CR⁴R⁸;
or when X is CR⁴R⁸, R⁸ and R⁷ together represent a bond;
U is selected from CO and CH₂;
R⁵ is an optionally substituted bicyclic carbocyclic or heterocyclic ring system (B): 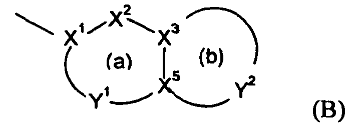 containing up to four heteroatoms in each ring in which
at least one of rings (a) and (b) is aromatic;
X¹ is C or N when part of an aromatic ring, or CR¹⁴ when part of a non-aromatic ring;
X² is N, NR¹³, O, S(O)ₓ, CO or CR¹⁴ when part of an aromatic or non-aromatic ring or may in addition be CR¹⁴R¹⁵ when part of a non aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring;
Y² is a 2 to 6 atom linker group, each atom of Y² being independently selected from N, NR¹³, O, S(O)ₓ, CO, CR¹⁴ when part of an aromatic or non-aromatic ring or may additionally be CR¹⁴R¹⁵ when part of a non aromatic ring;
each of R¹⁴ and R¹⁵ is independently selected from: H; (C₁₋₄)alkylthio; halo; carboxy(C₁₋₄)alkyl; (C₁₋₄)alkyl; (C₁₋₄)alkoxycarbonyl; (C₁₋₄)alkylcarbonyl; (C₁₋₄)alkoxy (C₁₋₄)alkyl; hydroxy; hydroxy(C₁₋₄)alkyl; (C₁₋₄)alkoxy; nitro; cyano; carboxy; amino or aminocarbonyl optionally mono- or di-substituted by (C₁₋₄)alkyl; or
R¹⁴ and R¹⁵ may together represent oxo;
each R¹³ is independently H; trifluoromethyl; (C₁₋₄₎alkyl optionally substituted by hydroxy, (C₁₋₆)alkoxy, (C₁₋₆)alkylthio, halo or trifluoromethyl; (C_{2- 4})alkenyl; (C₁₋₄)alkoxycarbonyl; (C₁₋₄)alkylcarbonyl; (C₁₋₆)alkylsulphonyl; or aminocarbonyl wherein the amino group is optionally mono or disubstituted by (C₁₋₄)alkyl;
each x is independently 0, 1 or 2; and
R⁹ is hydrogen or hydroxy.

2. A compound according to claim 1 wherein R^{1a} is methoxy, fluoro or cyano and R^{1b} is hydrogen.

3. A compound according to any preceding claim wherein R² is hydrogen.

4. A compound according to any preceding claim wherein R⁹ is hydrogen.

5. A compound according to any preceding claim wherein A is a group (ia) in which n is 1 and R³ is hydrogen or hydroxy.

6. A compound according to any of claims 1 to 4 wherein A is (ii), W¹ is a bond, X, W² and W³ are each CH₂ and R⁷ is H.

7. A compound according to any preceding claim wherein U is CH₂.

8. A compound according to any preceding claim wherein R⁵ is an aromatic heterocyclic ring (B) having 8-11 ring atoms including 2-4 heteroatoms of which at least one is N or NR¹³ in which Y² contains 2-3 heteroatoms, one of which is S and 1-2 are N, with one N bonded to X³, or the heterocyclic ring (B) has ring (a) aromatic selected from optionally substituted benzo, pyrido and pyridazino and ring (b) non aromatic and Y² has 3-5 atoms, including at least one heteroatom, with O, S, CH₂ or NR¹³ bonded to X⁵ where R¹³ is other than hydrogen, and either NHCO bonded via N to X³, or O, S, CH₂ or NH bonded to X³.

9. A compound according to any of claims 1 to 7 wherein R⁵ is selected from:
3-oxo-3,4-dihydro-2H-pyrido[3,2-b][1,4]oxazin-6-yl;
3-oxo-3,4-dihydro-2*H*pyrido[3,2-*b*][1,4]thiazin-6-yl;
6,7-Dihydro[1,4]dioxino[2,3-*c*]pyridazin-3-yl;
6,7-dihydro[1,4]oxathiino[2,3-*c*]pyridazin-3-yl;
6,7-dihydro[1,4]oxathiino[3,2*-c*]pyridazin-3-yl;
2,3-dihydro-[1,4]dioxino[2,3-c]pyridin-7-yl; and
[1,3]oxathiolo[5,4*-c*]pyridin-6-yl.

10. A compound according to claim 1 selected from:
1-({4-[(2,3-Dihydro[1,4]dioxino[2,3-c]pyridin-7-ylmethyl)amino]-1-piperidinyl}methyl)-9-fluoro-1,2-dihydro-4H-pyrrolo[3,2,1-ij]quinolin-4-one, Enantiomer E2;
1-({4-[(2,3-Dihydro[1,4]dioxino[2,3-c]pyridin-7-ylmethyl)amino]-1-piperidinyl}methyl)-9-fluoro-1,2-dihydro-4H-pyrrolo[3,2,1-ij]quinolin-4-one, Enantiomer E1;
1-({(3*R*,4*S*)-4-[(2,3-Dihydro[1,4]dioxino[2,3*-c*]pyridin-7-ylmethyl)amino]-3-hydroxy-1-piperidinyl}methyl)-9-fluoro-1,2-dihydro-4*H*-pyrrolo[3,2,1-*ij*]quinolin-4-one Diastereomer 1;
1-({(3*R*,4*S*)-4-[(2,3-Dihydro[1,4]dioxino[2,3*-c*]pyridin-7-ylmethyl)amino]-3-hydroxy-1-piperidinyl}methyl)-9-fluoro-1,2-dihydro-4*H*-pyrrolo[3,2,1-*ij*]quinolin-4-one Diastereomer 2;
9-Fluoro-1-({(3*R*,4*S*)-3-hydroxy-4-[([1,3]oxathiolo[5,4-*c*]pyridin-6-ylmethyl)amino]-1-piperidinyl}methyl)-1,2-dihydro-4*H-*pyrrolo[3,2,1-*ij*]quinolin-4-one;
9-Fluoro-1-[((3*R*,4*S*)-3-hydroxy-4-{[(6-oxo-6,7-dihydro-5*H*-pyridazino[3,4-*b*][1,4]thiazin-3-yl)methyl]amino}-1-piperidinyl)methyl]-1,2-dihydro-4*H-*pyrrolo[3,2,1-*ij*]quinolin-4-one;
1-({(3*R*,4*S*)-4-[(2,3-Dihydro-1*H*-pyrido[3,4-*b*][1,4]oxazin-7-ylmethyl)amino]-3-hydroxy-1-piperidinyl}methyl)-9-fluoro-1,2-dihydro-4*H*-pyrrolo[3,2,1-*ij*]quinolin-4-one;
1-({(3*R*,4*S*)-4-[(2,3-Dihydrofuro[2,3*-c*]pyridin-5-ylmethyl)amino]-3-hydroxy-1-piperidinyl}methyl)-9-fluoro-1,2-dihydro-4*H*-pyrrolo[3,2,1-*ij*]quinolin-4-one;
9-Fluoro-1-[((3*R*,4*S*)-3-hydroxy-4-{[(7-oxo-1,5,6,7-tetrahydro-1,8-naphthyridin-2-yl)methyl]amino}-1-piperidinyl)methyl]-1,2-dihydro-4*H* pyrrolo[3,2,1-*ij*]quinolin-4-one;
1-({4-[(6,7-Dihydro[1,4]dioxino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E2;
9-Fluoro-1-[(4-{[(3-oxo-3,4-dihydro-2*H*-pyrido[3,2-*b*][1,4]oxazin-6-yl)methyl]amino}-1-piperidinyl)methyl]-1,2-dihydro-4*H*-pyrrolo[3,2,1-*ij*]quinolin-4-one Enantiomer E1;
1-({4-[(2,3-dihydro[1,4]dioxino[2,3-*c*]pyridin-7-ylmethyl)amino]-1-piperidinyl}methyl)-4-oxo-1,2-dihydro-4*H*-pyrrolo[3,2,1-*ij*]quinoline-9-carbonitrile Enantiomer E1;
1-({4-[(2,3-Dihydro[1,4]dioxino[2,3*-c*]pyridin-7-ylmethyl)amino]-1-piperidinyl}methyl)-9-fluoro-1-hydroxy-1,2-dihydro-4*H*-pyrrolo[3,2,1-*ij*]quinolin-4-one Enantiomer E2;
1-({4-[(2,3-Dihydro[1,4]dioxino[2,3*-c*]pyridin-7-ylmethyl)amino]-1-piperidinyl}methyl)-9-fluoro-1-hydroxy-1,2-dihydro-4*H*-pyrrolo[3,2,1-*ij*]quinolin-4-one;
1-({4-[(2,3-Dihydro[1,4]oxathiino[2,3*-c*]pyridin-7-ylmethyl)amino]piperidin-1-yl}methyl)-9-fluoro-1-hydroxy-1,2-dihydro-4*H*-pyrrolo[3,2,1-*ij*]quinolin-4-one Enantiomer E 1;
9-Fluoro-1-hydroxy-1-[(4-{[(3-oxo-3,4-dihydro-2*H*-pyrido[3,2-*b*][1,4]oxazin-6-yl)methyl]amino}-1-piperidinyl)methyl]-1,2-dihydro-4*H*-pyrrolo[3,2,1-*ij*]quinolin-4-one;
1-({4-[(6,7-Dihydro[1,4]oxathiino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E 1;
1-({4-[(6,7-Dihydro[1,4]dioxino[2,3-*c*]pyridazin-3-ylmethyl)amino]-1-piperidinyl}methyl)-9-fluoro-1-hydroxy-1,2-dihydro-4*H*-pyrrolo[3,2,1-*ij*]quinolin-4-one;
1-({4-[(2,3-dihydro-1*H*-pyrido[3,4-*b*][1,4]oxazin-7-ylmethyl)amino]-1-piperidinyl}methyl)-9-fluoro-1,2-dihydro-4*H*-pyrrolo[3,2,1-*ij*]quinolin-4-one;
1-({4-[(2,3-Dihydro[1,4]oxathiino[2,3-*b*]pyridin-7-ylmethyl)amino]-1-piperidinyl}methyl)-9-fluoro-1,2-dihydro-4*H*-pyrrolo[3,2,1-*ij*]quinolin-4-one;
1-({4-[(2,3-Dihydro[1,4]dioxino[2,3-b]pyridin-7-ylmethyl)amino]-1-piperidinyl}methyl)-9-fluoro-1-hydroxy-1,2-dihydro-4*H*-pyrrolo[3,2,1-*ij*]quinolin-4-one Enantiomer E1;
1-({4-[(2,3-dihydro[1,4]dioxino[2,3-*b*]pyridin-7-ylmethyl)amino]-1-piperidinyl}methyl)-9-fluoro-1,2-dihydro-4*H*-pyrrolo[3,2,1-*ij*]quinolin-4-one;
1-({4-[(1,2,3-benzothiadiazol-5-ylmethyl)amino]-1-piperidinyl}methyl)-9-fluoro-1-hydroxy-1,2-dihydro-4*H*-pyrrolo[3,2,1-*ij*]quinolin-4-one;
1-({4-[(2,3-dihydro[1,4]dioxino[2,3*-c*]pyridin-7-ylmethyl)amino]-1-piperidinyl}methyl)-4-oxo-1,2-dihydro-4*H-*pyrrolo[3,2,1-*ij*]quinoline-9-carbonitrile Enantiomer E2;
1-({4-[(2,3-Dihydro[1,4]dioxino[2,3-*c*]pyridin-7-ylmethyl)amino]-1-piperidinyl}methyl)-9-fluoro-1-hydroxy-1,2-dihydro-4*H*-pyrrolo[3,2,1-*ij*]quinolin-4-one Enantiomer E1;
9-Fluoro-1-[(4-{[(5-oxo-1,2,3,5-tetrahydro-7-indolizinyl)methyl]amino}-1-piperidinyl)methyl]-1,2-dihydro-4*H*-pyrrolo[3,2,1-*ij*]quinolin-4-one;
1-({4-[(2,3-Dihydro[1,4]oxathiino[2,3-*c*]pyridin-7-ylmethyl)amino]-1-piperidinyl}methyl)-9-fluoro-1-hydroxy-1,2-dihydro-4*H*-pyrrolo[3,2,1-*ij*]quinolin-4-one Enantiomer E2;
1-({4-[(2,3-Dihydro[1,4]dioxino[2,3-*b*]pyridin-7-ylmethyl)amino]-1-piperidinyl}methyl)-9-fluoro-1-hydroxy-1,2-dihydro-4*H*-pyrrolo[3,2,1-*ij*]quinolin-4-one Enantiomer E2;
1-({4-[(6,7-Dihydro[1,4]oxathiino[2,3-*c*]pyridazin-3-ylmethyl)amino]-1-piperidinyl}methyl)-9-fluoro-1-hydroxy-1,2-dihydro-4*H*-pyrrolo[3,2,1-*ij*]quinolin-4-one Enantiomer E1;
1-({4-[(6,7-Dihydro[1,4]oxathiino[3,2-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E1;
1-({4-[(6,7-Dihydro-5H-pyrano[2,3-*c*]pyridazin-3-ylmethyl)amino]-1-piperidinyl}methyl)-9-fluoro-1,2-dihydro-4H-pyrrolo[3,2,1-*ij*]quinolin-4-one Enantiomer E 1;
9-Fluoro-1-[((3*R*)-3-{[([1,3]oxathiolo[5,4-*c*]pyridin-6-ylmethyl)amino]methyl}-1-pyrrolidinyl)methyl]-1,2-dihydro-4*H*-pyrrolo[3,2,1-*ij*]quinolin-4-one;
1-{[(3*R*)-3-({[(7-Chloro-3-oxo-3,4-dihydro-2*H*-pyrido[3,2-*b*][1,4]oxazin-6-yl)methyl]amino}methyl)-1-pyrrolidinyl]methyl}-9-fluoro-1,2-dihydro-4*H-*pyrrolo[3,2,1*-ij*]quinolin-4-one;
9-Fluoro-1-{[(3*R*)-3-({[(3-oxo-3,4-dihydro-2*H*-pyrido[3,2-*b*][1,4]thiazin-6-yl)methyl]amino}methyl)-1-pyrrolidinyl]methyl}-1,2-dihydro-4*H*-pyrrolo[3,2,1-*ij*]quinolin-4-one;
9-Fluoro-1-{[(3*R*)-3-({[(3-oxo-3,4-dihydro-2*H*-pyrido[3,2-*b*][1,4]oxazin-6-yl)methyl]amino}methyl)-1-pyrrolidinyl]methyl}-1,2-dihydro-4*H*-pyrrolo[3,2,1-ij]quinolin-4-one;
1-[((3*R*)-3-{[(2,3-Dihydro[1,4]dioxino[2,3*-c*]pyridin-7-ylmethyl)amino]methyl}-1-pyrrolidinyl)methyl]-9-fluoro-1,2-dihydro-4*H-*pyrrolo[3,2,1-*ij*]quinolin-4-one;
9-Fluoro-1-[(3-{[([1,3]oxathiolo[5,4-*c*]pyridin-6-ylmethyl)amino]methyl}-1-pyrrolidinyl)methyl]-1,2-dihydro-4*H*-pyrrolo[3,2,1-*ij*]quinolin-4-one;
1-{[3-({[(7-Chloro-3-oxo-3,4-dihydro-2*H*-pyrido[3,2-*b*][1,4]oxazin-6-yl)methyl]amino}methyl)-1-pyrrolidinyl]methyl}-9-fluoro-1,2-dihydro-4*H-*pyrrolo[3,2,1-*ij*]quinolin-4-one;
1-{[3-({[(7-Chloro-3-oxo-3,4-dihydro-2*H*-pyrido[3,2-*b*][1,4]oxazin-6-yl)methyl]amino}methyl)-1-pyrrolidinyl]methyl}-9-fluoro-1,2-dihydro-4*H-*pyrrolo[3,2,1-*ij*]quinolin-4-one Isomer 1, 2, 3 or 4;
1-[(4-{[(3-Oxo-3,4-dihydro-2*H*-pyrido[3,2-*b*][1,4]thiazin-6-yl)methyl]amino}-1-piperidinyl)methyl]-1,2-dihydro-4*H*-pyrrolo[3,2,1-*ij*]quinolin-4-one;
1-({4-[(2,3-Dihydro[1,4]dioxino[2,3*-c*]pyridin-7-ylmethyl)amino]-1-piperidinyl}methyl)-1,2-dihydro-4*H* pyrrolo[3,2,1-*ij*]quinolin-4-one;
1-({4-[([1,3]Oxathiolo[5,4*-c*]pyridin-6-ylmethyl)amino]-1-piperidinyl}methyl)-1,2-dihydro-4*H*-pyrrolo[3,2,1-*ij*]quinolin-4-one;
1-[(4-{[(7-Chloro-3-oxo-3,4-dihydro-2*H*-dihydro[3,2-*b*][1,4]oxazin-6-yl)methyl]amino}-1-piperidinyl)methyl]-1,2-dihydro-4*H*-pyrrolo[3,2,1-*ij*]quinolin-4-one;
1-({4-[(3,4-Dihydro-2*H*-pyrano[2,3*-c*]pyridin-6-ylmethyl)amino]-1-piperidinyl}methyl)-1,2-dihydro-4*H*-pyrrolo[3,2,1-*ij*]quinolin-4-one;
1-[(3-{[(2,3-Dihydro[1,4]dioxino[2,3-*c*]pyridin-7-ylmethyl)amino]methyl}-1-pyrrolidinyl)methyl]-9-fluoro-1,2-dihydro-4*H*-pyrrolo[3,2,1-*ij*]quinolin-4-one;
1-({4-[(6,7-Dihydro[1,4]oxathiino[2,3-*c*]pyridazin-3-ylmethyl)amino]-1-piperidinyl}methyl)-9-fluoro-1-hydroxy-1,2-dihydro-4*H*-pyrrolo[3,2,1-*ij*]quinolin-4-one Enantiomer E2;
9-Fluoro-1-{[3-({[(3-oxo-3,4-dihydro-2*H*-pyrido[3,2-*b*][1,4]thiazin-6-yl)methyl]amino}methyl)-1-pyrrolidinyl]methyl}-1,2-dihydro-4*H*-pyrrolo[3,2,1-ij]quinolin-4-one;
1-({4-[(6,7-Dihydro[1,4]dioxino[2,3*-c*]pyridazin-3-ylmethyl)amino]-1-piperidinyl}methyl)-4-oxo-1,2-dihydro-4*H*-pyrrolo[3,2,1-*ij*]quinoline-9-carbonitrile (Enantiomer E1);
1-({4-[(6,7-Dihydro[1,4]dioxino[2,3-*c*]pyridazin-3-ylmethyl)amino]-1-piperidinyl}methyl)-4-oxo-1,2-dihydro-4*H*-pyrrolo[3,2,1-*ij*]quinoline-9-carbonitrile (Enantiomer E2);
1-(R/S)-[(4-{[(3*S*)-2,3-Dihydro[1,4]dioxino[2,3-*b*]pyridin-3-ylmethyl]amino}-1-piperidinyl)methyl]-9-fluoro-1,2-dihydro-4*H*-pyrrolo[3,2,1-*ij*]quinolin-4-one;
1-({4-[(6,7-Dihydro-5*H*-pyrano[2,3-*c*]pyridazin-3-ylmethyl)amino]-1-piperidinyl}methyl)-4-oxo-1,2-dihydro-4*H*-pyrrolo[3,2,1-*ij*]quinoline-9-carbonitrile Enantiomer E1;
1-({4-[(6,7-dihydro-5*H*-pyrano[2,3-*c*]pyridazin-3-ylmethyl)amino]-1-piperidinyl}methyl)-9-fluoro-1-hydroxy-1,2-dihydro-4*H*-pyrrolo[3,2,1-*ij*]quinolin-4-one Enantiomer E1;
9-Fluoro-1-[(4-{[(6-oxo-6,7-dihydro-5*H*-pyridazino[3,4-*b*][1,4]thiazin-3-yl)methyl]amino}-1-piperidinyl)methyl]-1,2-dihydro-4*H*-pyrrolo[3,2,1-*ij*]quinolin-4-one Enantiomer E1;
1-({4-[(2,3-dihydro[1,4]dioxino[2,3-*c*]pyridin-7-ylmethyl)amino]-1-piperidinyl}methyl)-1-hydroxy-4-oxo-1,2-dihydro-4*H*-pyrrolo[3,2,1-*ij*]quinoline-9-carbonitrile Enantiomer E1;
1-({4-[(2,3-dihydro[1,4]dioxino[2,3-*c*]pyridin-7-ylmethyl)amino]-1-piperidinyl}methyl)-1-hydroxy-4-oxo-1,2-dihydro-4H-pyrrolo[3,2,1-*ij*]quinoline-9-carbonitrile Enantiomer E2;
1-[(4-{[(7-Chloro-3-oxo-3,4-dihydro-2*H*-pyrido[3,2-*b*][1,4]oxazin-6-yl)methyl]amino}-1-piperidinyl)methyl]-9-fluoro-1,2-dihydro-4*H*-pyrrolo[3,2,1-ij]quinolin-4-one Enantiomer E1;
1-({4-[(2,3-Dihydro[1,4]dioxino[2,3-*c*]pyridin-7-ylmethyl)amino]-1-piperidinyl}methyl)-9-(methyloxy)-1,2-dihydro-4H-pyrrolo[3,2,1-ij]quinolin-4-one Enantiomer E 1;
1-({4-[(2,3-Dihydro[1,4]dioxino[2,3-c]pyridin-7-ylmethyl)amino]-1-piperidinyl}methyl)-9-(methyloxy)-1,2-dihydro-4H-pyrrolo[3,2,1-ij]quinolin-4-one, Enantiomer E2;
1-({4-[(6,7-Dihydro[1,4]dioxino[2,3-*c*]pyridazin-3-ylmethyl)amino]-1-piperidinyl}methyl)-9-(methyloxy)-1,2-dihydro-4*H*-pyrrolo[3,2,1-*ij*]quinolin-4-one, Enantiomer 1;
9-Fluoro-1-{[4-({[(7*R*/*S*)-7-(hydroxymethyl)-6,7-dihydro[1,4]dioxino[2,3-*c*]pyridazin-3-yl]methyl}amino)-1-piperidinyl]methyl}-1,2-dihydro-4*H*-pyrrolo[3,2,1-*ij*]quinolin-4-one Diastereomer D1;
1-({4-[(5,6-Dihydrofuro[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E1;
1-({4-[(5,6-dihydrofuro[2,3-*c*]pyridazin-3-ylmethyl)amino]-1-piperidinyl}methyl)-9-fluoro-1-hydroxy-1,2-dihydro-4*H*-pyrrolo[3,2,1-*ij*]quinolin-4-one Enantiomer E1; and
(1*R*/*S*)-1-[(4-{[(7*S*)-6,7-dihydro[1,4]dioxino[2,3-*c*]pyridazin-7-ylmethyl]amino}-1-piperidinyl)methyl]-9-fluoro-1,2-dihydro-4*H*-pyrrolo[3,2,1-*ij*]quinolin-4-one;
or a pharmaceutically acceptable salt and/or solvate thereof.

11. A compound according to claim 1 which is 1-({4-[(6,7-dihydro[1,4]dioxino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E1 or a pharmaceutically acceptable salt and/or solvate thereof.

12. A compound according to claim 1 which is 1-({4-[(6,7-Dihydro[1,4]dioxino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E1 Mono 4-methylbenzene sulphonate salt.

13. A compound according to claim 11 which is 1-({4-[(6,7-Dihydro[1,4]dioxino[2,3-*c*]pyridazin-3-ylmethyl)amino]-1-piperidinyl}methyl)-9-fluoro-1,2-dihydro-4*H-*pyrrolo[3,2,1-*ij*]quinolin-4-one Enantiomer E1 Mono (2E)-2-butenedioate salt.

14. A compound according to claim 13 which is 1-({4-[(6,7-Dihydro[1,4]dioxino[2,3-*c*]pyridazin-3-ylmethyl)amino]-1-piperidinyl}methyl)-9-fluoro-1,2-dihydro-4*H-*pyrrolo[3,2,1*-ij*]quinolin-4-one Enantiomer E1 Mono (2E)-2-butenedioate salt anhydrate I.

15. A compound according to claim 13 which is 1-({4-[(6,7-Dihydro[1,4]dioxino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one Enantiomer E1 Mono (2E)-2-butenedioate salt trihydrate.

16. A compound according to any of claims 1 to 10 or 12 to 15 for use in the treatment of bacterial infections in mammals.

17. A compound according to claim 11 for use in the treatment of bacterial infections in mammals.

18. A compound according to claim 17 for use in the treatment of bacterial infections in mammals wherein the bacterial infection is caused by a gram positive organism selected from *Staphylococcus aureus, Streptococcus pneumoniae, Streptococcus pyogenes, Enterococcus faecalis* and *Enterococcus faecium;* or a gram negative organism selected from the group consisting of *Haemophilus influenzae, Moraxella catarrhalis, Escherichia coli, Pseudomonas aeruginosa, Proteus mirabilis, Legionella pneumophila, Chlamydia pneumoniae, Enterobacter cloacae, Enterobacter aerogenes, Klebsiella pneumoniae* and *Stenotrophomonas maltophilia.*

19. A pharmaceutical composition comprising a compound according to any one of claims 1 to 10 or 12 to 15 and a pharmaceutically acceptable carrier.

20. A pharmaceutical composition comprising a compound according to claim 11 and a pharmaceutically acceptable carrier.

21. A compound of formula (IIB): 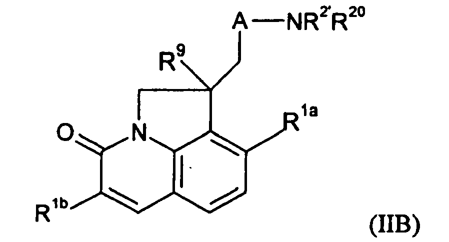 in which R²⁰ is hydrogen and R^{2'} is R² or a group convertible thereto, wherein A, R^{1a}, R^{1b}, R² and R⁹ are as defined in claim 1.

22. A compound according to claim 21 selected from:
1-[(4-Amino-1-piperidinyl)methyl]-9-fluoro-1,2-dihydro-4H-pyrrolo[3,2,1-ij]quinolin-4-one enantiomer E1;
1-[(4-Amino-1-piperidinyl)methyl]-9-fluoro-1,2-dihydro-4H-pyrrolo[3,2,1-ij]quinolin-4-one enantiomer E2;
1-{[(3R,4S)-4-amino-3-hydroxy-1-piperidinyl]methyl}-9-fluoro-1,2-dihydro-4H-pyrrolo[3,2,1-ij]quinolin-4-one;
1-[(4-Amino-1-piperidinyl)methyl]-9-fluoro-1-hydroxy-1,2-dihydro-4*H-*pyrrolo[3,2,1*-ij*]quinolin-4-one;
1-{[(3*R*)-3-(Aminomethyl)-1-pyrrolidinyl]methyl}-9-fluoro-1,2-dihydro-4*H-*pyrrolo[3,2,1*-ij*]quinolin-4-one;
1-[(4-Amino-1-piperidinyl)methyl]-1,2-dihydro-4*H*-pyrrolo[3,2,1-*ij*]quinolin-4-one;
1-[(4-Amino-1-piperidinyl)methyl]-4-oxo-1,2-dihydro-4H-pyrrolo[3,2,1-ij]quinoline-9-carbonitrile; and
1-[(4-Amino-1-piperidinyl)methyl]-9-(methyloxy)-1,2-dihydro-4-H-pyrrolo[3,2,1-ij]quinolin-4-one.

23. 6,7-Dihydro[1,4]dioxino[2,3-c]pyridazine-3-carbaldehyde.

## Patentansprüche

1. Eine Verbindung der Formel (I) oder ein pharmazeutisch verträgliches Salz und/oder Solvat davon: 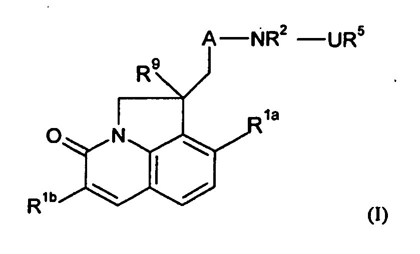 wobei:
R^{1a} und R^{1b} unabhängig ausgewählt sind aus Wasserstoff; Halogen; Cyano; (C₁₋₆)-Alkyl; (C₁-₆)-Alkylthio; Trifluormethyl; Trifluormethoxy; Carboxy; Hydroxy, gegebenenfalls substituiert mit (C₁₋₆)-Alkyl oder (C₁₋₆)-Alkoxy-substituiertem (C₁-₆)-Alkyl; (C₁₋₆)-Alkoxy-substituiertem (C₁₋₆)-Alkyl; Hydroxy-(C₁₋₆)-alkyl; einer Aminogruppe, gegebenenfalls N-substituiert mit einem oder zwei (C₁₋₆)-Alkyl-, Formyl-, (C₁₋₆)-Alkylcarbonyl- oder (C₁₋₆)-Alkylsulfonylresten; oder Aminocarbonyl, wobei die Aminogruppe gegebenenfalls mit (C₁₋₄)-Alkyl substituiert ist;
R² Wasserstoff oder (C₁₋₄)-Alkyl ist oder, zusammen mit R⁶, Y wie nachstehend definiert bildet;
A ein Rest (ia) oder (ib) ist: 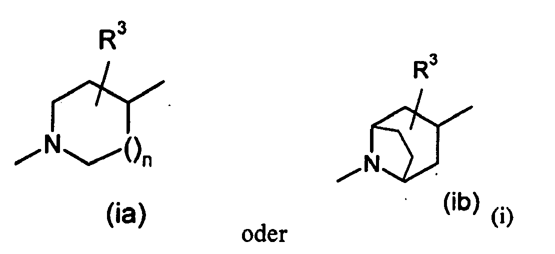 wobei R³ wie für R^{1a} oder R^{1b} definiert ist oder Oxo ist und n 1 oder 2 ist;
oder A ein Rest (ii) ist 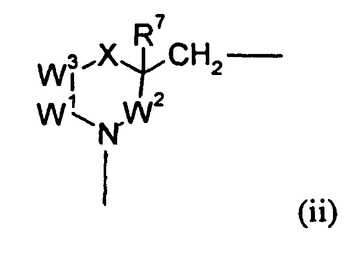 wobei:
W¹, W² und W³ für CR⁴R⁸ stehen;
oder W² und W³ CR⁴R⁸ sind und W¹ eine Bindung zwischen W³ und N darstellt;
X für O, CR⁴R⁸ oder NR⁶ steht;
ein Rest R⁴ wie für R^{1a} und R^{1b} definiert ist und der Restbestand und R⁸ Wasserstoff sind oder ein Rest R⁴ und R⁸ zusammen Oxo sind und der Restbestand Wasserstoff sind;
R⁶ Wasserstoff oder (C₁₋₆)-Alkyl ist oder zusammen mit R² den Rest Y bildet;
R⁷ Wasserstoff; Halogen; Hydroxy, gegebenenfalls substituiert mit (C₁₋₆)-Alkyl; oder (C₁₋₆)-Alkyl ist;
Y für CR⁴R⁸CH₂; CH₂CR⁴R⁸; (C=O); CR⁴R⁸; CR⁴R⁸(C=O) oder (C=O)CR⁴R⁸ steht; oder wenn X für CR⁴R⁸ steht, R⁸ und R⁷ zusammen eine Bindung darstellen;
U ausgewählt ist aus CO und CH₂;
R⁵ ein gegebenenfalls substituiertes, bicyclisch carbocyclisches oder heterocyclisches Ringsystem (B) ist: 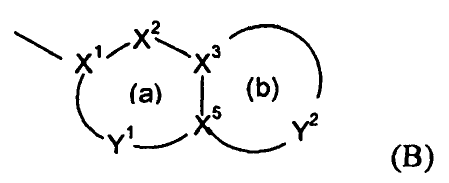 welches bis zu vier Heteroatome in jedem Ring enthält, wobei
mindestens einer der Ringe (a) und (b) aromatisch ist;
X¹ für C oder N steht, wenn es Teil eines aromatischen Rings ist, oder für CR¹⁴ steht, wenn es Teil eines nicht-aromatischen Rings ist;
X² für N, NR¹³, O, S(O)ₓ, CO oder CR¹⁴ steht, wenn es Teil eines aromatischen oder nicht-aromatischen Rings ist, oder zusätzlich CR¹⁴R¹⁵ sein kann, wenn es Teil eines nicht-aromatischen Rings ist;
X³ und X⁵ unabhängig N oder C sind;
Y¹ ein Linkerrest mit 0 bis 4 Atomen ist, wobei jedes Atom unabhängig ausgewählt ist aus N, NR¹³, O, S(O)ₓ, CO und CR¹⁴, wenn es Teil eines aromatischen oder nicht-aromatischen Rings ist, oder zusätzlich CR¹⁴R¹⁵ sein kann, wenn es Teil eines nicht-aromatischen Rings ist;
Y² ein Linkerrest mit 2 bis 6 Atomen ist, wobei jedes Atom von Y² unabhängig ausgewählt ist aus N, NR¹³, O, S(O)ₓ, CO, CR¹⁴, wenn es Teil eines aromatischen oder nicht-aromatischen Rings ist, oder zusätzlich CR¹⁴R¹⁵ sein kann, wenn es Teil eines nicht-aromatischen Rings ist;
R¹⁴ und R¹⁵ jeweils unabhängig ausgewählt sind aus H; (C₁₋₄)-Alkylthio; Halogen; Carboxy-(C₁₋₄)-alkyl; (C₁₋₄)-Alkyl; (C₁₋₄)-Alkoxycarbonyl; (C₁₋₄)-Alkylcarbonyl; (C₁₋₄)-Alkoxy-(C₁₋₄)-alkyl; Hydroxy; Hydroxy-(C₁₋₄)-alkyl; (C₁₋₄)-Alkoxy; Nitro; Cyano; Carboxy; Amino oder Aminocarbonyl, gegebenenfalls mit (C₁₋₄)-Alkyl mono- oder disubstituiert;
oder
R¹⁴ und R¹⁵ zusammen Oxo darstellen können;
jeder Rest R¹³ unabhängig H; Trifluormethyl; (C₁₋₄)-Alkyl, gegebenenfalls substituiert mit Hydroxy, (C₁₋₆)-Alkoxy, (C₁₋₆)-Alkylthio, Halogen oder Trifluormethyl; (C₂₋₄)-Alkenyl; (C₁₋₄)-Alkoxycarbonyl; (C₁₋₄)-Alkylcarbonyl; (C₁₋₆)-Alkylsulfonyl; oder Aminocarbonyl, wobei die Aminogruppe gegebenenfalls mit (C₁₋₄)-Alkyl mono- oder disubstituiert ist, ist;
jedes x unabhängig 0, 1 oder 2 ist; und
R⁹ Wasserstoff oder Hydroxy ist.

2. Eine Verbindung gemäß Anspruch 1, wobei R^{1a} Methoxy, Fluor oder Cyano ist und R^{1b} Wasserstoff ist.

3. Eine Verbindung gemäß einem der vorstehenden Ansprüche, wobei R² Wasserstoff ist.

4. Eine Verbindung gemäß einem der vorstehenden Ansprüche, wobei R⁹ Wasserstoff ist.

5. Eine Verbindung gemäß einem der vorstehenden Ansprüche, wobei A ein Rest (ia) ist, wobei n gleich 1 ist und R³ Wasserstoff oder Hydroxy ist.

6. Eine Verbindung gemäß einem der Ansprüche 1 bis 4, wobei A (ii) ist, W¹ eine Bindung ist, X, W² und W³ jeweils CH₂ sind und R⁷ für H steht.

7. Eine Verbindung gemäß einem der vorstehenden Ansprüche, wobei U für CH₂ steht.

8. Eine Verbindung gemäß einem der vorstehenden Ansprüche, wobei R⁵ ein aromatischer heterocyclischer Ring (B) mit 8- bis 11 Ringatomen, einschließlich 2 bis 4 Heteroatomen, von denen mindestens eines N oder NR¹³ ist, ist, wobei Y² 2 bis 3 Heteroatome enthält, von denen eines S ist und 1 bis 2 N sind, wobei ein N an X³ gebunden ist, oder der heterocyclische Ring (B) einen aromatischen Ring (a), ausgewählt aus gegebenenfalls substituiertem Benzo, Pyrido und Pyridazino, und einen nicht-aromatischen Ring (b) aufweist und Y² 3 bis 5 Atome aufweist, einschließlich mindestens einem Heteroatom, wobei O, S, CH₂ oder NR¹³ an X⁵ gebunden sind, wobei R¹³ von Wasserstoff verschieden ist und entweder NHCO durch N an X³ gebunden ist oder O, S, CH₂ oder NH an X³ gebunden ist.

9. Eine Verbindung gemäß einem der Ansprüche 1 bis 7, wobei R⁵ ausgewählt ist aus:
3-Oxo-3,4-dihydro-2H-pyrido[3,2-b][1,4]oxazin-6-yl;
3-Oxo-3,4-dihydro-2H-pyrido[3,2-b][1,4]thiazin-6-yl;
6,7-Dihydro[1,4]dioxino[2,3-c]pyridazin-3-yl;
6,7-Dihydro[1,4]oxathiino[2,3-c]pyridazin-3-yl;
6,7-Dihydro[1,4]oxathüno[3,2-c]pyridazin-3-yl;
2,3-Dihydro-[1,4]dioxino[2,3-c]pyridin-7-yl; und
[1,3]Oxathiolo[5,4-c]pyridin-6-yl.

10. Eine Verbindung gemäß Anspruch 1, ausgewählt aus
1-({4-[(2,3-Dihydro[1,4]dioxino[2,3-c]pyridin-7-ylmethyl)amino]-1-piperidinyl}methyl)-9-fluor-1,2-dihydro-4H-pyrrolo[3,2,1-ij]chinolin-4-on, Enantiomer E2;
1-({4-[(2,3-Dihydro[1,4]dioxino[2,3-c]pyridin-7-ylmethyl)amino]-1-piperidinyl}methyl)-9-fluor-1,2-dihydro-4H-pyrrolo[3,2,1-ij]chinolin-4-on, Enantiomer E1;
1-({(3R,4S)-4-[(2,3-Dihydro[1,4]dioxino[2,3-c]pyridin-7-ylmethyl)amino]-3-hydroxy-1-piperidinyl}methyl)-9-fluor-1,2-dihydro-4H-pyrrolo[3,2,1-ij]chinolin-4-on Diastereomer 1;
1-({(3R,4S)-4-[(2,3-Dihydro[1,4]dioxino[2,3-c]pyridin-7-ylmethyl)amino]-3-hydroxy-1-piperidinyl}methyl)-9-fluor-1,2-dihydro-4H-pyrrolo[3,2,1-ij]chinolin-4-on Diastereomer 2;
9-Fluor-1-({(3R,4S)-3-hydroxy-4-[([1,3]oxathiolo[5,4-c]pyridin-6-ylmethyl)amino]-1-piperidinyl}methyl)-1,2-dihydro-4H-pyrrolo[3,2,1-ij]chinolin-4-on;
9-Fluor-1-[((3R,4S)-3-hydroxy-4-{[(6-oxo-6,7-dihydro-5H-pyridazino[3,4-b][1,4]-thiazin-3-yl)methyl]amino}-1-piperidinyl)methyl]-1,2-dihydro-4H-pyrrolo[3,2,1-ij]chinolin-4-on;
1-({(3R,4S)-4-[(2,3-Dihydro-1H-pyrido[3,4-b][1,4]oxazin-7-ylmethyl)amino]-3-hydroxy-1-piperidinyl}methyl)-9-fluor-1,2-dihydro-4H-pyrrolo[3,2,1-ij]chinolin-4-on;
1-({(3R,4S)-4-[(2,3 -Dihydrofuro[2,3-c]pyridin-5-ylmethyl)amino]-3-hydroxy-1-piperidinyl}methyl)-9-fluor-1,2-dihydro-4H-pyrrolo[3,2,1-ij]chinolin-4-on;
9-Fluor-1-[((3R,4S)-3-hydroxy-4-{[(7-oxo-1,5,6,7-tetrahydro-1,8-naphthyridin-2-yl)methyl]amino}-1-piperidinyl)methyl]-1,2-dihydro-4H-pyrrolo[3,2,1-ij]chinolin-4-on;
1-({4-[(6,7-Dihydro[1,4]dioxino[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E2;
9-Fluor-1-[(4-{[(3-oxo-3,4-dihydro-2H-pyrido[3,2-b][1,4]oxazin-6-yl)methyl]amino}-1-piperidinyl)methyl]-1,2-dihydro-4H-pyrrolo[3,2,1-ij]chinolin-4-on Enantiomer E1;
1-({4-[(2,3-Dihydro[1,4]dioxino[2,3-c]pyridin-7-ylmethyl)amino]-1-piperidinyl}methyl)-4-oxo-1,2-dihydro-4H-pyrrolo[3,2,1-ij]chinolin-9-carbonitril Enantiomer E1;
1-({4-[(2,3-Dihydro[1,4]dioxino[2,3-c]pyridin-7-ylmethyl)amino]-1-piperidinyl}methyl)-9-fluor-1-hydroxy-1,2-dihydro-4H-pyrrolo[3,2,1-ij]chinolin-4-on Enantiomer E2;
1-({4-[(2,3-Dihydro[1,4]dioxino[2,3-c]pyridin-7-ylmethyl)amino]-1-piperidinyl}methyl)-9-fluor-1-hydroxy-1,2-dihydro-4H-pyrrolo[3,2,1-ij]chinolin-4-on;
1-({4-[(2,3-Dihydro[1,4]oxathiino[2,3-c]pyridin-7-ylmethyl)amino]piperidin-1-yl}methyly9-fluor-1-hydroxy-1,2-dihydro-4H-pyrrolo[3,2,1-ij]chinolin-4-on Enantiomer E1;
9-Fluor-1-hydroxy-1-[(4-{[(3-oxo-3,4-dihydro-2H-pyrido[3,2-b][1,4]oxazin-6-yl)methyl]amino}-1-piperidinyl)methyl]-1,2-dihydro-4H-pyrrolo[3,2,1-ij]chinolin-4-on;
1-({4-[(6,7-Dihydro[1,4]oxathiino[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E1;
1-({4-[(6,7-Dihydro[1,4]dioxino[2,3-c]pyridazin-3-ylmethyl)amino]-1-piperidinyl}-methyl)-9-fluor-1-hydroxy-1,2-dihydro-4H-pyrrolo[3,2,1-ij]chinolin-4-on;
1-({4-[(2,3-Dihydro-1H-pyrido[3,4-b][1,4]oxazin-7-ylmethyl)amino]-1-piperidinyl}-methyl)-9-fluor-1,2-dihydro-4H-pyrrolo[3,2,1-ij]chinolin-4-on;
1-({4-[(2,3-Dihydro[1,4]oxathiino[2,3-b]pyridin-7-ylmethyl)amino]-1-piperidinyl}-methyl)-9-fluor-1,2-dihydro-4H-pyrrolo[3,2,1-ij]chinolin-4-on;
1-({4-[(2,3-Dihydro[1,4]dioxino[2,3-b]pyridin-7-ylmethyl)amino]-1-piperidinyl}methyl)-9-fluor-1-hydroxy-1,2-dihydro-4H-pyrrolo[3,2,1-ij]chinolin-4-on Enantiomer E1;
1-({4-[(2,3-Dihydro[1,4]dioxino[2,3-b]pyridin-7-ylmethyl)amino]-1-piperidinyl}methyl)-9-fluor-1,2-dihydro-4H-pyrrolo[3,2,1-ij]chinolin-4-on;
1-({4-[(1,2,3-Benzothiadiazol-5-ylmethyl)amino]-1-piperidinyl}methyl)-9-fluor-1-hydroxy-1,2-dihydro-4H-pyrrolo[3,2,1-ij]chinolin-4-on;
1-({4-[(2,3-Dihydro[1,4]dioxino[2,3-c]pyridin-7-ylmethyl)amino]-1-piperidinyl}methyl)-4-oxo-1,2-dihydro-4H-pyrrolo[3,2,1-ij]chinolin-9-carbonitril Enantiomer E2;
1-({4-[(2,3-Dihydro[1,4]dioxino[2,3-c]pyridin-7-ylmethyl)amino]-1-piperidinyl}methyl)-9-fluor-1-hydroxy-1,2-dihydro-4H-pyrrolo[3,2,1-ij]chinolin-4-on Enantiomer E1;
9-Fluor-1-[(4-{[(5-oxo-1,2,3,5-tetrahydro-7-indolizinyl)methyl]amino}-1-piperidinyl)-methyl]-1,2-dihydro-4H-pyrrolo[3,2,1-ij]chinolin-4-on;
1-({4-[(2,3-Dihydro[1,4]oxathiino[2,3-c]pyridin-7-ylmethyl)amino]-1-piperidinyl}-methyl)-9-fluor-1-hydroxy-1,2-dihydro-4H-pyrrolo[3,2,1-ij]chinolin-4-on Enantiomer E2;
1-({4-[(2,3-Dihydro[1,4]dioxino[2,3-b]pyridin-7-ylmethyl)amino]-1-piperidinyl}methyl)-9-fluor-1-hydroxy-1,2-dihydro-4H-pyrrolo[3,2,1-ij]chinolin-4-on Enantiomer E2;
1-({4-[(6,7-Dihydro[1,4]oxathiino[2,3-c]pyridazin-3-ylmethyl)amino]-1-piperidinyl}-methyl)-9-ftuor-1-hydroxy-1,2-dihydro-4H-pyrrolo[3,2,1-ij]chinolin-4-on Enantiomer E1;
1-({4-[(6,7-Dihydro[1,4]oxathiino[3,2-c]pyridazin-3-ylmethyl)amino]-1-piperidinyl}-methyl)-9-fluor-1,2-dihydro-4H-pyrrolo[3,2,1-ij]chinolin-4-on Enantiomer E1;
1-({4-[(6,7-Dihydro-5H-pyrano[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E1;
9-Fluor-1-[((3R)-3-{[([1,3]oxathiolo[5,4-c]pyridin-6-ylmethyl)amino]methyl}-1-pyrrolidinyl)methyl]-1,2-dihydro-4H-pyrrolo[3,2,1-ij]chinolin-4-on;
1-{[(3R)-3-({[(7-Chlor-3-oxo-3,4-dihydro-2H-pyrido[3,2-b][1,4]oxazin-6-yl)methyl]-amino}methyl)-1-pyrrolidinyl]methyl}-9-fluor-1,2-dihydro-4H-pyrrolo[3,2,1-ij]chinolin-4-on;
9-Fluor-1-{[(3R)-3-({[(3-oxo-3,4-dihydro-2H-pyrido[3,2-b][1,4]thiazin-6-yl)methyl]-amino}methyl)-1-pyrrolidinyl]methyl}-1,2-dihydro-4H-pyrrolo[3,2,1-ij]chinolin-4-on;
9-Fluor-1-{[(3R)-3-({[(3-oxo-3,4-dihydro-2H-pyrido[3,2-b][1,4]oxazin-6-yl)methyl]-amino}methyl)-1-pyrrolidinyl]methyl}-1,2-dihydro-4H-pyrrolo[3,2,1-ij]chinolin-4-on;
1-[((3R)-3-{ [(2,3-Dihydro[1,4]dioxino[2,3-c]pyridin-7-ylmethyl)amino]methyl}-1-pyrrolidinyl)methyl]-9-fluor-1,2-dihydro-4H-pyrrolo[3,2,1-ij]chinolin-4-on;
9-Fluor-1-[(3-{[([1,3]oxathiolo[5,4-c]pyridin-6-ylmethyl)amino]methyl}-1-pyrrolidinyl)methyl]-1,2-dihydro-4H-pyrrolo[3,2,1-ij]chinolin-4-on;
1-{[3-({[(7-Chloro-3-oxo-3,4-dihydro-2H-pyrido[3,2-b][1,4]oxazin-6-yl)methyl]amino}-methyl)-1-pyrrolidinyl]methyl}-9-fluor-1,2-dihydro-4H-pyrrolo[3,2,1 -ij]chinolin-4-on;
1-{[3-({[(7-Chlor-3-oxo-3,4-dihydro-2H-pyrido[3,2-b][1,4]oxazin-6-yl)methyl]amino}-methyl)-1-pyrrolidinyl]methyl}-9-fluor-1,2-dihydro-4H-pyrrolo[3,2,1-ij]chinolin-4-on Isomer 1, 2, 3 oder 4;
1-[(4-{[(3-Oxo-3,4-dihydro-2H-pyrido[3,2-b][1,4]thiazin-6-yl)methyl]amino}-1-piperidinyl)methyl]-1,2-dihydro-4H-pyrrolo [3,2,1-ij]chinolin-4-on;
1-({4-[(2,3-Dihydro[1,4]dioxino[2,3-c]pyridin-7-ylmethyl)amino]-1-piperidinyl}methyl)-1,2-dihydro-4H-pyrrolo[3,2,1-ij]chinolin-4-on;
1-({4-[([1,3]Oxathiolo[5,4-c]pyridin-6-ylmethyl)amino]-1-piperidinyl}methyl)-1,2-dihydro-4H-pyrrolo[3,2,1-ij]chinolin-4-on;
1-[(4-{[(7-Chlor-3-oxo-3,4-dihydro-2H-pyrido[3,2-b][1,4]oxazin-6-yl)methyl]amino}-1-piperidinyl)methyl]-1,2-dihydro-4H-pyrrolo[3,2,1-ij]chinolin-4-on;
1-({4-[(3,4-Dihydro-2H-pyrano[2,3-c]pyridin-6-ylmethyl)amino]-1-piperidinyl}methyl)-1,2-dihydro-4H-pyrrolo[3,2,1-ij]chinolin-4-on;
1-[(3-{[(2,3-Dihydro[1,4]dioxino[2,3-c]pyridin-7-ylmethyl)amino]methyl}-1-pyrrolidinyl)methyl]-9-fluor-1,2-dihydro-4H-pyrrolo[3,2,1-ij]chinolin-4-on;
1-({4-[(6,7-Dihydro[1,4]oxathiino[2,3-c]pyridazin-3-ylmethyl)amino]-1-piperidinyl}-methyl)-9-fluor-1-hydroxy-1,2-dihydro-4H-pyrrolo[3,2,1-ij]chinolin-4-on Enantiomer E2;
9-Fluor-1-{[3-({[(3-oxo-3,4-dihydro-2H-pyrido[3,2-b][1,4]thiazin-6-yl)methyl]-amino}methyl)-1-pyrrolidinyl]methyl}-1,2-dihydro-4H-pyrrolo[3,2,1-ij]chinolin-4-on;
1-({4-[(6,7-Dihydro[1,4]dioxino[2,3-c]pyridazin-3-ylmethyl)amino]-1-piperidinyl}-methyl)-4-oxo-1,2-dihydro-4H-pyrrolo[3,2,1-ij]chinolin-9-carbonitril (Enantiomer E1);
1-({4-[(6,7-Dihydro[1,4]dioxino[2,3-c]pyridazin-3-ylmethyl)amino]-1-piperidinyl}-methyl)-4-oxo-1,2-dihydro-4H-pyrrolo[3,2,1-ij]chinolin-9-carbonitril (Enantiomer E2);
1-(R/S)-[(4-{[(3S)-2,3-Dihydro[1,4]dioxino[2,3-b]pyridin-3-ylmethyl]amino}-1-piperidinyl)methyl]-9-fluor-1,2-dihydro-4H-pyrrolo[3,2,1-ij]chinolin-4-on;
1-({4-[(6,7-Dihydro-5H-pyrano[2,3-c]pyridazin-3-ylmethyl)amino]-1-piperidinyl}-methyl)-4-oxo-1,2-dihydro-4H-pyrrolo[3,2,1-ij]chinolin-9-carbonitril Enantiomer E1;
1-({4-[(6,7-Dihydro-5H-pyrano[2,3-c]pyridazin-3-ylmethyl)amino]-1-piperidinyl}-methyl)-9-fluor-1-hydroxy-1,2-dihydro-4H-pyrrolo[3,2,1-ij]chinolin-4-on Enantiomer E1;
9-Fluor-1-[(4-{[(6-oxo-6,7-dihydro-5H-pyridazino[3,4-b][1,4]thiazin-3-yl)methyl]-amino}-1-piperidinyl)methyl]-1,2-dihydro-4H-pyrrolo[3,2,1-ij]chinolin-4-on Enantiomer E1;
1-({4-[(2,3-Dihydro[1,4]dioxino[2,3-c]pyridin-7-ylmethyl)amino]-1-piperidinyl}methyl)-1-hydroxy-4-oxo-1,2-dihydro-4H-pyrrolo[3,2,1-ij]chinolin-9-carbonitril Enantiomer E1;
1-({4-[(2,3-Dihydro[1,4]dioxino[2,3-c]pyridin-7-ylmethyl)amino]-1-piperidinyl}methyl)-1-hydroxy-4-oxo-1,2-dihydro-4H-pyrrolo[3,2,1-ij]chinolin-9-carbonitril Enantiomer E2;
1-[(4-{[(7-Chlor-3-oxo-3,4-dihydro-2H-pyrido[3,2-b][1,4]oxazin-6-yl)methyl]amino}-1-piperidinyl)methyl]-9-fluor-1,2-dihydro-4H-pyrrolo[3,2,1-ij]chinolin-4-on Enantiomer E1;
1-({4-[(2,3-Dihydro[1,4]dioxino[2,3-c]pyridin-7-ylmethyl)amino]-1-piperidinyl}methyl)-9-(methyloxy)-1,2-dihydro-4H-pyrrolo[3,2,1-ij]chinolin-4-on Enantiomer E1;
1-({4-[(2,3-Dihydro[1,4]dioxino[2,3-c]pyridin-7-ylmethyl)amino]-1-piperidinyl}methyl)-9-(methyloxy)-1,2-dihydro-4H-pyrrolo[3,2,1-ij]chinolin-4-on, Enantiomer E2;
1-({4-[(6,7-Dihydro[1,4]dioxino[2,3-c]pyridazin-3-ylmethyl)amino]-1-piperidinyl}-methyl)-9-(methyloxy)-1,2-dihydro-4H-pyrrolo[3,2,1-ij]chinolin-4-on, Enantiomer 1;
9-Fluor-1-{[4-({[(7R/S)-7-(hydroxymethyl)-6,7-dihydro[1,4]dioxino[2,3-c]pyridazin-3-yl]methyl}amino)-1-piperidinyl]methyl}-1,2-dihydro-4H-pyrrolo[3,2,1-ij]chinolin-4-on Diastereomer D1;
1-({4-[(5,6-Dihydrofuro[2,3-c]pyridazin-3-ylmethyl)amino]-1-piperidinyl}methyl)-9-fluor-1,2-dihydro-4H-pyrrolo[3,2,1-ij]chinolin-4-on Enantiomer E1;
1-({4-[(5,6-Dihydrofuro[2,3-c]pyridazin-3-ylmethyl)amino]-1-piperidinyl}methyl)-9-fluor-1-hydroxy-1,2-dihydro-4H-pyrrolo[3,2,1-ij]chinolin-4-on Enantiomer E1; und
(1R/S)-1-[(4-{([(7S)-6,7-Dihydro[1,4]dioxino[2,3-c]pyridazin-7-ylmethyl]amino}-1-piperidinyl)methyl]-9-fluor-1,2-dihydro-4H-pyrrolo[3,2,1-ij]chinolin-4-on;
oder ein pharmazeutisch verträgliches Salz und/oder Solvat davon.

11. Eine Verbindung gemäß Anspruch 1, nämlich
1-({4-[(6,7-Dihydro[l,4]dioxino[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E1 oder ein pharmazeutisch verträgliches Salz und/oder Solvat davon.

12. Eine Verbindung gemäß Anspruch 1, nämlich
1-({4-[(6,7-Dihydro[1,4]dioxino[2,3-c]pyridazin-3-ylmethyl)amino]-1-piperidinyl}-methyl)-9-fluor-l,2-dihydro-4H-pyrrolo[3,2,1-ij]chinolin-4-on Enantiomer E1 Mono-4-methylbenzolsulfonatsalz.

13. Eine Verbindung gemäß Anspruch 11, nämlich
1-({4-[(6,7-Dihydro[1,4]dioxino[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E1 Mono-(2E)-2-butendioatsalz.

14. Eine Verbindung gemäß Anspruch 13, nämlich
1-({4-[(6,7-Dihydro[1,4]dioxino[2,3-c]pyridazin-3-ylmethyl)amino]-1-piperidinyl}-methyl)-9-fluor-1,2-dihydro-4H-pyrrolo[3,2,1-ij]chinolin-4-on Enantiomer E1 Mono-(2E)-2-butendioatsalzanhydrat I.

15. Eine Verbindung gemäß Anspruch 13, nämlich
1-({4-[(6,7-Dihydro[1,4]dioxino[2,3-c]pyridazin-3-ylmethyl)amino]-1-piperidinyl}-methyl)-9-fluor-1,2-dihydro-4H-pyrrolo[3,2,1-ij]quinolin-4-on Enantiomer E1 Mono-(2E)-2-butendioatsalztrihydrat.

16. Eine Verbindung gemäß einem der Ansprüche 1 bis 10 oder 12 bis 15 zur Verwendung bei der Behandlung von bakteriellen Infektionen in Säugern.

17. Eine Verbindung gemäß Anspruch 11 zur Verwendung bei der Behandlung von bakteriellen Infektionen in Säugern.

18. Eine Verbindung gemäß Anspruch 17 zur Verwendung bei der Behandlung von bakteriellen Infektionen in Säugern, wobei die bakterielle Infektion durch einen grampositiven Organismus, ausgewählt aus *Staphylococcus aureus, Streptococcus pneumoniae, Streptococcus pyogenes, Enterococcus faecalis* und *Enterococcus faecium,* oder einen gram-negativen Organismus, ausgewählt aus der Gruppe bestehend aus *Haemophilus influenzae, Moraxella catarrhalis, Escherichia coli, Pseudomonas aeruginosa, Proteus mirabilis, Legionella pneumophila, Chlamydia pneumoniae, Enterobacter cloacae, Enterobacter aerogenes, Klebsiella pneumoniae* und *Stenotrophomonas maltophilia,* verursacht wird.

19. Ein Arzneimittel, umfassend eine Verbindung gemäß einem der Ansprüche 1 bis 10 oder 12 bis 15 und einen pharmazeutisch verträglichen Träger.

20. Ein Arzneimittel, umfassend eine Verbindung gemäß Anspruch 11 und einen pharmazeutisch verträglichen Träger.

21. Eine Verbindung der Formel (IIB): 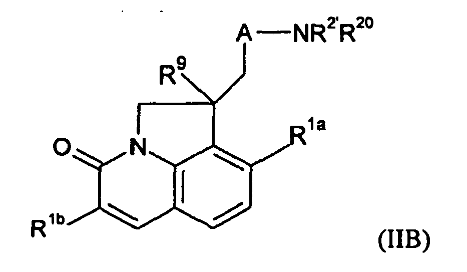 wobei R²⁰ Wasserstoff ist und R^{2'} für R² oder einen darin umwandelbaren Rest steht, wobei A, R^{1a}, R^{1b}, R² und R⁹ wie in Anspruch 1 definiert sind.

22. Eine Verbindung gemäß Anspruch 21, ausgewählt aus:
1-[(4-Amino-1-piperidinyl)methyl]-9-fluor-1,2-dihydro-4H-pyrrolo[3,2,1-ij]chinolin-4-on Enantiomer E1;
1-[(4-Amino-1-piperidinyl)methyl]-9-fluor-1,2-dihydro-4H-pyrrolo[3,2,1-ij]chinolin-4-on Enantiomer E2;
1-{[(3R,4S)-4-Amino-3-hydroxy-1-piperidinyl]methyl}-9-fluor-1,2-dihydro-4H-pyrrolo[3,2,1-ij]chinolin-4-on;
1-[(4-Amino-1-piperidinyl)methyl]-9-fluor-1-hydroxy-1,2-dihydro-4H-pyrrolo[3,2,1-ij]chinolin-4-on;
1-{[(3R)-3-(Aminomethyl)-1-pyrrolidinyl]methyl}-9-fluor-1,2-dihydro-4H-pyrrolo[3,2,1-ij]chinolin-4-on;
1-[(4-Amino-1-piperidinyl)methyl]-1,2-dihydro-4H-pyrrolo[3,2,1-ij]quinolin-4-on;
1-[(4-Amino-1-piperidinyl)methyl]-4-oxo-1,2-dihydro-4H-pyrrolo[3,2,1-ij]chinolin-9-carbonitril; und
1-[(4-Amino-1-piperidinyl)methyl]-9-(methyloxyh)-1,2-dihydro-4H-pyrrolo[3,2,1-ij]chinolin-4-on.

23. 6,7-Dihydro[1,4]dioxino[2,3-c]pyridazin-3-carbaldehyd.

## Revendications

1. Composé de formule (I) ou un de ses sels et/ou produits de solvatation pharmaceutiquement acceptables : 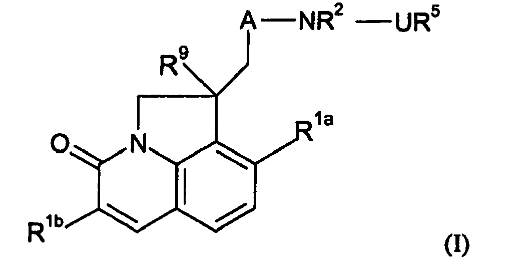 formule dans laquelle :
R^{1a} et R^{1b} sont choisis indépendamment entre un atome d'hydrogène ; un atome d'halogène ; des groupes cyano ; alkyle en C₁ à C₆ ; alkylthio en C₁ à C₆ ; trifluorométhyle ; trifluorométhoxy ; carboxy ; hydroxy facultativement substitué avec un substituant alkyle en C₁ à C₆ ou alkyle en C₁ à C₆ à substituant alkoxy en C₁ à C₆ ; alkyle en C₁ à C₆ à substituant alkoxy en C₁ à C₆ ; hydroxyalkyle en C₁ à C₆ ; un groupe amino facultativement N-substitué avec un ou deux groupes alkyle en C₁ à C₆, formyle, (alkyle en C₁ à C₆) carbonyle ou alkylsulfonyle en C₁ à C₆ ; ou un groupe aminocarbonyle dans lequel le groupe amino est facultativement substitué avec un substituant alkyle en C₁ à C₉ ;
R² représente un atome d'hydrogène ou un groupe alkyle en C₁ à C₄ ou bien, conjointement avec R⁶, forme un groupe Y répondant à la définition mentionnée ci-dessous ;
A représente un groupe (ia) ou (ib) : 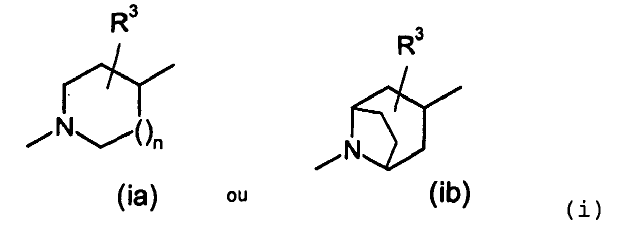 dans lequel : R³ est tel que défini pour R^{1a} ou R^{1b} ou bien représente un groupe oxo et n est égal à 1 ou 2 ;
ou bien A représente un groupe (ii) 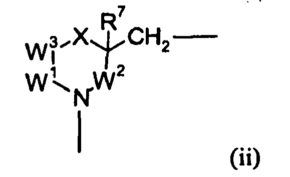 dans lequel :
W¹, W² et W³ représentent CR⁴R⁸ ;
ou W² et W³ représentent CR⁴R⁸ et W¹ représente une liaison entre W³ et N ;
X représente O, un groupe CR⁴R⁸ ou NR⁶ ;
un groupe R⁴ est tel que défini pour R^{1a} et R^{1b} et le reste et R⁸ représentent des atomes d'hydrogène ou bien un groupe R⁴ et un groupe R⁸ représentent ensemble un groupe oxo et le reste représente des atomes d'hydrogène ;
R⁶ représente un atome d'hydrogène ou un groupe alkyle en C₁ à C₆ ou bien, conjointement avec R², forme un groupe Y ;
R⁷ représente un atome d'hydrogène : un atome d'halogène ; un groupe hydroxy facultativement substitué avec un substituant alkyle en C₁ à C₆ ou un groupe alkyle en C₁ à C₆ ;
Y représente un groupe CR⁴R⁸CH₂ ; CH₂CR⁴R⁸; (C=O) ; CR⁴R⁸ ; CR⁴R⁸(C=O) ; ou (C=O)CR⁴R⁸ ;
ou bien, lorsque X représente un groupe CR⁴R⁸, R⁸ et R⁷ représentent conjointement une liaison ;
U est choisi entre un groupe CO et un groupe CH₂ ;
R⁵ représente un système de noyau carbocyclique ou hétérocyclique bicyclique, facultativement substitué, (B) : 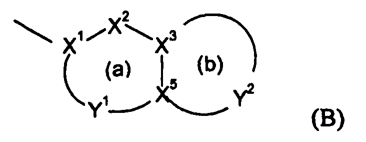 contenant jusqu'à quatre hétéroatomes dans chaque noyau, dans lequel
au moins un des noyaux (a) et (b) est aromatique ;
X¹ représente C ou N lorsqu'il fait partie d'un noyau aromatique, ou un groupe CR¹⁴ lorsqu'il fait partie d'un noyau non aromatique ;
X² représente N, un groupe NR¹³, O, un groupe S(O)ₓ, CO ou CR¹⁴, lorsqu'il fait partie d'un noyau aromatique ou non aromatique ou bien peut représenter en outre un groupe CR¹⁴R¹⁵ lorsqu'il fait partie d'un noyau non aromatique ;
X³ et X⁵ représentent indépendamment N ou C ;
Y¹ représente un groupe de liaison de 0 à 4 atomes dont chaque atome est choisi indépendamment entre N, NR¹³, O, S(O)ₓ, CO et CR¹⁴ lorsqu'il fait partie d'un noyau aromatique ou non aromatique ou peut représenter en outre un groupe CR¹⁴R¹⁵ lorsqu'il fait partie d'un noyau non aromatique ;
Y² représente un groupe de liaison de 2 à 6 atomes, chaque atome de Y² étant choisi indépendamment entre N, NR¹³, O, S(O)ₓ, CO et CR¹⁴ lorsqu'il fait partie d'un noyau aromatique ou non aromatique ou peut représenter en outre un groupe CR¹⁴R¹⁵ lorsqu'il fait partie d'un noyau non aromatique ;
chacun des groupes R¹⁴ et R¹⁵ est choisi indépendamment entre : H ; des groupes alkylthio en C₁ à C₄ ; halogéno ; carboxy(alkyle en C₁ à C₄) ; alkyle en C₁ à C₄ ; (alkoxy en C₁ à C₄) carbonyle ; (alkyle en C₁ à C₄) carbonyle ; (alkoxy en C₁ à C₄) (alkyle en C₁ à C₄) hydroxy ; hydroxyalkyle en C₁ à C₄ ; alkoxy en C₁ à C₄ ; nitro ; cyano ; carboxy ; amino ou aminocarbonyle facultativement mono- ou disubstitué avec un substituant alkyle en C₁ à C₄ ; ou bien
R¹⁴ et R¹⁵ peuvent représenter conjointement un groupe oxo ;
chaque groupe R¹³ représente indépendamment H ; un groupe trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; (alkoxy en C₁ à C₄) carbonyle ; (alkyle en C₁ à C₄) carbonyle ; alkylsulfonyle en C₁ à C₆ ; ou aminocarbonyle dans lequel le groupe amino est facultativement mono- ou disubstitué avec un substituant alkyle en C₁ à C₄ ;
chaque indice x est indépendamment égal à 0, 1 ou 2 et
R⁹ représente un atome d'hydrogène ou un groupe hydroxy.

2. Composé suivant la revendication 1, dans lequel R^{1a} représente un groupe méthoxy, fluoro ou cyano et R^{1b} représente un atome d'hydrogène.

3. Composé suivant l'une quelconque des revendications précédentes, dans lequel R² représente un atome d'hydrogène.

4. Composé suivant l'une quelconque des revendications précédentes, dans lequel R⁹ représente un atome d'hydrogène.

5. Composé suivant l'une quelconque des revendications précédentes, dans lequel A représente un groupe (ia) dans lequel n est égal à 1 et R³ représente un atome d'hydrogène ou un groupe hydroxy.

6. Composé suivant l'une quelconque des revendications 1 à 4, dans lequel A représente un groupe (ii), W¹ représente une liaison, X, W² et W³ représentent chacun un groupe CH₂ et R⁷ représente H.

7. Composé suivant l'une quelconque des revendications précédentes, dans lequel U représente un groupe CH₂.

8. Composé suivant l'une quelconque des revendications précédentes, dans lequel R⁵ représente un noyau hétérocyclique aromatique (B) ayant 8 à 11 atomes dans le noyau y compris 2 à 4 hétéroatomes dont au moins l'un est un atome de N ou groupe NR¹³, dans lequel Y² contient 2 ou 3 hétéroatomes, dont l'un est un atome de S et 1 ou 2 consistent en un atome de N, avec un atome de N lié à X³, ou bien le noyau hétérocyclique (B) possède un noyau (a) aromatique choisi entre des noyaux benzo, pyrido et pyridazino facultativement substitués et un noyau (b) non aromatique et Y² a 3 à 5 atomes, y compris au moins un hétéroatome, avec O, S, CH₂ ou NR¹³ lié à X⁵ lorsque R¹³ est autre que l' hydrogène, et soit NHCO lié par N à X³, soit O, S, CH₂ ou NH lié à X³.

9. Composé suivant l'une quelconque des revendications 1 à 7, dans lequel R⁵ est choisi entre des groupes :
3-oxo-3,4-dihydro-2H-pyrido[3,2-b][1,4]oxazine-6-yle ;
3-oxo-3,4-dihydro-2*H*-pyrido[3,2-*b*][1,4]thiazine-6-yle ;
6,7-dihydro[1,4]dioxino[2,3-*c*]pyridazine-3-yle ;
6,7-dihydro[1,4]oxathiino[2,3-*c*]pyridazine-3-yle ;
6,7-dihydro[1,4]oxathiino[3,2-*c*]pyridazine-3-yle ;
2,3-dihydro[1,4]dioxino[2,3-*c*]pyridine-7-yle ; et
[1,3]oxathiolo[5,4-*c*]pyridine-6-yle.

10. Composé suivant la revendication 1, choisi entre :
1-({4-[(2,3-dihydro[1,4]dioxino[2,3*-c*]pyridine-7-ylméthyl)-amino]-1-pipéridinyl}méthyl)-9-fluoro-1,2-dihydro-4H-pyrrolo[3,2,1-ij]quinoléine-4-one, énantiomère E2 ;
1-({4-[(2,3-dihydro[1,4]dioxino[2,3-c]pyridine-7-ylméthyl)-amino]-1-pipéridinyl}méthyl)-9-fluoro-1,2-dihydro-4H-pyrrolo[3,2,1-ij]quinoléine-4-one, énantiomère E1 ;
1-({(3*R*,4*S*)-4-[(2,3-dihydro[1,4]dioxino[2,3-*c*]pyridine-7-ylméthyl)amino]-3-hydroxy-1-pipéridinyl}méthyl)-9-fluoro-1,2-dihydro-4*H-*pyrrolo[3,2,1*-ij*]quinoléine-4-one, diastéréisomère 1 ;
1-({(3*R*,4*S*)-4-[(2,3-dihydro[1,4]dioxino[2,3-c]pyridine-7-ylméthyl)amino]-3-hydroxy-1-pipéridinyl}méthyl)-9-fluoro-1,2-dihydro-4H-pyrrolo-[3,2,1*-ij*]quinoléine-4-one, diastéréoisomère 2 ;
9-fluoro-1-({(3*R*,4*S*)-3-hydroxy-4-[([1,3]oxathiolo[5,4-c]pyridine-6-ylméthyl)amino]-1-pipéridinyl}méthyl)-1,2-dihydro-4*H-*pyrrolo[3,2,1*-ij*]quinoléine-4-one ;
9-fluoro-1-[((3*R*,4*S*)-3-hydroxy-4-{[(6-oxo-6,7-dihydro-5*H-*pyridazino[3,4-*b*][1,4]thiazine-3-yl)méthyl]amino}-1-pipéridinyl)méthyl]-1,2-dihydro-4*H-*pyrrolo[3,2,1*-ij*]-quinoléine-4-one ;
1-({(3*R*,4*S*)-4-[(2,3-dihydro-1*H*-pyrido[3,4-*b*][1,4]oxazine-7-ylméthyl)amino]-3-hydroxy-1-pipéridinyl}méthyl)-9-fluoro-1,2-dihydro-4*H*-pyrrolo[3,2,1-*ij*]quinoléine-4-one ;
1-({(3*R*,4*S*)-4-[(2,3-dihydrofuro[2,3-*c*]pyridine-5-ylméthyl)-amino]-3-hydroxy-1-pipéridinyl}méthyl)-9-fluoro-1,2-dihydro-4*H-*pyrrolo[3,2,1*-ij*]quinoléine-4-one ;
9-fluoro-1-[((3*R*,4*S*)-3-hydroxy-4-{[(7-oxo-1,5,6,7-tétrahydro-1,8-naphtyridine-2-yl)méthyl]amino}-1-pipéridinyl)-méthyl]-1,2-dihydro-4*H*-pyrrolo[3,2,1-*ij*]quinoléine-4-one ;
1-({4-[(6,7-dihydro[1,4]dioxino[2,3*-c*]pyridazine-3-yl-méthyl)amino]-1-pipéridinyl}méthyl)-9-fluoro-1,2-dihydro-4*H-*pyrrolo[3,2,1*-ij*]quinoléine-4-one, énantiomère E2 ;
9-fluoro-1-[(4-{[(3-oxo-3,4-dihydro-2*H-*pyrido[3,2-*b*]-[1,4]oxazine-6-yl)méthyl]amino}-1-pipéridinyl)méthyl]-1,2-dihydro-4*H-*pyrrolo[3,2,1*-ij*]quinoléine-4-one, énantiomère E1 ;
1-({4-[(2,3-dihydro[1,4]dioxino[2,3*-c*]pyridine-7-yl-méthyl)amino]-1-pipéridinyl}méthyl)-4-oxo-1,2-dihydro-4*H-*pyrrolo[3,2,1*-ij*]quinoléine-9-carbonitrile, énantiomère E1 ;
1-({4-[(2,3-dihydro[1,4]dioxino[2,3*-c*]pyridine-7-yl-méthyl)amino]-1-pipéridinyl}méthyl)-9-fluoro-1-hydroxy-1,2-dihydro-4*H-*pyrrolo[3,2,1*-ij*]quinoléine-4-one, énantiomère E2 ;
1-({4-[(2,3-dihydro[1,4]dioxino[2,3*-c*]pyridine-7-yl-méthyl)amino]-1-pipéridinyl}méthyl)-9-fluoro-1-hydroxy-1,2-dihydro-4*H-*pyrrolo[3,2,1*-ij*]quinoléine-4-one ;
1-({4-[(2,3-dihydro[1,4]oxathiino[2,3*-c*]pyridine-7-yl-méthyl)amino]pipéridine-1-yl}méthyl)-9-fluoro-1-hydroxy-1,2-dihydro-4*H-*pyrrolo[3,2,1*-ij*]quinoléine-4-one, énantiomère E1 ;
9-fluoro-1-hydroxy-1-[(4-{[(3-oxo-3,4-dihydro-2*H-*pyrido-[3,2*-b*][1,4]oxazine-6-yl)méthyl]amino}-1-pipéridinyl)-méthyl]-1,2-dihydro-4*H-*pyrrolo[3,2,1*-ij*]quinoléine-4-one ;
1-({4-[(6,7-dihydro[1,4]oxathiino[2,3-*c*]pyridazine-3-yl-méthyl)amino]-1-pipéridinyl}méthyl)-9-fluoro-1,2-dihydro-4*H-*pyrrolo[3,2,1*-ij*]quinoléine-4-one, énantiomère E1 ;
1-({4-[(6,7-dihydro[1,4]dioxino[2,3*-c*]pyridazine-3-yl-méthyl)amino]-1-pipéridinyl}méthyl)-9-fluoro-1-hydroxy-1,2-dihydro-4*H-*pyrrolo[3,2,1*-ij*]quinoléine-4-one ;
1-({4-[(2,3-dihydro-1*H-*pyrido[3,4*-b*][1,4]oxazine-7-yl-méthyl)amino]-1-pipéridinyl}méthyl)-9-fluoro-1,2-dihydro-4*H-*pyrrolo[3,2,1*-ij*]quinoléine-4-one ;
1-({4-[(2,3-dihydro[1,4]oxathiino[2,3-*b*]pyridine-7-yl-méthyl)amino]-1-pipéridinyl}méthyl)-9-fluoro-1,2-dihydro-4*H-*pyrrolo[3,2,1*-ij*]quinoléine-4-one ;
1-({4-[(2,3-dihydro[1,4]dioxino[2,3*-b*]pyridine-7-yl-méthyl)amino]-1-pipéridinyl}méthyl)-9-fluoro-1-hydroxy-1,2-dihydro-4*H-*pyrrolo[3,2,1*-ij*]quinoléine-4-one, énantiomère E1 ;
1-({4-[(2,3-dihydro[1,4]dioxino[2,3-*b*]pyridine-7-yl-méthyl)amino]-1-pipéridinyl}méthyl)-9-fluoro-1,2-dihydro-4*H-*pyrrolo[3,2,1-*ij*]quinoléine-4-one ;
1-({4-[(1,2,3-benzothiadiazole-5-ylméthyl)amino]-1-pipéridinyl}méthyl)-9-fluoro-1-hydroxy-1,2-dihydro-4*H*-pyrrolo-[3,2,1*-ij*]quinoléine-4-one ;
1-({4-[(2,3-dihydro[1,4]dioxino[2,3*-c*]pyridine-7-yl-méthyl)amino]-1-pipéridinyl}méthyl)-4-oxo-1,2-dihydro-4*H-*pyrrolo[3,2,1*-ij*]quinoléine-9-carbonitrile, énantiomère E2 ;
1-({4-[(2,3-dihydro[1,4]dioxino[2,3*-c*]pyridine-7-ylméthyl)-amino]-1-pipéridinyl}méthyl)-9-fluoro-1-hydroxy-1,2-dihydro-4*H-*pyrrolo[3,2,1*-ij*]quinoléine-4-one, énantiomère E1 ;
9-fluoro-1-[(4-{[(5-oxo-1,2,3,5-tétrahydro-7-indolizinyl)-méthyl]amino}-1-pipéridinyl)méthyl]-1,2-dihydro-4*H-*pyrrolo-[3,2,1*-ij*]quinoléine-4-one ;
1-({4-[(2,3-dihydro[1,4]oxathiino[2,3*-c*]pyridine-7-yl-méthyl)amino]-l-pipéridinyl}méthyl)-9-fluoro-1-hydroxy-1,2-dihydro-4*H-*pyrrolo[3,2,1*-ij*]quinoléine-4-one, énantiomère E2 ;
1-({4-[(2,3-dihydro[1,4]dioxino[2,3*-b*]pyridine-7-ylméthyl)-amino]-1-pipéridinyl}méthyl)-9-fluoro-1-hydroxy-1,2-dihydro-4*H-*pyrrolo[3,2,1*-ij*]quinoléine-4-one, énantiomère E2 ;
1-({4-[(6,7-dihydro[1,4]oxathiino[2,3-*c*]pyridazine-3-yl-méthyl)amino]-1-pipéridinyl}méthyl)-9-fluoro-1-hydroxy-1,2-dihydro-4*H-*pyrrolo[3,2,1*-ij*]quinoléine-4-one, énantiomère E1 ;
1-({4-[(6,7-dihydro[1,4]oxathiino[3,2-*c*]pyridazine-3-yl-méthyl)amino]-1-pipéridinyl}méthyl)-9-fluoro-1,2-dihydro-4*H-*pyrrolo[3,2,1*-ij*]quinoléine-4-one, énantiomère E1 ;
1-({4-[(6,7-dihydro-5H-pyrano[2,3-*c*]pyridazine-3-yl-méthyl)amino]-1-pipéridinyl}méthyl)-9-fluoro-1,2-dihydro-4*H*-pyrrolo[3,2,1-*ij*]quinoléine-4-one, énantiomère E1 :
9-fluoro-1-[((3*R*)-3-{[([1,3]oxathiolo[5,4-*c*]pyridine-6-ylméthyl)amino]méthyl}-1-pyrrolidinyl)méthyl]-1,2-dihydro-4*H-*pyrrolo[3,2,1*-ij*]quinoléine-4-one :
1-{[(3*R*)-3-({[(7-chloro-3-oxo-3,4-dihydro-2*H*-pyrido[3,2-*b*][1,4]oxazine-6-yl)méthyl]amino}méthyl)-1-pyrrolidinyl]-méthyl}-9-fluoro-1,2-dihydro-4*H*-pyrrolo[3,2,1-*ij*]-quinoléine-4-one ;
9-fluoro-1-{[(3*R*)-3-({[(3-oxo-3,4-dihydro-2*H*-pyrido[3,2-c][1,4]thiazine-6-yl)méthyl]amino}méthyl)-1-pyrrolidinyl]-méthyl}-1,2-dihydro-4*H-*pyrrolo[3,2,1*-ij*]quinoléine-4-one ;
9-fluoro-1-{[(3*R*)-3-({[(3-oxo-3,4-dihydro-2*H-*pyrido[3,2*-b*]-[1,4]oxazine-6-yl)méthyl]amino}méthyl)-1-pyrrolidinyl]-méthyl}-1,2-dihydro-4*H-*pyrrolo[3,2,1*-ij*]quinoléine-4-one ;
1-[((3*R*)-3-{[(2,3-dihydro[1,4]dioxino[2,3-*c*]pyridine-7-ylméthyl)amino]méthyl}-1-pyrrolidinyl)méthyl]-9-fluoro-1,2-dihydro-4*H*-pyrrolo[3,2,1-*ij*]-quinoléine-4-one ;
9-fluoro-1-[(3-{[([1,3]oxathiolo[5,4-*c*]pyridine-6-yl-méthyl)amino]méthyl}-1-pyrrolidinyl)méthyl]-1,2-dihydro-4*H-*pyrrolo[3,2,1*-ij*]quinoléine-4-one ;
1-{[3-({[(7-chloro-3-oxo-3,4-dihydro-2*H-*pyrido[3,2-b][1,4]oxazine-6-yl)méthyl]amino}méthyl)-1-pyrrolidinyl]-méthyl}-9-fluoro-1,2-dihydro-4*H-*pyrrolo[3,2,1*-ij*]-quinoléine-4-one ;
1-{[3-({[(7-chloro-3-oxo-3,4-dihydro-2*H*-pyrido[3,2-*b*]-[1,4]oxazine-6-yl)méthyl]amino}méthyl)-1-pyrrolidinyl]-méthyl}-9-fluoro-1,2-dihydro-4*H-*pyrrolo[3,2,1*-ij*]-quinoléine-4-one, isomère 1, 2, 3 ou 4 ;
1-[(4-{[(3-oxo-3,4-dihydro-2*H-*pyrido[3,2*-b*][1,4]thiazine-6-yl)méthyl]amino}-1-pipéridinyl)méthyl]-1,2-dihydro-4*H-*pyrrolo-[3,2,1*-ij*]quinoléine-4-one ;
1-({4-[(2,3-dihydro[1,4]dioxino[2,3*-c*]pyridine-7-ylméthyl)-amino]-1-pipéridinyl}méthyl)-1,2-dihydro-4*H*-pyrrolo[3,2,1-*ij*]-quinoléine-4-one ;
1-({4-[([1,3]oxathiolo[5,4-*c*]pyridine-6-ylméthyl)amino]-1-pipéridinyl}méthyl)-1,2-dihydro-4*H*-pyrrolo[3,2,1-*ij*]-quinoléine-4-one ;
1-[(4-{[(7-chloro-3-oxo-3,4-dihydro-2*H*-pyrido[3,2-*b*]-[1,4]oxazine-6-yl)méthyl]amino}-1-pipéridinyl)méthyl]-1,2-dihydro-4*H*-pyrrolo[3,2,1-*ij*]quinoléine-4-one ;
1-({4-[(3,4-dihydro-2*H*-pyrano[2,3-*c*]pyridine-6-ylméthyl)-amino]-1-pipéridinyl}méthyl)-1,2-dihydro-4*H*-pyrrolo[3,2,1-*ij*]-quinoléine-4-one ;
1-[(3-([(2,3-dihydro[1,4]dioxino[2,3-*c*]pyridine-7-ylméthyl)-amino]méthyl}-1-pyrrolidinyl)méthyl]-9-fluoro-1,2-dihydro-4*H*-pyrrolo[3,2,1-*ij*]quinoléine-4-one ;
1-({4-[(6,7-dihydro[1,4]oxathiino[2,3-*c*]pyridazine-3-ylméthyl)amino]-1-pipéridinyl}méthyl)-9-fluoro-1-hydroxy-1,2-dihydro-4*H*-pyrrolo[3,2,1-*ij*]quinoléine-4-one, énantiomère E2 ;
9-fluoro-1-{[3-({[(3-oxo-3,4-dihydro-2*H*-pyrido[3,2-*b*][1,4]-thiazine-6-yl)méthyl]amino}méthyl)-1-pyrrolidinyl]méthyl}-1,2-dihydro-4*H*-pyrrolo[3,2,1-*ij*]quinoléine-4-one ;
1-({4-[(6,7-dihydro[1,4]dioxino[2,3-*c*]pyridazine-3-ylméthyl)amino]-1-pipéridinyl}méthyl)-4-oxo-1,2-dihydro-4*H-*pyrrolo[3,2,1-*ij*]quinoléine-9-carbonitrile, (énantiomère E1);
1-({4-[(6,7-dihydro[1,4]dioxino[2,3-*c*]pyridazine-3-ylméthyl)-amino]-1-pipéridinyl}méthyl)-4-oxo-1,2-dihydro-4*H*-pyrrolo-[3,2,1-*ij*]quinoléine-9-carbonitrile, (énantiomère E2) ;
1-(R/S)-[(4-{[(3*S*)-2,3-dihydro[1,4]dioxino[2,3-*b*]pyridine-3-ylméthyl]amino}-1-pipéridinyl)méthyl]-9-fluoro-1,2-dihydro-4*H*-pyrrolo-[3,2,1-*ij*]quinoléine-4-one ;
1-({4-[(6,7-dihydro-5*H*-pyrano[2,3-*c*]pyridazine-3-yl-méthyl)-amino]-1-pipéridinyl}méthyl)-4-oxo-1,2-dihydro-4*H*-pyrrolo-[3,2,1-*ij*]-quinoléine-9-carbonitrile, énantiomère E1;
1-({4-[(6,7-dihydro-5*H*-pyrano[2,3-*c*]pyridazine-3-yl-méthyl)-amino]-1-pipéridinyl}méthyl)-9-fluoro-1-hydroxy-1,2-dihydro-4*H*-pyrrolo[3,2,1-*ij*]quinoléine-4-one, énantiomère E1;
9-fluoro-1-[(4-{[(6-oxo-6,7-dihydro-5*H*-pyridazino[3,4-*b*]-[1,4]thiazine-3-yl)méthyl]amino}-1-pipéridinyl)méthyl]-1,2-dihydro-4*H*-pyrrolo[3,2,1-*ij*]quinoléine-4-one, énantiomère E1;
1-({4-[(2,3-dihydro[1,4]dioxino[2,3-*c*]pyridine-7-yl-méthyl)amino]-1-pipéridinyl}méthyl)-1-hydroxy-4-oxo-1,2-dihydro-4*H*-pyrrolo[3,2,1-*ij*]quinoléine-9-carbonitrile, énantiomère E1;
1-({4-[(2,3-dihydro[1,4]dioxino[2,3-*c*]pyridine-7-yl-méthyl)amino]-1-pipéridinyl}méthyl)-1-hydroxy-4-oxo-1,2-dihydro-4*H*-pyrrolo[3,2,1-*ij*]quinoléine-9-carbonitrile, énantiomère E2 ;
1-[(4-{[(7-chloro-3-oxo-3,4-dihydro-2*H*-pyrido[3,2-*b*]-[1,4]oxazine-6-yl)méthyl]amino}-1-pipéridinyl)méthyl]-9-fluoro-1,2-dihydro-4*H*-pyrrolo-[3,2,1-ij]quinoléine-4-one, énantiomère E1;
1-({4-[(2,3-dihydro[1,4]dioxino[2,3-*c*]pyridine-7-ylméthyl)-amino]-1-pipéridinyl}méthyl)-9-(méthyloxy)-1,2-dihydro-4*H-*pyrrolo[3,2,1-ij]-quinoléine-4-one, énantiomère E1 ;
1-({4-[(2,3-dihydro[1,4]dioxino[2,3-*c*]pyridine-7-ylméthyl)-amino]-1-pipéridinyl}méthyl)-9-(méthyloxy)-1,2-dihydro-4*H-*pyrrolo[3,2,1-*ij*]-quinoléine-4-one, énantiomère E2;
1-({4-[(6,7-dihydro[1,4]dioxino[2,3-*c*]pyridazine-3-ylméthyl)amino]-1-pipéridinyl}méthyl)-9-(méthyloxy)-1,2-dihydro-4*H*-pyrrolo-[3,2,1*-ij*]quinoléine-4-one, énantiomère 1;
9-fluoro-1-{[4-({[(7R/*S*)-7-(hydroxyméthyl)-6,7-dihydro-[1,4]dioxino[2,3-*c*]pyridazine-3-yl]méthyl}amino)-1-pipéridinyl]méthyl}-1,2-dihydro-4*H*-pyrrolo[3,2,1-*ij*]quinoléine-4-one, diastéréoisomère D1;
1-({4-[(5,6-dihydrofuro[2,3-*c*]pyridazine-3-ylméthyl)amino]-1-pipéridinyl}méthyl)-9-fluoro-1,2-dihydro-4*H*-pyrrolo-[3,2,1-*ij*]quinoléine-4-one, énantiomère E1;
1-({4-[(5,6-dihydrofuro[2,3-*c*]pyridazine-3-ylméthyl)amino]-1-pipéridinyl}méthyl)-9-fluoro-1-hydroxy-1,2-dihydro-4*H-*pyrrolo[3,2,1-*ij*]quinoléine-4-one, énantiomère E1; et
(1*R*/*S*)-1-[(4-{[(7*S*)-6,7-dihydro[1,4]dioxino[2,3-*c*]-pyridazine-7-ylméthyllamino}-1-pipéridinyl)méthyl]-9-fluoro-1,2-dihydro-4*H*-pyrrolo[3,2,1-*ij*]quinoléine-4-one ;
ou un de ses sels et/ou produits de solvatation pharmaceutiquement acceptables.

11. Composé suivant la revendication 1, qui est l'énantiomère E1 de 1-({4-[(6,7-dihydro[1,4]dioxino[2,3-*c*]-pyridazine-3-ylméthyl)amino]-1-pipéridinyl}méthyl)-9-fluoro-1,2-dihydro-4*H*-pyrrolo[3,2,1-*ij*]quinoléine-4-one ou un de ses sels et/ou produits de solvatation pharmaceutiquement acceptables.

12. Composé suivant la revendication 1, qui est le mono-4-méthylbenzènesulfonate d'énantiomère E1 de 1-({4-[(6,7-dihydro[1,4]dioxino[2,3-*c*]pyridazine-3-ylméthyl)-amino]-1-pipéridinyl}méthyl)-9-fluoro-1,2-dihydro-4*H-*pyrrolo-[3,2,1-*ij*]quinoléine-4-one.

13. Composé suivant la revendication 11, qui est le mono-(2E)-2-butènedioate d'énantiomère E1 de 1-({4-[(6,7-dihydro[1,4]dioxino[2,3-*c*]pyridazine-3-ylméthyl)amino)-1-pipéridinyl}méthyl)-9-fluoro-1,2-dihydro-4*H*-pyrrolo-[3,2,1-ij]quinoléine-4-one.

14. Composé suivant la revendication 13, qui est la forme anhydre I de mono-(2E)-2-butènedioate d'énantiomère E1 de 1-({4-[(6,7-dihydro[1,4]dioxino[2,3-*c*]pyridazine-3-ylméthyl)amino]-1-pipéridinyl}méthyl)-9-fluoro-1,2-dihydro-4H-pyrrolo-[3,2,1-*ij*]quinoléine-4-one.

15. Composé suivant la revendication 13, qui est le trihydrate de mono-(2E)-2-butènedioate d'énantiomère E1 de 1-({4-[(6,7-dihydro[1,4]dioxino[2,3-*c*]pyridazine-3-ylméthyl)amino]-1-pipéridinyl}méthyl)-9-fluoro-1,2-dihydro-4*H*-pyrrolo-[3,2,1-*ij*]quinoléine-4-one.

16. Composé suivant l'une quelconque des revendications 1 à 10 ou 12 à 15, destiné à être utilisé dans le traitement d'infections bactériennes chez des mammifères.

17. Composé suivant la revendication 11, destiné à être utilisé dans le traitement d'infections bactériennes chez des mammifères.

18. Composé suivant la revendication 17, destiné à être utilisé dans le traitement d'infections bactériennes chez des mammifères, dans lequel l'infection bactérienne est provoquée par un organisme Gram-positif choisi parmi *Staphylococcus aureus, Streptococcus pneumoniae, Streptococcus pyogenes, Enterococcus faecalis* et *Enterococcus faecium ;* ou un organisme Gram-négatif choisi dans le groupe consistant en *Haemophilus influenzae, Moraxella catarrhalis, Escherichia coli, Pseudomonas aeruginosa, Proteus mirabilis, Legionella pneumophila, Chlamydia pneumoniae, Enterobacter cloacae, Enterobacter aerogenes, Klebsiella pneumoniae* et *Stenotrophomonas maltophilia.*

19. Composition pharmaceutique comprenant un composé suivant l'une quelconque des revendications 1 à 10 ou 12 à 15 et un support pharmaceutiquement acceptable.

20. Composition pharmaceutique comprenant un composé suivant la revendication 11 et un support pharmaceutiquement acceptable.

21. Composé de formule (IIB) : 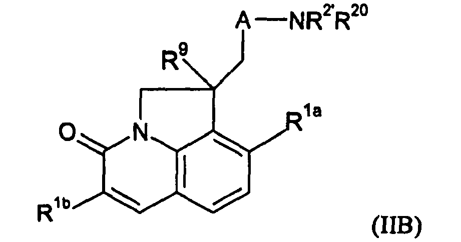 dans laquelle R²⁰ représente un atome d'hydrogène et R^{2'} représente un groupe R² ou un groupe pouvant être converti en celui-ci, où A, R^{1a}, R^{1b}, R² et R⁹ sont tels que définis dans la revendication 1.

22. Composé suivant la revendication 21, choisi entre:
1-[(4-amino-1-pipéridinyl)méthyl]-9-fluoro-1,2-dihydro-4H-pyrrolo[3,2,1-ij]quinoléine-4-one, énantiomère E1;
1-[(4-amino-1-pipéridinyl)méthyl]-9-fluoro-1,2-dihydro-4H-pyrrolo[3,2,1-ij]quinoléine-4-one, énantiomère E2 ;
1-{[(3R,4S)-4-amino-3-hydroxy-1-pipéridinyl]méthyl}-9-fluoro-1,2-dihydro-4H-pyrrolo[3,2,1-ij]quinoléine-4-one ;
1-[(4-amino-1-pipéridinyl)méthyl]-9-fluoro-1-hydroxy-1,2-dihydro-4*H*-pyrrolo[3,2,1-ij]quinoléine-4-one ;
1-{[(3*R*)-3-(aminométhyl)-1-pyrrolidinyl]méthyl}-9-fluoro-1,2-dihydro-4*H*-pyrrolo[3,2,1-*ij*] quinoléine-4-one ;
1-[(4-amino-1-pipéridinyl)méthyl]-1,2-dihydro-4*H*-pyrrolo-[3,2,1-*ij*]quinoléine-4-one ;
1-[(4-amino-1-pipéridinyl)méthyl]-4-oxo-1,2-dihydro-4H-pyrrolo[3,2,1-ij]quinoléine-9-carbonitrile ; et
1-[(4-amino-1-pipéridinyl)méthyl]-9-(méthyloxy)-1,2-dihydro-4H-pyrrolo[3,2,1-ij]quinoléine-4-one.

23. 6,7-dihydro[1,4]dioxino[2,3-c]pyridazine-3-carbaldéhyde.
